(19)

**Europäisches Patentamt**

**European Patent Office**

**Office européen des brevets**

(11) **EP 2 806 272 A1**

(12) **EUROPEAN PATENT APPLICATION**

(43) Date of publication:
26.11.2014 Bulletin 2014/48

(21) Application number: **14180229.8**

(22) Date of filing: **06.08.2010**

(51) Int Cl.:
**G01N 33/48** *(2006.01)*   **G01N 33/53** *(2006.01)*
**G01N 33/68** *(2006.01)*

(84) Designated Contracting States:
**AL AT BE BG CH CY CZ DE DK EE ES FI FR GB
GR HR HU IE IS IT LI LT LU LV MC MK MT NL NO
PL PT RO SE SI SK SM TR**

(30) Priority: **07.08.2009 US 232091 P
23.04.2010 US 327389 P**

(62) Document number(s) of the earlier application(s) in
accordance with Art. 76 EPC:
**10807278.6 / 2 462 438**

(71) Applicant: **Myriad RBM, Inc.
Austin TX 78759 (US)**

(72) Inventors:
• **Spain, Michael
Austin, TX 78729 (US)**
• **Mapes, James P.
Lakeway, TX 78734 (US)**
• **Labrie, Samuel
Austin, TX 78717 (US)**
• **McDade, Ralph
Austin, TX 78730 (US)**

• **Eisinger, Dominic
Keene, NY 12942 (US)**
• **Ballard, Karri
Austin, TX 78732 (US)**
• **Salomon, Daniel
San Diego, CA 92130 (US)**
• **Abecassis, Michael
Chicago, IL 60611 (US)**

(74) Representative: **Vossius & Partner
Siebertstrasse 4
81675 München (DE)**

Remarks:
•This application was filed on 07-08-2014 as a
divisional application to the application mentioned
under INID code 62.
•A request for correction of part of the description has
been filed pursuant to Rule 139 EPC. A decision on
the request will be taken during the proceedings
before the Examining Division (Guidelines for
Examination in the EPO, A-V, 3.).

(54) **Methods and devices for detecting kidney transplant rejection**

(57) Methods and devices for diagnosing, monitoring, or determining kidney transplant rejection or an associated disorder in a mammal are described. In particular, methods and devices for diagnosing, monitoring, or determining kidney transplant rejection or an associated disorder using measured concentrations of a combination of three or more analytes in a test sample taken from the mammal are described.

**EP 2 806 272 A1**

## Description

### CROSS REFERENCE TO RELATED APPLICATIONS

This application claims the priority of US provisional application serial no. 61/327,389, filed April 23, 2010, and US provisional application serial no. 61/232,091, filed August 7, 2009, each of which is hereby incorporated by reference in its entirety and is related to U.S. Patent Application Nos. [Not Yet Assigned], entitled Methods and Devices for Detecting Obstructive Uropathy and Associated Disorders, Methods and Devices for Detecting Kidney Damage, Devices for Detecting Renal Disorders, Computer Methods and Devices for Detecting Kidney Damage, Methods and Devices for Detecting Diabetic Nephropathy and Associated Disorders, and Methods and Devices for Detecting Glomerulonephritis and Associated Disorders, Attorney Docket Nos. 060075- , filed on the same date as this application, the entire contents of which are incorporated herein by reference..

### FIELD OF THE INVENTION

The invention encompasses methods and devices for diagnosing, monitoring, or determining kidney transplant rejection or an associated disorder in a mammal. In particular, the present invention provides methods and devices for diagnosing, monitoring, or determining kidney transplant rejection or an associated disorder using measured concentrations of a combination of three or more analytes in a test sample taken from the mammal.

### BACKGROUND OF THE INVENTION

The urinary system, in particular the kidneys, perform several critical functions such as maintaining electrolyte balance and eliminating toxins from the bloodstream. In the human body, the pair of kidneys together process roughly 20% of the total cardiac output, amounting to about 1 L/min in a 70-kg adult male. Because compounds in circulation are concentrated in the kidney up to 1000-fold relative to the plasma concentration, the kidney is especially vulnerable to injury due to exposure to toxic compounds.

Severe kidney damage that results in end-stage renal disease (ESRD) may be treated with a kidney transplant. ESRD is defined as a drop in the glomerular filtration rate (GFR) to 20-25% of normal. Common diseases leading to ESRD may include malignant hypertension, infections, diabetes mellitus, and focal segmental glomerulosclerosis; genetic causes include polycystic kidney disease, a number of inborn errors of metabolism, and autoimmune conditions such as lupus and Goodpasture's syndrome. Diabetes is the most common cause of kidney transplantation, accounting for approximately 25% of those in the US. Despite the success of a kidney transplant in extending the patient's life, rejection is still a significant complication to the procedure, and may result in failure of the transplant. Detecting early signs of a rejection may enable faster, more aggressive treatment, resulting in less damage to the kidney. Existing diagnostic tests such as BUN and serum creatine tests, however, typically detect only advanced stages of kidney damage. Other diagnostic tests such as kidney tissue biopsies or CAT scans have the advantage of enhanced sensitivity to earlier stages of kidney damage, but these tests are also generally costly, slow, and/or invasive.

A need exists in the art for a fast, simple, reliable, and sensitive method of detecting kidney transplant rejection or an associated disorder. In a clinical setting, the early detection of kidney damage would help medical practitioners to diagnose and treat kidney damage more quickly and effectively.

### SUMMARY OF THE INVENTION

The present invention provides methods and devices for diagnosing, monitoring, or determining a renal disorder in a mammal. In particular, the present invention provides methods and devices for diagnosing, monitoring, or determining a renal disorder using measured concentrations of a combination of three or more analytes in a test sample taken from the mammal.

One aspect of the invention encompasses a method for diagnosing, monitoring, or determining kidney transplant rejection or an associated disorder in a mammal. The method typically comprises providing a test sample comprising a sample of bodily fluid taken from the mammal. Then, the method comprises determining a combination of sample concentrations for three or more sample analytes in the test sample, wherein the sample analytes are selected from the group consisting of alpha-1 microglobulin, beta-2 microglobulin, calbindin, clusterin, CTGF, creatinine, cystatin C, GST-alpha, KIM-1, microalbumin, NGAL, osteopontin, THP, TIMP-1, TFF-3, VEGF, BLC, CD40, IGF BP2, MMP3, peptide YY, stem cell factor, TNF RII, AXL, Eotaxin 3, FABP, FGF basic, myoglobin, resistin, TRAIL R3, endothelin 1, NrCAM, Tenascin C, VCAM1, and cortisol. The combination of sample concentrations may be compared to a data set comprising at least one entry, wherein each entry of the data set comprises a list comprising three or more minimum diagnostic concentrations indicative of kidney transplant rejection or an associated disorder. Each minimum diagnostic concentration comprises a maximum of a range of analyte concentrations for a healthy mammal. Next, the method comprises determining a matching entry of the dataset in which all minimum diagnostic concentrations are less than the corresponding sample concentrations and identifying an indicated disorder comprising the particular disorder of the matching entry.

Another aspect of the invention encompasses a method for diagnosing, monitoring, or determining kidney transplant rejection or an associated disorder in a mammal. The method generally comprises providing a test sample comprising a sample of bodily fluid taken from the mammal. Then the method comprises determining the concentrations of three or more sample analytes in a panel of biomarkers in the test sample, wherein the sample analytes are selected from the group consisting of alpha-1 microglobulin, beta-2 microglobulin, calbindin, clusterin, CTGF, creatinine, cystatin C, GST-alpha, KIM-1, microalbumin, NGAL, osteopontin, THP, TIMP-1, TFF-3, VEGF, BLC, CD40, IGF BP2, MMP3, peptide YY, stem cell factor, TNF RII, AXL, Eotaxin 3, FABP, FGF basic, myoglobin, resistin, TRAIL R3, endothelin 1, NrCAM, Tenascin C, VCAM1, and cortisol. Diagnostic analytes are identified in the test sample, wherein the diagnostic analytes are the sample analytes whose concentrations are statistically different from concentrations found in a control group of humans who do not suffer from kidney transplant rejection or an associated disorder. The combination of diagnostic analytes is compared to a dataset comprising at least one entry, wherein each entry of the dataset comprises a combination of three or more diagnostic analytes reflective of kidney transplant rejection or an associated disorder. The particular disorder having the combination of diagnostic analytes that essentially match the combination of sample analytes is then identified.

An additional aspect of the invention encompasses a method for diagnosing, monitoring, or determining kidney transplant rejection or an associated disorder in a mammal. The method usually comprises providing an analyte concentration measurement device comprising three or more detection antibodies. Each detection antibody comprises an antibody coupled to an indicator, wherein the antigenic determinants of the antibodies are sample analytes associated with kidney transplant rejection or an associated disorder. The sample analytes are generally selected from the group consisting of alpha-1 microglobulin, beta-2 microglobulin, calbindin, clusterin, CTGF, creatinine, cystatin C, GST-alpha, KIM-1, microalbumin, NGAL, osteopontin, THP, TIMP-1, TFF-3, VEGF, BLC, CD40, IGF BP2, MMP3, peptide YY, stem cell factor, TNF RII, AXL, Eotaxin 3, FABP, FGF basic, myoglobin, resistin, TRAIL R3, endothelin 1, NrCAM, Tenascin C, VCAM1, and cortisol. The method next comprises providing a test sample comprising three or more sample analytes and a bodily fluid taken from the mammal. The test sample is contacted with the detection antibodies and the detection antibodies are allowed to bind to the sample analytes. The concentrations of the sample analytes are determined by detecting the indicators of the detection antibodies bound to the sample analytes in the test sample. The concentrations of each sample analyte correspond to a corresponding minimum diagnostic concentration reflective of kidney transplant rejection or an associated disorder.

Other aspects and iterations of the invention are described in more detail below.

## DESCRIPTION OF FIGURES

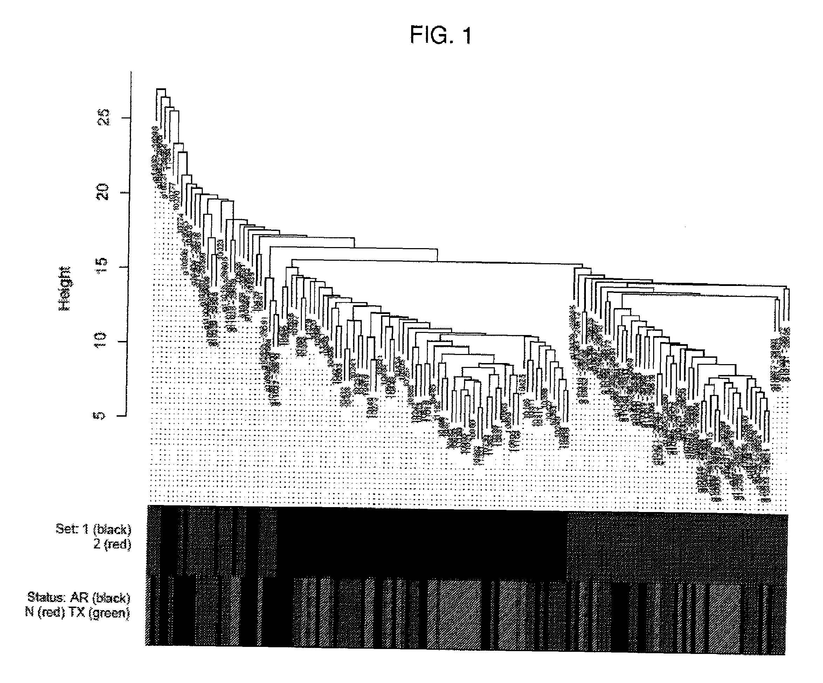

FIG.1 depicts a sample clustering tree (dendrogram) together with set and status indicators. Below the tree the set that each sample belongs to (black encodes Set 1, red Set 2; see Examples) is shown, and the patient status (black encodes AR (acute rejection), red CAN (chronic allograft nephropathy), green TX(successful, non-rejected transplant)). The sample tree contains two large branches, one of which corresponds to the Set 1 (the large black block in the set indicator color bar), and one that corresponds to Set 2 (the large red block in the set indicator color bar). This two-branch structure points to a batch effect.

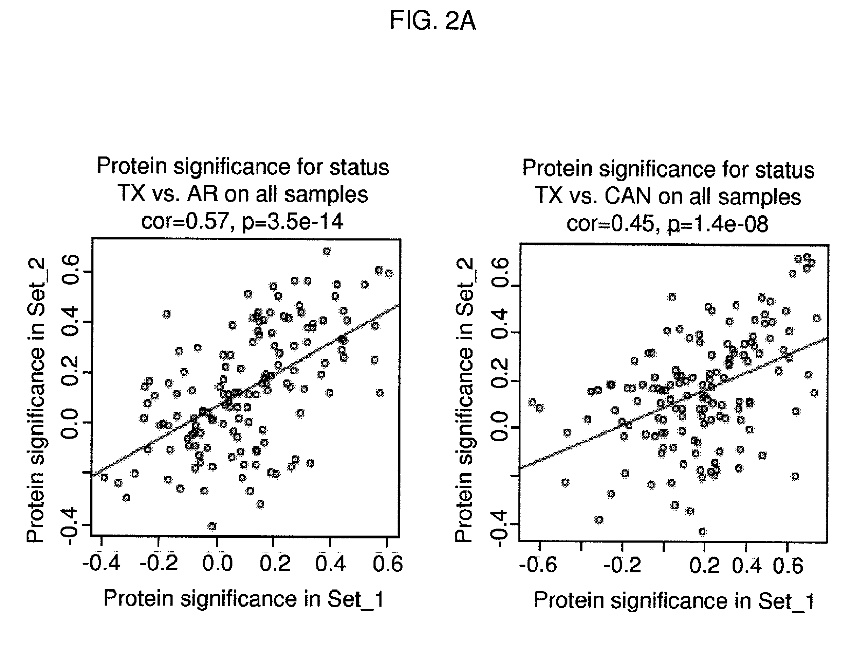

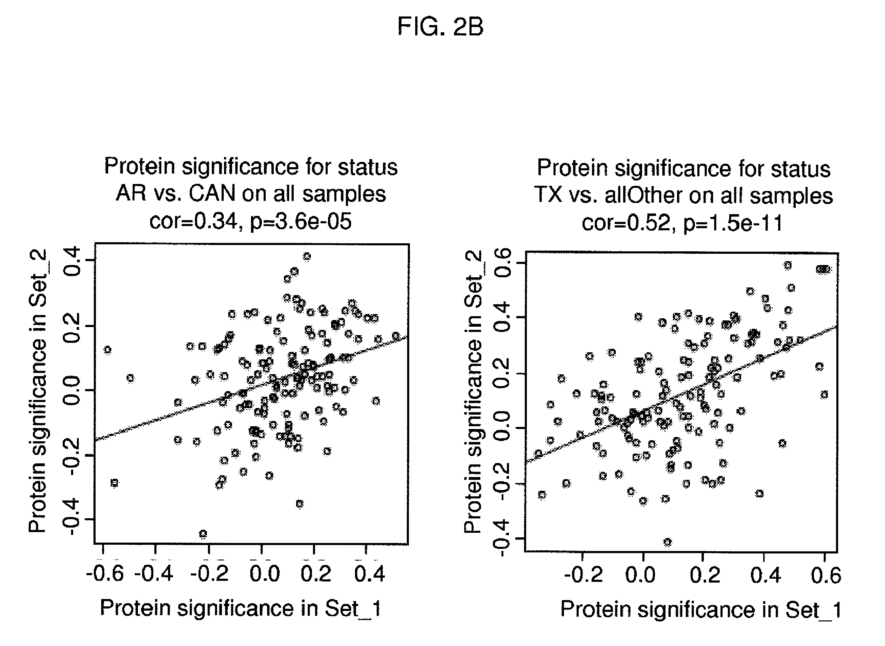

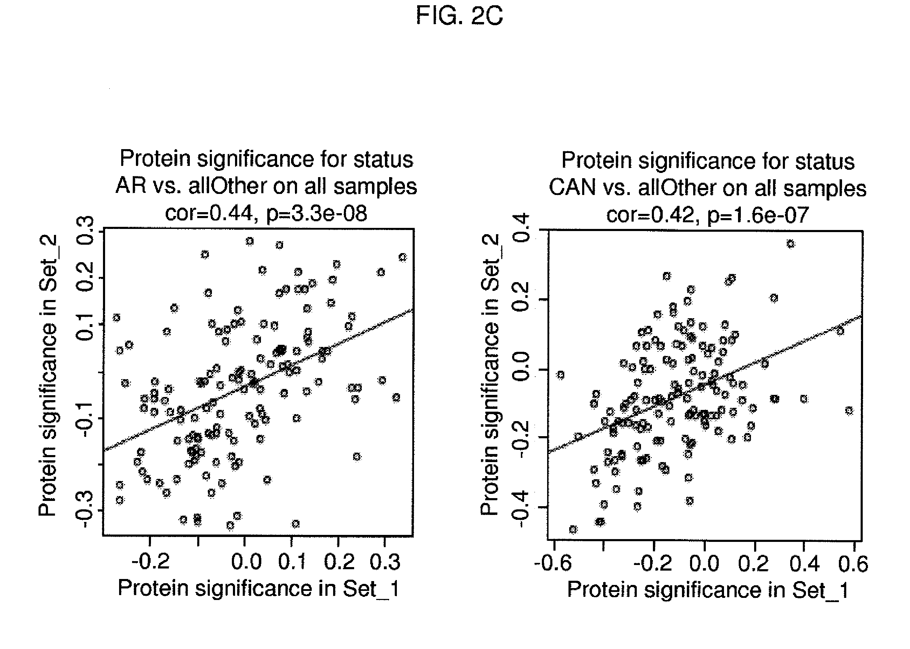

FIG. 2 depicts scatterplots of protein significance for the comparisons TX vs. AR, TX vs. CAN, AR vs. CAN (in each case, the samples belonging to the third group are ignored), and for the comparisons TX vs. all others, AR vs. all others, CAN vs. all others, in Set 2 (y-axis) vs. Set 1 (x-axis). Each dot represents a protein; protein significance is defined as biweight midcorreiation [1] of the protein level with status. The correlation and p-value, and a linear model fit line are also included.

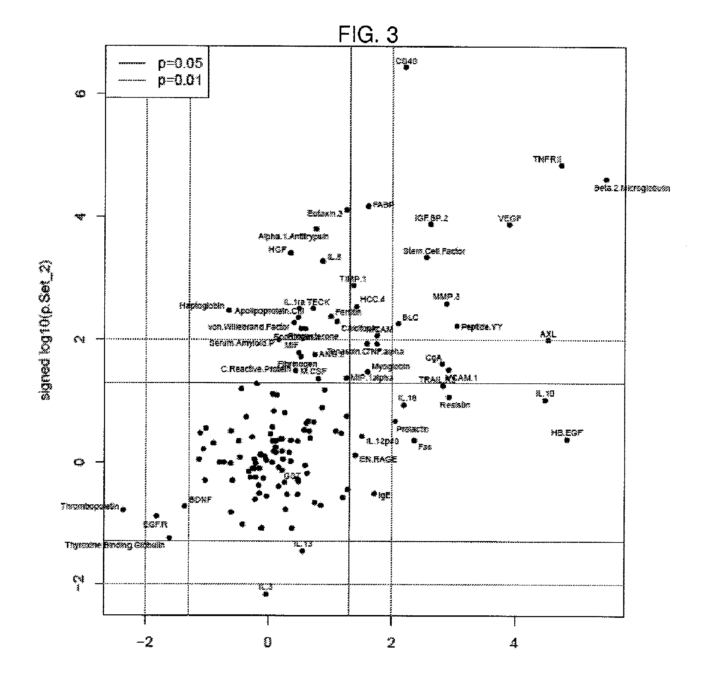

FIG. 3 depicts a scatterplot of protein significance for TX vs. AR in Set 2 (y-axis) vs. Set 1 (x-axis). Positive significance identifies proteins whose levels are higher in AR than TX, and negative the opposite. Each dot represents a protein; in this plot the negative logarithm of the association p-value multiplied by the sign of the robust correlation of the protein is plotted with TX vs. AR status. Kidney injury markers identified in previous work are plotted in blue, while all other proteins are black. Proteins with relatively high overall significance are labeled by their names or symbols. The green and red lines denote p-value thresholds of 0.01 and 0.05, respectively.

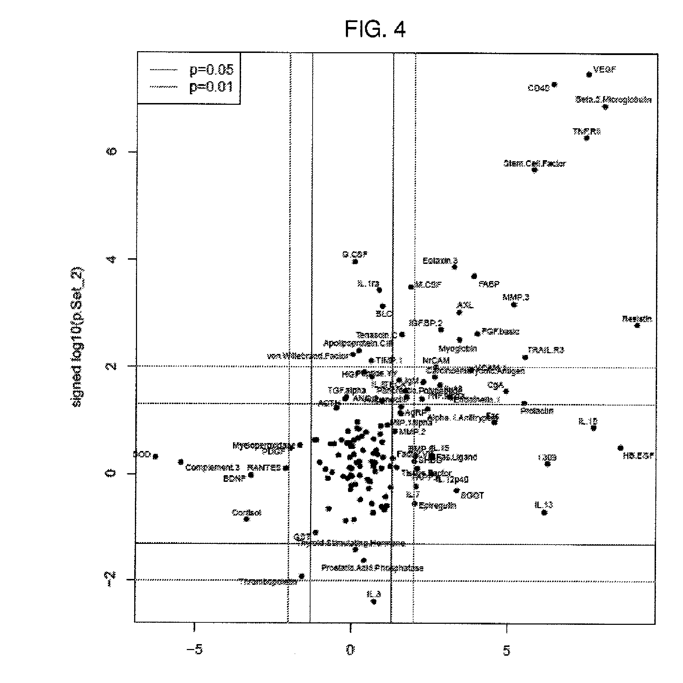

FIG. 4 depicts a scatterplot of protein significance for TX vs. CAN in Set 2 (y-axis) vs. Set 1 (x-axis). Positive significance identifies proteins whose levels are higher in CAN than TX, and negative the opposite. Each dot represents a protein; in this plot the negative logarithm of the association p-value multiplied by the sign of the robust correlation of the protein is plotted with TX vs. CAN status. Kidney injury markers identified in previous work are plotted in blue, while all other proteins are black. Proteins with relatively high overall significance are labeled by their names or symbols. The green and red lines denote p-value thresholds of 0.01 and 0.05, respectively.

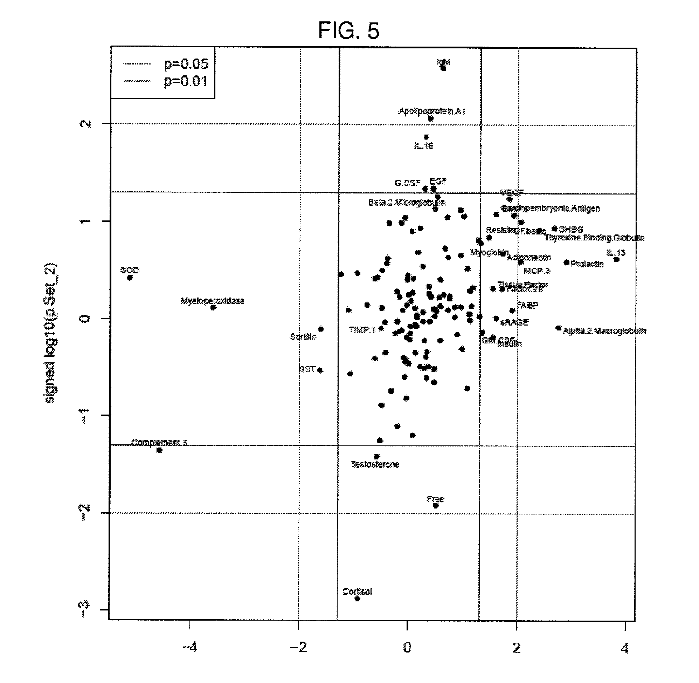

**FIG. 5** depicts a scatterplot of protein significance for AR vs. CAN in Set 2 (y-axis) vs. Set 1 (x-axis). Positive significance identifies proteins whose levels are higher in CAN than AR, and negative the opposite. Each dot represents a protein; in this plot the negative logarithm of the association p-value multiplied by the sign of the robust correlation of the protein is plotted with AR vs. CAN status. Kidney injury markers identified in previous work are plotted in blue, while all other proteins are black. Proteins with relatively high overall significance are labeled by their names or symbols. The green and red lines denote p-value thresholds of 0.01 and 0.05, respectively.

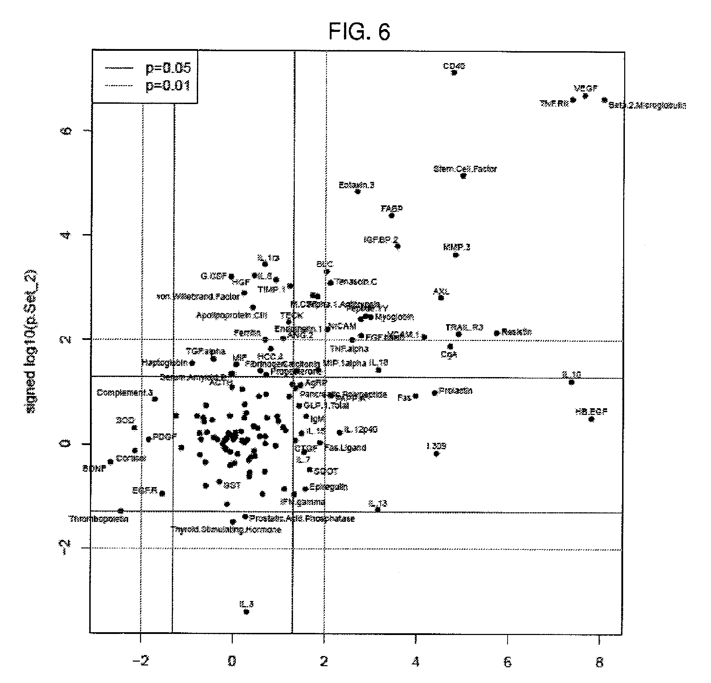

**FIG. 6** depicts a scatterplot of protein significance for TX vs. all others in Set 2 (y-axis) vs. Set 1 (x-axis). Positive significance identifies proteins whose levels are higher in others than TX, and negative the opposite. Each dot represents a protein; in this plot the negative logarithm of the association p-value multiplied by the sign of the robust correlation of the protein is plotted with TX vs. all others status. Kidney injury markers identified in previous work are plotted in blue, while all other proteins are black. Proteins with relatively high overall significance are labeled by their names or symbols. The green and red lines denote p-value thresholds of 0.01 and 0.05, respectively.

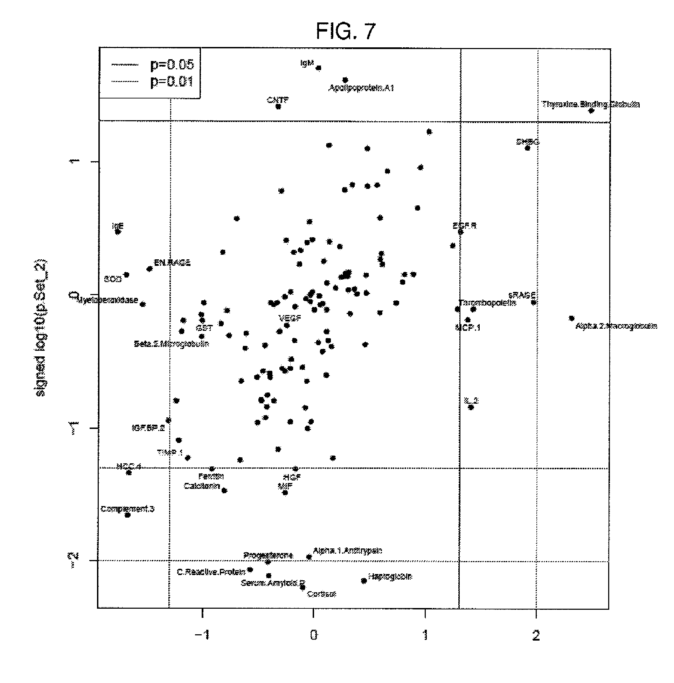

**FIG. 7** depicts a scatterplot of protein significance for AR vs. all others in Set 2 (y-axis) vs. Set 1 (x-axis). Positive significance identifies proteins whose levels are higher in others than AR, and negative the opposite. Each dot represents a protein; in this plot the negative logarithm of the association p-value multiplied by the sign of the robust correlation of the protein is plotted with AR vs. all others status. Kidney injury markers identified in previous work are plotted in blue, while all other proteins are black. Proteins with relatively high overall significance are labeled by their names or symbols. The green and red lines denote p-value thresholds of 0.01 and 0.05, respectively.

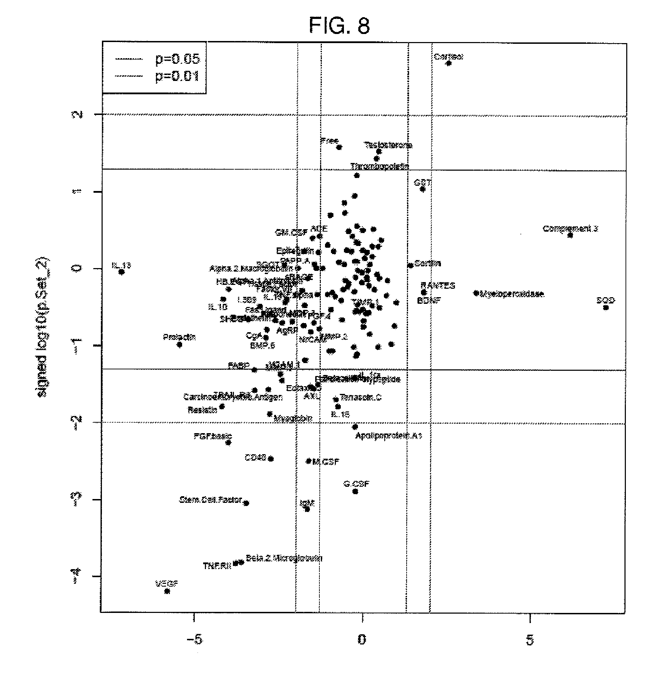

**FIG. 8** depicts a scatterplot of protein significance for CAN vs. all others in Set 2 (y-axis) vs. Set 1 (x-axis). Positive significance identifies proteins whose levels are higher in others than CAN, and negative the opposite. Each dot represents a protein; in this plot the negative logarithm of the association p-value multiplied by the sign of the robust correlation of the protein is plotted with CAN vs. all others status. Kidney injury markers identified in previous work are plotted in blue, while all other proteins are black. Proteins with relatively high overall significance are labeled by their names or symbols. The green and red lines denote p-value thresholds of 0.01 and 0.05, respectively.

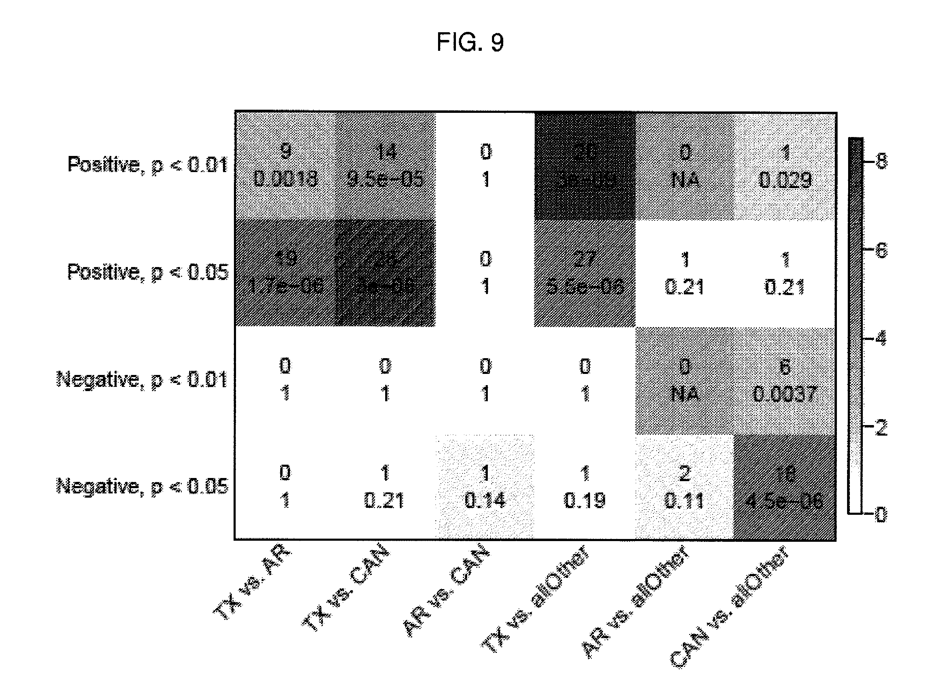

**FIG. 9** depicts a chart showing the p-values for finding the observed numbers of genes by chance. Each row corresponds to one significance level and sign of the relationship between protein level and trait, while each column corresponds to a comparison. Thus, for example, the p-value of finding 9 genes with p-values less than 0.01 in the TX vs AR comparison (upper left square) is 0.0018.

## DETAILED DESCRIPTION OF THE INVENTION

It has been discovered that a multiplexed panel of at least three, six, or preferably 16 biomarkers may be used to detect kidney transplant rejection and associated disorders. In particular, a panel or method of the invention may be used to detect acute kidney rejection or chronic allograft nephropathy. Importantly, a panel or method of the invention may be used to distinguish between an acute rejection reaction and a chronic allograft nephropathy. Alternatively, a panel or method of the invention may be used to distinguish between a successful transplant and rejection. As used herein, the term "rejection" refers to a recipient response to a foreign antigen derived from the transplanted kidney. The phrase "acute rejection" refers to an immune related response to the foreign kidney. The response is primarily T-cell driven and originates from an HLC mismatch between the donor and recipient. The phrase "chronic allograft nephropathy" refers to a chronic inflammatory and immune response mediated reaction to a foreign kidney. Chronic allograft nephropathy may result in damage to the kidney manifested by diffuse interstitial fibrosis glomerular changes, typically membranous and sclerotic in nature, as well as intimal fibrosis of the blood vessels with tubular atrophy and loss of tubular structures.

Additionally, the present invention encompasses biomarkers that may be used to detect a disorder associated with kidney transplant rejection. As used herein, the phrase "a disorder associated with kidney transplant rejection" refers to a disorder that stems from a host response to a foreign antigen derived from the transplated kidney. For instance, non-limiting examples of associated disorders may include chronic kidney failure and end-stage kidney disease.

The biomarkers included in a multiplexed panel of the invention are analytes known in the art that may be detected in the urine, serum, plasma and other bodily fluids of mammals. As such, the analytes of the multiplexed panel may be readily extracted from the mammal in a test sample of bodily fluid. The concentrations of the analytes within the test sample may be measured using known analytical techniques such as a multiplexed antibody-based immunological assay. The combination of concentrations of the analytes in the test sample may be compared to empirically determined combinations of minimum diagnostic concentrations and combinations of diagnostic concentration ranges associated with healthy kidney function or kidney transplant rejection or an associated disorder to determine whether kidney transplant rejection, and if so, what type of rejection, is indicated in the mammal.

One embodiment of the present invention provides a method for diagnosing, monitoring, or determining kidney transplant rejection or an associated disorder in a mammal that includes determining the presence or concentration of a combination of three or more sample analytes in a test sample containing the bodily fluid of the mammal. The measured concentrations of the combination of sample analytes is compared to the entries of a dataset in which each entry contains the minimum diagnostic concentrations of a combination of three of more analytes reflective of kidney transplant rejection or an associated disorder. Other embodiments provide computer-readable media encoded with applications containing executable modules, systems that include databases and processing devices containing executable modules configured to diagnose, monitor, or determine a renal disorder in a mammal. Still other embodiments provide antibody-based devices for diagnosing, monitoring, or determining kidney transplant rejection or an associated disorder in a mammal.

The analytes used as biomarkers in the multiplexed assay, methods of diagnosing, monitoring, or determining a renal disorder using measurements of the analytes, systems and applications used to analyze the multiplexed assay measurements, and antibody-based devices used to measure the analytes are described in detail below.

## I. Analytes in Multiplexed Assay

One embodiment of the invention measures the concentrations of three, six, sixteen, or more than 16 biomarker analytes within a test sample taken from a mammal and compares the measured analyte concentrations to minimum diagnostic concentrations to diagnose, monitor, or determine kidney transplant rejection or an associated disorder in a mammal. In this aspect, the biomarker analytes are known in the art to occur in the urine, plasma, serum and other bodily fluids of mammals. The biomarker analytes are proteins that have known and documented associations with early renal damage in humans. As defined herein, the biomarker analytes may include but are not limited to alpha-1 microglobulin, beta-2 microglobulin, calbindin, clusterin, CTGF, creatinine, cystatin C, GST-alpha, KIM-1, microalbumin, NGAL, osteopontin, THP, TIMP-1, TFF-3, VEGF, BLC, CD40, IGF BP2, MMP3, peptide YY, stem cell factor, TNF RII, AXL, Eotaxin 3, FABP, FGF basic, myoglobin, resistin, TRAIL R3, endothelin 1, NrCAM, Tenascin C, VCAM1, and cortisol. A description of each biomarker analyte is given below.

### (a) Alpha-1 Microglobulin (A1M)

Alpha-1 microglobulin (A1M, Swiss-Prot Accession Number P02760) is a 26 kDa glycoprotein synthesized by the liver and reabsorbed in the proximal tubules. Elevated levels of A1 M in human urine are indicative of glomerulotubular dysfunction. A1 M is a member of the lipocalin super family and is found in all tissues. Alpha-1-microglobulin exists in blood in both a free form and complexed with immunoglobulin A (IgA) and heme. Half of plasma A1 M exists in a free form, and the remainder exists in complexes with other molecules including prothrombin, albumin, immunoglobulin A and heme. Nearly all of the free A1M in human urine is reabsorbed by the megalin receptor in proximal tubular cells, where it is then catabolized. Small amounts of A1 M are excreted in the urine of healthy humans. Increased A1 M concentrations in human urine may be an early indicator of renal damage, primarily in the proximal tubule.

### (b) Beta-2 Microglobulin (B2M)

Beta-2 microglobulin (B2M, Swiss-Prot Accession Number P61769) is a protein found on the surfaces of all nucleated cells and is shed into the blood, particularly by tumor cells and lymphocytes. Due to its small size, B2M passes through the glomerular membrane, but normally less than 1% is excreted due to reabsorption of B2M in the proximal tubules of the kidney. Therefore, high plasma levels of B2M occur as a result of renal failure, inflammation, and neoplasms, especially those associated with B-lymphocytes.

### (c) Calbindin

Calbindin (Calbindin D-28K, Swiss-Prot Accession Number P05937) is a Ca-binding protein belonging to the troponin C superfamily. It is expressed in the kidney, pancreatic islets, and brain. Calbindin is found predominantly in subpopulations of central and peripheral nervous system neurons, in certain epithelial cells involved in Ca2+ transport such as distal tubular cells and cortical collecting tubules of the kidney, and in enteric neuroendocrine cells.

### (d) Clusterin

Clusterin (Swiss-Prot Accession Number P10909) is a highly conserved protein that has been identified independently by many different laboratories and named SGP2, S35-S45, apolipoprotein J, SP-40, 40, ADHC-9, gp80, GPIII, and testosterone-repressed prostate message (TRPM-2). An increase in clusterin levels has been consistently detected in apoptotic heart, brain, lung, liver, kidney, pancreas, and retinal tissue both *in vivo* and *in vitro,* establishing clusterin as a ubiquitous marker of apoptotic cell loss. However, clusterin protein has also been implicated in physiological processes that do not involve apoptosis, including the control of complement-mediated cell lysis, transport of beta-amyloid precursor protein, shuttling of aberrant beta-amyloid across the blood-brain barrier, lipid scavenging, membrane remodeling, cell aggregation, and protection from immune detection and tumor necrosis factor induced cell death.

### (e) Connective Tissue Growth Factor (CTGF)

Connective tissue growth factor (CTGF, Swiss-Prot Accession Number P29279) is a 349-amino acid cysteine-rich polypeptide belonging to the CCN family. *In vitro* studies have shown that CTGF is mainly involved in extracellular matrix synthesis and fibrosis. Up-regulation of CTGF mRNA and increased CTGF levels have been observed in various diseases, including diabetic nephropathy and cardiomyopathy, fibrotic skin disorders, systemic sclerosis, biliary atresia, liver fibrosis and idiopathic pulmonary fibrosis, and nondiabetic acute and progressive glomerular and tubulointerstitial lesions of the kidney. A recent cross-sectional study found that urinary CTGF may act as a progression promoter in diabetic nephropathy.

### (f) Creatinine

Creatinine is a metabolite of creatine phosphate in muscle tissue, and is typically produced at a relatively constant rate by the body. Creatinine is chiefly filtered out of the blood by the kidneys, though a small amount is actively secreted by the kidneys into the urine. Creatinine levels in blood and urine may be used to estimate the creatinine clearance, which is representative of the overall glomerular filtration rate (GFR), a standard measure of renal function. Variations in creatinine concentrations in the blood and urine, as well as variations in the ratio of urea to creatinine concentration in the blood, are common diagnostic measurements used to assess renal function.

### (g) Cystatin C (Cyst C)

Cystatin C (Cyst C, Swiss-Prot Accession Number P01034) is a 13 kDa protein that is a potent inhibitor of the C1 family of cysteine proteases. It is the most abundant extracellular inhibitor of cysteine proteases in testis, epididymis, prostate, seminal vesicles and many other tissues. Cystatin C, which is normally expressed in vascular wall smooth muscle cells, is severely reduced in both atherosclerotic and aneurismal aortic lesions.

### (h) Glutathione S-Transferase alpha (GST-alpha)

Glutathione S-transferase alpha (GST-alpha, Swiss-Prot Accession Number P08263) belongs to a family of enzymes that utilize glutathione in reactions contributing to the transformation of a wide range of compounds, including carcinogens, therapeutic drugs, and products of oxidative stress. These enzymes play a key role in the detoxification of such substances.

### (i) Kidney Injury Molecule-1 (KIM-1)

Kidney injury molecule-1 (KiM-1, Swiss-Prot Accession Number Q96D42) is an immunoglobulin superfamily cell-surface protein highly upregulated on the surface of injured kidney epithelial cells. It is also known as TIM-1 (T-cell immunoglobulin mucin domain-1), as it is expressed at low levels by subpopulations of activated T-cells and hepatitis A virus cellular receptor-1 (HAVCR-1). KIM-1 is increased in expression more than any other protein in the injured kidney and is localized predominantly to the apical membrane of the surviving proximal epithelial cells.

### (j) Microalbumin

Albumin is the most abundant plasma protein in humans and other mammals. Albumin is essential for maintaining the osmotic pressure needed for proper distribution of body fluids between intravascular compartments and body tissues. Healthy, normal kidneys typically filter out albumin from the urine. The presence of albumin in the urine may indicate damage to the kidneys. Albumin in the urine may also occur in patients with long-standing diabetes, especially type 1 diabetes. The amount of albumin eliminated in the urine has been used to differentially diagnose various renal disorders. For example, nephrotic syndrome usually results in the excretion of about 3.0 to 3.5 grams of albumin in human urine every 24 hours. Microalbuminuria, in which less than 300mg of albumin is eliminated in the urine every 24 hours, may indicate the early stages of diabetic nephropathy.

### (k) Neutrophil Gelatinase-Associated Lipocalin (NGAL)

Neutrophil gelatinase-associated lipocalin (NGAL, Swiss-Prot Accession Number P80188) forms a disulfide bond-linked heterodimer with MMP-9. It mediates an innate immune response to bacterial infection by sequestrating iron. Lipocalins interact with many different molecules such as cell surface receptors and proteases, and play a role in a variety of processes such as the progression of cancer and allergic reactions.

### (l) Osteopontin (OPN)

Osteopontin (OPN, Swiss-Prot Accession Number P10451) is a cytokine involved in enhancing production of interferon-gamma and IL-12, and inhibiting the production of IL-10. OPN is essential in the pathway that leads to type I immunity. OPN appears to form an integral part of the mineralized matrix. OPN is synthesized within the kidney and has been detected in human urine at levels that may effectively inhibit calcium oxalate crystallization. Decreased concentrations of OPN have been documented in urine from patients with renal stone disease compared with normal individuals.

### (m) Tamm-Horsfall Protein (THP)

Tamm-Horsfall protein (THP, Swiss-Prot Accession Number P07911), also known as uromodulin, is the most abundant protein present in the urine of healthy subjects and has been shown to decrease in individuals with kidney stones. THP is secreted by the thick ascending limb of the loop of Henley. THP is a monomeric glycoprotein of ~ 85 kDa with ~30% carbohydrate moiety that is heavily glycosylated. THP may act as a constitutive inhibitor of calcium crystallization in renal fluids.

### (n) Tissue Inhibitor of Metalloproteinase-1 (TIMP-1)

Tissue inhibitor of metalloproteinase-1 (TIMP-1, Swiss-Prot Accession Number P01033) is a major regulator of extracellular matrix synthesis and degradation. A certain balance of MMPs and TIMPs is essential for tumor growth and health. Fibrosis results from an imbalance of fibrogenesis and fibrolysis, highlighting the importance of the role of the inhibition of matrix degradation role in renal disease.

### (o) Trefoil Factor 3 (TFF3)

Trefoil factor 3 (TFF3, Swiss-Prot Accession Number Q07654), also known as intestinal trefoil factor, belongs to a small family of mucin-associated peptides that include TFF1, TFF2, and TFF3. TFF3 exists in a 60-amino acid monomeric form and a 118-amino acid dimeric form. Under normal conditions TFF3 is expressed by goblet cells of the intestine and the colon. TFF3 expression has also been observed in the human respiratory tract, in human goblet cells and in the human salivary gland. In addition, TFF3 has been detected in the human hypothalamus.

### (p) Vascular Endothelial Growth Factor (VEGF)

Vascular endothelial growth factor (VEGF, Swiss-Prot Accession Number P15692) is an important factor in the pathophysiology of neuronal and other tumors, most likely functioning as a potent promoter of angiogenesis. VEGF may also be involved in regulating blood-brain-barrier functions under normal and pathological conditions. VEGF secreted from the stromal cells may be responsible for the endothelial cell proliferation observed in capillary hemangioblastomas, which are typically composed of abundant microvasculature and primitive angiogenic elements represented by stromal cells.

### (q) B-lymphocyte Chemoattractant (BLC)

B-lymphocyte chemoattractant (BLC, Swiss-Prot Accession Number 043927) is also referred to as C-X-C motif chemokine 13, Small-inducible cytokine B13, B lymphocyte chemoattractant, CXC chemokine BLC, and B cell-attracting chemokine 1. BLC functions as a potent chemoattractant for B lymphocytes, but not T lymphocytes, monocytes, or neutrophils. Its specific receptor BLR1 is a G protein-coupled receptor originally isolated from Burkitt's lymphoma cells. Among cells of the hematopoietic lineages, the expression of BRL1, now designated CXCR5, is restricted to B lymphocytes and a subpopulation of T helper memory cells.

*(r) Cluster of Differentiation Surface Receptors 40 (CD40)*

Cluster of Differentiation Surface Receptors 40 (CD40, Swiss Prot Accession Number P25942) is also referred to TNFRSF5 (Tumor necrosis factor receptor superfamily member 5. CD40 is a member of the tumor necrosis factor-receptor superfamily of proteins. CD40 has been found to be essential in mediating a broad variety of immune and inflammatory responses including T cell-dependent immunoglobulin class switching, memory B cell development, and germinal center formation.

*(s) Insulin-like Growth Factor Binding Protein 2 (IGF BP2)*

Insulin-like Growth Factor Binding Protein 2 (IGF BP2, Swiss Prot Accession Number P18065) functions to prolong the half-life of the insulin growth factors and have been shown to either inhibit or stimulate the growth promoting effects of the insulin growth factors on cell culture. Specifically, during development, insulin-like growth factor binding protein-2 is expressed in a number of tissues with the highest expression level found in the central nervous system. IGFBP-2 exhibits a 2-10 fold higher affinity for IGF II than for IGF I.

*(t) Matrix Metalloproteinass-3 (MMP3)*

Matrix Metalloproteinase-3 (MMP3, Swiss Prot Accession Number P08254) is also known as stromelysin-1 and Transin-1. MMP3 is involved in the breakdown of extracellular matrix in normal physiological processes, such as embryonic development, reproduction, and tissue remodeling, as well as in disease processes, such as arthritis and metastasis. Most MMP's are secreted as inactive proproteins which are activated when cleaved by extracellular proteinases. MMP3 encodes an enzyme which degrades fibronectin, laminin, collagens III, IV, IX, and X, and cartilage proteoglycans. The enzyme is thought to be involved in wound repair, progression of atherosclerosis, and tumor initiation. MMP3 is part of a cluster of MMP genes which localize to chromosome 11q22.3.

*(u) Peptide YY (PYY)*

Peptide YY (PYY, Swiss-Prot Accession Number P10082) is also known as peptide tyrosine tyrosine and pancreatic peptide $YY_{3-36}$. Peptide YY exerts its action through neuropeptide Y receptors, inhibits gastric motility and increases water and electrolyte absorption in the colon. PYY may also suppress pancreatic secretion. It is secreted by the neuroendocrine cells in the ileum and colon in response to a meal, and has been shown to reduce appetite. PYY works by slowing the gastric emptying; hence, it increases efficiency of digestion and nutrient absorption after meal. Research has also indicated that PYY may be useful in removing aluminum accumulated in the brain.

*(v) Stem Cell Factor (SCF)*

Stem Cell Factor (SCF, UniProtKB/TrEMBL Q13528) is also known as kit-ligand, KL, and steel factor. SCF functions SCF plays an important role in the hematopoiesis during embryonic development. Sites where hematopoiesis takes place, such as the fetal liver and bone marrow, all express SCF. SCF may serve as guidance cues that direct hematopoietic stem cells (HSCs) to their stem cell niche (the microenvironment in which a stem cell resides), and it plays an important role in HSC maintenance. Non-lethal point mutants on the c-Kit receptor can cause anemia, decreased fertility, and decreased pigmentation. During development, the presence of the SCF also plays an important role in the localization of melanocytes, cells that produce melanin and control pigmentation. In melanogenisis, melanoblasts migrate from the neural crest to their appropriate locations in the epidermis. Melanoblasts express the Kit receptor, and it is believed that SCF guides these cells to their terminal locations. SCF also regulates survival and proliferation of fully differentiated melanocytes in adults. In spermatogenesis, c-Kit is expressed in primordial germ cells, spermatogonia, and in primordial oocytes. It is also expressed in the primordial germ cells of females. SCF is expressed along the pathways that the germ cells use to reach their terminal destination in the body. It is also expressed in the final destinations for these cells. Like for melanoblasts, this helps guide the cells to their appropriate locations in the body

*(w) Tumor Necrosis Factor Receptor Type II (TNF RII)*

Tumor Necrosis Factor Receptor Type II (TNF RII, Swiss-Prot Accession Number P20333) is also known as p75, p80 TNF alpha receptor, and TNFRSF1 B. TNF RII is a protein that in humans is encoded by the TNFRSF1 B gene. The protein encoded by this gene is a member of the Tumor necrosis factor receptor superfamily, which also contains TNFRSF1 A. The protein encoded by this gene is a member of the TNF-receptor superfamily. This protein and TNF-receptor 1 form a heterocomplex that mediates the recruitment of two anti-apoptotic proteins, c-IAP1 and c-IAP2, which possess E3 ubiquitin ligase activity. The function of IAPs in TNF-receptor signaling is unknown; however, c-IAP1 is thought to potentiate TNF-induced apoptosis by the ubiquitination and degradation of TNF-receptor-associated factor 2, which mediates anti-apoptotic signals. Knockout studies in mice also suggest a role of this protein in protecting neurons from apoptosis by stimulating antioxidative pathways.

### (x) AXL Oncogene

AXL (Swiss-Prot Accession Number P30530) is also known as UFO, ARK, and tyrosine-protein kinase receptor UFO. The protein encoded by AXL is a member of the receptor tyrosine kinase subfamily. Although it is similar to other receptor tyrosine kinases, the AXL protein represents a unique structure of the extracellular region that juxtaposes IgL and FNIII repeats. AXL transduces signals from the extracellular matrix into the cytoplasm by binding growth factors like vitamin K-dependent protein growth-arrest-specific gene 6. It is involved in the stimulation of cell proliferation. This receptor can also mediate cell aggregation by homophilic binding. AXL is a chronic myelogenous leukemia-associated oncogene and also associated with colon cancer and melanoma.

### (y) Eotaxin 3

Eotaxin 3 (Swiss-Prot Accession Number P51671) is also known as C-C motif chemokine 11 (CCL11), small inducible cytokine A11, and eosinophil chemotactic protein. Eotaxin 3 is a small cytokine belonging to the CC chemokine family that is also called Eotaxin-3, Macrophage inflammatory protein 4-alpha (MIP-4-alpha), Thymic stroma chemokine-1 (TSC-1), and IMAC. It is expressed by several tissues including heart, lung and ovary, and in endothelial cells that have been stimulated with the cytokine interleukin 4.[1][2] CCL26 is chemotactic for eosinophils and basophils and elicits its effects by binding to the cell surface chemokine receptor CCR3.

### (z) Fatty Acid Binding Protein (FABP)

Fatty Acid Binding Protein (FABP, Swiss-Prot Accession Number Q01469) is also known as epidermal-type fatty acid binding protein, and fatty-acid binding protein 5. This gene encodes the fatty acid binding protein found in epidermal cells, and was first identified as being upregulated in psoriasis tissue. Fatty acid binding proteins are a family of small, highly conserved, cytoplasmic proteins that bind longchain fatty acids and other hydrophobic ligands. It is thought that FABPs roles include fatty acid uptake, transport, and metabolism..

### (aa) Basic Fibroblast Growth Factor (FGF basic)

Basic Fibroblast Growth Factor (FGF basic, Swiss-Prot Accession NumberP09038) is also known as heparin-binding growth factor. In normal tissue, basic fibroblast growth factor is present in basement membranes and in the subendothelial extracellular matrix of blood vessels. It stays membrane-bound as long as there is no signal peptide. It has been hypothesized that, during both wound healing of normal tissues and tumor development, the action of heparan sulfate-degrading enzymes activates FGF basic, thus mediating the formation of new blood vessels. Additionally, FGF basic is a critical component of human embryonic stem cell culture medium; the growth factor is necessary for the cells to remain in an undifferentiated state, although the mechanisms by which it does this are poorly defined. It has been demonstrated to induce gremlin expression which in turn is known to inhibit the induction of differentiation by bone morphogenetic proteins. It is necessary in mouse-feeder cell dependent culture systems, as well as in feeder and serum-free culture systems.

### (bb) Myoglobin

Myoglobin (Swiss-Prot Accession Number P02144) is released from damaged muscle tissue (rhabdomyolysis), which has very high concentrations of myoglobin. The released myoglobin is filtered by the kidneys but is toxic to the renal tubular epithelium and so may cause acute renal failure. Myoglobin is a sensitive marker for muscle injury, making it a potential marker for heart attack in patients with chest pain.

### (cc) Resistin (RETN)

Resistin (RETN, UniProtKB/TrEMBL Q76B53) is theorized to participate in the inflammatory response. Resistin has also been shown to increase transcriptional events leading to an increased expression of several pro-inflammatory cytokines including (but not limited to) interleukin-1 (IL-1), interleukin-6 (IL-6), interleukin-12 (IL-12), and tumor necrosis factor-$\alpha$ (TNF-$\alpha$) in an NF-$\kappa$B-mediated fashion. It has also been demonstrated that resistin upregulates intracellular adhesion molecule-1 (ICAM1) vascular cell-adhesion molecule-1 (VCAM1) and CCL2, all of which are occupied in chemotactic pathways involved in leukocyte recruitment to sites of infection. Resistin itself can be upregulated by interleukins and also by microbial antigens such as lipopolysaccharide, which are recognized by leukocytes. Taken together, because resistin is reputed to contribute to insulin resistance, results such as those mentioned suggest that resistin may be a link in the well-known association between inflammation and insulin resistance. In fact, recent data have shown positive correlations between obesity, insulin resistance, and chronic inflammation which is believed to be directed in part by resistin signaling.

### (dd) Tumor Necrosis Factor-Related Apoptosis-Inducing Ligand Receptor 3 (TRAIL R3)

TRAIL R3 (Swiss-Prot Accession Number P83626 (mouse)) is also known as tumor necrosis factor-related apoptosis-inducing ligand receptor 3, and tumor necrosis factor receptor mouse homolog. TRAIL R3 is a decoy receptor for TRAIL, a member of the tumor necrosis factor family. In several cell types decoy receptors inhibit TRAIL-induced apoptosis by binding TRAIL and thus preventing its binding to proapoptotic TRAIL receptors.

### (ee) Endothelin 1 (ET1)

Endothelin 1 (ET1, UniProtKB/TrEMBL Q6FH53) is also known as EDN1 and EDN1 protein. Endothelin 1 is a protein that constricts blood vessels and raises blood pressure. It is normally kept in balance by other mechanisms, but when over-expressed, it contributes to high blood pressure (hypertension) and heart disease. Endothelin 1 peptides and receptors are implicated in the pathogenesis of a number of disease states, including cancer and heart disease.

### (ff) Neuronal Cell Adhesion Molecule (NrCAM)

Neuronal Cell Adhesion Molecule (NrCAM, UniProtKB/TrEMBL Q14CA1) encodes a neuronal cell adhesion molecule with multiple immunoglobulin-like C2-type domains and fibronectin type-III domains. This ankyrin-binding protein is involved in neuron-neuron adhesion and promotes directional signaling during axonal cone growth. This gene is also expressed in non-neural tissues and may play a general role in cell-cell communication via signaling from its intracellular domain to the actin cytoskeleton during directional cell migration. Allelic variants of this gene have been associated with autism and addiction vulnerability.

### (gg) Tenascin C (TN-C)

Tenascin C (TN-C, UniProt/TrEMBL Q99857) has anti-adhesive properties, causing cells in tissue culture to become rounded after it is added to the medium. One mechanism to explain this may come from its ability to bind to the extracellular matrix glycoprotein fibronectin and block fibronectin's interactions with specific syndecans. The expression of tenasan-C in the stroma of certain tumors is associated with a poor prognosis.

### (hh) Vascular Cell Adhesion Molecule 1 (VCAM1)

Vascular Cell Adhesion Molecule 1 (VCAM1, Swiss-Prot Accession Number P19320) is also known as vascular cell adhesion protein 1. VCAM1 mediates the adhesion of lymphocytes, monocytes, eosinophils, and basophils to vascular endothelium. It also functions in leukocyte-endothelial cell signal transduction, and it may play a role in the development of atherosclerosis and rheumatoid arthritis. Upregulation of VCAM-1 in endothelial cells by cytokines occurs as a result of increased gene transcription (e.g., in response to Tumor necrosis factor-alpha (TNF-$\alpha$) and Interleukin-1 (IL-1)) and through stabilization of Messenger RNA (mRNA) (e.g., Interleukin-4 (IL-4)). The promoter region of the VCAM-1 gene contains functional tandem NF-$\kappa$B (nuclear factor-kappa B) sites. The sustained expression of VCAM-1 lasts over 24 hours. Primarily, the VCAM-1 protein is an endothelial ligand for VLA-4 (Very Late Antigen-4 or $\alpha4\beta1$) of the $\beta1$ subfamily of integrins, and for integrin $\alpha4\beta7$. VCAM-1 expression has also been observed in other cell types (e.g., smooth muscle cells). It has also been shown to interact with EZR and Moesin. Certain melanoma cells can use VCAM-1 to adhere to the endothelium, and VCAM-1 may participate in monocyte recruitment to atherosclerotic sites.

### (ii) Cortisol

Cortisol (Swiss-Prot Accession Number P08185) is also known as corticosteroid-binding globulin, transcortin, and Serpin A6. Cortisol is a steroid hormone or glucocorticoid produced by the adrenal gland. It is released in response to stress, and to a low level of blood glucocorticoids. Its primary functions are to increase blood sugar through gluconeogenesis, suppress the immune system, and aid in fat, protein and carbohydrate metabolism. It also decreases bone formation. In addition, cortisol can weaken the activity of the immune system. Cortisol prevents proliferation of T-cells by rendering the interleukin-2 producer T-cells unresponsive to interleukin-1 (IL-1), and unable to produce the T-cell growth factor. Cortisol also has a negative feedback effect on interleukin-1. IL-1 must be especially useful in combating some diseases; however, endotoxin bacteria have gained an advantage by forcing the hypothalamus to increase cortisol levels via forcing secretion of CRH hormone, thus antagonizing IL-1 in this case. The suppressor cells are not affected by GRMF, so that the effective set point for the immune cells may be even higher than the set point for physiological processes. It reflects leukocyte redistribution to lymph nodes, bone marrow, and skin.

## II. Combinations of Analytes Measured by Multiplexed Assay

The method for diagnosing, monitoring, or determining a transplant rejection involves determining the presence or concentrations of a combination of sample analytes in a test sample. The combinations of sample analytes, as defined herein, are any group of three or more analytes selected from the biomarker analytes, including but not limited to alpha-1 microglobulin, beta-2 microglobulin, calbindin, clusterin, CTGF, creatinine, cystatin C, GST-alpha, KIM-1, microalbumin, NGAL, osteopontin, THP, TIMP-1, TFF-3, VEGF, BLC, CD40, IGF BP2, MMP3, peptide YY, stem cell factor, TNF RII, AXL, Eotaxin 3, FABP, FGF basic, myoglobin, resistin, TRAIL R3, endothelin 1, NrCAM, Tenascin C, VCAM1, and cortisol. In one embodiment, the combination of analytes may be selected to provide a group of analytes associated with kidney transplant rejection or an associated disorder.

In one embodiment, the combination of sample analytes may be any three of the biomarker analytes. In other embodiments, the combination of sample analytes may be any four, any five, any six, any seven, any eight, any nine, any ten, any eleven, any twelve, any thirteen, any fourteen, any fifteen, any sixteen, any seventeen, any eighteen, any nineteen, any twenty, or more of biomarker analytes listed in Section I above. In some embodiments, the combination of sample analytes comprises B2M and VEGF. In another embodiment, the combination of sample analytes may be a combination listed in **Table A** below.

**Table A**

| | | |
|---|---|---|
| BLC | CD40 | IGF BP2 |
| BLC | CD40 | MMP3 |
| BLC | CD40 | peptide YY |
| BLC | CD40 | stem cell factor |
| BLC | CD40 | TNF RII |
| BLC | CD40 | AXL |
| BLC | CD40 | Eotaxin 3 |
| BLC | CD40 | FABP |
| BLC | CD40 | FGF basic |
| BLC | CD40 | myoglobin |
| BLC | CD40 | resistin |
| BLC | CD40 | TRAIL R3 |
| BLC | CD40 | endothilin 1 |
| BLC | CD40 | NrCAM |
| BLC | CD40 | Tenascin C |
| BLC | CD40 | VCAM1 |
| BLC | CD40 | cortisol |
| BLC | IGF BP2 | MMP3 |
| BLC | IGF BP2 | peptide YY |
| BLC | IGF BP2 | stem cell factor |
| BLC | IGF BP2 | TNF RII |
| BLC | IGF BP2 | AXL |

(continued)

| BLC | IGF BP2 | Eotaxin 3 |
|---|---|---|
| BLC | IGF BP2 | FABP |
| BLC | IGF BP2 | FGF basic |
| BLC | IGF BP2 | myoglobin |
| BLC | IGF BP2 | resistin |
| BLC | IGF BP2 | TRAIL R3 |
| BLC | IGF BP2 | endothilin 1 |
| BLC | IGF BP2 | NrCAM |
| BLC | IGF BP2 | Tenascin C |
| BLC | IGF BP2 | VCAM1 |
| BLC | IGF BP2 | cortisol |
| BLC | MMP3 | peptide YY |
| BLC | MMP3 | stem cell factor |
| BLC | MMP3 | TNF RII |
| BLC | MMP3 | AXL |
| BLC | MMP3 | Eotaxin 3 |
| BLC | MMP3 | FABP |
| BLC | MMP3 | FGF basic |
| BLC | MMP3 | myoglobin |
| BLC | MMP3 | resistin |
| BLC | MMP3 | TRAIL R3 |
| BLC | MMP3 | endothilin 1 |
| BLC | MMP3 | NrCAM |
| BLC | MMP3 | Tenascin C |
| BLC | MMP3 | VCAM1 |
| BLC | MMP3 | cortisol |
| BLC | peptide YY | stem cell factor |
| BLC | peptide YY | TNF RII |
| BLC | peptide YY | AXL |
| BLC | peptide YY | Eotaxin 3 |
| BLC | peptide YY | FABP |
| BLC | peptide YY | FGF basic |
| BLC | peptide YY | myoglobin |
| BLC | peptide YY | resistin |
| BLC | peptide YY | TRAIL R3 |
| BLC | peptide YY | endothilin 1 |
| BLC | peptide YY | NrCAM |
| BLC | peptide YY | Tenascin C |
| BLC | peptide YY | VCAM1 |

(continued)

| BLC | peptide YY | cortisol |
|-----|-----|-----|
| BLC | stem cell factor | TNF RII |
| BLC | stem cell factor | AXL |
| BLC | stem cell factor | Eotaxin 3 |
| BLC | stem cell factor | FABP |
| BLC | stem cell factor | FGF basic |
| BLC | stem cell factor | myoglobin |
| BLC | stem cell factor | resistin |
| BLC | stem cell factor | TRAIL R3 |
| BLC | stem cell factor | endothilin 1 |
| BLC | stem cell factor | NrCAM |
| BLC | stem cell factor | Tenascin C |
| BLC | stem cell factor | VCAM1 |
| BLC | stem cell factor | cortisol |
| BLC | TNF RII | AXL |
| BLC | TNF RII | Eotaxin 3 |
| BLC | TNF RII | FABP |
| BLC | TNF RII | FGF basic |
| BLC | TNF RII | myoglobin |
| BLC | TNF RII | resistin |
| BLC | TNF RII | TRAIL R3 |
| BLC | TNF RII | endothilin 1 |
| BLC | TNF RII | NrCAM |
| BLC | TNF RII | Tenascin C |
| BLC | TNF RII | VCAM1 |
| BLC | TNF RII | cortisol |
| BLC | AXL | Eotaxin 3 |
| BLC | AXL | FABP |
| BLC | AXL | FGF basic |
| BLC | AXL | myoglobin |
| BLC | AXL | resistin |
| BLC | AXL | TRAIL R3 |
| BLC | AXL | endothilin 1 |
| BLC | AXL | NrCAM |
| BLC | AXL | Tenascin C |
| BLC | AXL | VCAM1 |
| BLC | AXL | cortisol |
| BLC | Eotaxin 3 | FABP |
| BLC | Eotaxin 3 | FGF basic |

(continued)

| BLC | Eotaxin 3 | myoglobin |
|---|---|---|
| BLC | Eotaxin 3 | resistin |
| BLC | Eotaxin 3 | TRAIL R3 |
| BLC | Eotaxin 3 | endothilin 1 |
| BLC | Eotaxin 3 | NrCAM |
| BLC | Eotaxin 3 | Tenascin C |
| BLC | Eotaxin 3 | VCAM1 |
| BLC | Eotaxin 3 | cortisol |
| BLC | FABP | FGF basic |
| BLC | FABP | myoglobin |
| BLC | FABP | resistin |
| BLC | FABP | TRAIL R3 |
| BLC | FABP | endothilin 1 |
| BLC | FABP | NrCAM |
| BLC | FABP | Tenascin C |
| BLC | FABP | VCAM1 |
| BLC | FABP | cortisol |
| BLC | FGF basic | myoglobin |
| BLC | FGF basic | resistin |
| BLC | FGF basic | TRAIL R3 |
| BLC | FGF basic | endothilin 1 |
| BLC | FGF basic | NrCAM |
| BLC | FGF basic | Tenascin C |
| BLC | FGF basic | VCAM1 |
| BLC | FGF basic | cortisol |
| BLC | myoglobin | resistin |
| BLC | myoglobin | TRAIL R3 |
| BLC | myoglobin | endothilin 1 |
| BLC | myoglobin | NrCAM |
| BLC | myoglobin | Tenascin C |
| BLC | myoglobin | VCAM1 |
| BLC | myoglobin | cortisol |
| BLC | resistin | TRAIL R3 |
| BLC | resistin | endothilin 1 |
| BLC | resistin | NrCAM |
| BLC | resistin | Tenascin C |
| BLC | resistin | VCAM1 |
| BLC | resistin | cortisol |
| BLC | TRAIL R3 | endothelin 1 |

(continued)

| | | |
|---|---|---|
| BLC | TRAIL R3 | NrCAM |
| BLC | TRAIL R3 | Tenascin C |
| BLC | TRAIL R3 | VCAM1 |
| BLC | TRAIL R3 | cortisol |
| BLC | endothilin 1 | NrCAM |
| BLC | endothiln 1 | Tenascin C |
| BLC | endothilin 1 | VCAM1 |
| BLC | endothilin 1 | cortisol |
| BLC | NrCAM | Tenascin C |
| BLC | NrCAM | VCAM1 |
| BLC | NrCAM | cortisol |
| BLC | Tenascin C | VCAM1 |
| BLC | Tenascin C | cortisol |
| BLC | VCAM1 | cortisol |
| CD40 | IGF BP2 | MMP3 |
| CD40 | IGF BP2 | peptide YY |
| CD40 | IGF BP2 | stem cell factor |
| CD40 | IGF BP2 | TNF RII |
| CD40 | IGF BP2 | AXL |
| CD40 | IGF BP2 | Eotaxin 3 |
| CD40 | IGF BP2 | FABP |
| CD40 | IGF BP2 | FGF basic |
| CD40 | IGF BP2 | myoglobin |
| CD40 | IGF BP2 | resistin |
| CD40 | IGF BP2 | TRAIL R3 |
| CD40 | IGF BP2 | endothilin 1 |
| CD40 | IGF BP2 | NrCAM |
| CD40 | IGF BF2 | Tenascin C |
| CD40 | IGF BP2 | VCAM1 |
| CD40 | IGF BP2 | cortisol |
| CD40 | MMP3 | peptide YY |
| CD40 | MMP3 | stem cell factor |
| CD40 | MMP3 | TNF RII |
| CD40 | MMP3 | AXL |
| CD40 | MMP3 | Eotaxin 3 |
| CD40 | MMP3 | FABP |
| CD40 | MMP3 | FGF basic |
| CD40 | MMP3 | myoglobin |
| CD40 | MMP3 | resistin |

(continued)

| CD40 | MMP3 | TRAIL R3 |
|------|------|----------|
| CD40 | MMP3 | endothelin 1 |
| CD40 | MMP3 | NrCAM |
| CD40 | MMP3 | Tenascin C |
| CD40 | MMP3 | VCAM1 |
| CD40 | MMP3 | cortisol |
| CD40 | peptide YY | stem cell factor |
| CD40 | peptide YY | TNF RII |
| CD40 | peptide YY | AXL |
| CD40 | peptide YY | Eotaxin 3 |
| CD40 | peptide YY | FABP |
| CD40 | peptide YY | FGF basic |
| CD40 | peptide YY | myoglobin |
| CD40 | peptide YY | resistin |
| CD40 | peptide YY | TRAIL R3 |
| CD40 | peptide YY | endothilin 1 |
| CD40 | peptide YY | NrCAM |
| CD40 | peptide YY | Tenascin C |
| CD40 | peptide YY | VCAM1 |
| CD40 | peptide YY | cortisol |
| CD40 | stem cell factor | TNF RII |
| CD40 | stem cell factor | AXL |
| CD40 | stem cell factor | Eotaxin 3 |
| CD40 | stem cell factor | FABP |
| CD40 | stem cell factor | FGF basic |
| CD40 | stem cell factor | myoglobin |
| CD40 | stem cell factor | resistin |
| CD40 | stem cell factor | TRAIL R3 |
| CD40 | stem cell factor | endothilin 1 |
| CD40 | stem cell factor | NrCAM |
| CD40 | stem cell factor | Tenascin C |
| CD40 | stem cell factor | VCAM1 |
| CD40 | stem cell factor | cortisol |
| CD40 | TNF RII | AXL |
| CD40 | TNF RII | Eotaxin 3 |
| CD40 | TNF RII | FABP |
| CD40 | TNF RII | FGF basic |
| CD40 | TNF RII | myoglobin |
| CD40 | TNF RII | resistin |

(continued)

| CD40 | TNF RII | TRAIL R3 |
|------|---------|----------|
| CD40 | TNF RII | endothilin 1 |
| CD40 | TNF RII | NrCAM |
| CD40 | TNF RII | Tenascin C |
| CD40 | TNF RII | VCAM1 |
| CD40 | TNF RII | cortisol |
| CD40 | AXL | Eotaxin 3 |
| CD40 | AXL | FABP |
| CD40 | AXL | FGF basic |
| CD40 | AXL | myoglobin |
| CD40 | AXL | resistin |
| CD40 | AXL | TRAIL R3 |
| CD40 | AXL | endothilin 1 |
| CD40 | AXL | NrCAM |
| CD40 | AXL | Tenascin C |
| CD40 | AXL | VCAM1 |
| CD40 | AXL | cortisol |
| CD40 | Eotaxin 3 | FABP |
| CD40 | Eotaxin 3 | FGF basic |
| CD40 | Eotaxin 3 | myoglobin |
| CD40 | Eotaxin 3 | resistin |
| CD40 | Eotaxin 3 | TRAIL R3 |
| CD40 | Eotaxin 3 | endothilin 1 |
| CD40 | Eotaxin 3 | NrCAM |
| CD40 | Eotaxin 3 | Tenascin C |
| CD40 | Eotaxin 3 | VCAM1 |
| CD40 | Eotaxin 3 | cortisol |
| CD40 | FABP | FGF basic |
| CD40 | FABP | myoglobin |
| CD40 | FABP | resistin |
| CD40 | FABP | TRAIL R3 |
| CD40 | FABP | endothilin 1 |
| CD40 | FABP | NrCAM |
| CD40 | FABP | Tenascin C |
| CD40 | FABP | VCAM1 |
| CD40 | FABP | cortisol |
| CD40 | FGF basic | myoglobin |
| CD40 | FGF basic | resistin |
| CD40 | FGF basic | TRAIL R3 |

(continued)

| CD40 | FGF basic | endothilin 1 |
|------|-----------|--------------|
| CD40 | FGF basic | NrCAM |
| CD40 | FGF basic | Tenascin C |
| CD40 | FGF basic | VCAM1 |
| CD40 | FGF basic | cortisol |
| CD40 | myoglobin | resistin |
| CD40 | myoglobin | TRAIL R3 |
| CD40 | myoglobin | endothilin 1 |
| CD40 | myoglobin | NrCAM |
| CD40 | myoglobin | Tenascin C |
| CD40 | myoglobin | VCAM1 |
| CD40 | myoglobin | cortisol |
| CD40 | resistin | TRAIL R3 |
| CD40 | resistin | endothilin 1 |
| CD40 | resistin | NrCAM |
| CD40 | resistin | Tenascin C |
| CD40 | resistin | VCAM1 |
| CD40 | resistin | cortisol |
| CD40 | TRAIL R3 | endothilin 1 |
| CD40 | TRAIL R3 | NrCAM |
| CD40 | TRAIL R3 | Tenascin C |
| CD40 | TRAIL R3 | VCAM1 |
| CD40 | TRAIL R3 | cortisol |
| CD40 | endothilin 1 | NrCAM |
| CD40 | endothilin 1 | Tenascin C |
| CD40 | endothilin 1 | VCAM1 |
| CD40 | endothilin 1 | cortisol |
| CD40 | NrCAM | Tenascin C |
| CD40 | NrCAM | VCAM1 |
| CD40 | NrCAM | cortisol |
| CD40 | Tenascin C | VCAM1 |
| CD40 | Tenascin C | cortisol |
| CD40 | VCAM1 | cortisol |
| IGF BP2 | MMP3 | peptide YY |
| IGF BP2 | MMP3 | stem cell factor |
| IGF BP2 | MMP3 | TNF RII |
| IGF BP2 | MMP3 | AXL |
| IGF BP2 | MMP3 | Eotaxin 3 |
| IGF BP2 | MMP3 | FABP |

(continued)

| IGF BP2 | MMP3 | FGF basic |
|---|---|---|
| IGF BP2 | MMP3 | myoglobin |
| IGF BP2 | MMP3 | resistin |
| IGF BP2 | MMP3 | TRAIL R3 |
| IGF BP2 | MMP3 | endothilin 1 |
| IGF BP2 | MMP3 | NrCAM |
| IGF BP2 | MMP3 | Tenascin C |
| IGF BP2 | MMP3 | VCAM1 |
| IGF BP2 | MMP3 | cortisol |
| IGF BP2 | peptide YY | stem cell factor |
| IGF BP2 | peptide YY | TNF RII |
| IGF BP2 | peptide YY | AXL |
| IGF BP2 | peptide YY | Eotaxin 3 |
| IGF BP2 | peptide YY | FABP |
| IGF BP2 | peptide YY | FGF basic |
| IGF BP2 | peptide YY | myoglobin |
| IGF BP2 | peptide YY | resistin |
| IGF BP2 | peptide YY | TRAIL R3 |
| IGF BP2 | peptide YY | endothilin 1 |
| IGF BP2 | peptide YY | NrCAM |
| IGF BP2 | peptide YY | Tenascin C |
| IGF BP2 | peptide YY | VCAM1 |
| IGF BP2 | peptide YY | cortisol |
| IGF BP2 | stem cell factor | TNF RII |
| IGF BP2 | stem cell factor | AXL |
| IGF BP2 | stem cell factor | Eotaxin 3 |
| IGF BP2 | stem cell factor | FABP |
| IGF BP2 | stem cell factor | FGF basic |
| IGF BP2 | stem cell factor | myoglobin |
| IGF BP2 | stem cell factor | resistin |
| IGF BP2 | stem cell factor | TRAIL R3 |
| IGF BP2 | stem cell factor | endothilin 1 |
| IGF BP2 | stem cell factor | NrCAM |
| IGF BP2 | stem cell factor | Tenascin C |
| IGF BP2 | stem cell factor | VCAM1 |
| IGF BP2 | stem cell factor | cortisol |
| IGF BP2 | TNF RII | AXL |
| IGF BP2 | TNF RII | Eotaxin 3 |
| IGF BP2 | TNF RII | FABP |

(continued)

| IGF BP2 | TNF RII | FGF basic |
|---------|---------|-----------|
| IGF BP2 | TNF RII | myoglobin |
| IGF BP2 | TNF RII | resistin |
| IGF BP2 | TNF RII | TRAIL R3 |
| IGF BP2 | TNF RII | endothilin 1 |
| IGF BP2 | TNF RII | NrCAM |
| IGF BP2 | TNF RII | Tenascin C |
| IGF BP2 | TNF RII | VCAM1 |
| IGF BP2 | TNF RII | cortisol |
| IGF BP2 | AXL | Eotaxin 3 |
| IGF BP2 | AXL | FABP |
| IGF BP2 | AXL | FGF basic |
| IGF BP2 | AXL | myoglobin |
| IGF BP2 | AXL | resistin |
| IGF BP2 | AXL | TRAIL R3 |
| IGF BP2 | AXL | endothilin 1 |
| IGF BP2 | AXL | NrCAM |
| IGF BP2 | AXL | Tenascin C |
| IGF BP2 | AXL | VCAM1 |
| IGF BP2 | AXL | cortisol |
| IGF BP2 | Eotaxin 3 | FABP |
| IGF BP2 | Eotaxin 3 | FGF basic |
| IGF BP2 | Eotaxin 3 | myoglobin |
| IGF BP2 | Eotaxin 3 | resistin |
| IGF BP2 | Eotaxin 3 | TRAIL R3 |
| IGF BP2 | Eotaxin 3 | endothilin 1 |
| IGF BP2 | Eotaxin 3 | NrCAM |
| IGF BP2 | Eotaxin 3 | Tenascin C |
| IGF BP2 | Eotaxin 3 | VCAM1 |
| IGF BP2 | Eotaxin 3 | cortisol |
| IGF BP2 | FABP | FGF basic |
| IGF BP2 | FABP | myoglobin |
| IGF BP2 | FABP | resistin |
| IGF BP2 | FABP | TRAIL R3 |
| IGF BP2 | FABP | endothilin 1 |
| IGF BP2 | FABP | NrCAM |
| IGF BP2 | FABP | Tenascin C |
| IGF BP2 | FABP | VCAM1 |
| IGF BP2 | FABP | cortisol |

(continued)

| IGF BP2 | FGF basic | myoglobin |
|---------|-----------|-----------|
| IGF BP2 | FGF basic | resistin |
| IGF BP2 | FGF basic | TRAIL R3 |
| IGF BP2 | FGF basic | endothilin 1 |
| IGF BP2 | FGF basic | NrCAM |
| IGF BP2 | FGF basic | Tenascin C |
| IGF BP2 | FGF basic | VCAM1 |
| IGF BP2 | FGF basic | cortisol |
| IGF BP2 | myoglobin | resistin |
| IGF BP2 | myoglobin | TRAIL R3 |
| IGF BP2 | myoglobin | endothilin 1 |
| IGF BP2 | myoglobin | NrCAM |
| IGF BP2 | myoglobin | Tenascin C |
| IGF BP2 | myoglobin | VCAM1 |
| IGF BP2 | myoglobin | cortisol |
| IGF BP2 | resistin | TRAIL R3 |
| IGF BP2 | resistin | endothilin 1 |
| IGF BP2 | resistin | NrCAM |
| IGF BP2 | resistin | Tenascin C |
| IGF BP2 | resistin | VCAM1 |
| IGF BP2 | resistin | cortisol |
| IGF BP2 | TRAIL R3 | endothilin 1 |
| IGF BP2 | TRAIL R3 | NrCAM |
| IGF BP2 | TRAIL R3 | Tenascin C |
| IGF BP2 | TRAIL R3 | VCAM1 |
| IGF BP2 | TRAIL R3 | cortisol |
| IGF BP2 | endothilin 1 | NrCAM |
| IGF BP2 | endothilin 1 | Tenascin C |
| IGF BP2 | endothilin 1 | VCAM1 |
| IGF BP2 | endothilin 1 | cortisol |
| IGF BP2 | NrCAM | Tenascin C |
| IGF BP2 | NrCAM | VCAM1 |
| IGF BP2 | NrCAM | cortisol |
| IGF BP2 | Tenascin C | VCAM1 |
| IGF BP2 | Tenascin C | cortisol |
| IGF BP2 | VCAM1 | cortisol |
| MMP3 | peptide YY | stem cell factor |
| MMP3 | peptide YY | TNF RII |
| MMP3 | peptide YY | AXL |

(continued)

| MMP3 | peptide YY | Eotaxin 3 |
|---|---|---|
| MMP3 | peptide YY | FABP |
| MMP3 | peptide YY | FGF basic |
| MMP3 | peptide YY | myoglobin |
| MMP3 | peptide YY | resistin |
| MMP3 | peptide YY | TRAIL R3 |
| MMP3 | peptide YY | endothilin 1 |
| MMP3 | peptide YY | NrCAM |
| MMP3 | peptide YY | Tenascin C |
| MMP3 | peptide YY | VCAM1 |
| MMP3 | peptide YY | cortisol |
| MMP3 | stem cell factor | TNF RII |
| MMP3 | stem cell factor | AXL |
| MMP3 | stem cell factor | Eotaxm 3 |
| MMP3 | stem cell factor | FABP |
| MMP3 | stem cell factor | FGF basic |
| MMP3 | stem cell factor | myoglobin |
| MMP3 | stem cell factor | resistin |
| MMP3 | stem cell factor | TRAIL R3 |
| MMP3 | stem cell factor | endothilin 1 |
| MMP3 | stem cell factor | NrCAM |
| MMP3 | stem cell factor | Tenascin C |
| MMP3 | stem cell factor | VCAM1 |
| MMP3 | stem cell factor | cortisol |
| MMP3 | TNF RII | AXL |
| MMP3 | TNF RII | Eotaxin 3 |
| MMP3 | TNF RII | FABP |
| MMP3 | TNF RII | FGF basic |
| MMP3 | TNF RII | myoglobin |
| MMP3 | TNF RII | resistin |
| MMP3 | TNF RII | TRAIL R3 |
| MMP3 | TNF RII | endothilin 1 |
| MMP3 | TNF RII | NrCAM |
| MMP3 | TNF RII | Tenascin C |
| MMP3 | TNF RII | VCAM1 |
| MMP3 | TNF RII | cortisol |
| MMP3 | AXL | Eotaxin 3 |
| MMP3 | AXL | FABP |
| MMP3 | AXL | FGF basic |

(continued)

| MMP3 | AXL | myoglobin |
|------|-----|-----------|
| MMP3 | AXL | resistin |
| MMP3 | AXL | TRAIL R3 |
| MMP3 | AXL | endothilin 1 |
| MMP3 | AXL | NrCAM |
| MMP3 | AXL | Tenascin C |
| MMP3 | AXL | VCAM1 |
| MMP3 | AXL | cortisol |
| MMP3 | Eotaxin 3 | FABP |
| MMP3 | Eotaxin 3 | FGF basic |
| MMP3 | Eotaxin 3 | myoglobin |
| MMP3 | Eotaxin 3 | resistin |
| MMP3 | Eotaxin 3 | TRAIL R3 |
| MMP3 | Eotaxin 3 | endothilin 1 |
| MMP3 | Eotaxin 3 | NrCAM |
| MMP3 | Eotaxin 3 | Tenascin C |
| MMP3 | Eotaxin 3 | VCAM1 |
| MMP3 | Eotaxin 3 | cortisol |
| MMP3 | FABP | FGF basic |
| MMP3 | FABP | myoglobin |
| MMP3 | FABP | resistin |
| MMP3 | FABP | TRAIL R3 |
| MMP3 | FABP | endothilin 1 |
| MMP3 | FABP | NrCAM |
| MMP3 | FABP | Tenascin C |
| MMP3 | FABP | VCAM1 |
| MMP3 | FABP | cortisol |
| MMP3 | FGF basic | myoglobin |
| MMP3 | FGF basic | resisti n |
| MMP3 | FGF basic | TRAIL R3 |
| MMP3 | FGF basic | endothilin 1 |
| MMP3 | FGF basic | NrCAM |
| MMP3 | FGF basic | Tenascin C |
| MMP3 | FGF basic | VCAM1 |
| MMP3 | FGF basic | cortisol |
| MMP3 | myoglobin | resistin |
| MMP3 | myoglobin | TRAIL R3 |
| MMP3 | myoglobin | endothilin 1 |
| MMP3 | myoglobin | NrCAM |

(continued)

| MMP3 | myoglobin | Tenascin C |
|---|---|---|
| MMP3 | myoglobin | VCAM1 |
| MMP3 | myoglobin | cortisol |
| MMP3 | resistin | TRAIL R3 |
| MMP3 | resistin | endothilin 1 |
| MMP3 | resistin | NrCAM |
| MMP3 | resistin | Tenascin C |
| MMP3 | resistin | VCAM1 |
| MMP3 | resistin | cortisol |
| MMP3 | TRAIL R3 | endothilin 1 |
| MMP3 | TRAIL R3 | NrCAM |
| MMP3 | TRAIL R3 | Tenascin C |
| MMP3 | TRAIL R3 | VCAM1 |
| MMP3 | TRAIL R3 | cortisol |
| MMP3 | endothilin 1 | NrCAM |
| MMP3 | endothilin 1 | Tenascin C |
| MMP3 | endothilin 1 | VCAM1 |
| MMP3 | endothilin 1 | cortisol |
| MMP3 | NrCAM | Tanascin C |
| MMP3 | NrCAM | VCAM1 |
| MMP3 | NrCAM | cortisol |
| MMP3 | Tenascin C | VCAM1 |
| MMP3 | Tenascin C | cortisol |
| MMP3 | VCAM1 | cortisol |
| peptide YY | stem cell factor | TNF RII |
| peptide YY | stem cell factor | AXL |
| peptide YY | stem cell factor | Eotaxin 3 |
| peptide YY | stem cell factor | FABP |
| peptide YY | stem cell factor | FGF basic |
| peptide YY | stem cell factor | myoglobin |
| peptide YY | stem cell factor | resistin |
| peptide YY | stem cell factor | TRAIL R3 |
| peptide YY | stem cell factor | endothilin 1 |
| peptide YY | stem cell factor | NrCAM |
| peptide YY | stem cell factor | Tenascin C |
| peptide YY | stem cell factor | VCAM1 |
| peptide YY | stem cell factor | cortisol |
| peptide YY | TNF RII | AXL |
| peptide YY | TNF RII | Eotaxin 3 |

(continued)

| peptide YY | TNF RII | FABP |
|---|---|---|
| peptide YY | TNF RII | FGF basic |
| peptide YY | TNF RII | myoglobin |
| peptide YY | TNF RII | resistin |
| peptide YY | TNF RII | TRAIL R3 |
| peptide YY | TNF RII | endothilin 1 |
| peptide YY | TNF RII | NrCAM |
| peptide YY | TNF RII | Tenascin C |
| peptide YY | TNF RII | VCAM1 |
| peptide YY | TNF RII | cortisol |
| peptide YY | AXL | Eotaxin 3 |
| peptide YY | AXL | FABP |
| peptide YY | AXL | FGF basic |
| peptide YY | AXL | myoglobin |
| peptide YY | AXL | resistin |
| peptide YY | AXL | TRAIL R3 |
| peptide YY | AXL | endothilin 1 |
| peptide YY | AXL | NrCAM |
| peptide YY | AXL | Tenascin C |
| peptide YY | AXL | VCAM1 |
| peptide YY | AXL | cortisol |
| peptide YY | Eotaxin 3 | FABP |
| peptide YY | Eotaxin 3 | FGF basic |
| peptide YY | Eotaxin 3 | myoglobin |
| peptide YY | Eotaxin 3 | resistin |
| peptide YY | Eotaxin 3 | TRAIL R3 |
| peptide YY | Eotaxm 3 | endothilin 1 |
| peptide YY | Eotaxin 3 | NrCAM |
| peptide YY | Eotaxin 3 | Tenascin C |
| peptide YY | Eotaxin 3 | VCAM1 |
| peptide YY | Eotaxin 3 | cortisol |
| peptide YY | FABP | FGF basic |
| peptide YY | FABP | myoglobin |
| peptide YY | FABP | resistin |
| peptide YY | FABP | TRAIL R3 |
| peptide YY | FABP | endothilin 1 |
| peptide YY | FABP | NrCAM |
| peptide YY | FABP | Tenascin C |
| peptide YY | FABP | VCAM1 |

(continued)

| peptide YY | FABP | cortisol |
|---|---|---|
| peptide YY | FGF basic | myoglobin |
| peptide YY | FGF basic | resistin |
| peptide YY | FGF basic | TRAIL R3 |
| peptide YY | FGF basic | endothilin 1 |
| peptide YY | FGF basic | NrCAM |
| peptide YY | FGF basic | Tenascin C |
| peptide YY | FGF basic | VCAM1 |
| peptide YY | FGF basic | cortisol |
| peptide YY | myoglobin | resistin |
| peptide YY | myoglobin | TRAIL R3 |
| peptide YY | myoglobin | endothilin 1 |
| peptide YY | myoglobin | NrCAM |
| peptide YY | myoglobin | Tenascin C |
| peptide YY | myoglobin | VCAM1 |
| peptide YY | myoglobin | cortisol |
| peptide YY | resistin | TRAIL R3 |
| peptide YY | resistin | endothilin 1 |
| peptide YY | resistin | NrCAM |
| peptide YY | resistin | Tenascin C |
| peptide YY | resistin | VCAM1 |
| peptide YY | resistin | cortisol |
| peptide YY | TRAIL R3 | endothilin 1 |
| peptide YY | TRAIL R3 | NrCAM |
| peptide YY | TRAIL R3 | Tenascin C |
| peptide YY | TRAIL R3 | VCAM1 |
| peptide YY | TRAIL R3 | cortisol |
| peptide YY | endothilin 1 | NrCAM |
| peptide YY | endothilin 1 | Tenascin C |
| peptide YY | endothilin 1 | VCAM1 |
| peptide YY | endothilin 1 | cortisol |
| peptide YY | NrCAM | Tenascin C |
| peptide YY | NrCAM | VCAM1 |
| peptide YY | NrCAM | cortisol |
| peptide YY | Tenascin C | VCAM1 |
| peptide YY | Tenascin C | cortisol |
| peptide YY | VCAM1 | cortisol |
| stem cell factor | TNF RII | AXL |
| stem cell factor | TNF RII | Eotaxin 3 |

(continued)

| stem cell factor | TNF RII | FABP |
|---|---|---|
| stem cell factor | TNF RII | FGF basic |
| stem cell factor | TNF RII | myoglobin |
| stem cell factor | TNF RII | resistin |
| stem cell factor | TNF RII | TRAIL R3 |
| stem cell factor | TNF RII | endothilin 1 |
| stem cell factor | TNF RII | NrCAM |
| stem cell factor | TNF RII | Tenascin C |
| stem cell factor | TNF RII | VCAM1 |
| stem cell factor | TNF RII | cortisol |
| stem cell factor | AXL | Eotaxin 3 |
| stem cell factor | AXL | FABP |
| stem cell factor | AXL | FGF basic |
| stem cell factor | AXL | myoglobin |
| stem cell factor | AXL | resistin |
| stem cell factor | AXL | TRAIL R3 |
| stem cell factor | AXL | endothilin 1 |
| stem cell factor | AXL | NrCAM |
| stem cell factor | AXL | Tenascin C |
| stem cell factor | AXL | VCAM1 |
| stem cell factor | AXL | cortisol |
| stem cell factor | Eotaxin 3 | FABP |
| stem cell factor | Eotaxin 3 | FGF basic |
| stem cell factor | Eotaxin 3 | myoglobin |
| stem cell factor | Eotaxin 3 | resistin |
| stem cell factor | Eotaxin 3 | TRAIL R3 |
| stem cell factor | Eotaxin 3 | endothilin 1 |
| stem cell factor | Eotaxin 3 | NrCAM |
| stem cell factor | Eotaxin 3 | Tenascin C |
| stem cell factor | Eotaxin 3 | VCAM1 |
| stem cell factor | Eotaxin 3 | cortisol |
| stem cell factor | FABP | FGF basic |
| stem cell factor | FABP | myoglobin |
| stem cell factor | FABP | resistin |
| stem cell factor | FABP | TRAIL R3 |
| stem cell factor | FABP | endothilin 1 |
| stem cell factor | FABP | NrCAM |
| stem cell factor | FABP | Tenascin C |
| stem cell factor | FABP | VCAM1 |

(continued)

| stem cell factor | FABP | cortisol |
|---|---|---|
| stem cell factor | FGF basic | myoglobin |
| stem cell factor | FGF basic | resistin |
| stem cell factor | FGF basic | TRAIL R3 |
| stem cell factor | FGF basic | endothilin 1 |
| stem cell factor | FGF basic | NrCAM |
| stem cell factor | FGF basic | Tenascin C |
| stem cell factor | FGF basic | VCAM1 |
| stem cell factor | FGF basic | cortisol |
| stem cell factor | myoglobin | resistin |
| stem cell factor | myoglobin | TRAIL R3 |
| stem cell factor | myoglobin | endothilin 1 |
| stem cell factor | myoglobin | NrCAM |
| stem cell factor | myoglobin | Tenascin C |
| stem cell factor | myoglobin | VCAM1 |
| stem cell factor | myoglobin | cortisol |
| stem cell factor | resistin | TRAIL R3 |
| stem cell factor | resistin | endothilin 1 |
| stem cell factor | resistin | NrCAM |
| stem cell factor | resistin | Tenascin C |
| stem cell factor | resistin | VCAM1 |
| stem cell factor | resistin | cortisol |
| stem cell factor | TRAIL R3 | endothilin 1 |
| stem cell factor | TRAIL R3 | NrCAM |
| stem cell factor | TRAIL R3 | Tenascin C |
| stem cell factor | TRAIL R3 | VCAM1 |
| stem cell factor | TRAIL R3 | cortisol |
| stem cell factor | endothilin 1 | NrCAM |
| stem cell factor | endothilin 1 | Tenascin C |
| stem cell factor | endothilin 1 | VCAM1 |
| stem cell factor | endothilin 1 | cortisol |
| stem cell factor | NrCAM | Tenascin C |
| stem cell factor | NrCAM | VCAM1 |
| stem cell factor | NrCAM | cortisol |
| stem cell factor | Tenascin C | VCAM1 |
| stem cell factor | Tenascin C | cortisol |
| stem cell factor | VCAM1 | cortisol |
| TNF RII | AXL | Eotaxin 3 |
| TNF RII | AXL | FABP |

(continued)

| TNF RII | AXL | FGF basic |
|---------|-----------|-------------|
| TNF RII | AXL | myoglobin |
| TNF RII | AXL | resistin |
| TNF RII | AXL | TRAIL R3 |
| TNF RII | AXL | endothilin 1 |
| TNF RII | AXL | NrCAM |
| TNF RII | AXL | Tenascin C |
| TNF RII | AXL | VCAM1 |
| TNF RII | AXL | cortisol |
| TNF RII | Eotaxin 3 | FABP |
| TNF RII | Eotaxin 3 | FGF basic |
| TNF RII | Eotaxin 3 | myoglobin |
| TNF RII | Eotaxin 3 | resistin |
| TNF RII | Eotaxin 3 | TRAIL R3 |
| TNF RII | Eotaxin 3 | endothilin 1 |
| TNF RII | Eotaxin 3 | NrCAM |
| TNF RII | Eotaxin 3 | Tenascin C |
| TNF RII | Eotaxin 3 | VCAM1 |
| TNF RII | Eotaxin 3 | cortisol |
| TNF RII | FABP | FGF basic |
| TNF RII | FABP | myoglobin |
| TNF RII | FABP | resistin |
| TNF RII | FABP | TRAIL R3 |
| TNF RII | FABP | endothilin 1 |
| TNF RII | FABP | NrCAM |
| TNF RII | FABP | Tenascin C |
| TNF RII | FABP | VCAM1 |
| TNF RII | FABP | cortisol |
| TNF RII | FGF basic | myoglobin |
| TNF RII | FGF basic | resistin |
| TNF RII | FGF basic | TRAIL R3 |
| TNF RII | FGF basic | endothilin 1 |
| TNF RII | FGF basic | NrCAM |
| TNF RII | FGF basic | Tenascin C |
| TNF RII | FGF basic | VCAM1 |
| TNF RII | FGF basic | cortisol |
| TNF RII | myoglobin | resistin |
| TNF RII | myoglobin | TRAIL R3 |
| TNF RII | myoglobin | endothilin 1 |

(continued)

| TNF RII | myoglobin | NrCAM |
|---------|-----------|-------|
| TNF RII | myoglobin | Tenascin C |
| TNF RII | myoglobin | VCAM1 |
| TNF RII | myoglobin | cortisol |
| TNF RII | resistin | TRAIL R3 |
| TNF RII | resistin | endothilin 1 |
| TNF RII | resistin | NrCAM |
| TNF RII | resistin | Tenascin C |
| TNF RII | resistin | VCAM1 |
| TNF RII | resistin | cortisol |
| TNF RII | TRAIL R3 | endothilin 1 |
| TNF RII | TRAIL R3 | NrCAM |
| TNF RII | TRAIL R3 | Tenascin C |
| TNF RII | TRAIL R3 | VCAM1 |
| TNF RII | TRAIL R3 | cortisol |
| TNF RII | endothilin 1 | NrCAM |
| TNF RII | endothilin 1 | Tenascin C |
| TNF RII | endothilin 1 | VCAM1 |
| TNF RII | endothilin 1 | cortisol |
| TNF RII | NrCAM | Tenascin C |
| TNF RII | NrCAM | VCAM1 |
| TNF RII | NrCAM | cortisol |
| TNF RII | Tenascin C | VCAM1 |
| TNF RII | Tenascin C | cortisol |
| TNF RII | VCAM1 | cortisol |
| AXL | Eotaxin 3 | FABP |
| AXL | Eotaxin 3 | FGF basic |
| AXL | Eotaxin 3 | myoglobin |
| AXL | Eotaxin 3 | resistin |
| AXL | Eotaxin 3 | TRAIL R3 |
| AXL | Eotaxin 3 | endothilin 1 |
| AXL | Eotaxin 3 | NrCAM |
| AXL | Eotaxm 3 | Tenascin C |
| AXL | Eotaxin 3 | VCAM1 |
| AXL | Eotaxin 3 | cortisol |
| AXL | FABP | FGF basic |
| AXL | FABP | myoglobin |
| AXL | FABP | resistin |
| AXL | FABP | TRAIL R3 |

(continued)

| AXL | FABP | endothilin 1 |
|-----|------|--------------|
| AXL | FABP | NrCAM |
| AXL | FABP | Tenascin C |
| AXL | FABP | VCAM1 |
| AXL | FABP | cortisol |
| AXL | FGF basic | myoglobin |
| AXL | FGF basic | resistin |
| AXL | FGF basic | TRAIL R3 |
| AXL | FGF basic | endothilin 1 |
| AXL | FGF basic | NrCAM |
| AXL | FGF basic | Tenascin C |
| AXL | FGF basic | VCAM1 |
| AXL | FGF basic | cortisol |
| AXL | myoglobin | resistin n |
| AXL | myoglobin | TRAIL R3 |
| AXL | myoglobin | endothilin 1 |
| AXL | myoglobin | NrCAM |
| AXL | myoglobin | Tenascin C |
| AXL | myoglobin | VCAM1 |
| AXL | myoglobin | cortisol |
| AXL | resistin | TRAIL R3 |
| AXL | resistin | endothilin 1 |
| AXL | resistin | NrCAM |
| AXL | resistin | Tenascin C |
| AXL | resistin | VCAM1 |
| AXL | resistin | cortisol |
| AXL | TRAIL R3 | endothilin 1 |
| AXL | TRAIL R3 | NrCAM |
| AXL | TRAIL R3 | Tenascin C |
| AXL | TRAIL R3 | VCAM1 |
| AXL | TRAIL R3 | cortisol |
| AXL | endothilin 1 | NrCAM |
| AXL | endothilin 1 | Tenascin C |
| AXL | endothilin 1 | VCAM1 |
| AXL | endothilin 1 | cortisol |
| AXL | NrCAM | Tenascin C |
| AXL | NrCAM | VCAM1 |
| AXL | NrCAM | cortisol |
| AXL | Tenascin C | VCAM1 |

(continued)

| AXL | Tenascin C | cortisol |
|---|---|---|
| AXL | VCAM1 | cortisol |
| Eotaxin 3 | FABP | FGF basic |
| Eotaxin 3 | FABP | myoglobin |
| Eotaxin 3 | FABP | resistin |
| Eotaxin 3 | FABP | TRAIL R3 |
| Eotaxin 3 | FABP | endothilin 1 |
| Eotaxin 3 | FABP | NrCAM |
| Eotaxin 3 | FABP | Tenascin C |
| Eotaxin 3 | FABP | VCAM1 |
| Eotaxin 3 | FABP | cortisol |
| Eotaxin 3 | FGF basic | myoglobin |
| Eotaxin 3 | FGF basic | resistin |
| Eotaxin 3 | FGF basic | TRAIL R3 |
| Eotaxin 3 | FGF basic | endothilin 1 |
| Eotaxin 3 | FGF basic | NrCAM |
| Eotaxin 3 | FGF basic | Tenascin C |
| Eotaxin 3 | FGF basic | VCAM1 |
| Eotaxin 3 | FGF basic | cortisol |
| Eotaxin 3 | myoglobin | resistin |
| Eotaxin 3 | myoglobin | TRAIL R3 |
| Eotaxin 3 | myoglobin | endothilin 1 |
| Eotaxin 3 | myoglobin | NrCAM |
| Eotaxin 3 | myoglobin | Tenascin C |
| Eotaxin 3 | myoglobin | VCAM1 |
| Eotaxin 3 | myoglobin | cortisol |
| Eotaxin 3 | resistin | TRAIL R3 |
| Eotaxin 3 | resistin | endothilin 1 |
| Eotaxin 3 | resistin | NrCAM |
| Eotaxin 3 | resistin | Tenascin C |
| Eotaxin 3 | resistin | VCAM1 |
| Eotaxin 3 | resistin | cortisol |
| Eotaxin 3 | TRAIL R3 | endothilin 1 |
| Eotaxin 3 | TRAIL R3 | NrCAM |
| Eotaxin 3 | TRAIL R3 | Tenascin C |
| Eotaxin 3 | TRAIL R3 | VCAM1 |
| Eotaxin 3 | TRAIL R3 | cortisol |
| Eotaxin 3 | endothilin 1 | NrCAM |
| Eotaxin 3 | endothilin 1 | Tenascin C |

(continued)

| Eotaxin 3 | endothilin 1 | VCAM1 |
|-----------|--------------|-------|
| Eotaxin 3 | endothilin 1 | cortisol |
| Eotaxin 3 | NrCAM | Tenascin C |
| Eotaxin 3 | NrCAM | VCAM1 |
| Eotaxin 3 | NrCAM | cortisol |
| Eotaxin 3 | Tenascin C | VCAM1 |
| Eotaxin 3 | Tenascin C | cortisol |
| Eotaxin 3 | VCAM1 | cortisol |
| FABP | FGF basic | myoglobin |
| FABP | FGF basic | resistin |
| FABP | FGF basic | TRAIL R3 |
| FABP | FGF basic | endothilin 1 |
| FABP | FGF basic | NrCAM |
| FABP | FGF basic | Tenascin C |
| FABP | FGF basic | VCAM1 |
| FABP | FGF basic | cortisol |
| FABP | myoglobin | resistin |
| FABP | myoglobin | TRAIL R3 |
| FABP | myoglobin | endothilin 1 |
| FABP | myoglobin | NrCAM |
| FABP | myoglobin | Tenascin C |
| FABP | myoglobin | VCAM1 |
| FABP | myoglobin | cortisol |
| FABP | resistin | TRAIL R3 |
| FABP | resistin | endothilin 1 |
| FABP | resistin | NrCAM |
| FABP | resistin | Tenascin C |
| FABP | resistin | VCAM1 |
| FABP | resistin | cortisol |
| FABP | TRAIL R3 | endothilin 1 |
| FABP | TRAIL R3 | NrCAM |
| FABP | TRAIL R3 | Tenascin C |
| FABP | TRAIL R3 | VCAM1 |
| FABP | TRAIL R3 | cortisol |
| FABP | endothilin 1 | NrCAM |
| FABP | endothilin 1 | Tenascin C |
| FABP | endothilin 1 | VCAM1 |
| FABP | endothilin 1 | cortisol |
| FABP | NrCAM | Tenascin C |

(continued)

| FABP | NrCAM | VCAM1 |
|---|---|---|
| FABP | NrCAM | cortisol |
| FABP | Tenascin C | VCAM1 |
| FABP | Tenascin C | cortisol |
| FABP | VCAM1 | cortisol |
| FGF basic | myoglobin | resistin |
| FGF basic | myoglobin | TRAIL R3 |
| FGF basic | myoglobin | endothelin 1 |
| FGF basic | Myoglobin | NrCAM |
| FGF basic | myoglobin | Tenascin C |
| FGF basic | myoglobin | VCAM1 |
| FGF basic | myoglobin | cortisol |
| FGF basic | resistin | TRAIL R3 |
| FGF basic | resistin | endothilin 1 |
| FGF basic | resistin | NrCAM |
| FGF basic | resistin | Tenascin C |
| FGF basic | resistin | VCAM1 |
| FGF basic | resistin | cortisol |
| FGF basic | TRAIL R3 | endothilin 1 |
| FGF basic | TRAIL R3 | NrCAM |
| FGF basic | TRAIL R3 | Tenascin C |
| FGF basic | TRAIL R3 | VCAM1 |
| FGF basic | TRAIL R3 | cortisol |
| FGF basic | endothilin 1 | NrCAM |
| FGF basic | endothilin 1 | Tenascin C |
| FGF basic | endothilin 1 | VCAM1 |
| FGF basic | endothilin 1 | cortisol |
| FGF basic | NrCAM | Tenascin C |
| FGF basic | NrCAM | VCAM1 |
| FGF basic | NrCAM | cortisol |
| FGF basic | Tenascin C | VCAM1 |
| FGF basic | Tenascin C | cortisol |
| FGF basic | VCAM1 | cortisol |
| myoglobin | resistin | TRAIL R3 |
| myoglobin | resistin | endothilin 1 |
| myoglobin | resistin | NrCAM |
| myoglobin | resistin | Tenascin C |
| myoglobin | resistin | VCAM1 |
| myoglobin | resistin | cortisol |

(continued)

| | | |
|---|---|---|
| myoglobin | TRAIL R3 | endothilin 1 |
| myoglobin | TRAIL R3 | NrCAM |
| myoglobin | TRAIL R3 | Tenascin C |
| myoglobin | TRAIL R3 | VCAM1 |
| myoglobin | TRAIL R3 | cortisol |
| myoglobin | endothilin 1 | NrCAM |
| myoglobin | endothilin 1 | Tenascin C |
| myoglobin | endothilin 1 | VCAM1 |
| myoglobin | endothilin 1 | cortisol |
| myoglobin | NrCAM | Tenascin C |
| myoglobin | NrCAM | VCAM1 |
| myoglobin | NrCAM | cortisol |
| myoglobin | Tenascin C | VCAM1 |
| myoglobin | Tenascin C | cortisol |
| myoglobin | VCAM1 | cortisol |
| resistin | TRAIL R3 | endothelin 1 |
| resistin | TRAIL R3 | NrCAM |
| resistin | TRAIL R3 | Tenascin C |
| resistin | TRAIL R3 | VCAM1 |
| resistin | TRAIL R3 | cortisol |
| resistin | endothilin 1 | NrCAM |
| resistin | endothilin 1 | Tenascin C |
| resistin | endothilin 1 | VCAM1 |
| resistin | endothilin 1 | cortisol |
| resistin | NrCAM | Tenascin C |
| resistin | NrCAM | VCAM1 |
| resistin | NrCAM | cortisol |
| resistin | Tenascin C | VCAM1 |
| resistin | Tenascin C | cortisol |
| resistin | VCAM1 | cortisol |
| TRAIL R3 | endothilin 1 | NrCAM |
| TRAIL R3 | endothilin 1 | Tenascin C |
| TRAIL R3 | endothilm 1 | VCAM1 |
| TRAIL R3 | endothilin 1 | cortisol |
| TRAIL R3 | NrCAM | Tenascin C |
| TRAIL R3 | NrCAM | VCAM1 |
| TRAIL R3 | NrCAM | cortisol |
| TRAIL R3 | Tenascin C | VCAM1 |
| TRAIL R3 | Tenascin C | cortisol |

(continued)

| TRAIL R3 | VCAM1 | cortisol |
|---|---|---|
| endothilin 1 | NrCAM | Tenascin C |
| andothilin 1 | NrCAM | VCAM1 |
| endothilin 1 | NrCAM | cortisol |
| endothilin 1 | Tenascin C | VCAM1 |
| endothilin 1 | Tenascin C | cortisol |
| endothilin 1 | VCAM1 | cortisol |
| NrCAM | Tenascin C | VCAM1 |
| NrCAM | Tenascin C | cortisol |
| NrCAM | VCAM1 | cortisol |
| Tenascin C | VCAM1 | cortisol |

In one exemplary embodiment, the combination of sample analytes may include Beta 2 Microglobulin, BLC, CD40, IGF BP2, MMP3, Peptide YY, Stem Cell Factor, TNF RII, and VEGF. In another exemplary embodiment, the combination of sample analytes may include AXL, Beta 2 Microglobulin, CD40, Eotaxin 3, FABP, FGF basic, IGF BP2, MMP3, Myoglobin, Resistin, Stem Cell Factor, TNF RII, TRAIL R3, and VEGF. In yet another exemplary embodiment, the combination of sample analytes may include AXL, Beta 2 Microglobulin, BLC, CD40, Endothelin 1, Eotaxin 3, FABP, FGF basic, IGF BP2, MMP3, Myoglobin, NrCAM, Peptide YY, Resistin, Stem Cell Factor, Tenascin C, TNF RII, TRAIL R3, VCAM 1, and VEGF. In still yet another exemplary embodiment, the combination of sample analytes may include Beta 2 Microglobulin, CD40, Cortisol, FGF.basic, Stem Cell Factor, TNF RII, and VEGF.

**III. Test Sample**

The method for diagnosing, monitoring, or determining a renal disorder involves determining the presence of sample analytes in a test sample. A test sample, as defined herein, is an amount of bodily fluid taken from a mammal. Non-limiting examples of bodily fluids include urine, blood, plasma, serum, saliva, semen, perspiration, tears, mucus, and tissue lysates. In an exemplary embodiment, the bodily fluid contained in the test sample is urine, plasma, or serum.

**(a) Mammals**

A mammal, as defined herein, is any organism that is a member of the class Mammalia. Non-limiting examples of mammals appropriate for the various embodiments may include humans, apes, monkeys, rats, mice, dogs, cats, pigs, and livestock including cattle and oxen. In an exemplary embodiment, the mammal is a human.

**(b) Devices and Methods of Taking Bodily Fluids from Mammals**

The bodily fluids of the test sample may be taken from the mammal using any known device or method so long as the analytes to be measured by the multiplexed assay are not rendered undetectable by the multiplexed assay. Non-limiting examples of devices or methods suitable for taking bodily fluid from a mammal include urine sample cups, urethral catheters, swabs, hypodermic needles, thin needle biopsies, hollow needle biopsies, punch biopsies, metabolic cages, and aspiration.

In order to adjust the expected concentrations of the sample analytes in the test sample to fall within the dynamic range of the multiplexed assay, the test sample may be diluted to reduce the concentration of the sample analytes prior to analysis. The degree of dilution may depend on a variety of factors including but not limited to the type of multiplexed assay used to measure the analytes, the reagents utilized in the multiplexed assay, and the type of bodily fluid contained in the test sample. In one embodiment, the test sample is diluted by adding a volume of diluent ranging from about ½ of the original test sample volume to about 50,000 times the original test sample volume.

In one exemplary embodiment, if the test sample is human urine and the multiplexed assay is an antibody-based capture-sandwich assay, the test sample is diluted by adding a volume of diluent that is about 100 times the original test sample volume prior to analysis. In another exemplary embodiment, if the test sample is human serum and the multiplexed assay is an antibody-based capture-sandwich assay, the test sample is diluted by adding a volume of diluent that is about 5 times the original test sample volume prior to analysis. In yet another exemplary embodiment, if the test sample is human plasma and the multiplexed assay is an antibody-based capture-sandwich assay, the test sample is diluted by adding a volume of diluent that is about 2,000 times the original test sample volume prior to analysis.

The diluent may be any fluid that does not interfere with the function of the multiplexed assay used to measure the concentration of the analytes in the test sample. Non-limiting examples of suitable diluents include deionized water, distilled water, saline solution, Ringer's solution, phosphate buffered saline solution, TRIS-buffered saline solution, standard saline citrate, and HEPES-buffered saline.

**IV. Multiplexed Assay Device**

In one embodiment, the concentration of a combination of sample analytes is measured using a multiplexed assay device capable of measuring the concentrations of up to sixteen of the biomarker analytes. A multiplexed assay device, as defined herein, is an assay capable of simultaneously determining the concentration of three or more different sample analytes using a single device and/or method. Any known method of measuring the concentration of the biomarker analytes may be used for the multiplexed assay device. Non-limiting examples of measurement methods suitable for the multiplexed assay device may include electrophoresis, mass spectrometry, protein microarrays, surface plasmon resonance and immunoassays including but not limited to western blot, immunohistochemical staining, enzyme-linked immunosorbent assay (ELISA) methods, and particle-based capture-sandwich immunoassays.

*(a) Multiplexed Immunoassay Device*

In one embodiment, the concentrations of the analytes in the test sample are measured using a multiplexed immunoassay device that utilizes capture antibodies marked with indicators to determine the concentration of the sample analytes.

**(I) capture antibodies**

In the same embodiment, the multiplexed immunoassay device includes three or more capture antibodies. Capture antibodies, as defined herein, are antibodies in which the antigenic determinant is one of the biomarker analytes. Each of the at least three capture antibodies has a unique antigenic determinant that is one of the biomarker analytes. When contacted with the test sample, the capture antibodies form antigen-antibody complexes in which the analytes serve as antigens.

The term "antibody," as used herein, encompasses a monoclonal ab, an antibody fragment, a chimeric antibody, and a single-chain antibody.

In some embodiments, the capture antibodies may be attached to a substrate in order to immobilize any analytes captured by the capture antibodies. Non-limiting examples of suitable substrates include paper, cellulose, glass, or plastic strips, beads, or surfaces, such as the inner surface of the well of a microtitration tray. Suitable beads may include polystyrene or latex microspheres.

**(ii) indicators**

In one embodiment of the multiplexed immunoassay device, an indicator is attached to each of the three or more capture antibodies. The indicator, as defined herein, is any compound that registers a measurable change to indicate the presence of one of the sample analytes when bound to one of the capture antibodies. Non-limiting examples of indicators include visual indicators and electrochemical indicators.

Visual indicators, as defined herein, are compounds that register a change by reflecting a limited subset of the wavelengths of light illuminating the indicator, by fluorescing light after being illuminated, or by emitting light via chemiluminescence. The change registered by visual indicators may be in the visible light spectrum, in the infrared spectrum, or in the ultraviolet spectrum. Non-limiting examples of visual indicators suitable for the multiplexed immunoassay device include nanoparticulate gold, organic particles such as polyurethane or latex microspheres loaded with dye compounds, carbon black, fluorophores, phycoerythrin, radioactive isotopes, nanoparticles, quantum dots, and enzymes such as horseradish peroxidase or alkaline phosphatase that react with a chemical substrate to form a colored or chemiluminescent product.

Electrochemical indicators, as defined herein, are compounds that register a change by altering an electrical property. The changes registered by electrochemical indicators may be an alteration in conductivity, resistance, capacitance, current conducted in response to an applied voltage, or voltage required to achieve a desired current. Non-limiting examples of electrochemical indicators include redox species such as ascorbate (vitamin C), vitamin E, glutathione, polyphenols, catechols, quercetin, phytoestrogens, penicillin, carbazole, murranes, phenols, carbonyls, benzoates, and trace metal ions such as nickel, copper, cadmium, iron and mercury.

In this same embodiment, the test sample containing a combination of three or more sample analytes is contacted with the capture antibodies and allowed to form antigen-antibody complexes in which the sample analytes serve as the antigens. After removing any uncomplexed capture antibodies, the concentrations of the three or more analytes are determined by measuring the change registered by the indicators attached to the capture antibodies.

In one exemplary embodiment, the indicators are polyurethane or latex microspheres loaded with dye compounds and phycoerythrin.

*(b) Multiplexed Sandwich Immunoassay Device*

In another embodiment, the multiplexed immunoassay device has a sandwich assay format. In this embodiment, the multiplexed sandwich immunoassay device includes three or more capture antibodies as previously described. However, in this embodiment, each of the capture antibodies is attached to a capture agent that includes an antigenic moiety. The antigenic moiety serves as the antigenic determinant of a detection antibody, also included in the multiplexed immunoassay device of this embodiment. In addition, an indicator is attached to the detection antibody.

In this same embodiment, the test sample is contacted with the capture antibodies and allowed to form antigen-antibody complexes in which the sample analytes serve as antigens. The detection antibodies are then contacted with the test sample and allowed to form antigen-antibody complexes in which the capture agent serves as the antigen for the detection antibody. After removing any uncomplexed detection antibodies the concentration of the analytes are determined by measuring the changes registered by the indicators attached to the detection antibodies.

*(c) Multiplexing Approaches*

In the various embodiments of the multiplexed immunoassay devices, the concentrations of each of the sample analytes may be determined using any approach known in the art. In one embodiment, a single indicator compound is attached to each of the three or more antibodies. In addition, each of the capture antibodies having one of the sample analytes as an antigenic determinant is physically separated into a distinct region so that the concentration of each of the sample analytes may be determined by measuring the changes registered by the indicators in each physically separate region corresponding to each of the sample analytes.

In another embodiment, each antibody having one of the sample analytes as an antigenic determinant is marked with a unique indicator. In this manner, a unique indicator is attached to each antibody having a single sample analyte as its antigenic determinant. In this embodiment, all antibodies may occupy the same physical space. The concentration of each sample analyte is determined by measuring the change registered by the unique indicator attached to the antibody having the sample analyte as an antigenic determinant.

*(d) Microsphere-Based Capture-Sandwich immunoassay Device*

In an exemplary embodiment, the multiplexed immunoassay device is a microsphere-based capture-sandwich immunoassay device. In this embodiment, the device includes a mixture of three or more capture-antibody microspheres, in which each capture-antibody microsphere corresponds to one of the biomarker analytes. Each capture-antibody microsphere includes a plurality of capture antibodies attached to the outer surface of the microsphere. In this same embodiment, the antigenic determinant of all of the capture antibodies attached to one microsphere is the same biomarker analyte.

In this embodiment of the device, the microsphere is a small polystyrene or latex sphere that is loaded with an indicator that is a dye compound. The microsphere may be between about 3 $\mu$m and about 5 $\mu$m in diameter. Each capture-antibody microsphere corresponding to one of the biomarker analytes is loaded with the same indicator. In this manner, each capture-antibody microsphere corresponding to a biomarker analyte is uniquely color-coded.

In this same exemplary embodiment, the multiplexed immunoassay device further includes three or more biotinylated detection antibodies in which the antigenic determinant of each biotinylated detection antibody is one of the biomarker analytes. The device further includes a plurality of streptaviden proteins complexed with a reporter compound. A reporter compound, as defined herein, is an indicator selected to register a change that is distinguishable from the indicators used to mark the capture-antibody microspheres.

The concentrations of the sample analytes may be determined by contacting the test sample with a mixture of capture-antigen microspheres corresponding to each sample analyte to be measured. The sample analytes are allowed to form antigen-antibody complexes in which a sample analyte serves as an antigen and a capture antibody attached to the microsphere serves as an antibody. In this manner, the sample analytes are immobilized onto the capture-antigen microspheres. The biotinylated detection antibodies are then added to the test sample and allowed to form antigen-antibody complexes in which the analyte serves as the antigen and the biotinylated detection antibody serves as the antibody. The streptaviden-reporter complex is then added to the test sample and allowed to bind to the biotin moieties of the biotinylated detection antibodies. The antigen-capture microspheres may then be rinsed and filtered.

in this embodiment, the concentration of each analyte is determined by first measuring the change registered by the indicator compound embedded in the capture-antigen microsphere in order to identify the particular analyte. For each microsphere corresponding to one of the biomarker analytes, the quantity of analyte immobilized on the microsphere is determined by measuring the change registered by the reporter compound attached to the microsphere.

For example, the indicator embedded in the microspheres associated with one sample analyte may register an emission of orange light, and the reporter may register an emission of green light. In this example, a detector device may measure the intensity of orange light and green light separately. The measured intensity of the green light would determine the concentration of the analyte captured on the microsphere, and the intensity of the orange light would determine the specific analyte captured on the microsphere.

Any sensor device may be used to detect the changes registered by the indicators embedded in the microspheres and the changes registered by the reporter compound, so long as the sensor device is sufficiently sensitive to the changes registered by both indicator and reporter compound. Non-limiting examples of suitable sensor devices include spectrophotometers, photosensors, colorimeters, cyclic coulometry devices, and flow cytometers. In an exemplary embodiment, the sensor device is a flow cytometer.

**V. Method for Diagnosing, Monitoring, or Determining a Renal Disorder**

In one embodiment, a method is provided for diagnosing, monitoring, or determining kidney transplant rejection or an associated disorder that includes providing a test sample, determining the concentration of a combination of three or more sample analytes, comparing the measured concentrations of the combination of sample analytes to the entries of a dataset, and identifying kidney transplant rejection or an associated disorder based on the comparison between the concentrations of the sample analytes and the minimum diagnostic concentrations contained within each entry of the dataset.

*(a) Diagnostic Dataset*

In an embodiment, the concentrations of the sample analytes are compared to the entries of a dataset. In this embodiment, each entry of the dataset includes a combination of three or more minimum diagnostic concentrations indicative of a particular renal disorder. A minimum diagnostic concentration, as defined herein, is the concentration of an analyte that defines the limit between the concentration range corresponding to normal, healthy renal function and the concentration reflective of a particular renal disorder. In one embodiment, each minimum diagnostic concentration is the maximum concentration of the range of analyte concentrations for a healthy, normal individual. The minimum diagnostic concentration of an analyte depends on a number of factors including but not limited to the particular analyte and the type of bodily fluid contained in the test sample. As an illustrative example, Table 1 lists the expected normal ranges of the biomarker analytes in human plasma, serum, and urine.

**TABLE 1: Normal Concentration Ranges In Human Plasma, Serum, and Urine Samples**

| Analyte | Units | Plasma | | Sera | | Urine | |
|---|---|---|---|---|---|---|---|
| | | low | high | low | high | low | high |
| Calbindin | ng/ml | - | < 5.0 | - | < 2.6 | 4.2 | 233 |
| Clusterin | μg/ml | 86 | 134 | 37 | 152 | - | < 0.089 |
| CTGF | ng/ml | 2.8 | 7.5 | - | < 8.2 | - | < 0.90 |
| GST-alpha | ng/ml | 6.7 | 62 | 1.2 | 52 | - | < 26 |
| KIM-1 | ng/ml | 0.053 | 0.57 | - | < 0.35 | 0.023 | 0.67 |
| VEGF | pg/ml | 222 | 855 | 219 | 1630 | 69 | 517 |
| B2M | μg/ml | 0.68 | 2.2 | 1.00 | 2.6 | | < 0.17 |
| Cyst C | ng/ml | 608 | 1170 | 476 | 1250 | 3.9 | 79 |
| NGAL | ng/ml | 89 | 375 | 102 | 822 | 2.9 | 81 |
| OPN | ng/ml | 4.1 | 25 | 0.49 | 12 | 291 | 6130 |
| TIMP-1 | ng/ml | 50 | 131 | 100 | 246 | - | < 3.9 |

(continued)

| Analyte | Units | Plasma | | Sera | | Urine | |
|---|---|---|---|---|---|---|---|
| | | low | high | low | high | low | high |
| A1M | μg/ml | 6.2 | 16 | 5.7 | 17 | - | < 4.2 |
| THP | μg/ml | 0.0084 | 0.052 | 0.007 9 | 0.053 | 0.39 | 2.6 |
| TFF3 | μg/ml | 0.040 | 0.49 | 0.021 | 0.17 | - | < 21 |
| Creatinine | mg/dL | - | - | - | - | 13 | 212 |
| Microalbumin | μg/ml | - | - | - | - | - | > 16 |

In one embodiment, the high values shown for each of the biomarker analytes in Table 1 for the analytic concentrations in human plasma, sera and urine are the minimum diagnostics values for the analytes in human plasma, sera, and urine, respectively. In one exemplary embodiment, the minimum diagnostic concentration in human plasma of alpha-1 microglobulin is about 16 μg/ml, beta-2 microglobulin is about 2.2 μg/ml, calbindin is greater than about 5 ng/ml, clusterin is about 134 μg/ml, CTGF is about 16 ng/ml, cystatin C is about 1170 ng/ml, GST-alpha is about 62 ng/ml, KIM-1 is about 0.57 ng/ml, NGAL is about 375 ng/ml, osteopontin is about 25 ng/ml, THP is about 0.052 μg/ml, TIMP-1 is about 131 ng/ml, TFF-3 is about 0.49 μg/ml, and VEGF is about 855 pg/ml.

In another exemplary embodiment, the minimum diagnostic concentration in human sera of alpha-1 microglobulin is about 17 μg/ml, beta-2 microglobulin is about 2.6 μg/ml, calbindin is greater than about 2.6 ng/ml, clusterin is about 152 μg/ml, CTGF is greater than about 8.2 ng/ml, cystatin C is about 1250 ng/ml, GST-alpha is about 52 ng/ml, KIM-1 is greater than about 0.35 ng/ml, NGAL is about 822 ng/ml, osteopontin is about 12 ng/ml, THP is about 0.053 μg/ml, TIMP-1 is about 246 ng/ml, TFF-3 is about 0.17 μg/ml, and VEGF is about 1630 pg/ml.

In yet another exemplary embodiment, the minimum diagnostic concentration in human urine of alpha-1 microglobulin is about 233 μg/ml, beta-2 microglobulin is greater than about 0.17 μg/ml, calbindin is about 233 ng/ml, clusterin is greater than about 0.089 μg/ml, CTGF is greater than about 0.90 ng/ml, cystatin C is about 1170 ng/ml, GST-alpha is greater than about 26 ng/ml, KIM-1 is about 0.67 ng/ml, NGAL is about 81 ng/ml, osteopontin is about 6130 ng/ml, THP is about 2.6 μg/ml, TIMP-1 is greater than about 3.9 ng/ml, TFF-3 is greater than about 21 μg/ml, and VEGF is about 517 pg/ml.

In one embodiment, the minimum diagnostic concentrations represent the maximum level of analyte concentrations falling within an expected normal range. Kidney transplant rejection or an associated disorder may be indicated if the concentration of an analyte is higher than the minimum diagnostic concentration for the analyte.

If diminished concentrations of a particular analyte are known to be associated with kidney transplant rejection or an associated disorder, the minimum diagnostic concentration may not be an appropriate diagnostic criterion for identifying kidney transplant rejection or an associated disorder indicated by the sample analyte concentrations. In these cases, a maximum diagnostic concentration may define the limit between the expected normal concentration range for the analyte and a sample concentration reflective of kidney transplant rejection or an associated disorder. In those cases in which a maximum diagnostic concentration is the appropriate diagnostic criterion, sample concentrations that fall below a maximum diagnostic concentration may indicate kidney transplant rejection or an associated disorder.

A critical feature of the method of the multiplexed analyte panel is that a combination of sample analyte concentrations may be used to diagnose kidney transplant rejection or an associated disorder. In addition to comparing subsets of the biomarker analyte concentrations to diagnostic criteria, the analytes may be algebraically combined and compared to corresponding diagnostic criteria. In one embodiment, two or more sample analyte concentrations may be added and/or subtracted to determine a combined analyte concentration. In another embodiment, two or more sample analyte concentrations may be multiplied and/or divided to determine a combined analyte concentration. To identify kidney transplant rejection or an associated disorder, the combined analyte concentration may be compared to a diagnostic criterion in which the corresponding minimum or maximum diagnostic concentrations are combined using the same algebraic operations used to determine the combined analyte concentration.

In yet another embodiment, the analyte concentration measured from a test sample containing one type of body fluid may be algebraically combined with an analyte concentration measured from a second test sample containing a second type of body fluid to determine a combined analyte concentration. For example, the ratio of urine calbindin to plasma calbindin may be determined and compared to a corresponding minimum diagnostic urine: plasma calbindin ratio to identify a particular renal disorder.

A variety of methods known in the art may be used to define the diagnostic criteria used to identify kidney transplant rejection or an associated disorder. In one embodiment, any sample concentration falling outside the expected normal range indicates kidney transplant rejection or an associated disorder. In another embodiment, the multiplexed analyte panel may be used to evaluate the analyte concentrations in test samples taken from a population of patients having kidney transplant rejection or an associated disorder and compared to the normal expected analyte concentration ranges. In this same embodiment, any sample analyte concentrations that are significantly higher or lower than the expected normal concentration range may be used to define a minimum or maximum diagnostic concentration, respectively. A number of studies comparing the biomarker concentration ranges of a population of patients having a renal disorder to the corresponding analyte concentrations from a population of normal healthy subjects are described in the examples section below.

## VI. Automated Method for Diagnosing, Monitoring, or Determining a Renal Disorder

In one embodiment, a system for diagnosing, monitoring, or determining kidney transplant rejection or an associated disorder in a mammal is provided that includes a database to store a plurality of kidney transplant rejection or an associated disorder database entries, and a processing device that includes the modules of a kidney transplant rejection or an associated disorder determining application. In this embodiment, the modules are executable by the processing device, and include an analyte input module, a comparison module, and an analysis module.

The analyte input module receives three or more sample analyte concentrations that include the biomarker analytes. In one embodiment, the sample analyte concentrations are entered as input by a user of the application. In another embodiment, the sample analyte concentrations are transmitted directly to the analyte input module by the sensor device used to measure the sample analyte concentration via a data cable, infrared signal, wireless connection or other methods of data transmission known in the art.

The comparison module compares each sample analyte concentration to an entry of a kidney transplant rejection or an associated disorder database. Each entry of the kidney transplant rejection or an associated disorder database includes a list of minimum diagnostic concentrations reflective of a particular type of kidney transplant rejection or an associated disorder. The entries of the kidney transplant rejection or an associated disorder database may further contain additional minimum diagnostic concentrations to further define diagnostic criteria including but not limited to minimum diagnostic concentrations for additional types of bodily fluids, additional types of mammals, and severities of a particular kidney transplant rejection or an associated disorder.

The analysis module determines a most likely kidney transplant rejection or an associated disorder by combining the particular renal disorders identified by the comparison module for all of the sample analyte concentrations. In one embodiment, the most likely kidney transplant rejection or an associated disorder is the particular type of kidney transplant rejection or an associated disorder from the database entry having the most minimum diagnostic concentrations that are less than the corresponding sample analyte concentrations. In another embodiment, the most likely type of kidney transplant rejection or an associated disorder is the particular renal disorder from the database entry having minimum diagnostic concentrations that are all less than the corresponding sample analyte concentrations. In yet other embodiments, the analysis module combines the sample analyte concentrations algebraically to calculate a combined sample analyte concentration that is compared to a combined minimum diagnostic concentration calculated from the corresponding minimum diagnostic criteria using the same algebraic operations. Other combinations of sample analyte concentrations from within the same test sample, or combinations of sample analyte concentrations from two or more different test samples containing two or more different bodily fluids may be used to determine a particular type of kidney transplant rejection or an associated disorder in still other embodiments.

The system includes one or more processors and volatile and/or nonvolatile memory and can be embodied by or in one or more distributed or integrated components or systems. The system may include computer readable media (CRM) on which one or more algorithms, software, modules, data, and/or firmware is loaded and/or operates and/or which operates on the one or more processors to implement the systems and methods identified herein. The computer readable media may include volatile media, nonvolatile media, removable media, non-removable media, and/or other media or mediums that can be accessed by a general purpose or special purpose computing device. For example, computer readable media may include computer storage media and communication media, including but not limited to computer readable media. Computer storage media further may include volatile, nonvolatile, removable, and/or non-removable media implemented in a method or technology for storage of information, such as computer readable instructions, data structures, program modules, and/or other data. Communication media may, for example, embody computer readable instructions, data structures, program modules, algorithms, and/or other data, including but not limited to as or in a modulated data signal. The communication media may be embodied in a carrier wave or other transport mechanism and may include an information delivery method. The communication media may include wired and wireless connections and technologies and may be used to transmit and/or receive wired or wireless communications. Combinations and/or subcombinations of the above and systems, components, modules, and methods and processes described herein may be made.

The following examples are included to demonstrate preferred embodiments of the invention.

**EXAMPLES**

The following examples illustrate various iterations of the invention.

**Example 1: Least Detectable Dose and Lower Limit of Quantitation of Assay for Analytes Associated with Renal Disorders**

To assess the least detectable doses (LDD) and lower limits of quantitation (LLOQ) of a variety of analytes associated with renal disorders, the following experiment was conducted. The analytes measured were alpha-1 microglobulin (A1 M), beta-2 microglobulin (B2M), calbindin, clusterin, CTGF, cystatin C, GST-alpha, KIM-1, NGAL, osteoopontin (OPN), THP, TIMP-1, TFF-3, and VEGF.

The concentrations of the analytes were measured using a capture-sandwich assay using antigen-specific antibodies. For each analyte, a range of standard sample dilutions ranging over about four orders of magnitude of analyte concentration were measured using the assay in order to obtain data used to construct a standard dose response curve. The dynamic range for each of the analytes, defined herein as the range of analyte concentrations measured to determine its dose response curve, is presented below.

To perform the assay, 5 μL of a diluted mixture of capture-antibody microspheres were mixed with 5 μL of blocker and 10 μL of pre-diluted standard sample in each of the wells of a hard-bottom microtiter plate. After incubating the hard-bottom plate for 1 hour, 10 μL of biotinylated detection antibody was added to each well, and then the hard-bottom plate was incubated for an additional hour. 10 μL of diluted streptavidin-phycoerythrin was added to each well and then the hard-bottom plate was incubated for another 60 minutes.

A filter-membrane microtiter plate was pre-wetted by adding 100 μL wash buffer, and then aspirated using a vacuum manifold device. The contents of the wells of the hard-bottom plate were then transferred to the corresponding wells of the filter-membrane plate. All wells of the hard-bottom plate were vacuum-aspirated and the contents were washed twice with 100 μL of wash buffer. After the second wash, 100 μL of wash buffer was added to each well, and then the washed microspheres were resuspended with thorough mixing. The plate was then analyzed using a Luminex 100 Analyzer (Luminex Corporation, Austin, Texas, USA). Dose response curves were constructed for each analyte by curve-fitting the median fluorescence intensity (MFI) measured from the assays of diluted standard samples containing a range of analyte concentrations.

The least detectable dose (LDD) was determined by adding three standard deviations to the average of the MFI signal measured for 20 replicate samples of blank standard solution (i.e. standard solution containing no analyte). The MFI signal was converted to an LDD concentration using the dose response curve and multiplied by a dilution factor of 2.

The lower limit of quantification (LLOQ), defined herein as the point at which the coefficient of variation (CV) for the analyte measured in the standard samples was 30%, was determined by the analysis of the measurements of increasingly diluted standard samples. For each analyte, the standard solution was diluted by 2 fold for 8 dilutions. At each stage of dilution, samples were assayed in triplicate, and the CV of the analyte concentration at each dilution was calculated and plotted as a function of analyte concentration. The LLOQ was interpolated from this plot and multiplied by a dilution factor of 2.

The LDD and LLOQ results for each analyte are summarized in **Table 2**:

**TABLE 2: LDD, LLOQ, and Dynamic Range of Analyte Assay**

| Analyte | Units | LDD | LLOQ | Dynamic Range | |
|---|---|---|---|---|---|
| | | | | minimum | maximum |
| Calbindin | ng/mL | 1.1 | 3.1 | 0.516 | 2580 |
| Clusterin | ng/mL | 2.4 | 2.3 | 0.676 | 3378 |
| CTGF | ng/mL | 1.3 | 3.8 | 0.0794 | 400 |
| GST-alpha | ng/mL | 1.4 | 3.6 | 0.24 | 1,200 |
| KIM-1 | ng/mL | 0.016 | 0.028 | 0.00478 | 24 |
| VEGF | pg/mL | 4.4 | 20 | 8.76 | 44,000 |
| β-2M | μg/mL | 0.012 | 0.018 | 0.0030 | 15 |
| Cystatin C | ng/mL | 2.8 | 3.7 | 0.60 | 3,000 |
| NGAL | ng/mL | 4.1 | 7.8 | 1.2 | 6,000 |

(continued)

| Analyte | Units | LDD | LLOQ | Dynamic Range | |
| --- | --- | --- | --- | --- | --- |
| | | | | minimum | maximum |
| Osteopontin | ng/mL | 29 | 52 | 3.9 | 19,500 |
| TIMP-1 | ng/mL | 0.71 | 1.1 | 0.073 | 365 |
| A-1 M | μg/mL | 0.059 | 0.29 | 0.042 | 210 |
| THP | μg/mL | 0.46 | 0.30 | 0.16 | 800 |
| TFF-3 | μg/mL | 0.06 | 0.097 | 0.060 | 300 |

The results of this experiment characterized the least detectible dose and the lower limit of quantification for fourteen analytes associated with various renal disorders using a capture-sandwich assay.

**Example 2: Precision of Assay for Analytes Associated with Renal Disorders**

To assess the precision of an assay used to measure the concentration of analytes associated with renal disorders, the following experiment was conducted. The analytes measured were alpha-1 microglobulin (A1M), beta-2 microglobulin (B2M), calbindin, clusterin, CTGF, cystatin C, GST-alpha, KIM-1, NGAL, osteopontin (OPN), THP, TIMP-1, TFF-3, and VEGF. For each analyte, three concentration levels of standard solution were measured in triplicate during three runs using the methods described in Example 1. The percent errors for each run at each concentration are presented in **Table 3** for all of the analytes tested:

**TABLE 3: Precision of Analyte Assay**

| Analyte | Average concentration (ng/mL) | Run 1 Error (%) | Run 2 Error (%) | Run 2 Error (%) | Interrun Error (%) |
| --- | --- | --- | --- | --- | --- |
| Calbindin | 4.0 | 6 | 2 | 6 | 13 |
| | 36 | 5 | 3 | 2 | 7 |
| | 281 | 1 | 6 | 0 | 3 |
| Clusterin | 4.4 | 4 | 9 | 2 | 6 |
| | 39 | 5 | 1 | 6 | 8 |
| | 229 | 1 | 3 | 0 | 2 |
| CTGF | 1.2 | 10 | 17 | 4 | 14 |
| | 2.5 | 19 | 19 | 14 | 14 |
| | 18 | 7 | 5 | 13 | 9 |
| GST-alpha | 3.9 | 14 | 7 | 5 | 10 |
| | 16 | 13 | 7 | 10 | 11 |
| | 42 | 1 | 16 | 6 | 8 |
| KIM-1 | 0.035 | 2 | 0 | 5 | 13 |
| | 0.32 | 4 | 5 | 2 | 8 |
| | 2.9 | 0 | 5 | 7 | 4 |
| VEGF | 65 | 10 | 1 | 6 | 14 |
| | 534 | 9 | 2 | 12 | 7 |
| | 5,397 | 1 | 13 | 14 | 9 |

(continued)

| Analyte | Average concentration (ng/mL) | Run 1 Error (%) | Run 2 Error (%) | Run 2 Error (%) | Interrun Error (%) |
|---|---|---|---|---|---|
| β-2M | 0.040 | 6 | 1 | 8 | 5 |
| | 0.43 | 2 | 2 | 0 | 10 |
| | 6.7 | 6 | 5 | 11 | 6 |
| Cystatin C | 10.5 | 4 | 1 | 7 | 13 |
| | 49 | 0 | 0 | 3 | 9 |
| | 424 | 2 | 6 | 2 | 5 |
| NGAL | 18.1 | 11 | 3 | 6 | 13 |
| | 147 | 0 | 0 | 6 | 5 |
| | 1,070 | 5 | 1 | 2 | 5 |
| Osteopontin | 44 | 1 | 10 | 2 | 11 |
| | 523 | 9 | 9 | 9 | 7 |
| | 8,930 | 4 | 10 | 1 | 10 |
| TIMP-1 | 2.2 | 13 | 6 | 3 | 13 |
| | 26 | 1 | 1 | 4 | 14 |
| | 130 | 1 | 3 | 1 | 4 |
| A-1 M | 1.7 | 11 | 7 | 7 | 14 |
| | 19 | 4 | 1 | 8 | 9 |
| | 45 | 3 | 5 | 2 | 4 |
| THP | 9.4 | 3 | 10 | 11 | 11 |
| | 15 | 3 | 7 | 8 | 6 |
| | 37 | 4 | 5 | 0 | 5 |
| TFF-3 | 0.3 | 13 | 3 | 11 | 12 |
| | 4.2 | 5 | 8 | 5 | 7 |
| | 1.2 | 3 | 7 | 0 | 13 |

The results of this experiment characterized the precision of a capture-sandwich assay for fourteen analytes associated with various renal disorders over a wide range of analyte concentrations. The precision of the assay varied between about 1 % and about 15% error within a given run, and between about 5% and about 15% error between different runs. The percent errors summarized in Table 2 provide information concerning random error to be expected in an assay measurement caused by variations in technicians, measuring instruments, and times of measurement.

**Example 3: Linearity of Assay for Analytes Associated with Renal Disorders**

To assess the linearity of an assay used to measure the concentration of analytes associated with renal disorders, the following experiment was conducted. The analytes measured were alpha-1 microglobulin (A1M), beta-2 microglobulin (B2M), calbindin, clusterin, CTGF, cystatin C, GST-alpha, KIM-1, NGAL, osteopontin (OPN), THP, TIMP-1, TFF-3, and VEGF. For each analyte, three concentration levels of standard solution were measured in triplicate during three runs using the methods described in Example 1. Linearity of the assay used to measure each analyte was determined by measuring the concentrations of standard samples that were serially-diluted throughout the assay range. The % recovery was calculated as observed vs. expected concentration based on the dose-response curve. The results of the linearity analysis are summarized in **Table 4.**

**TABLE 4: Linearity of Analyte Assay**

| Analyte | Dilution | Expected concentration | Observed concentration | Recovery (%) |
|---|---|---|---|---|
| Calbindin (ng/mL) | 1:2 | 61 | 61 | 100 |
| | 1:4 | 30 | 32 | 106 |
| | 1:8 | 15 | 17 | 110 |
| Clusterin (ng/mL) | 1:2 | 41 | 41 | 100 |
| | 1:4 | 21 | 24 | 116 |
| | 1:8 | 10 | 11 | 111 |
| CTGF (ng/mL) | 1:2 | 1.7 | 1.7 | 100 |
| | 1:4 | 0.84 | 1.0 | 124 |
| | 1:8 | 0.42 | 0.51 | 122 |
| GST-alpha (ng/mL) | 1:2 | 25 | 25 | 100 |
| | 1:4 | 12 | 14 | 115 |
| | 1:8 | 6.2 | 8.0 | 129 |
| KIM-1 (ng/mL) | 1:2 | 0.87 | 0.87 | 100 |
| | 1:4 | 0.41 | 0.41 | 101 |
| | 1:8 | 0.21 | 0.19 | 93 |
| VEGF (pg/mL) | 1:2 | 2,525 | 2,525 | 100 |
| | 1:4 | 1,263 | 1,340 | 106 |
| | 1:8 | 631 | 686 | 109 |
| $\beta$-2M ($\mu$g/mL) | 1:100 | 0.63 | 0.63 | 100 |
| | 1:200 | 0.31 | 0.34 | 106 |
| | 1:400 | 0.16 | 0.17 | 107 |
| Cystatin C (ng/mL) | 1:100 | 249 | 249 | 100 |
| | 1:200 | 125 | 122 | 102 |
| | 1:400 | 62 | 56 | 110 |
| NGAL (ng/mL) | 1:100 | 1,435 | 1,435 | 100 |
| | 1:200 | 718 | 775 | 108 |
| | 1:400 | 359 | 369 | 103 |
| Osteoponti n (ng/mL) | 1:100 | 6,415 | 6,415 | 100 |
| | 1:200 | 3,208 | 3,275 | 102 |
| | 1:400 | 1,604 | 1,525 | 95 |
| TIMP-1 (ng/mL) | 1:100 | 35 | 35 | 100 |
| | 1:200 | 18 | 18 | 100 |
| | 1:400 | 8.8 | 8.8 | 100 |
| A-1 M ($\mu$g/mL) | 1:2000 | 37 | 37 | 100 |
| | 1:4000 | 18 | 18 | 99 |
| | 1:8000 | 9.1 | 9.2 | 99 |

(continued)

| Analyte | Dilution | Expected concentration | Observed concentration | Recovery (%) |
|---|---|---|---|---|
| THP (µg/mL) | 1:2000 | 28 | 28 | 100 |
| | 1:4000 | 14 | 14 | 96 |
| | 1:8000 | 6.7 | 7.1 | 94 |
| TFF-3 (µg/mL) | 1:2000 | 8.8 | 8.8 | 100 |
| | 1:4000 | 3.8 | 4.4 | 86 |
| | 1:8000 | 1.9 | 2.2 | 86 |

The results of this experiment demonstrated reasonably linear responses of the sandwich-capture assay to variations in the concentrations of the analytes in the tested samples.

**Example 4: Spike Recovery of Analytes Associated with Renal Disorders**

To assess the recovery of analytes spiked into urine, serum, and plasma samples by an assay used to measure the concentration of analytes associated with renal disorders, the following experiment was conducted. The analytes measured were alpha-1 microglobulin (A1 M), beta-2 microglobulin (B2M), calbindin, clusterin, CTGF, cystatin C, GST-alpha, KIM-1, NGAL, osteopontin (OPN), THP, TIMP-1, TFF-3, and VEGF. For each analyte, three concentration levels of standard solution were spiked into known urine, serum, and plasma samples. Prior to analysis, all urine samples were diluted 1:2000 (sample: diluent), all plasma samples were diluted 1:5 (sample: diluent), and all serum samples were diluted 1:2000 (sample: diluent).

The concentrations of the analytes in the samples were measured using the methods described in Example 1. The average % recovery was calculated as the proportion of the measurement of analyte spiked into the urine, serum, or plasma sample (observed) to the measurement of analyte spiked into the standard solution (expected). The results of the spike recovery analysis are summarized in **Table 5.**

**TABLE 5: Spike Recovery of Analyte Assay in Urine, Serum, and Plasma Samples**

| Analyte | Spike Concentration | Recovery in Urine Sample (%) | Recovery in Serum Sample (%) | Recovery in Plasma Sample (%) |
|---|---|---|---|---|
| Calbindin (ng/mL) | 66 | 76 | 82 | 83 |
| | 35 | 91 | 77 | 71 |
| | 18 | 80 | 82 | 73 |
| | average | 82 | 80 | 76 |
| Clusterin (ng/mL) | 80 | 72 | 73 | 75 |
| | 37 | 70 | 66 | 72 |
| | 20 | 90 | 73 | 70 |
| | average | 77 | 70 | 72 |
| CTGF (ng/mL) | 8.4 | 91 | 80 | 79 |
| | 4.6 | 114 | 69 | 78 |
| | 2.4 | 76 | 80 | 69 |
| | average | 94 | 77 | 75 |
| GST-alpha (ng/mL) | 27 | 75 | 84 | 80 |
| | 15 | 90 | 75 | 81 |
| | 7.1 | 82 | 84 | 72 |
| | average | 83 | 81 | 78 |

(continued)

| Analyte | Spike Concentration | Recovery in Urine Sample (%) | Recovery in Serum Sample (%) | Recovery in Plasma Sample (%) |
|---|---|---|---|---|
| KIM-1 (ng/mL) | 0.63 | 87 | 80 | 83 |
| | .029 | 119 | 74 | 80 |
| | 0.14 | 117 | 80 | 78 |
| | average | 107 | 78 | 80 |
| VEGF (pg/mL) | 584 | 88 | 84 | 82 |
| | 287 | 101 | 77 | 86 |
| | 123 | 107 | 84 | 77 |
| | average | 99 | 82 | 82 |
| β-2 M (μg/mL) | 0.97 | 117 | 98 | 98 |
| | 0.50 | 124 | 119 | 119 |
| | 0.24 | 104 | 107 | 107 |
| | average | 115 | 108 | 105 |
| Cystatin C (ng/mL) | 183 | 138 | 80 | 103 |
| | 90 | 136 | 97 | 103 |
| | 40 | 120 | 97 | 118 |
| | average | 131 | 91 | 108 |
| NGAL (ng/mL) | 426 | 120 | 105 | 111 |
| | 213 | 124 | 114 | 112 |
| | 103 | 90 | 99 | 113 |
| | average | 111 | 106 | 112 |
| Osteopontin (ng/mL) | 1,245 | 204 | 124 | 68 |
| | 636 | 153 | 112 | 69 |
| | 302 | 66 | 103 | 67 |
| | average | 108 | 113 | 68 |
| TIMP-1 (ng/mL) | 25 | 98 | 97 | 113 |
| | 12 | 114 | 89 | 103 |
| | 5.7 | 94 | 99 | 113 |
| | average | 102 | 95 | 110 |
| A-1 M (μg/mL) | 0.0028 | 100 | 101 | 79 |
| | 0.0012 | 125 | 80 | 81 |
| | 0.00060 | 118 | 101 | 82 |
| | Average | 114 | 94 | 81 |
| THP (μg/mL) | 0.0096 | 126 | 108 | 90 |
| | 0.0047 | 131 | 93 | 91 |
| | 0.0026 | 112 | 114 | 83 |
| | average | 123 | 105 | 88 |

(continued)

| Analyte | Spike Concentration | Recovery in Urine Sample (%) | Recovery in Serum Sample (%) | Recovery in Plasma Sample (%) |
|---|---|---|---|---|
| TFF-3 (μg/mL) | 0.0038 | 105 | 114 | 97 |
| | 0.0019 | 109 | 104 | 95 |
| | 0.0010 | 102 | 118 | 93 |
| | average | 105 | 112 | 95 |

The results of this experiment demonstrated that the sandwich-type assay is reasonably sensitive to the presence of all analytes measured, whether the analytes were measured in standard samples, urine samples, plasma samples, or serum samples.

**Example 5: Matrix Interferences of Analytes Associated with Renal Disorders**

To assess the matrix interference of hemoglobin, bilirubin, and triglycerides spiked into standard samples, the following experiment was conducted. The analytes measured were alpha-1 microglobulin (A1M), beta-2 microglobulin (B2M), calbindin, clusterin, CTGF, cystatin C, GST-alpha, KIM-1, NGAL, osteopontin (OPN), THP, TIMP-1, TFF-3, and VEGF. For each analyte, three concentration levels of standard solution were spiked into known urine, serum, and plasma samples. Matrix interference was assessed by spiking hemoglobin, bilirubin, and triglyceride into standard analyte samples and measuring analyte concentrations using the methods described in Example 1. A % recovery was determined by calculating the ratio of the analyte concentration measured from the spiked sample (observed) divided by the analyte concentration measured form the standard sample (expected). The results of the matrix interference analysis are summarized in **Table 6.**

**TABLE 6: Matrix Interference of Hemoglobin, Bilirubin, and Triglyceride on the Measurement of Analytes**

| Analyte | Matrix Compound Spiked into Sample | Maximum Spike Concentration | Overall Recovery (%) |
|---|---|---|---|
| Calbindin (mg/mL) | Hemoglobin | 500 | 110 |
| | Bilirubin | 20 | 98 |
| | Triglyceride | 500 | 117 |
| Clusterin (mg/mL) | Hemoglobin | 500 | 125 |
| | Bilirubin | 20 | 110 |
| | Triglyceride | 500 | 85 |
| CTGF (mg/mL) | Hemoglobin | 500 | 91 |
| | Bilirubin | 20 | 88 |
| | Triglyceride | 500 | 84 |
| GST-alpha (mg/mL) | Hemoglobin | 500 | 100 |
| | Bilirubin | 20 | 96 |
| | Triglyceride | 500 | 96 |
| KIM-1 (mg/mL) | Hemoglobin | 500 | 108 |
| | Bilirubin | 20 | 117 |
| | Triglyceride | 500 | 84 |
| VEGF (mg/mL) | Hemoglobin | 500 | 112 |
| | Bilirubin | 20 | 85 |
| | Triglyceride | 500 | 114 |

(continued)

| Analyte | Matrix Compound Spiked into Sample | Maximum Spike Concentration | Overall Recovery (%) |
|---|---|---|---|
| β-2 M (μg/mL) | Hemoglobin | 500 | 84 |
| | Bilirubin | 20 | 75 |
| | Triglyceride | 500 | 104 |
| Cystatin C (ng/mL) | Hemoglobin | 500 | 91 |
| | Bilirubin | 20 | 102 |
| | Triglyceride | 500 | 124 |
| NGAL (ng/mL) | Hemoglobin | 500 | 99 |
| | Bilirubin | 20 | 92 |
| | Triglyceride | 500 | 106 |
| Osteopontin (ng/mL) | Hemoglobin | 500 | 83 |
| | Bilirubin | 20 | 86 |
| | Triglyceride | 500 | 106 |
| TIMP-1 (ng/mL) | Hemoglobin | 500 | 87 |
| | Bilirubin | 20 | 86 |
| | Triglyceride | 500 | 93 |
| A-1 M (μg/mL) | Hemoglobin | 500 | 103 |
| | Bilirubin | 20 | 110 |
| | Triglyceride | 500 | 112 |
| THP (μg/mL) | Hemoglobin | 500 | 108 |
| | Bilirubin | 20 | 101 |
| | Triglyceride | 500 | 121 |
| TFF-3 (μg/mL) | Hemoglobin | 500 | 101 |
| | Bilirubin | 20 | 101 |
| | Triglyceride | 500 | 110 |

The results of this experiment demonstrated that hemoglobin, bilirubin, and triglycerides, three common compounds found in urine, plasma, and serum samples, did not significantly degrade the ability of the sandwich-capture assay to detect any of the analytes tested.

**Example 6: Sample Stability of Analytes Associated with Renal Disorders**

To assess the ability of analytes spiked into urine, serum, and plasma samples to tolerate freeze-thaw cycles, the following experiment was conducted. The analyses measured were alpha-1 microglobulin (A1M), beta-2 microglobulin (B2M), calbindin, clusterin, CTGF, cystatin C, GST-alpha, KIM-1, NGAL, osteopontin (OPN), THP, TIMP-1, TFF-3, and VEGF. Each analyte was spiked into known urine, serum, and plasma samples at a known analyte concentration. The concentrations of the analytes in the samples were measured using the methods described in Example 1 after the initial addition of the analyte, and after one, two and three cycles of freezing and thawing. In addition, analyte concentrations in urine, serum and plasma samples were measured immediately after the addition of the analyte to the samples as well as after storage at room temperature for two hours and four hours, and after storage at 4o C for 2 hours, four hours, and 24 hours.
The results of the freeze-thaw stability analysis are summarized in **Table 7.** The % recovery of each analyte was calculated as a percentage of the analyte measured in the sample prior to any freeze-thaw cycles.

TABLE 7: Freeze-Thaw Stability of the Analytes in Urine, Serum, and Plasma

| Analyte | Period and Temp | Urine Sample | | Serum Sample | | Plasma Sample | |
|---|---|---|---|---|---|---|---|
| | | Concentration | Recovery (%) | Concentration | Recovery (%) | Concentraction | Recovery (%) |
| Calbindi n (ng/mL) | Control | 212 | 100 | 31 | 100 | 43 | 100 |
| | 1X | 221 | 104 | 30 | 96 | 41 | 94 |
| | 2X | 203 | 96 | 30 | 99 | 39 | 92 |
| | 3X | 234 | 110 | 30 | 97 | 40 | 93 |
| Clusterin (ng/mL) | 0 | 315 | 100 | 232 | 100 | 187 | 100 |
| | 1X | 329 | 104 | 227 | 98 | 177 | 95 |
| | 2X | 341 | 108 | 240 | 103 | 175 | 94 |
| | 3X | 379 | 120 | 248 | 107 | 183 | 98 |
| CTGF (ng/mL) | 0 | 6.7 | 100 | 1.5 | 100 | 1.2 | 100 |
| | 1X | 7.5 | 112 | 1.3 | 82 | 1.2 | 94 |
| | 2X | 6.8 | 101 | 1.4 | 90 | 1.2 | 100 |
| | 3X | 7.7 | 115 | 1.2 | 73 | 1.3 | 107 |
| GST-alpha (ng/mL) | 0 | 12 | 100 | 23 | 100 | 11 | 100 |
| | 1X | 13 | 104 | 24 | 105 | 11 | 101 |
| | 2X | 14 | 116 | 21 | 92 | 11 | 97 |
| | 3X | 14 | 111 | 23 | 100 | 12 | 108 |
| KIM-1 (ng/mL) | 0 | 1.7 | 100 | 0.24 | 100 | 0.24 | 100 |
| | 1X | 1.7 | 99 | 0.24 | 102 | 0.22 | 91 |
| | 2X | 1.7 | 99 | 0.22 | 94 | 0.19 | 78 |
| | 3X | 1.8 | 107 | 0.23 | 97 | 0.22 | 93 |
| VEGF (pg/mL) | 0 | 1,530 | 100 | 1,245 | 100 | 674 | 100 |
| | 1X | 1,575 | 103 | 1,205 | 97 | 652 | 97 |
| | 2X | 1,570 | 103 | 1,140 | 92 | 612 | 91 |
| | 3X | 1,700 | 111 | 1,185 | 95 | 670 | 99 |

| Analyte | Period and Temp | Urine Sample | | Serum Sample | | Plasma Sample | |
|---|---|---|---|---|---|---|---|
| | | Concentration | Recovery (%) | Concentration | Recovery (%) | Concentraction | Recovery (%) |
| β3-2 M (μg/mL) | 0 | 0.0070 | 100 | 1.2 | 100 | 15 | 100 |
| | 1X | 0.0073 | 104 | 1.1 | 93 | 14 | 109 |
| | 2X | 0.0076 | 108 | 1.2 | 103 | 15 | 104 |
| | 3X | 0.0076 | 108 | 1.1 | 97 | 13 | 116 |
| Cystatin C (ng/mL) | 0 | 1,240 | 100 | 1,330 | 100 | 519 | 100 |
| | 1X | 1,280 | 103 | 1,470 | 111 | 584 | 113 |
| | 2X | 1,410 | 114 | 1,370 | 103 | 730 | 141 |
| | 3X | 1,420 | 115 | 1,380 | 104 | 589 | 113 |
| NGAL (ng/mL) | 0 | 45 | 100 | 245 | 100 | 84 | 100 |
| | 1X | 46 | 102 | 179 | 114 | 94 | 112 |
| | 2X | 47 | 104 | 276 | 113 | 91 | 108 |
| | 3X | 47 | 104 | 278 | 113 | 91 | 109 |
| Osteopo ntin (ng/mL) | 0 | 38 | 100 | 1.7 | 100 | 5.0 | 100 |
| | 1X | 42 | 110 | 1.8 | 102 | 5.5 | 110 |
| | 2X | 42 | 108 | 1.5 | 87 | 5.5 | 109 |
| | 3X | 42 | 110 | 1.3 | 77 | 5.4 | 107 |
| TIMP-1 (ng/mL) | 0 | 266 | 100 | 220 | 100 | 70 | 100 |
| | 1X | 265 | 100 | 220 | 10 | 75 | 108 |
| | 2X | 255 | 96 | 215 | 98 | 77 | 110 |
| | 3X | 295 | 111 | 228 | 104 | 76 | 109 |
| A-1 M (μg/mL) | 0 | 14 | 100 | 26 | 100 | 4.5 | 100 |
| | 1X | 13 | 92 | 25 | 96 | 4.2 | 94 |
| | 2X | 15 | 107 | 25 | 96 | 4.3 | 97 |
| | 3X | 16 | 116 | 23 | 88 | 4.0 | 90 |

| Analyte | Period and Temp | Urine Sample | | Serum Sample | | Plasma Sample | |
|---|---|---|---|---|---|---|---|
| | | Concentration | Recovery (%) | Concentration | Recovery (%) | Concentraction | Recovery (%) |
| THP (µg/mL) | 0 | 4.6 | 100 | 31 | 100 | 9.2 | 100 |
| | 1X | 4.4 | 96 | 31 | 98 | 8.8 | 95 |
| | 2X | 5.0 | 110 | 31 | 100 | 9.2 | 100 |
| | 3X | 5.2 | 114 | 27 | 85 | 9.1 | 99 |
| TFF-3 (µg/mL) | 0 | 4.6 | 100 | 24 | 100 | 22 | 100 |
| | 1X | 4.4 | 96 | 23 | 98 | 22 | 103 |
| | 2X | 5.0 | 110 | 24 | 103 | 22 | 101 |
| | 3X | 5.2 | 114 | 19 | 82 | 22 | 102 |

The results of the short-term stability assessment are summarized in **Table 8.** The % recovery of each analyte was calculated as a percentage of the analyte measured in the sample prior to any short-term storage.

TABLE 8: Short-Term Stability of Analytes in Urine, Serum, and Plasma

| Analyte | Storage Time/ Temp | Urine Sample | | Serum Sample | | Plasma Sample | |
|---|---|---|---|---|---|---|---|
| | | Sample Conc. | Recovery (%) | Sample Conc. | Recovery (%) | Sample Conc. | Recovery (%) |
| Calbindin (ng/mL) | Control | 226 | 100 | 33 | 100 | 7 | 100 |
| | 2 hr/ room temp | 242 | 107 | 30 | 90 | 6.3 | 90 |
| | 2 hr. @ 4° C | 228 | 101 | 29 | 89 | 6.5 | 93 |
| | 4 hr @ room temp | 240 | 106 | 28 | 84 | 5.6 | 79 |
| | 4 hr. @ 4°C | 202 | 89 | 29 | 86 | 5.5 | 79 |
| | 24 hr. @ 4°C | 199 | 88 | 26 | 78 | 7.1 | 101 |
| Clusterin (ng/mL) | Control | 185 | 100 | 224 | 100 | 171 | 100 |
| | 2 hr @ room temp | 173 | 94 | 237 | 106 | 180 | 105 |
| | 2 hr. @ 4° C | 146 | 79 | 225 | 100 | 171 | 100 |
| | 4 hr @ room temp | 166 | 89 | 214 | 96 | 160 | 94 |
| | 4 hr. @ 4° C | 157 | 85 | 198 | 88 | 143 | 84 |
| | 24 hr. @ 4° C | 185 | 100 | 207 | 92 | 162 | 94 |
| CTGF (ng/mL) | Control | 1.9 | 100 | 8.8 | 100 | 1.2 | 100 |
| | 2 hr. @ room temp | 1.9 | 99 | 6.7 | 76 | 1 | 83 |
| | 2 hr. @ 4° C | 1.8 | 96 | 8.1 | 92 | 1.1 | 89 |
| | 4 hr. @ room temp | 2.1 | 113 | 5.6 | 64 | 1 | 84 |
| | 4 hr. @ 4° C | 1.7 | 91 | 6.4 | 74 | 0.9 | 78 |
| | 24 hr. @ 4° C | 2.2 | 116 | 5.9 | 68 | 1.1 | 89 |
| GST-alpha (ng/mL) | Control | 14 | 100 | 21 | 100 | 11 | 100 |
| | 2 hr. @ room temp | 11 | 75 | 23 | 107 | 11 | 103 |
| | 2 hr. @ 4° C | 13 | 93 | 22 | 104 | 9.4 | 90 |
| | 4 hr. @ room temp | 11 | 79 | 21 | 100 | 11 | 109 |
| | 4 hr. @ 4° C | 12 | 89 | 21 | 98 | 11 | 100 |
| | 24 hr. @ 4° C | 13 | 90 | 22 | 103 | 14 | 129 |

(continued)

| Analyte | Storage Time/ Temp | Urine Sample | | Serum Sample | | Plasma Sample | |
|---|---|---|---|---|---|---|---|
| | | Sample Conc. | Recovery (%) | Sample Conc. | Recovery (%) | Sample Conc. | Recovery (%) |
| KIM-1 (ng/mL) | Control | 1.5 | 100 | 0.23 | 100 | 0.24 | 100 |
| | 2 hr. @ room temp | 1.2 | 78 | 0.2 | 86 | 0.22 | 90 |
| | 2 hr. @ 4° C | 1.6 | 106 | 0.23 | 98 | 0.21 | 85 |
| | 4 hr. @ room temp | 1.3 | 84 | 0.19 | 82 | 0.2 | 81 |
| | 4 hr. @ 4° C | 1.4 | 90 | 0.22 | 93 | 0.19 | 80 |
| | 24 hr. @ 4° C | 1.1 | 76 | 0.18 | 76 | 0.23 | 94 |
| VEGF | Control | 851 | 100 | 1215 | 100 | 670 | 100 |
| (pg/mL) | 2 hr @ room temp | 793 | 93 | 1055 | 87 | 622 | 93 |
| | 2 hr. @ 4° C | 700 | 82 | 1065 | 88 | 629 | 94 |
| | 4 hr @ room temp | 704 | 83 | 1007 | 83 | 566 | 84 |
| | 4 hr. @ 4° C | 618 | 73 | 1135 | 93 | 544 | 81 |
| | 24 hr. @ 4° C | 653 | 77 | 1130 | 93 | 589 | 88 |
| β-2 M (μg/mL) | Control | 0.064 | 100 | 2.6 | 100 | 1.2 | 100 |
| | 2 hr @ room temp | 0.062 | 97 | 2.4 | 92 | 1.1 | 93 |
| | 2 hr. @ 4° C | 0.058 | 91 | 2.2 | 85 | 1.2 | 94 |
| | 4 hr @ room temp | 0.064 | 101 | 2.2 | 83 | 1.2 | 94 |
| | 4 hr. @ 4° C | 0.057 | 90 | 2.2 | 85 | 1.2 | 98 |
| | 24 hr. @ 4° C | 0.06 | 94 | 2.5 | 97 | 1.3 | 103 |
| Cystatin C (ng/mL) | Control | 52 | 100 | 819 | 100 | 476 | 100 |
| | 2 hr @ room temp | 50 | 96 | 837 | 102 | 466 | 98 |
| | 2 hr. @ 4° C | 44 | 84 | 884 | 108 | 547 | 115 |
| | 4 hr @ room temp | 49 | 93 | 829 | 101 | 498 | 105 |
| | 4 hr. @ 4° C | 46 | 88 | 883 | 108 | 513 | 108 |
| | 24 hr. @ 4° C | 51 | 97 | 767 | 94 | 471 | 99 |
| NGAL | Control | 857 | 100 | 302 | 100 | 93 | 100 |

(continued)

| Analyte | Storage Time/ Temp | Urine Sample | | Serum Sample | | Plasma Sample | |
|---|---|---|---|---|---|---|---|
| | | Sample Conc. | Recovery (%) | Sample Conc. | Recovery (%) | Sample Conc. | Recovery (%) |
| (ng/mL) | 2 hr @ room temp | 888 | 104 | 287 | 95 | 96 | 104 |
| | 2 hr. @ 4° C | 923 | 108 | 275 | 91 | 92 | 100 |
| | 4 hr @ room temp | 861 | 101 | 269 | 89 | 88 | 95 |
| | 4 hr. @ 4° C | 842 | 98 | 283 | 94 | 94 | 101 |
| | 24 hr. @ 4° C | 960 | 112 | 245 | 81 | 88 | 95 |
| Osteopontin (ng/mL) | Control | 2243 | 100 | 6.4 | 100 | 5.2 | 100 |
| | 2 hr @ room temp | 2240 | 100 | 6.8 | 107 | 5.9 | 114 |
| | 2 hr. @ 4° C | 2140 | 95 | 6.4 | 101 | 6.2 | 120 |
| | 4 hr @ room temp | 2227 | 99 | 6.9 | 108 | 5.8 | 111 |
| | 4 hr. @ 4° C | 2120 | 95 | 7.7 | 120 | 5.2 | 101 |
| | 24 hr. @ 4° C | 2253 | 100 | 6.5 | 101 | 6 | 116 |
| TIMP-1 (ng/mL) | Control | 17 | 100 | 349 | 100 | 72 | 100 |
| | 2 hr @ room temp | 17 | 98 | 311 | 89 | 70 | 98 |
| | 2 hr. @ 4° C | 16 | 94 | 311 | 89 | 68 | 95 |
| | 4 hr @ room temp | 17 | 97 | 306 | 88 | 68 | 95 |
| | 4 hr. @ 4° C | 16 | 93 | 329 | 94 | 74 | 103 |
| | 24 hr. @ 4° C | 18 | 105 | 349 | 100 | 72 | 100 |
| A-1 M | Control | 3.6 | 100 | 2.2 | 100 | 1 | 100 |
| (μg/mL) | 2 hr @ room temp | 3.5 | 95 | 2 | 92 | 1 | 105 |
| | 2 hr. @ 4° C | 3.4 | 92 | 2.1 | 97 | 0.99 | 99 |
| | 4 hr @ room temp | 3.2 | 88 | 2.2 | 101 | 0.99 | 96 |
| | 4 hr. @ 4° C | 3 | 82 | 2.2 | 99 | 0.97 | 98 |
| | 24 hr. @ 4° C | 3 | 83 | 2.2 | 100 | 1 | 101 |

(continued)

| Analyte | Storage Time/ Temp | Urine Sample | | Serum Sample | | Plasma Sample | |
|---|---|---|---|---|---|---|---|
| | | Sample Conc. | Recovery (%) | Sample Conc. | Recovery (%) | Sample Conc. | Recovery (%) |
| THP ($\mu$g/mL) | Control | 1.2 | 100 | 34 | 100 | 2.1 | 100 |
| | 2 hr @ room temp | 1.2 | 99 | 34 | 99 | 2 | 99 |
| | 2 hr. @ 4° C | 1.1 | 90 | 34 | 100 | 2 | 98 |
| | 4 hr @ room temp | 1.1 | 88 | 27 | 80 | 2 | 99 |
| | 4 hr. @ 4° C | 0.95 | 79 | 33 | 97 | 2 | 95 |
| | 24 hr. @ 4° C | 0.91 | 76 | 33 | 98 | 2.4 | 116 |
| TFF-3 ($\mu$g/mL) | Control | 1230 | 100 | 188 | 100 | 2240 | 100 |
| | 2 hr @ room temp | 1215 | 99 | 179 | 95 | 2200 | 98 |
| | 2 hr. @ 4° C | 1200 | 98 | 195 | 104 | 2263 | 101 |
| | 4 hr @ room temp | 1160 | 94 | 224 | 119 | 2097 | 94 |
| | 4 hr. @ 4° C | 1020 | 83 | 199 | 106 | 2317 | 103 |
| | 24 hr. @ 4° C | 1030 | 84 | 229 | 122 | 1940 | 87 |

The results of this experiment demonstrated that the analytes associated with renal disorders tested were suitably stable over several freeze/thaw cycles, and up to 24 hrs of storage at a temperature of 4° C.

**Example 8: Diagnosis of Renal Damage Using Detection of Analytes in Human Plasma Samples**

To assess the effectiveness of a human kidney toxicity panel to detect renal damage due to disease states, the following experiment was conducted. Plasma samples were obtained from healthy control patients as well as patients experiencing acute kidney rejection (AR) or chronic allograft nephropathy (CAN). All plasma samples were diluted as described in Example 4 and subjected to a sandwich-capture assay as described in Example 1. Plasma concentrations of analytes included in a human kidney toxicity panel were measured by the assay, including alpha-1 microglobulin (A1M), beta-2 microglobulin (B2M), calbindin, clusterin, CTGF, cystatin C, GST-alpha, KIM-1, NGAL, osteopontin (OPN), THP, TIMP-1, TFF-3, and VEGF.

**FIG. 1** summarizes the plasma concentrations of those analytes that differed significantly from control plasma concentrations. The plasma analyte concentrations of the AR and CAN groups are expressed as a percentage difference relative to the corresponding control plasma analyte concentrations. The A1M plasma concentrations of the CAN and AR groups were both significantly lower than control concentrations. Plasma levels of B2M, KIM-1, NGAL, and OPN had changes between about 100% and 200% of control levels for both the CAN and AR groups. VEGF and Cyst C plasma levels were between about 30% and about 100% higher than control levels for both the CAN and AR groups.

The results of this experiment demonstrated that panels of analytes detected in plasma samples were capable of identifying patients having renal damage caused by CAN and AR conditions.

**Example 9: Analysis of the statistical importance of proteins associated with kidney transplant.**

To assess the statistical importance of the proteins associated with kidney transplant success, the following experiments were conducted. Six two-way comparisons were performed: TX vs. AR, TX vs. CAN, AR vs. CAN. TX vs. all other, AR vs. all other, and CAN vs. all other where TX= successful, non-rejected transplant, AR=acute rejection, and CAN=chronic allograft nephropathy. Two different sets of patient data were evaluated. Set characterics are in **Table 9** below.

## Table 9

|  | Set 1 | Set 2 | total |
| --- | --- | --- | --- |
| AR |  | 25 | 20 | 45 |
| CAN/IFTA |  | 25 | 48 | 73 |
| TX |  | 18 | 3 | 21 |
| acute dysfunction no rejection (ADNR) |  | 0 | 47 | 47 |
|  | 68 | 118 | 186 |

In **FIG. 2**, samples were clustered to check for batch effects. A moderate batch effect was identified. Because robust statistics are used to identify proteins associated with status, there is no attempt to remove outliers. All protein levels are scaled to mean zero and variance one to equalize their units. The resulting sample dendrogram is shown in **FIG. 2**. The sample dendrogram shows evidence of a moderate batch effect since samples tend to cluster together with other samples from the same data set.

In **FIGS. 4-9**, the statistical association of protein levels with status is studied. The following proteins were found to be related to clinical status at the level of 0.01:

Table 10

| Comparison | Significant Proteins |
| --- | --- |
| TX vs. AR | Beta.2.Microglobulin, BLC, CD40, IGF.BP.2, MMP.3, Peptide.YY, Stem.Cell.Factor, TNF.RII, VEGF |
| TX vs. CAN | AXL, Beta.2.Microglobulin, CD40, Eotaxin.3, FABP, FGF.basic, IGF.BP.2, MMP.3, Myoglobin, Resistin, Stem.Cell.Factor, TNF.RII, TRAIL.R3, VEGF |
| AR vs. CAN | None |
| TX vs. all Other | AXL, Beta.2.Microglobulin, BLC, CD40, Endothelin.1, Eotaxin.3, FABP, FGF.basic, IGF.BP.2, MMP.3, Myoglobin, NrCAM, Peptide.YY, Resistin, Stem.Cell.Factor, Tenascin.C, TNF.RII, TRAIL.R3, VCAM.1, VEGF |
| AR vs. all Other | None |
| CAN vs. all Other | Beta.2.Microglobulin, CD40, Cortisol, FGF.basic, Stem.Cell.Factor, TNF.RII, VEGF |

The necessary variables for calculation of protein significances were prepared and then the clinical traits for two-way comparisons were defined. One clinical trait is defined for each of the three comparisons TX vs. AR, TX vs. CAN, AR vs. CAN (in each case, the samples belonging to the third group are ignored), and for the comparisons TX vs. all others, AR vs. all others, and CAN vs. all others. The protein significances for each of the 6 "clinical traits" are checked to see how well they agree in the two data sets. The significance scatterplots are shown in **FIG. 3**. In the analysis of gene expression data it was found that subtracting several principal components from the full matrices of the expression data in Test and Validation sets improved the concordance of gene significance for status in the two data sets. Another reason to perform subtraction of principal components was that the histograms of association p-values exhibited anomalies suggesting that the data contained systematic bias(es) that may be removed by subtracting the first few principal components. No significant evidence of such anomalies in the protein data was found, however, and the concordance of protein significance in the Test and Validation data does not improve significantly upon subtracting principal components of the data. Hence, such a subtraction is not performed here.

For each protein and clinical trait, the following information is contained in **Table 11**: correlation with the trait in set 1, correlation with the trait in set 2, the corresponding Z scores in sets 1 and 2, a combined ("meta-analysis") Z score determined using the formula

$$Z = \frac{Z_1 + Z_2}{\sqrt{2}}, \tag{1}$$

p-values in set 1 and 2, and meta-analysis determined from the Z scores, and q-values in set 1, set 2, and meta-analysis determined from the corresponding p-values. The q-values are estimates of false discovery rate (FDR). All correlations reported in Table 10 are robust (that is, outlier resistant) biweight midcorrelations. The results are presented in graphical form in **FIGS. 4-9**.

Lastly, the statistical significance of the observed significant proteins was studied. For example, in the TX vs. AR comparison 9 proteins were found to be significant at the level of 0.01 or better in both data sets. Provided herein, in **FIG. 10** are the p-values for the null hypothesis that the protein significances in the two data sets are not related and the 9 proteins were significant in both sets by chance. The p-values were calculated in the plotting code above and are contained in the variable pTable. **FIG. 10** illustrates that most of the findings are highly significant.

The results of this experiment demonstrate that the three kidney transplant success options (AR, CAN, and TX) can be distinguished via a limited set of significant proteins differentially expressed between the three transplant options.

| probeID | cor.TX-vs-AR.Set_1 | cor.TX-vs-AR.Set_2 | Z.TX-vs-AR.Set_1 | Z.TX-vs-AR.Set_2 | Z.TX-vs-AR.Meta |
|---|---|---|---|---|---|
| Alpha.1.Antitrypsin | 0.19651782 | 0.544745877 | 1.365014973 | 3.863540936 | 3.697147339 |
| ACE..Angiotensin.Converting.Enzyme. | -0.056591672 | -0.039749567 | 0.388387932 | 0.251530867 | 0.452490922 |
| ACTH..Adrenocorticotropic.Hormone. | 0.029720353 | 0.223744359 | 0.203812495 | 1.439433005 | 1.161950036 |
| Adiponectin | 0.010447683 | 0.145646186 | 0.071628312 | 0.92774491 | 0.706663583 |
| AgRP..Agouti.related.Protein. | 0.213314725 | 0.234643509 | 1.485219539 | 1.51218797 | 2.119487175 |
| Alpha.2.Macroglobulin | -0.244305568 | 0.07626922 | 1.709442987 | 0.483307489 | 1.55050873 |
| Alpha.Fetoprotein | 0.044502555 | 0.062135881 | 0.3052958 | 0.39348874 | 0.494115287 |
| Amphiregulin | -0.032895242 | -0.095582587 | 0.225599813 | 0.60636848 | 0.588290422 |
| ANG.2..Angiopoietin.2. | 0.192265313 | 0.360428016 | 1.334716268 | 2.386746362 | 2.631471461 |
| Angiotensinogen | 0.189583743 | 0.190254475 | 1.315636851 | 1.218116867 | 1.791634436 |
| Apolipoprotein.A1 | -0.056282705 | -0.160553086 | 0.386262988 | 1.02428934 | 0.997411116 |
| Apolipoprotein.CIII | 0.140143385 | 0.425209453 | 0.967139742 | 2.871564279 | 2.714373645 |
| Apolipoprotein.H | -0.086880527 | -0.045498485 | 0.597128338 | 0.287956498 | 0.62584949 |
| AXL | 0.554118882 | 0.387462077 | 4.280025149 | 2.585543542 | 4.854690178 |
| Beta.2.Microglobulin | 0.603466542 | 0.596675835 | 4.789232561 | 4.351099666 | 6.4631909 |
| Betacellulin | -0.015920759 | -0.169877855 | 0.109156446 | 1.084919821 | 0.844339426 |
| BLC..B.Lymphocyte.Chemoattractant. | 0.370923325 | 0.415254861 | 2.670231546 | 2.795101853 | 3.864574308 |
| BMP.6 | 0.097069574 | -0.162008604 | 0.667577519 | 1.033740689 | 1.203013642 |
| BDNF..Brain.Derived.Neurotrophic.Factor. | -0.286713691 | -0.203614904 | 2.022294107 | 1.306026587 | 2.353478133 |
| Complement.3 | 0.045145682 | 0.269820563 | 0.309713726 | 1.749816529 | 1.45630781 |
| C.Reactive.Protein | 0.127880901 | 0.325559186 | 0.881533821 | 2.13676921 | 2.134262541 |
| Calcitonin | 0.250584789 | 0.418596757 | 1.755299073 | 2.820684937 | 3.235709324 |
| Cancer.Antigen.125 | -0.203022866 | -0.006483253 | 1.411465258 | 0.041004264 | 1.027051049 |
| Cancer.Antigen.19.9 | -0.114647199 | 0.205070411 | 0.789452655 | 1.315633155 | 1.488520451 |
| Carcinoembryonic.Antigen | 0.039388379 | 0.085216233 | 0.270172896 | 0.540265091 | 0.573066196 |
| CD40 | 0.381789854 | 0.687101268 | 2.757023753 | 5.328082139 | 5.717033203 |
| CD40.Ligand | -0.219592046 | 0.108533326 | 1.530370269 | 0.689139481 | 1.569430395 |
| CgA..Chromogranin.A. | 0.436070921 | 0.341187247 | 3.204119198 | 2.247971959 | 3.855210629 |
| CNTF..Ciliary.Neurotrophic.Factor. | 0.116635413 | -0.26836516 | 0.803267918 | 1.739893195 | 1.798286468 |
| Cortisol | -0.167877174 | 0.157496866 | 1.161906421 | 1.00445859 | 1.53185139 |
| Creatine.Kinase.MB | 0.13826237 | 0.116509942 | 0.953989327 | 0.740235241 | 1.197997681 |
| CTGF..Connective.Tissue.Growth.Factor. | 0.247052383 | 0.1578506 | 1.729483843 | 1.006752835 | 1.93481151 |
| EGF | -0.137649862 | 0.028490054 | 0.94970872 | 0.180235695 | 0.798991358 |
| EGF.R | -0.342512876 | -0.236710848 | 2.447034166 | 1.52603192 | 2.809381972 |

| | | | | | |
|---|---|---|---|---|---|
| ENA.78 | -0.163250369 | -0.108029871 | 1.129292592 | 0.685917574 | 1.283547418 |
| Endothelin.1 | 0.147454702 | 0.406768863 | 1.018322217 | 2.730521359 | 2.650832715 |
| EN.RAGE | 0.292001087 | 0.044015515 | 2.061855147 | 0.278558546 | 1.654922392 |
| Eotaxin | 0.029235107 | 0.068717319 | 0.200482927 | 0.435292511 | 0.449561124 |
| Eotaxin.3 | 0.2720196 | 0.565748629 | 1.913025953 | 4.05560777 | 4.22046138 |
| Epiregulin | 0.192670037 | -0.192386614 | 1.337597654 | 1.232114051 | 1.817060572 |
| Erythropoietin | -0.189360236 | -0.00534035 | 1.314047505 | 0.033775662 | 0.953054901 |
| Factor.VII | -0.017829181 | 0.02127869 | 0.122243658 | 0.13459857 | 0.181614881 |
| Fas | 0.395920739 | 0.12113867 | 2.871162164 | 0.769929203 | 2.574640396 |
| Fas.Ligand | 0.169381265 | -0.073827426 | 1.172519757 | 0.467776749 | 1.159864782 |
| FABP | 0.31695717 | 0.569528815 | 2.250442714 | 4.090881595 | 4.48399342 |
| Ferritin | 0.235522216 | 0.427630472 | 1.645549201 | 2.890279169 | 3.207314998 |

| probeID | p.TX-vs-AR.Set_1 | p.TX-vs-AR.Set_2 | p.TX-vs-AR.Meta | q.TX-vs-AR.Set_1 | q.TX-vs-AR.Set_2 | q.TX-vs-AR.Meta |
|---|---|---|---|---|---|---|
| Alpha.1.Antitrypsin | 0.171367015 | 0.000158886 | 0.000218036 | 0.177198839 | 0.001563766 | 0.000106 |
| ACE..Angiotensin.Converting.Enzyme. | 0.696273788 | 0.800210605 | 0.65091536 | 0.332945168 | 0.500045635 | 0.042832 |
| ACTH..Adrenocorticotropic.Hormone. | 0.837662442 | 0.149213752 | 0.245255761 | 0.366221249 | 0.204283128 | 0.022133 |
| Adiponectin | 0.942594229 | 0.351385527 | 0.479775566 | 0.382906795 | 0.321707586 | 0.035171 |
| AgRP..Agouti.related.Protein. | 0.136911446 | 0.129885276 | 0.034049316 | 0.152674292 | 0.182619764 | 0.005401 |
| Alpha.2.Macroglobulin | 0.087298478 | 0.626894456 | 0.12101946 | 0.112836688 | 0.440709451 | 0.013535 |
| Alpha.Fetoprotein | 0.758939672 | 0.692231429 | 0.621224762 | 0.34808227 | 0.465740328 | 0.042338 |
| Amphiregulin | 0.820592822 | 0.542056445 | 0.556337373 | 0.364597827 | 0.395181531 | 0.039139 |
| ANG.2..Angiopoietin.2. | 0.181009349 | 0.017584436 | 0.008501601 | 0.18060197 | 0.041955643 | 0.001766 |
| Angiotensinogen | 0.187286716 | 0.221700639 | 0.073191543 | 0.181041825 | 0.252984397 | 0.009121 |
| Apolipoprotein.A1 | 0.697852653 | 0.303721912 | 0.318564998 | 0.332945168 | 0.297054343 | 0.027245 |
| Apolipoprotein.CIII | 0.331697419 | 0.004477577 | 0.006640123 | 0.227793158 | 0.018555187 | 0.001448 |
| Apolipoprotein.H | 0.548548959 | 0.772036613 | 0.531413667 | 0.308880402 | 0.500045635 | 0.037998 |
| AXL | 2.98E-05 | 0.010257245 | 1.21E-06 | 0.000378445 | 0.027848938 | 1.75E-06 |
| Beta.2.Microglobulin | 3.50E-06 | 2.41E-05 | 1.03E-10 | 0.000199245 | 0.000632919 | 8.94E-10 |
| Betacellulin | 0.912618541 | 0.276119505 | 0.398479761 | 0.374357111 | 0.292391201 | 0.030762 |
| BLC..B.Lymphocyte.Chemoattractant. | 0.00800596 | 0.005622454 | 0.000111283 | 0.023963826 | 0.020122369 | 6.90E-05 |
| BMP.6 | 0.502468214 | 0.299301108 | 0.228971041 | 0.294600304 | 0.297054343 | 0.02162 |
| BDNF..Brain.Derived.Neurotrophic.Factor. | 0.043518996 | 0.19032556 | 0.018598702 | 0.075 | 0.23124681 | 0.003245 |
| Complement.3 | 0.755562875 | 0.080150828 | 0.145307548 | 0.34808227 | 0.129706391 | 0.015327 |
| C.Reactive.Protein | 0.376143256 | 0.03314323 | 0.032821296 | 0.245541216 | 0.069889967 | 0.005302 |

| | | | | | | |
|---|---|---|---|---|---|---|
| Calcitonin | 0.079223506 | 0.005212604 | 0.001213409 | 0.107275636 | 0.019870757 | 0.000407 |
| Cancer.Antigen.125 | 0.157346199 | 0.967087687 | 0.304396427 | 0.166042746 | 0.551302217 | 0.026291 |
| Cancer.Antigen.19.9 | 0.427898478 | 0.187108314 | 0.136613693 | 0.267421106 | 0.23124681 | 0.014713 |
| Carcinoembryonic.Antigen | 0.78594594 | 0.586906775 | 0.566599874 | 0.351953498 | 0.416314969 | 0.039324 |
| CD40 | 0.006221717 | 3.61E-07 | 1.08E-08 | 0.020543178 | 2.84E-05 | 3.15E-08 |
| CD40.Ligand | 0.12546158 | 0.488447891 | 0.116547686 | 0.14561664 | 0.367796104 | 0.013204 |
| CgA..Chromogranin.A. | 0.001547727 | 0.02515291 | 0.00011563 | 0.007335157 | 0.056584317 | 6.90E-05 |
| CNTF..Ciliary.Neurotrophic.Factor. | 0.419876518 | 0.081857321 | 0.072131623 | 0.265323312 | 0.129706391 | 0.009119 |
| Cortisol | 0.243881693 | 0.313139689 | 0.125559111 | 0.203970152 | 0.297054343 | 0.013865 |
| Creatine.Kinase.MB | 0.338292712 | 0.456856369 | 0.23091792 | 0.227793158 | 0.360957043 | 0.02162 |
| CTGF..Connective.Tissue.Growth.Factor. | 0.083690413 | 0.312040284 | 0.05301343 | 0.110688776 | 0.297054343 | 0.007226 |
| EGF | 0.340457785 | 0.856090208 | 0.424295423 | 0.227793158 | 0.526605563 | 0.032468 |
| EGF.R | 0.014899123 | 0.126440621 | 0.004963672 | 0.040349463 | 0.181008852 | 0.001203 |
| ENA.78 | 0.257307978 | 0.490479139 | 0.199300358 | 0.204286052 | 0.367796104 | 0.019318 |
| Endothelin.1 | 0.306833111 | 0.006791176 | 0.00802936 | 0.227793158 | 0.020565891 | 0.001708 |
| EN.RAGE | 0.039626912 | 0.779278196 | 0.097940241 | 0.072698402 | 0.500045635 | 0.011537 |
| Eotaxin | 0.840278075 | 0.661495266 | 0.653026925 | 0.366221249 | 0.456875397 | 0.042832 |
| Eotaxin.3 | 0.056001109 | 7.70E-05 | 2.44E-05 | 0.086356261 | 0.001212281 | 1.93E-05 |
| Epiregulin | 0.180075217 | 0.216468259 | 0.069207839 | 0.18060197 | 0.25064624 | 0.009011 |
| Erythropoietin | 0.18781687 | 0.972887263 | 0.340562255 | 0.181041825 | 0.551302217 | 0.028844 |
| Factor.VII | 0.902192375 | 0.892266152 | 0.855884969 | 0.374357111 | 0.533558766 | 0.054104 |
| Fas | 0.004426033 | 0.439044296 | 0.010034435 | 0.015765461 | 0.360091599 | 0.001989 |
| Fas.Ligand | 0.239621608 | 0.638000542 | 0.246103863 | 0.203970152 | 0.444547822 | 0.022133 |
| FABP | 0.024915474 | 6.72E-05 | 7.33E-06 | 0.052262132 | 0.001212281 | 7.92E-06 |
| Ferritin | 0.099655777 | 0.004232034 | 0.001339802 | 0.120587128 | 0.018511967 | 0.000433 |

| probeID | cor.TX-vs-CAN.Set_1 | cor.TX-vs-CAN.Set_2 | Z.TX-vs-CAN.Set_1 | Z.TX-vs-CAN.Set_2 | Z.TX-vs-CAN.Meta |
|---|---|---|---|---|---|
| Alpha.1.Antitrypsin | 0.403528206 | 0.286268152 | 2.933234801 | 1.862559632 | 3.391138765 |
| ACE..Angiotensin.Converting.Enzyme. | 0.249434132 | -0.139050564 | 1.746884618 | 0.885167625 | 1.86114199 |
| ACTH..Adrenocorticotropic.Hormone. | -0.075783974 | 0.314686872 | 0.520546819 | 2.060154139 | 1.824831148 |
| Adiponectin | 0.312608285 | 0.293823322 | 2.217348989 | 1.91473215 | 2.921822594 |
| AgRP..Agouti.related.Protein. | 0.312389911 | 0.275500502 | 2.215689871 | 1.788624921 | 2.831478143 |
| Alpha.2.Macroglobulin | 0.183884999 | 0.052359602 | 1.27515657 | 0.331454316 | 1.136045452 |
| Alpha.Fetoprotein | -0.006831019 | 0.037721445 | 0.046831837 | 0.238684616 | 0.201890621 |

| Amphiregulin | -0.007138139 | -0.081358506 | 0.048937449 | 0.515696219 | 0.399256295 |
|---|---|---|---|---|---|
| ANG.2..Angiopoietin.2. | 0.232399349 | 0.309748595 | 1.62290013 | 2.025548253 | 2.579842592 |
| Angiotensinogen | -0.057492856 | 0.170215517 | 0.394586302 | 1.087118979 | 1.047723852 |
| Apolipoprotein.A1 | 0.057734809 | 0.249520832 | 0.396250578 | 1.612140311 | 1.420146817 |
| Apolipoprotein.CIII | 0.074687063 | 0.420587018 | 0.512983964 | 2.835962121 | 2.368062487 |
| Apolipoprotein.H | -0.012802317 | -0.015198422 | 0.087773059 | 0.096130665 | 0.130039571 |
| AXL | 0.48432 | 0.4861663 | 3.623986832 | 3.35851425 | 4.937373865 |
| Beta.2.Microglobulin | 0.708882508 | 0.704831924 | 6.066801827 | 5.545613163 | 8.211217385 |
| Betacellulin | 0.25521347 | 0.238464408 | 1.789200026 | 1.537785902 | 2.352534311 |
| BLC..B.Lymphocyte.Chemoattractant. | 0.231502704 | 0.494793252 | 1.616403509 | 3.430361128 | 3.568601498 |
| BMP.6 | 0.365975701 | 0.113504072 | 2.630984183 | 0.72096962 | 2.370189265 |
| BDNF..Brain.Derived.Neurotrophic.Factor. | -0.469665334 | -0.018371957 | 3.493921767 | 0.116207535 | 2.55274691 |
| Complement.3 | -0.604283485 | 0.08099202 | 4.798047873 | 0.513362986 | 3.755734637 |
| C.Reactive.Protein | 0.000771532 | 0.159532746 | 0.005289357 | 1.017666508 | 0.723339029 |
| Calcitonin | 0.183886745 | 0.187188875 | 1.275168955 | 1.19801225 | 1.748803201 |
| Cancer.Antigen.125 | -0.130092925 | 0.115908416 | 0.896955278 | 0.736378779 | 1.154941588 |
| Cancer.Antigen.19.9 | -0.178600465 | 0.167765145 | 1.237697025 | 1.07116587 | 1.63261261 |
| Carcinoembryonic.Antigen | 0.423344093 | 0.366571377 | 3.09710585 | 2.431516135 | 3.909326096 |
| CD40 | 0.647728239 | 0.720128266 | 5.288280108 | 5.742135675 | 7.7996818 |
| CD40.Ligand | -0.254880457 | 0.183465098 | 1.786758159 | 1.173623322 | 2.09330582 |
| CgA..Chromogranin.A. | 0.577392073 | 0.333524738 | 4.514734138 | 2.193285808 | 4.743286392 |
| CNTF..Ciliary.Neurotrophic.Factor. | 0.086442548 | -0.013245829 | 0.594102988 | 0.083778879 | 0.479334865 |
| Cortisol | -0.478326963 | -0.23121892 | 3.570511538 | 1.489286369 | 3.577817412 |
| Creatine.Kinase.MB | 0.269727283 | 0.051127151 | 1.896066231 | 0.323638689 | 1.569568401 |
| CTGF..Connective.Tissue.Growth.Factor. | 0.270754049 | -0.092497474 | 1.903659952 | 0.586682401 | 1.760937966 |
| EGF | -0.0140503 | 0.159857205 | 0.096330346 | 1.019772265 | 0.789203724 |
| EGF.R | -0.190238772 | -0.189118241 | 1.320295523 | 1.210662477 | 1.789657565 |
| ENA.78 | -0.170335194 | 0.012874577 | 1.179253854 | 0.081430475 | 0.891438438 |
| Endothelin.1 | 0.461212433 | 0.318755037 | 3.419944788 | 2.088752274 | 3.895237048 |
| EN.RAGE | 0.183565096 | 0.128343284 | 1.272886808 | 0.816215627 | 1.477218499 |
| Eotaxin | 0.08500313 | 0.083306843 | 0.584161781 | 0.528102688 | 0.786489749 |
| Eotaxin.3 | 0.472591422 | 0.549187985 | 3.519704939 | 3.903628615 | 5.249089495 |
| Epiregulin | 0.364310979 | -0.169763681 | 2.61781567 | 1.084176283 | 2.617703614 |
| Erythropoietin | -0.021159826 | 0.106709643 | 0.145086112 | 0.677470332 | 0.581635239 |
| Factor.VII | 0.280903304 | 0.101899999 | 1.978970326 | 0.646716832 | 1.856641194 |
| Fas | 0.556635954 | 0.247374844 | 4.304977883 | 1.597674992 | 4.173805875 |

| Fas.Ligand | 0.416300541 | 0.107624193 | 3.038485324 | 0.683321657 | 2.831714955 |
|---|---|---|---|---|---|
| FABP | 0.516340296 | 0.536936453 | 3.916887217 | 3.79372791 | 5.452228244 |
| Ferritin | 0.106030964 | 0.199676933 | 0.729654311 | 1.280065005 | 1.421086156 |

| probeID | p.TX-vs-CAN.Set_1 | p.TX-vs-CAN.Set_2 | p.TX-vs-CAN.Meta | q.TX-vs-CAN.Set_1 | q.TX-vs-CAN.Set_2 | q.TX-vs-CAN.Meta |
|---|---|---|---|---|---|---|
| Alpha.1.Antitrypsin | 0.003662189 | 0.062739931 | 0.000696029 | 0.007837826 | 0.082680224 | 0.000167 |
| ACE..Angiotensin.Converting.Enzyme. | 0.080657463 | 0.373853681 | 0.062724128 | 0.092951011 | 0.246336788 | 0.00693 |
| ACTH..Adrenocorticotropic.Hormone. | 0.600923403 | 0.039852544 | 0.068026514 | 0.39833304 | 0.059364258 | 0.007256 |
| Adiponectin | 0.027085358 | 0.055824398 | 0.003479897 | 0.038440557 | 0.076910709 | 0.000683 |
| AgRP..Agouti.related.Protein. | 0.027198354 | 0.073755746 | 0.00463334 | 0.038440557 | 0.095129113 | 0.00089 |
| Alpha.2.Macroglobulin | 0.201140587 | 0.73878983 | 0.255937542 | 0.171894045 | 0.370044586 | 0.019229 |
| Alpha.Fetoprotein | 0.962448347 | 0.810211967 | 0.840002235 | 0.522421762 | 0.377853768 | 0.052593 |
| Amphiregulin | 0.960761329 | 0.604009183 | 0.689704367 | 0.522421762 | 0.321288138 | 0.044142 |
| ANG.2..Angiopoietin.2. | 0.104360888 | 0.043246653 | 0.009884536 | 0.11156188 | 0.062419265 | 0.001675 |
| Angiotensinogen | 0.691675916 | 0.275151949 | 0.294765855 | 0.431761907 | 0.206125327 | 0.021583 |
| Apolipoprotein.A1 | 0.690443323 | 0.106605017 | 0.155564943 | 0.431761907 | 0.129247776 | 0.013577 |
| Apolipoprotein.CIII | 0.606217218 | 0.004981073 | 0.017881516 | 0.39833304 | 0.015097616 | 0.002628 |
| Apolipoprotein.H | 0.929685968 | 0.922939664 | 0.89653512 | 0.519702006 | 0.407633958 | 0.055728 |
| AXL | 0.00036546 | 0.000948795 | 7.92E-07 | 0.001244342 | 0.004424299 | 4.89E-07 |
| Beta.2.Microglobulin | 8.38E-09 | 1.32E-07 | 2.19E-16 | 2.09E-07 | 2.67E-06 | 9.46E-16 |
| Betacellulin | 0.073656939 | 0.123572863 | 0.018645969 | 0.086666091 | 0.144057278 | 0.002641 |
| BLC..B.Lymphocyte.Chemoattractant. | 0.10574291 | 0.000704778 | 0.000358892 | 0.11156188 | 0.003757316 | 9.12E-05 |
| BMP.6 | 0.008955433 | 0.468629468 | 0.017778982 | 0.016361596 | 0.289880472 | 0.002628 |
| BDNF..Brain.Derived.Neurotrophic.Factor. | 0.000579461 | 0.906913337 | 0.010687713 | 0.001669452 | 0.404242826 | 0.001742 |
| Complement.3 | 3.37E-06 | 0.605645086 | 0.000172834 | 1.80E-05 | 0.321288138 | 5.15E-05 |
| C.Reactive.Protein | 0.995757217 | 0.306845734 | 0.46947162 | 0.53505063 | 0.216290319 | 0.032193 |
| Calcitonin | 0.201136236 | 0.229375635 | 0.080325043 | 0.171894045 | 0.193127626 | 0.008165 |
| Cancer.Antigen.125 | 0.367873357 | 0.459199476 | 0.248114347 | 0.272834871 | 0.286975678 | 0.018971 |
| Cancer.Antigen.19.9 | 0.21462049 | 0.282224043 | 0.102550497 | 0.18063591 | 0.206125327 | 0.009845 |
| Carcinoembryonic.Antigen | 0.002189573 | 0.015616193 | 9.26E-05 | 0.005290792 | 0.032643191 | 3.03E-05 |
| CD40 | 3.69E-07 | 5.23E-08 | 6.21E-15 | 3.95E-06 | 1.58E-06 | 1.34E-14 |
| CD40.Ligand | 0.074046795 | 0.238940751 | 0.036321863 | 0.086666091 | 0.193127626 | 0.004551 |
| CgA..Chromogranin.A. | 1.13E-05 | 0.028842796 | 2.10E-06 | 5.31E-05 | 0.049955667 | 1.14E-06 |
| CNTF..Ciliary.Neurotrophic.Factor. | 0.550574078 | 0.932815641 | 0.631700423 | 0.375560341 | 0.407633958 | 0.040732 |
| Cortisol | 0.000442371 | 0.135745025 | 0.000346475 | 0.001380698 | 0.146793727 | 9.07E-05 |

| | | | | | | |
|---|---|---|---|---|---|---|
| Creatine.Kinase.MB | 0.058185878 | 0.744729306 | 0.116515555 | 0.073873469 | 0.370044586 | 0.01071 |
| CTGF..Connective.Tissue.Growth.Factor. | 0.057198956 | 0.555230637 | 0.078248903 | 0.073872538 | 0.314561151 | 0.008049 |
| EGF | 0.922851486 | 0.305850175 | 0.429992945 | 0.519702006 | 0.216290319 | 0.029832 |
| EGF.R | 0.185739167 | 0.224524361 | 0.073508978 | 0.165633097 | 0.193127626 | 0.007746 |
| ENA.78 | 0.236946251 | 0.93469456 | 0.372694004 | 0.190799565 | 0.407633958 | 0.02618 |
| Endothelin.1 | 0.000749031 | 0.037222795 | 9.81E-05 | 0.002078064 | 0.057945364 | 3.03E-05 |
| EN.RAGE | 0.20193918 | 0.412099983 | 0.139617077 | 0.171894045 | 0.268617986 | 0.012436 |
| Eotaxin | 0.557254626 | 0.595344675 | 0.431580622 | 0.37605695 | 0.321288138 | 0.029832 |
| Eotaxin.3 | 0.000529376 | 0.000136858 | 1.53E-07 | 0.00158616 | 0.001185187 | 1.32E-07 |
| Epiregulin | 0.009296074 | 0.276447168 | 0.008852366 | 0.016579567 | 0.206125327 | 0.00153 |
| Erythropoietin | 0.884036307 | 0.495826451 | 0.560812401 | 0.501671103 | 0.291815199 | 0.037273 |
| Factor.VII | 0.048150986 | 0.515557342 | 0.063362219 | 0.063278073 | 0.295470986 | 0.00693 |
| Fas | 2.70E-05 | 0.109750897 | 3.00E-05 | 0.000118763 | 0.130452782 | 1.13E-05 |
| Fas.Ligand | 0.00263811 | 0.49211907 | 0.008495511 | 0.006026802 | 0.291815199 | 0.001498 |
| FABP | 0.000123838 | 0.000205523 | 4.97E-08 | 0.000488227 | 0.001557347 | 5.37E-08 |
| Ferritin | 0.463644768 | 0.199226142 | 0.155291714 | 0.330649678 | 0.182985986 | 0.013577 |

| probeID | cor.AR-vs-CAN.Set_1 | cor.AR-vs-CAN.Set_2 | Z.AR-vs-CAN.Set_1 | Z.AR-vs-CAN.Set_2 | Z.AR-vs-CAN.Meta |
|---|---|---|---|---|---|
| Alpha.1.Antitrypsin | 0.136992841 | -0.14875832 | 0.945117837 | 1.027459807 | 1.394823029 |
| ACE..Angiotensin.Converting.Enzyme. | 0.232155898 | -0.097879991 | 1.621135924 | 0.673186751 | 1.622331122 |
| ACTH..Adrenocorticotropic.Hormone. | -0.169924977 | 0.124075593 | 1.176357722 | 0.855025205 | 1.436404643 |
| Adiponectin | 0.330238709 | 0.178540172 | 2.352148761 | 1.237270059 | 2.538102388 |
| AgRP..Agouti.related.Protein. | 0.253734302 | 0.093056001 | 1.778357211 | 0.639810884 | 1.709903059 |
| Alpha.2.Macroglobulin | 0.431188103 | -0.033871249 | 3.162891986 | 0.23229845 | 2.40076218 |
| Alpha.Fetoprotein | -0.06999934 | -0.010748584 | 0.480677417 | 0.073691417 | 0.391997961 |
| Amphiregulin | 0.050159657 | -0.027988775 | 0.344166116 | 0.1919315 | 0.37907826 |
| ANG.2..Angiopoietin.2. | 0.055405474 | 0.013407126 | 0.380230183 | 0.091920133 | 0.33386069 |
| Angiotensinogen | -0.251895593 | 0.03412023 | 1.764890946 | 0.234007352 | 1.413434541 |
| Apolipoprotein.A1 | 0.120303498 | 0.367502109 | 0.828773036 | 2.643074881 | 2.454967206 |
| Apolipoprotein.CIII | -0.07300405 | -0.010749203 | 0.501382539 | 0.073695664 | 0.406641697 |
| Apolipoprotein.H | 0.043364499 | 0.039796017 | 0.297478586 | 0.272971913 | 0.403369416 |
| AXL | 0.033240726 | 0.125177011 | 0.227970923 | 0.862695276 | 0.771217466 |
| Beta.2.Microglobulin | 0.1402783 | 0.254781493 | 0.968083226 | 1.786032577 | 1.94745396 |
| Betacellulin | 0.225867951 | 0.253082628 | 1.575641742 | 1.773582976 | 2.36825951 |

| | | | | | |
|---|---|---|---|---|---|
| BLC..B.Lymphocyte.Chemoattractant. | -0.074356718 | 0.090476337 | 0.510706586 | 0.621975394 | 0.800927109 |
| BMP.6 | 0.353408403 | 0.245916539 | 2.532021413 | 1.721193193 | 3.007476889 |
| BDNF..Brain.Derived.Neurotrophic.Factor. | -0.120532581 | 0.169252235 | 0.830366659 | 1.171609057 | 1.415610604 |
| Complement.3 | -0.556801163 | -0.285627368 | 4.306619204 | 2.014182349 | 4.469481641 |
| C.Reactive.Protein | -0.144505591 | -0.217864375 | 0.997663879 | 1.517930818 | 1.778794069 |
| Calcitonin | -0.019739972 | -0.204916753 | 0.135348013 | 1.425012717 | 1.103341653 |
| Cancer.Antigen.125 | 0.083343568 | 0.151902623 | 0.572703209 | 1.049514502 | 1.147081144 |
| Cancer.Antigen.19.9 | -0.061310956 | -0.045948962 | 0.420854605 | 0.315232191 | 0.520491965 |
| Carcinoembryonic.Antigen | 0.316075125 | 0.248096186 | 2.243722466 | 1.737107046 | 2.814871543 |
| CD40 | 0.113278859 | 0.104709925 | 0.77994835 | 0.720496039 | 1.060974402 |
| CD40.Ligand | 0.001134959 | 0.083705309 | 0.007780892 | 0.575200599 | 0.412230166 |
| CgA..Chromogranin.A. | 0.193956508 | 0.069870528 | .346759641 | 0.479789494 | 1.291565627 |
| CNTF..Ciliary.Neurotrophic.Factor. | -0.052357978 | 0.233851704 | 0.359276756 | 1.633429205 | 1.409055898 |
| Cortisol | -0.223298671 | -0.442328039 | 1.55709203 | 3.257268465 | 3.404266953 |
| Creatine.Kinase.MB | 0.100819535 | -0.118353967 | 0.69354016 | 0.815214661 | 1.066850765 |
| CTGF..Connective.Tissue.Growth.Factor. | 0.012853541 | -0.030586603 | 0.088124288 | 0.209756617 | 0.210633608 |
| EGF | 0.131457611 | 0.284173355 | 0.906473883 | 2.003333557 | 2.057544572 |
| EGF.R | 0.135750136 | 0.037557578 | 0.936436835 | 0.257602948 | 0.844313628 |
| ENA.78 | 0.021290661 | 0.21978659 | 0.145983481 | 1.531771632 | 1.186352018 |
| Endothelin.1 | 0.278121163 | 0.010337303 | 1.958280469 | 0.070871504 | 1.43482712 |
| EN.RAGE | -0.270341451 | 0.134778632 | 1.900607929 | 0.929652409 | 2.001296278 |
| Eotaxin | 0.055013877 | 0.006065001 | 0.37753732 | 0.04158006 | 0.296360741 |
| Eotaxin.3 | 0.229327065 | 0.043924253 | 1.600651801 | 0.301323395 | 1.344899559 |
| Epiregulin | 0.138407422 | 0.036299336 | 0.955003154 | 0.2489661 | 0.851334117 |
| Erythropoietin | 0.192826 | 0.079081482 | 1.338708136 | 0.543289776 | 1.330773486 |
| Factor.VII | 0.329855758 | 0.099473564 | 2.349202438 | 0.684219165 | 2.144952985 |
| Fas | 0.173030867 | 0.081557022 | 1.198295729 | 0.560371431 | 1.243565475 |
| Fas.Ligand | 0.246060385 | 0.147903054 | 1.722242865 | 1.021464491 | 1.940094077 |
| FABP | 0.350453011 | 0.034267974 | 2.508896037 | 0.235021418 | 1.94024264 |
| Ferritin | -0.170380908 | -0.124416508 | 1.179576616 | 0.857399044 | 1.440359303 |

| probeID | p.AR-vs-CAN.Set_1 | p.AR-vs-CAN.Set_2 | p.AR-vs-CAN.Meta | q.AR-vs-CAN.Set_1 | q.AR-vs-CAN.Set_2 | q.AR-vs-CAN.Meta |
|---|---|---|---|---|---|---|
| Alpha.1.Antitrypsin | 0.342789738 | 0.302528826 | 0.163069213 | 0.417256265 | 0.718387019 | 0.012314 |
| ACE..Angiotensin.Converting.Enzyme. | 0.104734745 | 0.498890163 | 0.104732468 | 0.253213935 | 0.797303461 | 0.010453 |

| | | | | | |
|---|---|---|---|---|---|
| ACTH..Adrenocorticotropic.Hormone. | 0.238094216 | 0.390627196 | 0.150887233 | 0.393405488 | 0.718387019 | 0.012314 |
| Adiponectin | 0.019169947 | 0.214777839 | 0.011145537 | 0.119167562 | 0.707339939 | 0.003576 |
| AgRP..Agouti.related.Protein. | 0.075401087 | 0.5203802 | 0.087283801 | 0.218272788 | 0.821380598 | 0.009256 |
| Alpha.2.Macroglobulin | 0.001770857 | 0.815361479 | 0.016360966 | 0.027309377 | 0.865859107 | 0.003955 |
| Alpha.Fetoprotein | 0.629064408 | 0.940943787 | 0.69505972 | 0.54399002 | 0.886846611 | 0.027007 |
| Amphiregulin | 0.729395862 | 0.847003984 | 0.70462975 | 0.592022265 | 0.866331559 | 0.027171 |
| ANG.2..Angiopoietin.2. | 0.702342308 | 0.926373122 | 0.738484684 | 0.581902126 | 0.886846611 | 0.027883 |
| Angiotensinogen | 0.077614516 | 0.814028221 | 0.157527995 | 0.218272788 | 0.865859107 | 0.012314 |
| Apolipoprotein.A1 | 0.405303538 | 0.008652634 | 0.014089744 | 0.446458098 | 0.348403945 | 0.003955 |
| Apolipoprotein.CIII | 0.614378627 | 0.94094039 | 0.684271167 | 0.54399002 | 0.886846611 | 0.027007 |
| Apolipoprotein.H | 0.764926039 | 0.7837837 | 0.686676482 | 0.599814602 | 0.865859107 | 0.027007 |
| AXL | 0.818740153 | 0.386401575 | 0.440578043 | 0.626095116 | 0.718387019 | 0.020733 |
| Beta.2.Microglobulin | 0.331227487 | 0.074162966 | 0.051480334 | 0.417256265 | 0.554575803 | 0.006835 |
| Betacellulin | 0.114751891 | 0.076179789 | 0.017871995 | 0.253213935 | 0.554575803 | 0.003955 |
| BLC..B.Lymphocyte.Chemoattractant. | 0.607815447 | 0.532058689 | 0.423173845 | 0.54399002 | 0.829572568 | 0.020321 |
| BMP.6 | 0.011816011 | 0.085167951 | 0.002634262 | 0.097098932 | 0.554575803 | 0.001341 |
| BDNF..Brain.Derived.Neurotrophic.Factor. | 0.404403234 | 0.23998506 | 0.156889545 | 0.446458098 | 0.707339939 | 0.012314 |
| Complement.3 | 2.68E-05 | 0.044356141 | 7.84E-06 | 0.001238694 | 0.554575803 | 3.99E-05 |
| C.Reactive.Protein | 0.316714685 | 0.128538176 | 0.07527353 | 0.417256265 | 0.566686451 | 0.008329 |
| Calcitonin | 0.891769567 | 0.153427033 | 0.269878824 | 0.63965019 | 0.618687053 | 0.015435 |
| Cancer.Antigen.125 | 0.565004005 | 0.292307793 | 0.251348086 | 0.520281791 | 0.718387019 | 0.014706 |
| Cancer.Antigen.19.9 | 0.67231939 | 0.751351518 | 0.602720727 | 0.576011929 | 0.865859107 | 0.025147 |
| Carcinoembryonic.Antigen | 0.025343376 | 0.082350396 | 0.004879673 | 0.123421399 | 0.554575803 | 0.001911 |
| CD40 | 0.433469212 | 0.460264154 | 0.288701534 | 0.459053854 | 0.789427922 | 0.015801 |
| CD40.Ligand | 0.993758703 | 0.563310577 | 0.680170729 | 0.661673607 | 0.841835746 | 0.027007 |
| CgA..Chromogranin.A. | 0.177128993 | 0.629697231 | 0.196507614 | 0.339367833 | 0.85129144 | 0.013142 |
| CNTF..Ciliary.Neurotrophic.Factor. | 0.718016792 | 0.102151863 | 0.15881864 | 0.587943717 | 0.554575803 | 0.012314 |
| Cortisol | 0.119048819 | 0.001298593 | 0.000663419 | 0.256174698 | 0.166028116 | 0.000844 |
| Creatine.Kinase.MB | 0.486022727 | 0.413012072 | 0.286039231 | 0.48356318 | 0.733396688 | 0.015801 |
| CTGF..Connective.Tissue.Growth.Factor. | 0.929405344 | 0.832997399 | 0.833173185 | 0.645312732 | 0.865859107 | 0.03051 |
| EGF | 0.362826467 | 0.045497182 | 0.039633872 | 0.427173306 | 0.554575803 | 0.006304 |
| EGF.R | 0.347227634 | 0.795676579 | 0.398494173 | 0.417256265 | 0.865859107 | 0.019503 |
| ENA.78 | 0.883324234 | 0.125118652 | 0.235483312 | 0.638542447 | 0.566686451 | 0.014617 |
| Endothelin.1 | 0.050506748 | 0.943199723 | 0.151336368 | 0.186934299 | 0.886846611 | 0.012314 |
| EN.RAGE | 0.05759391 | 0.350721153 | 0.045360471 | 0.197375114 | 0.718387019 | 0.006601 |
| Eotaxin | 0.704349759 | 0.9666568 | 0.766954603 | 0.581902126 | 0.892409011 | 0.028289 |

| | | | | | |
|---|---|---|---|---|---|
| Eotaxin.3 | 0.109154691 | 0.761979913 | 0.178657673 | 0.253213935 | 0.865859107 | 0.012673 |
| Epiregulin | 0.337781243 | 0.802382069 | 0.394583778 | 0.417256265 | 0.865859107 | 0.0195 |
| Erythropoietin | 0.179716172 | 0.585132507 | 0.183263555 | 0.339367833 | 0.841835746 | 0.012673 |
| Factor.VII | 0.019318336 | 0.49189285 | 0.031956606 | 0.119167562 | 0.796071569 | 0.005887 |
| Fas | 0.229496835 | 0.573401992 | 0.213659529 | 0.393244392 | 0.841835746 | 0.013656 |
| Fas.Ligand | 0.08497971 | 0.305348334 | 0.052368258 | 0.21841997 | 0.718387019 | 0.006835 |
| FABP | 0.01259262 | 0.81323732 | 0.052350209 | 0.097098932 | 0.865859107 | 0.006835 |
| Ferritin | 0.236818561 | 0.389316371 | 0.149765771 | 0.393405488 | 0.718387019 | 0.012314 |

| probeID | cor.TX-vs-allOther.Set_1 | cor.TX-vs-allOther.Set_2 | Z.TX-vs-allOther.Set_1 | Z.TX-vs-allOther.Set_2 | Z.TX-vs-allOther.Meta |
|---|---|---|---|---|---|
| Alpha.1.Antitrypsin | 0.279845384 | 0.37852713 | 2.439639828 | 3.211514328 | 3.995969425 |
| ACE..Angiotensin.Converting.Enzyme. | 0.096793335 | -0.07453388 | 0.823898167 | 0.60202782 | 1.008281935 |
| ACTH..Adrenocorticotropic.Hormone. | -0.008857328 | 0.243255635 | 0.075158883 | 2.001307259 | 1.46828329 |
| Adiponectin | 0.150760242 | 0.193249538 | 1.289069248 | 1.577869157 | 2.027231587 |
| AgRP..Agouti.related.Protein. | 0.244110103 | 0.218743366 | 2.114023215 | 1.792529679 | 2.762350042 |
| Alpha.2.Macroglobulin | -0.029520198 | 0.053719263 | 0.250559987 | 0.433514073 | 0.483714823 |
| Alpha.Fetoprotein | 0.013300076 | 0.0420251 | 0.112861545 | 0.339016865 | 0.319526288 |
| Amphiregulin | -0.021654836 | -0.106406644 | 0.183776104 | 0.8611377 | 0.738865637 |
| ANG.2..Angiopoietin.2. | 0.200518646 | 0.311350656 | 1.724827486 | 2.596372357 | 3.055549712 |
| Angiotensinogen | 0.06674839 | 0.164801807 | 0.567222262 | 1.340903321 | 1.349248539 |
| Apolipoprotein.A1 | -0.005345643 | 0.046367386 | 0.045359717 | 0.374094066 | 0.296598614 |
| Apolipoprotein.CIII | 0.101490396 | 0.362351782 | 0.864149763 | 3.060356401 | 2.775044921 |
| Apolipoprotein.H | -0.053080003 | -0.025333444 | 0.450822475 | 0.204288469 | 0.463233391 |
| AXL | 0.461681682 | 0.376089168 | 4.23794338 | 3.188596164 | 5.251356472 |
| Beta.2.Microglobulin | 0.605006599 | 0.578746564 | 5.948242023 | 5.325733403 | 7.971904475 |
| Betacellulin | 0.105269722 | 0.092410471 | 0.896564881 | 0.747168765 | 1.162295208 |
| BLC..B.Lymphocyte.Chemoattractant. | 0.297784477 | 0.411667496 | 2.605717357 | 3.528183612 | 4.337322971 |
| BMP.6 | 0.180999006 | -0.010129333 | 1.552936642 | 0.081668085 | 1.155840087 |
| BDNF..Brain.Derived.Neurotrophic.Factor. | -0.349230415 | -0.092979006 | 3.093453605 | 0.751792171 | 2.718999363 |
| Complement.3 | -0.2706015 | 0.17998084 | 2.354773631 | 1.467031696 | 2.702424464 |
| C.Reactive.Protein | 0.05320534 | 0.208196643 | 0.451889004 | 1.703438464 | 1.524046668 |
| Calcitonin | 0.234709966 | 0.250195483 | 2.029409834 | 2.060885113 | 2.892275294 |
| Cancer.Antigen.125 | -0.155863586 | 0.058945623 | 1.333414996 | 0.475786373 | 1.279298556 |
| Cancer.Antigen.19.9 | -0.133090685 | 0.163958853 | 1.136051568 | 1.333918472 | 1.746532565 |

| | | | | | |
|---|---|---|---|---|---|
| Carcinoembryonic.Antigen | 0.2251489 | 0.220092828 | 1.943752193 | 1.803959686 | 2.650032484 |
| CD40 | 0.474698782 | 0.598050125 | 4.379411479 | 5.563812856 | 7.030921354 |
| CD40.Ligand | -0.222090096 | 0.129578406 | 1.916431141 | 1.050601135 | 2.098008642 |
| CgA..Chromogranin.A. | 0.471759454 | 0.298897829 | 4.347273166 | 2.485665551 | 4.831617302 |
| CNTF..Ciliary.Neurotrophic.Factor. | 0.089789189 | -0.13317264 | 0.763943961 | 1.08008773 | 1.303927314 |
| Cortisol | -0.307729535 | -0.04205582 | 2.698620745 | 0.339264977 | 2.148109594 |
| Creatine.Kinase.MB | 0.20812614 | 0.072874268 | 1.792191902 | 0.588574521 | 1.683456082 |
| CTGF..Connective.Tissue.Growth.Factor. | 0.232700389 | 0.019731599 | 2.011372848 | 0.159101889 | 1.534757405 |
| EGF | -0.08973474 | 0.025948738 | 0.763478194 | 0.209252389 | 0.687824392 |
| EGF.R | -0.25408677 | -0.19976983 | 2.204279389 | 1.617439541 | 2.702363371 |
| ENA.78 | -0.154268945 | -0.062454233 | 1.319550638 | 0.504178328 | 1.289571119 |
| Endothelin.1 | 0.298151046 | 0.328411061 | 2.609130868 | 2.749602207 | 3.789196496 |
| EN.RAGE | 0.200737016 | 0.084824994 | 1.726758132 | 0.685528324 | 1.705744112 |
| Eotaxin | 0.049731896 | 0.067289204 | 0.422337541 | 0.543323925 | 0.682825771 |
| Eotaxin.3 | 0.350311634 | 0.499530074 | 3.103906907 | 4.423694432 | 5.322817953 |
| Epiregulin | 0.255240675 | -0.183283852 | 2.214749624 | 1.494569957 | 2.622885029 |
| Erythropoietin | -0.103689303 | 0.039769296 | 0.883006573 | 0.32079951 | 0.851219444 |
| Factor.VII | 0.130171342 | 0.046297232 | 1.110843322 | 0.37352725 | 1.049608497 |
| Fas | 0.432201918 | 0.192532372 | 3.925301664 | 1.571863743 | 3.887082936 |
| Fas.Ligand | 0.285196169 | 0.007882392 | 2.488981239 | 0.063551189 | 1.804912989 |
| FABP | 0.400382262 | 0.476220161 | 3.598642512 | 4.176929622 | 5.498159783 |
| Ferritin | 0.147124719 | 0.309648215 | 1.257520911 | 2.581182369 | 2.71437312 |

| probeID | p.TX-vs-allOther.Set_1 | p.TX-vs-allOther.Set_2 | p.TX-vs-allOther.Meta | q.TX-vs-allOther.Set_1 | q.TX-vs-allOther.Set_2 | q.TX-vs-allOther.Meta |
|---|---|---|---|---|---|---|
| Alpha.1.Antitrypsin | 0.015031282 | 0.001458204 | 6.44E-05 | 0.029332251 | 0.004336026 | 1.16E-06 |
| ACE..Angiotensin.Converting.Enzyme. | 0.408737237 | 0.545798246 | 0.313319133 | 0.307990131 | 0.321376701 | 0.001164 |
| ACTH..Adrenocorticotropic.Hormone. | 0.939880702 | 0.045621081 | 0.142027288 | 0.525954037 | 0.057739158 | 0.000658 |
| Adiponectin | 0.19667374 | 0.114346203 | 0.042638734 | 0.196945813 | 0.109239885 | 0.000266 |
| AgRP..Agouti.related.Protein. | 0.034807476 | 0.073111562 | 0.005738692 | 0.055188078 | 0.080518547 | 5.62E-05 |
| Alpha.2.Macroglobulin | 0.801494611 | 0.663502761 | 0.628588272 | 0.487982854 | 0.34919492 | 0.002099 |
| Alpha.Fetoprotein | 0.90983043 | 0.733650722 | 0.74932746 | 0.517096004 | 0.357651942 | 0.002339 |
| Amphiregulin | 0.8536933 | 0.387786017 | 0.45998859 | 0.503646861 | 0.250673222 | 0.001638 |
| ANG.2..Angiopoietin.2. | 0.084537923 | 0.009753545 | 0.002246484 | 0.102123336 | 0.017895869 | 2.48E-05 |
| Angiotensinogen | 0.569366912 | 0.179272495 | 0.177257149 | 0.388933497 | 0.149947391 | 0.000777 |
| Apolipoprotein.A1 | 0.963694925 | 0.707312058 | 0.766772969 | 0.531464737 | 0.356691103 | 0.002376 |

| | | | | | | |
|---|---|---|---|---|---|---|
| Apolipoprotein.CIII | 0.38626523 | 0.00239305 | 0.005519408 | 0.29396768 | 0.006468933 | 5.53E-05 |
| Apolipoprotein.H | 0.651064027 | 0.837521539 | 0.643197084 | 0.416380812 | 0.382039757 | 0.002115 |
| AXL | 3.06E-05 | 0.00157379 | 1.51E-07 | 0.000193849 | 0.004456883 | 5.42E-09 |
| Beta.2.Microglobulin | 8.97E-09 | 2.35E-07 | 1.56E-15 | 6.04E-07 | 3.49E-06 | 6.73E-16 |
| Betacellulin | 0.368731918 | 0.453547364 | 0.245115573 | 0.286350948 | 0.280966823 | 0.000987 |
| BLC..B.Lymphocyte.Chemoattractant. | 0.009466628 | 0.000487057 | 1.44E-05 | 0.020584543 | 0.002602755 | 3.27E-07 |
| BMP.6 | 0.120175908 | 0.934660958 | 0.247746573 | 0.13650752 | 0.408713347 | 0.000987 |
| BDNF..Brain.Derived.Neurotrophic.Factor. | 0.002133873 | 0.4507619 | 0.006547973 | 0.006246105 | 0.280966823 | 6.05E-05 |
| Complement.3 | 0.018869074 | 0.141923213 | 0.006883582 | 0.035900836 | 0.122322957 | 6.05E-05 |
| C.Reactive.Protein | 0.650293852 | 0.088431877 | 0.127497054 | 0.416380812 | 0.092265134 | 0.000604 |
| Calcitonin | 0.042670902 | 0.039612565 | 0.003824627 | 0.064949496 | 0.052682717 | 3.92E-05 |
| Cancer.Antigen.125 | 0.181770358 | 0.633019083 | 0.200791941 | 0.194262302 | 0.339154396 | 0.000832 |
| Cancer.Antigen.19.9 | 0.254989931 | 0.181538098 | 0.080718454 | 0.223057971 | 0.149947391 | 0.000433 |
| Carcinoembryonic.Antigen | 0.052123571 | 0.071314727 | 0.008048403 | 0.073460583 | 0.080021554 | 6.80E-05 |
| CD40 | 1.69E-05 | 7.23E-08 | 2.05E-12 | 0.000128322 | 3.49E-06 | 2.21E-13 |
| CD40.Ligand | 0.055487099 | 0.29226379 | 0.035904382 | 0.07677915 | 0.213937108 | 0.000227 |
| CgA..Chromogranin.A. | 1.93E-05 | 0.013287693 | 1.35E-06 | 0.00013371 | 0.022577984 | 3.89E-08 |
| CNTF..Ciliary.Neurotrophic.Factor. | 0.443625148 | 0.278967894 | 0.192258367 | 0.327992983 | 0.213937108 | 0.000812 |
| Cortisol | 0.007236427 | 0.733463263 | 0.03170505 | 0.01776546 | 0.357651942 | 0.000204 |
| Creatine.Kinase.MB | 0.073160397 | 0.554800902 | 0.092286834 | 0.092798033 | 0.323474919 | 0.000479 |
| CTGF..Connective.Tissue.Growth.Factor. | 0.044529964 | 0.873109673 | 0.124843429 | 0.066325284 | 0.385246844 | 0.000603 |
| EGF | 0.443902722 | 0.833631007 | 0.491563374 | 0.327992983 | 0.382039757 | 0.001733 |
| EGF.R | 0.027825734 | 0.105598873 | 0.006884847 | 0.047059585 | 0.105875255 | 6.05E-05 |
| ENA.78 | 0.18633628 | 0.612886861 | 0.197199609 | 0.194262302 | 0.33828268 | 0.000825 |
| Endothelin.1 | 0.009374834 | 0.006252799 | 0.000151135 | 0.020584543 | 0.013772549 | 2.25E-06 |
| EN.RAGE | 0.084192861 | 0.491605456 | 0.088055733 | 0.102123336 | 0.298328157 | 0.000463 |
| Eotaxin | 0.671770502 | 0.585607616 | 0.494716943 | 0.426043214 | 0.334870587 | 0.001733 |
| Eotaxin.3 | 0.002062402 | 1.45E-05 | 1.02E-07 | 0.006246105 | 0.000143365 | 4.31E-09 |
| Epiregulin | 0.027100436 | 0.134632362 | 0.008718869 | 0.046874599 | 0.118264344 | 6.96E-05 |
| Erythropoietin | 0.376004089 | 0.747457718 | 0.394647463 | 0.289048914 | 0.361397502 | 0.001417 |
| Factor.VII | 0.265654088 | 0.707734974 | 0.293898149 | 0.224640451 | 0.356691103 | 0.001111 |
| Fas | 0.000107887 | 0.115722593 | 0.000101456 | 0.00051317 | 0.109239885 | 1.56E-06 |
| Fas.Ligand | 0.01313349 | 0.949132864 | 0.071088299 | 0.027764612 | 0.409026599 | 0.000393 |
| FABP | 0.000371847 | 4.05E-05 | 3.84E-08 | 0.001572192 | 0.000343725 | 2.36E-09 |
| Ferritin | 0.207812107 | 0.010182338 | 0.006640133 | 0.19769461 | 0.017895869 | 6.05E-05 |

# EP 2 806 272 A1

| probeID | cor.AR-vs-allOther.Set_1 | cor.AR-vs-allOther.Set_2 | Z.AR-vs-allOther.Set_1 | Z.AR-vs-allOther.Set_2 | Z.AR-vs-allOther.Meta |
|---|---|---|---|---|---|
| Alpha.1.Antitrypsin | -0.015020208 | -0.307755635 | 0.127460281 | 2.564316643 | 1.903373716 |
| ACE..Angiotensin.Converting.Enzyme. | 0.131089293 | -0.041373071 | 1.118767622 | 0.333750881 | 1.027085684 |
| ACTH..Adrenocorticotropic.Hormone. | -0.098596298 | -0.01976877 | 0.83934424 | 0.159401686 | 0.706220017 |
| Adiponectin | 0.154783528 | -0.021205629 | 1.324023829 | 0.170990882 | 1.05713504 |
| AgRP..Agouti.related.Protein. | 0.032147075 | -0.035899665 | 0.272871003 | 0.289556788 | 0.397696505 |
| Alpha.2.Macroglobulin | 0.321309921 | -0.053016296 | 2.8265079 | 0.427832184 | 2.301165942 |
| Alpha.Fetoprotein | -0.051408652 | -0.029073126 | 0.436601772 | 0.234461112 | 0.474513117 |
| Amphiregulin | 0.031598383 | 0.028128856 | 0.268210458 | 0.22684193 | 0.3500549 |
| ANG.2..Angiopoietin.2. | -0.061621128 | -0.132504236 | 0.523535935 | 1.074602085 | 1.130054232 |
| Angiotensinogen | -0.213724215 | -0.057191036 | 1.841905004 | 0.461592571 | 1.628818756 |
| Apolipoprotein.A1 | 0.070827041 | 0.273180253 | 0.601995356 | 2.259829403 | 2.023615694 |
| Apolipoprotein.CIII | -0.103374911 | -0.165591028 | 0.880309966 | 1.347446642 | 1.575261805 |
| Apolipoprotein.H | 0.064692791 | 0.040509472 | 0.54970426 | 0.326776632 | 0.619765582 |
| AXL | -0.215531099 | -0.077721104 | 1.857977295 | 0.627873852 | 1.757762203 |
| Beta.2.Microglobulin | -0.193725087 | -0.085647421 | 1.664851473 | 0.692207466 | 1.666692359 |
| Betacellulin | 0.126547444 | 0.176609189 | 1.079578372 | 1.438956049 | 1.780872767 |
| BLC..B.Lymphocyte.Chemoattractant. | -0.220523901 | -0.172981467 | 1.902457135 | 1.408786602 | 2.3414029 |
| BMP.6 | 0.184340403 | 0.195886242 | 1.582268107 | 1.599963372 | 2.250177459 |
| BDNF..Brain.Derived.Neurotrophic.Factor. | 0.071041332 | 0.170791175 | 0.603822865 | 1.390590282 | 1.410263061 |
| Complement.3 | -0.266162383 | -0.277991552 | 2.314182666 | 2.301807578 | 3.263998004 |
| C.Reactive.Protein | -0.130290384 | -0.316680813 | 1.11187086 | 2.644046582 | 2.655834693 |
| Calcitonin | -0.165940828 | -0.258618175 | 1.42119667 | 2.133490824 | 2.513543632 |
| Cancer.Antigen.125 | 0.132940363 | 0.084656334 | 1.134753067 | 0.684158704 | 1.286164847 |
| Cancer.Antigen.19.9 | 0.02159707 | -0.10932198 | 0.183285715 | 0.884918657 | 0.755334555 |
| Carcinoembryonic.Antigen | 0.134925791 | 0.06625662 | 1.151907711 | 0.534961681 | 1.192796787 |
| CD40 | -0.119795444 | -0.196292102 | 1.02140292 | 1.603366376 | 1.855992169 |
| CD40.Ligand | 0.11006274 | 0.005028794 | 0.93771205 | 0.040543776 | 0.691731328 |
| CgA..Chromogranin.A. | -0.107762746 | -0.100729093 | 0.917961667 | 0.814867384 | 1.225295172 |
| CNTF..Ciliary.Neurotrophic.Factor. | -0.086620159 | 0.251013443 | 0.736842977 | 2.067921627 | 1.983268071 |
| Cortisol | -0.03013108 | -0.328388361 | 0.255748105 | 2.749397073 | 2.124958533 |
| Creatine.Kinase.MB | -0.05296598 | -0.092535283 | 0.449852228 | 0.748183708 | 0.847139335 |
| CTGF..Connective.Tissue.Growth.Factor. | -0.104714625 | -0.179620584 | 0.891802224 | 1.464030188 | 1.665825074 |
| EGF | 0.141901042 | 0.191277863 | 1.212251015 | 1.561362838 | 1.961241164 |

| EGF.R | 0.227598963 | 0.118434867 | 1.965664577 | 0.95935489 | 2.0683011 |
| FNA.78 | 0.085011001 | 0.176339171 | 0.723087518 | 1.436709119 | 1.527206848 |
| Endothelin.1 | 0.031548778 | -0.142378563 | 0.267789125 | 1.155744966 | 1.006590609 |
| EN.RAGE | -0.246965422 | 0.058022258 | 2.139805826 | 0.468316413 | 1.844220921 |
| Eotaxin | 0.015384566 | -0.024153432 | 0.130552669 | 0.194769079 | 0.230037214 |
| Eotaxin.3 | -0.020265865 | -0.201668158 | 0.17198511 | 1.64849649 | 1.287274885 |
| Epiregulin | -0.023343932 | 0.103359423 | 0.198115821 | 0.836296943 | 0.73144028 |
| Erythropoietin | 0.175989425 | 0.04664348 | 1.509029962 | 0.37632483 | 1.333147158 |
| Factor.VII | 0.164819084 | 0.047190003 | 1.411410704 | 0.380740761 | 1.267242454 |
| Fas | -0.086941287 | -0.021388199 | 0.739588515 | 0.172463478 | 0.644918149 |
| Fas.Ligand | 0.037921142 | 0.104568491 | 0.321925926 | 0.84615127 | 0.825955306 |
| FABP | -0.011280521 | -0.194764318 | 0.095722456 | 1.590559354 | 1.192381303 |
| Ferritin | -0.181175703 | -0.240084592 | 1.554486802 | 1.974155604 | 2.495126974 |

| probeID | p.AR-vs-allOther.Set_1 | p.AR-vs-allOther.Set_2 | p.AR-vs-allOther.Meta | q.AR-vs-allOther.Set_1 | q.AR-vs-allOther.Set_2 | q.AR-vs-allOther.Meta |
|---|---|---|---|---|---|---|
| Alpha.1.Antitrypsin | 0.898227154 | 0.010678177 | 0.056991798 | 0.652419976 | 0.202616126 | 0.041592 |
| ACE..Angiotensin.Converting.Enzyme. | 0.262268954 | 0.737633147 | 0.304380119 | 0.497506659 | 0.761984693 | 0.077566 |
| ACTH..Adrenocorticotropic.Hormone. | 0.400022698 | 0.872872619 | 0.480051324 | 0.531527436 | 0.77994156 | 0.098993 |
| Adiponectin | 0.1848539 | 0.863717909 | 0.290449961 | 0.453458513 | 0.77994156 | 0.076046 |
| AgRP..Agouti.related.Protein. | 0.784240954 | 0.771325554 | 0.690853914 | 0.63490951 | 0.770308729 | 0.122888 |
| Alpha.2.Macroglobulin | 0.004941305 | 0.667646372 | 0.021382253 | 0.236366098 | 0.756466542 | 0.027681 |
| Alpha.Fetoprotein | 0.661368837 | 0.813938592 | 0.635134047 | 0.596913512 | 0.77994156 | 0.116182 |
| Amphiregulin | 0.787836566 | 0.819878632 | 0.726297497 | 0.63490951 | 0.77994156 | 0.128214 |
| ANG.2..Angiopoietin.2. | 0.599450473 | 0.281409536 | 0.258453374 | 0.572272522 | 0.528322812 | 0.068437 |
| Angiotensinogen | 0.065598927 | 0.643188567 | 0.103351393 | 0.298848437 | 0.754909175 | 0.047221 |
| Apolipoprotein.A1 | 0.545952043 | 0.024196331 | 0.043009709 | 0.553515905 | 0.30608012 | 0.041592 |
| Apolipoprotein.CIII | 0.377461007 | 0.177169383 | 0.115196026 | 0.531527436 | 0.438489264 | 0.049593 |
| Apolipoprotein.H | 0.581342959 | 0.742918381 | 0.535412131 | 0.572272522 | 0.761984693 | 0.102264 |
| AXL | 0.063297639 | 0.52871003 | 0.078787979 | 0.298848437 | 0.684670431 | 0.044623 |
| Beta.2.Microglobulin | 0.095843577 | 0.48739986 | 0.095575593 | 0.356984243 | 0.667465551 | 0.044623 |
| Betacellulin | 0.279304848 | 0.149667163 | 0.074933238 | 0.513864574 | 0.419530935 | 0.044623 |
| BLC..B.Lymphocyte.Chemoattractant. | 0.057276687 | 0.158346378 | 0.019211423 | 0.298848437 | 0.419530935 | 0.026333 |
| BMP.6 | 0.113370866 | 0.10939383 | 0.024437683 | 0.384790107 | 0.409993732 | 0.029971 |
| BDNF..Brain.Derived.Neurotrophic.Factor. | 0.544734889 | 0.163763747 | 0.158462022 | 0.553515905 | 0.419530935 | 0.053514 |

64

| | | | | | | |
|---|---|---|---|---|---|---|
| Complement.3 | 0.020992334 | 0.021714687 | 0.001098519 | 0.256481419 | 0.30608012 | 0.012799 |
| C.Reactive.Protein | 0.265213438 | 0.008511066 | 0.007911238 | 0.497506659 | 0.202616126 | 0.02106 |
| Calcitonin | 0.154783076 | 0.033216727 | 0.011952499 | 0.425139878 | 0.336242863 | 0.02106 |
| Cancer.Antigen.125 | 0.255531823 | 0.492470272 | 0.198385536 | 0.497506659 | 0.667465551 | 0.061579 |
| Cancer.Antigen.19.9 | 0.854079287 | 0.374840671 | 0.45004827 | 0.643380681 | 0.652574559 | 0.09809 |
| Carcinoembryonic.Antigen | 0.248437681 | 0.591387107 | 0.232948969 | 0.497506659 | 0.723964092 | 0.063162 |
| CD40 | 0.305964634 | 0.108646469 | 0.063454674 | 0.528950714 | 0.409993732 | 0.044623 |
| CD40.Ligand | 0.347206367 | 0.967535124 | 0.489106069 | 0.528950714 | 0.80403378 | 0.099106 |
| CgA..Chromogranin.A. | 0.357436112 | 0.413748276 | 0.220464027 | 0.528950714 | 0.652574559 | 0.063162 |
| CNTF..Ciliary.Neurotrophic.Factor. | 0.459941265 | 0.038949744 | 0.047337494 | 0.553515905 | 0.336242863 | 0.041592 |
| Cortisol | 0.797473465 | 0.006256602 | 0.033590073 | 0.63578246 | 0.202616126 | 0.037272 |
| Creatine.Kinase.MB | 0.651765 | 0.452935054 | 0.396917463 | 0.594262315 | 0.663885683 | 0.091697 |
| CTGF..Connective.Tissue.Growth.Factor. | 0.37127633 | 0.142735998 | 0.095748261 | 0.531527436 | 0.419530935 | 0.044623 |
| EGF | 0.224589331 | 0.118160814 | 0.049850897 | 0.497506659 | 0.409993732 | 0.041592 |
| EGF.R | 0.049551513 | 0.336088159 | 0.038611718 | 0.298848437 | 0.607352446 | 0.040897 |
| ENA.78 | 0.468350416 | 0.150300693 | 0.126709584 | 0.553515905 | 0.419530935 | 0.050907 |
| Endothelin.1 | 0.78816185 | 0.246770855 | 0.314131549 | 0.63490951 | 0.528322812 | 0.078709 |
| EN.RAGE | 0.032673898 | 0.63836256 | 0.065150951 | 0.298848437 | 0.754909175 | 0.044623 |
| Eotaxin | 0.895772005 | 0.844993606 | 0.818062852 | 0.652419976 | 0.77994156 | 0.142019 |
| Eotaxin.3 | 0.862983608 | 0.099115132 | 0.197998494 | 0.64500952 | 0.409993732 | 0.061579 |
| Epiregulin | 0.842422405 | 0.401597571 | 0.464510269 | 0.643380681 | 0.652574559 | 0.0984 |
| Erythropoietin | 0.130959409 | 0.705648514 | 0.182483516 | 0.404155562 | 0.756466542 | 0.059059 |
| Factor.VII | 0.157630552 | 0.702359613 | 0.20506861 | 0.425139878 | 0.756466542 | 0.062059 |
| Fas | 0.458273075 | 0.862555946 | 0.51898023 | 0.553515905 | 0.77994156 | 0.102045 |
| Fas.Ligand | 0.746689919 | 0.396083288 | 0.408829445 | 0.63490951 | 0.652574559 | 0.09256 |
| FABP | 0.923477385 | 0.11148043 | 0.233111768 | 0.652723645 | 0.409993732 | 0.063162 |
| Ferritin | 0.11980838 | 0.048606235 | 0.012591207 | 0.384790107 | 0.336242863 | 0.02106 |

| probeID | cor.CAN-vs-allOther.Set_1 | cor.CAN-vs-allOther.Set_2 | Z.CAN-vs-allOther.Set_1 | Z.CAN-vs-allOther.Set_2 | Z.CAN-vs-allOther.Meta |
|---|---|---|---|---|---|
| Alpha.1.Antitrypsin | -0.264825175 | -0.038593443 | 2.301975644 | 0.311304902 | 1.847868395 |
| ACE..Angiotensin.Converting.Enzyme. | -0.227882629 | 0.109570932 | 1.968203233 | 0.886950041 | 2.018898241 |
| ACTH..Adrenocorticotropic.Hormone. | 0.107453626 | -0.20280805 | 0.915307965 | 1.658078499 | 1.819659019 |
| Adiponectin | -0.30554377 | -0.15561604 | 2.678149022 | 1.26489378 | 2.788152303 |
| AgRP..Agouti.related.Protein. | -0.276257178 | -0.164248638 | 2.406641679 | 1.336319453 | 2.646673198 |

| | | | | | |
|---|---|---|---|---|---|
| Alpha.2.Macroglobulin | -0.291789723 | 0.003863608 | 2.550008918 | 0.03114956 | 1.825154663 |
| Alpha.Fetoprotein | 0.038108576 | -0.00937948 | 0.323518659 | 0.075622002 | 0.282235068 |
| Amphiregulin | -0.009943547 | 0.06923231 | 0.084376575 | 0.55906309 | 0.454980551 |
| ANG.2..Angiopoietin.2. | -0.138897518 | -0.152378943 | 1.186252769 | 1.238161483 | 1.714319758 |
| Angiotensinogen | 0.146975825 | -0.093601204 | 1.256229583 | 0.756852528 | 1.423464012 |
| Apolipoprotein.A1 | -0.065481399 | -0.315606014 | 0.556424284 | 2.634419033 | 2.256266947 |
| Apolipoprotein.CIII | 0.001884515 | -0.165957515 | 0.015990657 | 1.350482943 | 0.966242749 |
| Apolipoprotein.H | -0.011612788 | -0.017329588 | 0.098542204 | 0.139729592 | 0.168483603 |
| AXL | -0.246150583 | -0.266397259 | 2.132444166 | 2.200849981 | 3.064101676 |
| Beta.2.Microglobulin | -0.411281512 | -0.44390072 | 3.709363342 | 3.846328724 | 5.342681096 |
| Betacellulin | -0.231817166 | -0.261163977 | 2.003451129 | 2.155502494 | 2.94082431 |
| BLC..B.Lymphocyte.Chemoattractant. | -0.077260576 | -0.203690761 | 0.656886839 | 1.665501802 | 1.642176756 |
| BMP.6 | -0.365339409 | -0.186617989 | 3.250151157 | 1.522402727 | 3.374705214 |
| BDNF..Brain.Derived.Neurotrophic.Factor. | 0.278189084 | -0.085716181 | 2.424399154 | 0.692765928 | 2.204168568 |
| Complement.3 | 0.536763883 | 0.113310627 | 5.087762893 | 0.917479613 | 4.246347699 |
| C.Reactive.Protein | 0.077085045 | 0.12618267 | 0.655388485 | 1.02276865 | 1.18663629 |
| Calcitonin | -0.068769139 | 0.029691454 | 0.584447978 | 0.239450541 | 0.58258423 |
| Cancer.Antigen.125 | 0.022923224 | -0.138591073 | 0.194544084 | 1.124594421 | 0.932771782 |
| Cancer.Antigen.19.9 | 0.111493615 | -0.040698956 | 0.950004276 | 0.328306827 | 0.903902449 |
| Carcinoembryonic.Antigen | -0.360074691 | -0.26763967 | 3.198711085 | 2.211635624 | 3.825692846 |
| CD40 | -0.354903338 | -0.350918632 | 3.148400966 | 2.954745005 | 4.315575903 |
| CD40.Ligand | 0.112027355 | -0.123591924 | 0.954590497 | 1.00155044 | 1.383200522 |
| CgA..Chromogranin.A. | -0.363996708 | -0.172759857 | 3.23701047 | 1.406944884 | 3.283772322 |
| CNTF..Ciliary.Neurotrophic.Factor. | -0.00316903 | -0.12916162 | 0.026890203 | 1.047183696 | 0.759484937 |
| Cortisol | 0.337860615 | 0.366869076 | 2.98406547 | 3.102360109 | 4.3037528 |
| Creatine.Kinase.MB | -0.15516016 | 0.025855952 | 1.3272983 | 0.208503822 | 1.085976096 |
| CTGF..Connective.Tissue.Growth.Factor. | -0.127985764 | 0.16156634 | 1.091983849 | 1.314104502 | 1.701361388 |
| EGF | -0.052166302 | -0.215020735 | 0.443047934 | 1.761035409 | 1.558522278 |
| EGF.R | 0.026487808 | 0.062705829 | 0.224809088 | 0.506214736 | 0.516911904 |
| ENA.78 | 0.069257945 | -0.119194084 | 0.588615484 | 0.965563542 | 1.098970528 |
| Endothelin.1 | -0.329699824 | -0.15811474 | 2.906136164 | 1.285547171 | 2.963967711 |
| EN.RAGE | 0.046228406 | -0.1356364 | 0.39254082 | 1.100316638 | 1.055609632 |
| Eotaxin | -0.065116461 | -0.037415612 | 0.553314428 | 0.301795189 | 0.604653809 |
| Eotaxin.3 | -0.33004577 | -0.255405809 | 2.909430038 | 2.105760115 | 3.546274966 |
| Epiregulin | -0.231896743 | 0.064343729 | 2.004164719 | 0.519473417 | 1.784481639 |
| Erythropoietin | -0.072300123 | -0.083032045 | 0.61455921 | 0.670970553 | 0.909006813 |

| | | | | | |
|---|---|---|---|---|---|
| Factor.VII | -0.294990425 | -0.089551574 | 2.579725775 | 0.723927199 | 2.336035421 |
| Fas | -0.345260631 | -0.15477727 | 3.055150186 | 1.257964499 | 3.049832642 |
| Fas.Ligand | -0.323117311 | -0.111780811 | 2.843620811 | 0.904987614 | 2.650666437 |
| FABP | -0.389101741 | -0.240973044 | 3.485253628 | 1.981758475 | 3.865761331 |
| Ferritin | 0.034050984 | -0.043240878 | 0.28904393 | 0.348836628 | 0.451049669 |

| probeID | p.CAN-vs-allOther.Set_1 | p.CAN-vs-allOther.Set_2 | p.CAN-vs-allOther.Meta | q.CAN-vs-allOther.Set_1 | q.CAN-vs-allOther.Set_2 | q.CAN-vs-allOther.Meta |
|---|---|---|---|---|---|---|
| Alpha.1.Antitrypsin | 0.02167059 | 0.754686534 | 0.064621384 | 0.031409745 | 0.409435518 | 0.000178 |
| ACE..Angiotensin.Converting.Enzyme. | 0.049260576 | 0.373747487 | 0.043497797 | 0.05367358 | 0.309219617 | 0.000127 |
| ACTH..Adrenocorticotropic.Hormone. | 0.35882502 | 0.097180608 | 0.068810948 | 0.244729503 | 0.178671735 | 0.000181 |
| Adiponectin | 0.00768246 | 0.205088234 | 0.005300962 | 0.015391274 | 0.250612008 | 2.75E-05 |
| AgRP..Agouti.related.Protein. | 0.016432536 | 0.180756914 | 0.008128787 | 0.027331201 | 0.249248422 | 3.69E-05 |
| Alpha.2.Macroglobulin | 0.011083135 | 0.975054572 | 0.067977693 | 0.020858575 | 0.467658228 | 0.000181 |
| Alpha.Fetoprotein | 0.745480251 | 0.939488433 | 0.77776327 | 0.367452772 | 0.457226373 | 0.001017 |
| Amphiregulin | 0.93252426 | 0.574801897 | 0.649123231 | 0.426132272 | 0.355892534 | 0.000891 |
| ANG.2..Angiopoietin.2. | 0.234654162 | 0.21478613 | 0.086470016 | 0.186839884 | 0.250612008 | 0.000214 |
| Angiotensinogen | 0.208277639 | 0.447724403 | 0.154601687 | 0.170202128 | 0.336477099 | 0.000319 |
| Apolipoprotein.A1 | 0.576734855 | 0.00874984 | 0.024053918 | 0.326829083 | 0.052648521 | 8.74E-05 |
| Apolipoprotein.CIII | 0.987197478 | 0.176199789 | 0.333922726 | 0.438634338 | 0.249248427 | 0.000547 |
| Apolipoprotein.H | 0.921230499 | 0.888450989 | 0.866202842 | 0.426132272 | 0.443264974 | 0.001124 |
| AXL | 0.033271279 | 0.028099977 | 0.002183247 | 0.042170558 | 0.094656849 | 1.42E-05 |
| Beta.2.Microglobulin | 0.000246756 | 0.000149614 | 9.16E-08 | 0.00139319 | 0.003300871 | 5.55E-09 |
| Betacellulin | 0.045367942 | 0.0314627 | 0.003273401 | 0.05122976 | 0.094656849 | 1.80E-05 |
| BLC..B.Lymphocyte.Chemoattractant. | 0.509995976 | 0.095702913 | 0.100553378 | 0.317338943 | 0.178671735 | 0.000237 |
| BMP.6 | 0.001268653 | 0.127563356 | 0.000738948 | 0.004335056 | 0.211078835 | 5.84E-06 |
| BDNF..Brain.Derived.Neurotrophic.Factor. | 0.015664739 | 0.487049107 | 0.027512491 | 0.027024434 | 0.338260484 | 9.80E-05 |
| Complement.3 | 6.90E-07 | 0.357558257 | 2.17E-05 | 1.43E-05 | 0.30898559 | 3.59E-07 |
| C.Reactive.Protein | 0.510960633 | 0.305201338 | 0.235371115 | 0.317338943 | 0.296895462 | 0.000447 |
| Calcitonin | 0.557707556 | 0.810054562 | 0.560173224 | 0.326070399 | 0.425522443 | 0.000789 |
| Cancer.Antigen.125 | 0.845226816 | 0.259694965 | 0.350937813 | 0.400717124 | 0.296356379 | 0.000567 |
| Cancer.Antigen.19.9 | 0.340935248 | 0.741757711 | 0.36604713 | 0.243382071 | 0.405747117 | 0.00057 |
| Carcinoembryonic.Antigen | 0.001508097 | 0.027347712 | 0.000130405 | 0.004683118 | 0.094656849 | 1.39E-06 |
| CD40 | 0.001782239 | 0.003346014 | 1.59E-05 | 0.005031289 | 0.024607303 | 3.05E-07 |
| CD40.Ligand | 0.338614323 | 0.315317778 | 0.16660339 | 0.243382071 | 0.296895462 | 0.00034 |
| CgA..Chromogranin.A. | 0.00132621 | 0.158888392 | 0.001024276 | 0.004335056 | 0.239011192 | 7.75E-06 |

| | | | | | | |
|---|---|---|---|---|---|---|
| CNTF..Ciliary.Neurotrophic.Factor. | 0.978472728 | 0.293832046 | 0.447562521 | 0.438634338 | 0.296895462 | 0.000656 |
| Cortisol | 0.00303159 | 0.002089145 | 1.68E-05 | 0.007845039 | 0.019753734 | 3.05E-07 |
| Creatine.Kinase.MB | 0.183774349 | 0.834217445 | 0.277489568 | 0.15423719 | 0.433193936 | 0.000485 |
| CTGF..Connective.Tissue.Growth.Factor. | 0.273832013 | 0.18808138 | 0.088875147 | 0.207398828 | 0.250612008 | 0.000215 |
| EGF | 0.656689569 | 0.078256414 | 0.119109491 | 0.339871542 | 0.167084819 | 0.000263 |
| EGF.R | 0.821529763 | 0.611453887 | 0.605217658 | 0.392478508 | 0.35814922 | 0.000839 |
| ENA.78 | 0.554904422 | 0.33297754 | 0.271780921 | 0.326070399 | 0.297476426 | 0.000482 |
| Endothelin.1 | 0.003870077 | 0.197818186 | 0.003037002 | 0.008902085 | 0.250612008 | 1.72E-05 |
| EN.RAGE | 0.693702006 | 0.270089791 | 0.291146598 | 0.345474026 | 0.296895462 | 0.000501 |
| Eotaxin | 0.578865212 | 0.76194862 | 0.545409045 | 0.326829083 | 0.410014597 | 0.000774 |
| Eotaxin.3 | 0.003830725 | 0.035545968 | 0.000390718 | 0.008902085 | 0.101743848 | 3.74E-06 |
| Epiregulin | 0.045291913 | 0.602160863 | 0.074345436 | 0.05122976 | 0.35814922 | 0.00019 |
| Erythropoietin | 0.537611812 | 0.500839528 | 0.36334653 | 0.326070399 | 0.338260484 | 0.00057 |
| Factor.VII | 0.010192464 | 0.467697012 | 0.019489396 | 0.01978177 | 0.336477099 | 7.38E-05 |
| Fas | 0.002415748 | 0.207570681 | 0.002289689 | 0.006523185 | 0.250612008 | 1.43E-05 |
| Fas.Ligand | 0.004690577 | 0.364128008 | 0.008033314 | 0.01004533 | 0.30898559 | 3.69E-05 |
| FABP | 0.00056027 | 0.047754136 | 0.000110743 | 0.002298337 | 0.117064704 | 1.26E-06 |
| Ferritin | 0.771799924 | 0.72624381 | 0.651953752 | 0.37448178 | 0.401863318 | 0.000891 |

| probeID | cor.TX-vs-AR.Set_1 | cor.TX-vs-AR.Set_2 | Z.TX-vs-AR.Set_1 | Z.TX-vs-AR.Set_2 | Z.TX-vs-AR.Meta |
|---|---|---|---|---|---|
| FGF.basic | 0.219765 | 0.280834 | 1.531617 | 1.825185 | 2.373617£ |
| FGF.4 | -0.09574 | -0.09341 | 0.658352 | 0.592513 | 0.884495( |
| Fibrinogen | 0.147901 | 0.355842 | 1.021453 | 2.353476 | 2.386435: |
| FSH..Follicle.Stimulating.Hormone. | -0.04907 | 0.042841 | 0.336711 | 0.271115 | 0.4297977 |
| G.CSF | -0.067 | 0.297749 | 0.460046 | 1.941938 | 1.698459` |
| GLP.1.Total | 0.271827 | 0.209249 | 1.911599 | 1.343247 | 2.301523: |
| Glucagon | 0.179278 | 0.128055 | 1.242499 | 0.814361 | 1.454419< |
| GST | 0.084586 | -0.1146 | 0.581283 | 0.727987 | 0.925794: |
| GM.CSF | -0.05902 | -0.12861 | 0.405107 | 0.817916 | 0.864808( |
| GRO.alpha | -0.04953 | 0.047844 | 0.339806 | 0.30282 | 0.4544055 |
| Growth.Hormone | 0.175739 | 0.158776 | 1.217445 | 1.012758 | 1.576991£ |
| Haptoglobin | -0.1762 | 0.437479 | 1.220683 | 2.966907 | 2.961073£ |
| HB.EGF | 0.571612 | 0.126062 | 4.45559 | 0.801549 | 3.717358: |
| HCC.4 | 0.294018 | 0.443851 | 2.07698 | 3.016915 | 3.601927< |

67

| | | | | | |
|---|---|---|---|---|---|
| HGF..Hepatocyte.growth.factor. | 0.108491 | 0.516245 | 0.746715 | 3.612628 | 3.082520? |
| I.309 | 0.275878 | -0.14381 | 1.941623 | 0.915873 | 2.020555? |
| ICAM.1 | 0.019896 | 0.1718 | 0.136418 | 1.097441 | 0.872469? |
| IFN.gamma | 0.263659 | -0.17397 | 1.851278 | 1.111559 | 2.095042? |
| IgA | -0.07277 | -0.00945 | 0.499804 | 0.059789 | 0.395691? |
| IgE | 0.329333 | -0.1608 | 2.34518 | 1.025895 | 2.383710? |
| IGF.BP.2 | 0.419807 | 0.550135 | 3.067613 | 3.912215 | 4.935483? |
| IGF.1 | 0.072756 | 0.067393 | 0.499675 | 0.426875 | 0.655169? |
| IgM | 0.137752 | -0.1032 | 0.950423 | 0.655044 | 1.13523? |
| IL.10 | 0.551368 | 0.256702 | 4.252866 | 1.660665 | 4.18149? |
| IL.12p40 | 0.305293 | 0.135979 | 2.161907 | 0.86537 | 2.140607? |
| IL.13 | 0.154889 | -0.32294 | 1.070484 | 2.118248 | 2.254773? |
| IL.15 | 0.111104 | 0.005267 | 0.764845 | 0.033312 | 0.564382 |
| IL.16 | 0.057034 | -0.03483 | 0.39143 | 0.220367 | 0.432606? |
| IL.18 | 0.379917 | 0.243251 | 2.742004 | 1.569923 | 3.048992? |
| IL.1alpha | -0.12688 | -0.25815 | 0.874532 | 1.670507 | 1.789614? |
| IL.1beta | 0.209684 | -0.20336 | 1.45916 | 1.304315 | 1.954071? |
| IL.1ra | 0.14329 | 0.439819 | 0.989152 | 2.985227 | 2.810310? |
| IL.2 | -0.25366 | 0.14853 | 1.777779 | 0.946386 | 1.92627? |
| IL.3 | -0.01549 | -0.40686 | 0.106214 | 2.731189 | 2.00634? |
| IL.4 | 0.063458 | 0.123913 | 0.435629 | 0.787744 | 0.86505? |
| IL.5 | 0.140371 | -0.11078 | 0.968729 | 0.703536 | 1.182470? |
| IL.6 | 0.173364 | 0.194216 | 1.200654 | 1.244133 | 1.728725? |
| IL.7 | 0.163832 | -0.02217 | 1.133391 | 0.140247 | 0.90059? |
| IL.8 | 0.214186 | 0.506815 | 1.491476 | 3.531852 | 3.552029? |
| Insulin | -0.1697 | -0.01107 | 1.174786 | 0.070041 | 0.880225? |
| Leptin | -0.01574 | 0.011589 | 0.107883 | 0.073299 | 0.128115? |
| LH..Luteinizing.Hormone. | -0.07687 | 0.020608 | 0.528024 | 0.130355 | 0.465544? |
| Lymphotactin | -0.07625 | -0.18185 | 0.52376 | 1.163044 | 1.192751? |
| MCP.1 | -0.23898 | 0.169045 | 1.67065 | 1.079496 | 1.944646? |
| MCP.3 | -0.10347 | -0.06091 | 0.711899 | 0.385735 | 0.776144? |
| M.CSF | 0.202673 | 0.308927 | 1.410397 | 2.019803 | 2.425517? |

| probeID | p.TX-vs-AR.Set_1 | p.TX-vs-AR.Set_2 | p.TX-vs.AR.Meta | q.TX-vs-AR.Set_1 | q.TX-vs-AR.Set_2 |
|---|---|---|---|---|---|
| FGF.basic | 0.125156 | 0.068123 | 0.017615 | 0.145617 | 0.116603? |
| FGF.4 | 0.508383 | 0.551312 | 0.376429 | 0.295027 | 0.398241? |
| Fibrinogen | 0.305354 | 0.019187 | 0.017013 | 0.227793 | 0.044319? |
| FSH..Follicle.Stimulating.Hormone. | 0.735032 | 0.785028 | 0.667343 | 0.342644 | 0.500045? |
| G.CSF | 0.643847 | 0.052481 | 0.089421 | 0.324042 | 0.098384? |
| GLP.1.Total | 0.056182 | 0.178087 | 0.021362 | 0.086356 | 0.226160? |
| Glucagon | 0.212857 | 0.41316 | 0.14583 | 0.198451 | 0.353595? |
| GST | 0.559196 | 0.464321 | 0.354553 | 0.309054 | 0.360957? |
| GM.CSF | 0.683898 | 0.411129 | 0.387144 | 0.332945 | 0.353595? |
| GRO.alpha | 0.73269 | 0.760625 | 0.649537 | 0.342644 | 0.49907? |
| Growth.Hormone | 0.222177 | 0.309175 | 0.114797 | 0.200565 | 0.297054? |
| Haptoglobin | 0.220956 | 0.00335 | 0.003066 | 0.200565 | 0.015516? |
| HB.EGF | 1.45E-05 | 0.420528 | 0.000201 | 0.000356 | 0.356031? |
| HCC.4 | 0.03822 | 0.00287 | 0.000316 | 0.072454 | 0.015516? |
| HGF..Hepatocyte.growth.factor. | 0.453278 | 0.000395 | 0.002053 | 0.274242 | 0.003452? |
| I.309 | 0.052474 | 0.357561 | 0.043326 | 0.086356 | 0.323599? |
| ICAM.1 | 0.890919 | 0.270642 | 0.382952 | 0.374357 | 0.291909? |
| IFN.gamma | 0.064302 | 0.264557 | 0.036167 | 0.096236 | 0.289309? |
| IgA | 0.615493 | 0.952025 | 0.692332 | 0.312583 | 0.551169? |
| IgE | 0.019523 | 0.302968 | 0.017139 | 0.046262 | 0.297054? |
| IGF.BP.2 | 0.002406 | 0.000133 | 8.00E-07 | 0.010524 | 0.001490? |
| IGF.1 | 0.615584 | 0.66764 | 0.512359 | 0.312583 | 0.45710? |
| IgM | 0.340096 | 0.510174 | 0.256276 | 0.227793 | 0.375414? |
| IL.10 | 3.33E-05 | 0.096577 | 2.90E-05 | 0.000378 | 0.143474? |
| IL.12p40 | 0.031091 | 0.384601 | 0.032306 | 0.060972 | 0.34411? |
| IL.13 | 0.28281 | 0.034669 | 0.024148 | 0.21735 | 0.06999? |
| IL.15 | 0.442408 | 0.97326 | 0.572494 | 0.272928 | 0.551302? |
| IL.16 | 0.694016 | 0.824527 | 0.665301 | 0.332945 | 0.511183? |
| IL.18 | 0.006502 | 0.115994 | 0.002296 | 0.020543 | 0.169128? |
| IL.1alpha | 0.379936 | 0.094639 | 0.071922 | 0.245541 | 0.143295? |
| IL.1beta | 0.143881 | 0.190903 | 0.050693 | 0.157361 | 0.23124? |

| probeID | | | | | |
|---|---|---|---|---|---|
| IL.1ra | 0.320847 | 0.003166 | 0.004949 | 0.227793 | 0.0155166 |
| IL.2 | 0.075495 | 0.341827 | 0.05407 | 0.10472 | 0.316638( |
| IL.3 | 0.914965 | 0.006778 | 0.044819 | 0.374357 | 0.0205658 |
| IL.4 | 0.661527 | 0.428555 | 0.387009 | 0.330019 | 0.3587827 |
| IL.5 | 0.330906 | 0.479428 | 0.237019 | 0.227793 | 0.3664899 |
| IL.6 | 0.228586 | 0.212047 | 0.083858 | 0.203126 | 0.2504325 |
| IL.7 | 0.255593 | 0.887775 | 0.367802 | 0.204286 | 0.5335587 |
| IL.8 | 0.135278 | 0.000524 | 0.000382 | 0.152674 | 0.0037513 |
| Insulin | 0.238719 | 0.943812 | 0.378737 | 0.20397 | 0.5504624 |
| Leptin | 0.913634 | 0.941203 | 0.898058 | 0.374357 | 0.5504624 |
| LH..Luteinizing.Hormone. | 0.59571 | 0.895643 | 0.641542 | 0.312583 | 0.5335587 |
| Lymphotactin | 0.59868 | 0.243177 | 0.232967 | 0.312583 | 0.2697746 |
| MCP.1 | 0.094643 | 0.278516 | 0.051818 | 0.117011 | 0.2923912 |
| MCP.3 | 0.474574 | 0.697991 | 0.437664 | 0.281144 | 0.465740; |
| M.CSF | 0.157658 | 0.043833 | 0.015287 | 0.166043 | 0.084177£ |

| probeID | cor.TX-vs-CAN.Set_1 | cor.TX-vs-CAN.Set_2 | Z.TX-vs-CAN.Set_1 | Z.TX-vs-CAN.Set_2 | Z.TX-vs-CAN.Meta |
|---|---|---|---|---|---|
| FGF.basic | 0.523011 | 0.450677 | 3.979537 | 3.070885 | 4.98540; |
| FGF.4 | 0.19822 | 0.140206 | 1.377157 | 0.892622 | 1.604976( |
| Fibrinogen | 0.133795 | 0.213512 | 0.922786 | 1.371466 | 1.6222809 |
| FSH..Follicle.Stimulating.Hormone. | 0.055921 | 0.080129 | 0.383775 | 0.507869 | 0.6304877 |
| G.CSF | 0.033182 | 0.555745 | 0.22757 | 3.963316 | 2.9634036 |
| GLP.1.Total | 0.237364 | 0.107079 | 1.658923 | 0.679835 | 3751€ |
| Glucagon | 0.230253 | 0.1744 | 1.607354 | 1.114393 | 4566( |
| GST | -0.2575 | -0.27313 | 1.805987 | 1.772432 | 0323€ |
| GM.CSF | 0.244815 | -0.19464 | 1.713154 | 1.246921 | 3089; |
| GRO.alpha | -0.26424 | 0.185897 | 1.855569 | 1.189547 | 3222; |
| Growth.Hormone | 0.227667 | 0.039176 | 1.588642 | 0.247895 | 8628( |
| Haptoglobin | -0.15782 | 0.168342 | 1.091104 | 1.07492 | 1610; |
| HB.EGF | 0.726564 | 0.156639 | 6.316865 | 0.998894 | 7302; |
| HCC.4 | 0.061449 | 0.227028 | 0.421805 | 1.461314 | 1566; |
| HGF..Hepatocyte.growth.factor. | 0.118935 | 0.378144 | 0.819253 | 2.516489 | 8726; |

| | | | | | |
|---|---|---|---|---|---|
| I.309 | 0.641185 | 0.07338 | 5.21155 | 0.46493 | 4.013877! |
| ICAM.1 | -0.09089 | -0.02329 | 0.624859 | 0.147308 | 0.54600< |
| IFN.gamma | 0.256517 | -0.17739 | 1.798762 | 1.133885 | 2.073694 |
| IgA | 0.086241 | 0.054202 | 0.592714 | 0.343138 | 0.661747 |
| IgE | 0.231625 | -0.18461 | 1.617289 | 1.181108 | 1.97876! |
| IGF.BP.2 | 0.439736 | 0.457948 | 3.23521 | 3.128831 | 4.50005; |
| IGF.1 | 0.079227 | 0.226027 | 0.544291 | 1.45464 | 1.413457< |
| IgM | 0.389396 | 0.356356 | 2.818277 | 2.3572 | 3.659614! |
| IL.10 | 0.69784 | 0.229915 | 5.916964 | 1.480573 | 5.230848; |
| IL.12p40 | 0.414912 | -0.00237 | 3.026981 | 0.014993 | 2.1510002 |
| IL.13 | 0.636678 | -0.20022 | 5.159341 | 1.283613 | 4.555856< |
| IL.15 | 0.415472 | 0.118059 | 3.031616 | 0.750169 | 2.674126; |
| IL.16 | 0.221892 | 0.206032 | 1.546943 | 1.321984 | 2.028637; |
| IL.18 | 0.436546 | 0.349692 | 3.208145 | 2.30905 | 3.901246< |
| IL.1alpha | 0.033517 | -0.23162 | 0.22987 | 1.491993 | 1.217541; |
| IL.1beta | 0.092569 | -0.15227 | 0.636445 | 0.970606 | 1.136356! |
| IL.1ra | 0.213197 | 0.517812 | 1.48437 | 3.626157 | 3.613688; |
| IL.2 | -0.04594 | -0.03644 | 0.315167 | 0.230572 | 0.385895; |
| IL.3 | 0.191177 | -0.43052 | 1.32697 | 2.91265 | 2.997864; |
| IL.4 | 0.070873 | 0.193002 | 0.486699 | 1.236155 | 1.218241£ |
| IL.5 | 0.175286 | -0.17177 | 1.21424 | 1.097247 | 1.634467! |
| IL.6 | 0.193244 | 0.081119 | 1.341682 | 0.514173 | 1.312287£ |
| IL.7 | 0.367248 | -0.08808 | 2.641063 | 0.558525 | 2.26245( |
| IL.8 | 0.172353 | 0.367558 | 1.193504 | 2.438725 | 2.568374( |
| Insulin | 0.162448 | -0.05786 | 1.123641 | 0.36632 | 1.053561£ |
| Leptin | -0.01036 | -0.10244 | 0.071 | 0.650197 | 0.50996; |
| LH..Luteinizing.Hormone. | -0.02933 | 0.046301 | 0.201117 | 0.29304 | 0.34942; |
| Lymphotactin | -0.19403 | -0.03644 | 1.347266 | 0.230572 | 1.115700( |
| MCP.1 | 0.02529 | 0.121622 | 0.173415 | 0.773029 | 0.6692; |
| MCP.3 | 0.208628 | 0.124022 | 1.451591 | 0.788443 | 1.58394; |
| M.CSF | 0.348659 | 0.523007 | 2.494882 | 3.671215 | 4.36008£ |

| probeID | p.TX-vs-CAN.Set_1 | p.TX-vs-CAN.Set_2 | p.TX-vs-CAN.Meta | q.TX-vs-CAN.Set_1 | q.TX-vs-CAN.Set_2 |
|---|---|---|---|---|---|
| FGF.basic | 9.75E-05 | 0.002423 | 6.18E-07 | 0.000406 | 0.009012? |
| FGF.4 | 0.167614 | 0.369856 | 0.108499 | 0.15893 | 0.246336? |
| Fibrinogen | 0.35428 | 0.169211 | 0.104743 | 0.265381 | 0.165444( |
| FSH..Follicle.Stimulating.Hormone. | 0.699703 | 0.609505 | 0.528376 | 0.433163 | 0.321288? |
| G.CSF | 0.819054 | 0.000109 | 0.003043 | 0.483094 | 0.00110? |
| GLP.1.Total | 0.096959 | 0.494326 | 0.098178 | 0.106807 | 0.291815? |
| Glucagon | 0.107692 | 0.263347 | 0.054284 | 0.112041 | 0.206125? |
| GST | 0.071023 | 0.076371 | 0.011396 | 0.086666 | 0.096450( |
| GM.CSF | 0.086621 | 0.211032 | 0.036341 | 0.098311 | 0.190936? |
| GRO.alpha | 0.063693 | 0.232664 | 0.031301 | 0.079518 | 0.193127? |
| Growth.Hormone | 0.111815 | 0.803038 | 0.194072 | 0.114736 | 0.377853? |
| Haptoglobin | 0.273677 | 0.280549 | 0.125619 | 0.213546 | 0.206125? |
| HB.EGF | 2.33E-09 | 0.315817 | 2.30E-07 | 8.72E-08 | 0.220055( |
| HCC.4 | 0.671623 | 0.14318 | 0.183003 | 0.428923 | 0.14898? |
| HGF..Hepatocyte.growth.factor. | 0.410707 | 0.012412 | 0.018338 | 0.298688 | 0.027868? |
| I.309 | 5.26E-07 | 0.640046 | 5.97E-05 | 4.38E-06 | 0.328809? |
| ICAM.1 | 0.530162 | 0.882165 | 0.585063 | 0.367712 | 0.402081? |
| IFN.gamma | 0.072147 | 0.25513 | 0.038108 | 0.086666 | 0.203499? |
| IgA | 0.551505 | 0.729941 | 0.508133 | 0.37556 | 0.368741? |
| IgE | 0.105554 | 0.235976 | 0.047842 | 0.111562 | 0.193127? |
| IGF.BP.2 | 0.001397 | 0.002016 | 6.79E-06 | 0.003738 | 0.008145? |
| IGF.1 | 0.584442 | 0.145 | 0.157521 | 0.390883 | 0.14898? |
| IgM | 0.005189 | 0.019001 | 0.000253 | 0.010797 | 71562 |
| IL.10 | 1.78E-08 | 0.138028 | 1.69E-07 | 3.33E-07 | 6793? |
| IL.12p40 | 0.002736 | 0.987963 | 0.031476 | 0.006027 | 7787? |
| IL.13 | 6.69E-07 | 0.197992 | 5.22E-06 | 5.01E-06 | 2985? |
| IL.15 | 0.002696 | 0.450852 | 0.007492 | 0.006027 | 4694? |
| IL.16 | 0.121453 | 0.185004 | 0.042495 | 0.121303 | 8014? |
| IL.18 | 0.001527 | 0.021527 | 9.57E-05 | 0.003945 | 0779? |
| IL.1alpha | 0.817257 | 0.135042 | 0.223398 | 0.483094 | 6793? |
| IL.1beta | 0.522574 | 0.329661 | 0.255807 | 0.365836 | 4918? |
| IL.1ra | 0.137134 | 0.000376 | 0.000302 | 0.135162 | 0228? |
| IL.2 | 0.751401 | 0.816544 | 0.699574 | 0.453914 | 0.377853? |
| IL.3 | 0.183539 | 0.003955 | 0.002719 | 0.165633 | 0.012617? |
| IL.4 | 0.624778 | 0.214975 | 0.223132 | 0.406959 | 0.191643? |
| IL.5 | 0.22339 | 0.270726 | 0.102161 | 0.185928 | 0.206125? |
| IL.6 | 0.178757 | 0.605077 | 0.189423 | 0.163295 | 0.321288? |
| IL.7 | 0.008702 | 0.574345 | 0.02367 | 0.016297 | 0.321288? |
| IL.8 | 0.231355 | 0.015318 | 0.010218 | 0.190441 | 0.032643? |
| Insulin | 0.259686 | 0.71249 | 0.292084 | 0.204762 | 0.362950? |
| Leptin | 0.943097 | 0.513304 | 0.610077 | 0.521567 | 0.295470? |
| LH..Luteinizing.Hormone. | 0.839779 | 0.768128 | 0.726773 | 0.487639 | 0.372511? |
| Lymphotactin | 0.176967 | 0.816544 | 0.26455 | 0.163295 | 0.377853? |
| MCP.1 | 0.861606 | 0.437206 | 0.503344 | 0.492675 | 0.278984? |
| MCP.3 | 0.145957 | 0.428146 | 0.113207 | 0.140169 | 0.276108? |
| M.CSF | 0.013085 | 0.00032 | 1.30E-05 | 0.021782 | 0.002157? |

| probeID | cor.AR-vs-CAN.Set_1 | cor.AR-vs-CAN.Set_2 | Z.AR-vs-CAN.Set_1 | Z.AR-vs-CAN.Set_2 | Z.AR-vs-CAN.Meta |
|---|---|---|---|---|---|
| FGF.basic | 0.366515 | 0.234035 | 2.635256 | 1.634758 | 3.019355? |
| FGF.4 | 0.275622 | 0.204225 | 1.939727 | 1.420062 | 2.375729? |
| Fibrinogen | -0.0223 | -0.12613 | 0.152872 | 0.869299 | 0.722784? |
| FSH..Follicle.Stimulating.Hormone. | 0.13974 | 0.012692 | 0.964316 | 0.087017 | 0.743404? |
| G.CSF | 0.09501 | 0.284384 | 0.653326 | 2.004906 | 1.87965? |
| GLP.1.Total | 0.161859 | -0.07719 | 1.119494 | 0.530258 | 1.166550? |
| Glucagon | 0.14622 | 0.030897 | 1.009673 | 0.211889 | 3774? |
| GST | -0.31924 | -0.15219 | 2.267855 | 1.051507 | 7143? |
| GM.CSF | 0.284358 | -0.05099 | 2.004708 | 0.349847 | 4922? |
| GRO.alpha | -0.12571 | 0.158639 | 0.866405 | 1.096836 | 8220? |
| Growth.Hormone | 0.045327 | -0.10947 | 0.31096 | 0.753501 | 2687? |
| Haptoglobin | 0.028959 | -0.26489 | 0.19859 | 1.860386 | 5916? |
| HB.EGF | 0.208938 | 0.007886 | 1.453811 | 0.054066 | 6230? |
| HCC.4 | -0.24625 | -0.15785 | 1.723613 | 1.09132 | 0458? |
| HGF..Hepatocyte.growth.factor. | 0.009374 | -0.07342 | 0.06427 | 0.504256 | 2008? |
| I.309 | 0.178034 | 0.18999 | 1.233687 | 1.318526 | 4686? |

| | | | | | |
|---|---|---|---|---|---|
| ICAM.1 | -0.1297 | -0.1094 | 0.894223 | 0.752996 | 1.1647600 |
| IFN.gamma | -0.14298 | 0.040963 | 0.986954 | 0.280989 | 0.8965706 |
| IgA | 0.154911 | 0.074028 | 1.070634 | 0.508437 | 1.1165721 |
| IgE | -0.19691 | 0.050105 | 1.367807 | 0.343791 | 1.2102825 |
| IGF.BP.2 | -0.04179 | 0.033413 | 0.286672 | 0.229151 | 0.3647416 |
| IGF.1 | 0.070726 | 0.224356 | 0.485684 | 1.564724 | 1.4498574 |
| IgM | 0.168308 | 0.416427 | 1.164944 | 3.039532 | 2.9730138 |
| IL.10 | 0.253973 | -0.00542 | 1.780104 | 0.037135 | 1.284982 |
| IL.12p40 | 0.070853 | -0.14277 | 0.486561 | 0.985502 | 1.0409052 |
| IL.13 | 0.509434 | 0.169467 | 3.852638 | 1.173125 | 3.5537514 |
| IL.15 | 0.261816 | 0.103229 | 1.837707 | 0.710231 | 1.8016638 |
| IL.16 | 0.098611 | 0.346756 | 0.678245 | 2.480047 | 2.2332502 |
| IL.18 | -0.00769 | 0.131812 | 0.052689 | 0.908949 | 0.6799805 |
| IL.1alpha | 0.160743 | 0.048178 | 1.111642 | 0.330545 | 1.0197801 |
| IL.1beta | -0.11252 | 0.232568 | 0.774667 | 1.62412 | 1.6961985 |
| IL.1ra | -0.01402 | 0.05154 | 0.096101 | 0.353653 | 0.3180246 |
| IL.2 | 0.246858 | -0.18707 | 1.728061 | 1.297802 | 2.1396087 |
| IL.3 | 0.209462 | 0.046199 | 1.457568 | 0.316949 | 1.2547729 |
| IL.4 | 0.018434 | 0.06579 | 0.126393 | 0.451689 | 0.4087656 |
| IL.5 | -0.01354 | -0.06831 | 0.092799 | 0.469043 | 0.3972820 |
| IL.6 | -0.03488 | -0.1224 | 0.23921 | 0.843345 | 0.7654821 |
| IL.7 | 0.217022 | -0.05833 | 1.511867 | 0.400359 | 1.3521486 |
| IL.8 | -0.08272 | -0.05253 | 0.568424 | 0.36049 | 0.65684 |
| Insulin | 0.310197 | -0.06794 | 2.19904 | 0.466501 | 1.8848224 |
| Leptin | -0.00109 | -0.13677 | 0.007501 | 0.943564 | 0.672504 |
| LH..Luteinizing.Hormone. | 0.028652 | 0.088215 | 0.196485 | 0.606351 | 0.5676910 |
| Lymphotactin | -0.14318 | 0.142857 | 0.988402 | 0.986124 | 1.3962009 |
| MCP.1 | 0.251706 | 0.017496 | 1.763503 | 0.11996 | 1.3318096 |
| MCP.3 | 0.366232 | 0.162495 | 2.633011 | 1.123976 | 2.6565913 |
| M.CSF | 0.185109 | 0.243428 | 1.283843 | 1.703048 | 2.1120507 |

| probeID | p.AR-vs-CAN.Set_1 | p.AR-vs-CAN.Set_2 | p.AR-vs-CAN.Meta | q.AR-vs-CAN.Set_1 | q.AR-vs-CAN.Set_2 |
|---|---|---|---|---|---|
| FGF.basic | 0.008847 | 0.101876 | 0.002533 | 0.082388 | 0.5545758 |
| FGF.4 | 0.052702 | 0.15485 | 0.017514 | 0.187555 | 0.6186870 |
| Fibrinogen | 0.877861 | 0.382786 | 0.469812 | 0.638542 | 0.7183870 |
| FSH..Follicle.Stimulating.Hormone. | 0.333107 | 0.93029 | 0.457237 | 0.417256 | 0.8868466 |
| G.CSF | 0.511621 | 0.04533 | 0.060155 | 0.493124 | 0.5545758 |
| GLP.1.Total | 0.261441 | 0.594157 | 0.243392 | 0.39584 | 0.8418357 |
| Glucagon | 0.310945 | 0.831325 | 0.387712 | 0.417256 | 0.8658591 |
| GST | 0.023836 | 0.291396 | 0.018918 | 0.123421 | 0.7183870 |
| GM.CSF | 0.045351 | 0.72511 | 0.095928 | 0.182449 | 0.8658591 |
| GRO.alpha | 0.384368 | 0.271176 | 0.16507 | 0.43615 | 0.7138412 |
| Growth.Hormone | 0.754611 | 0.449192 | 0.451638 | 0.596784 | 0.7766084 |
| Haptoglobin | 0.841766 | 0.063016 | 0.145416 | 0.628537 | 0.5545758 |
| HB.EGF | 0.145346 | 0.956653 | 0.28632 | 0.292364 | 0.8924090 |
| HCC.4 | 0.084735 | 0.273583 | 0.04654 | 0.21842 | 0.7138412 |
| HGF..Hepatocyte.growth.factor. | 0.948483 | 0.612353 | 0.687678 | 0.645313 | 0.8418357 |
| I.309 | 0.216102 | 0.186326 | 0.071124 | 0.377278 | 0.6806341 |
| ICAM.1 | 0.36933 | 0.449495 | 0.244116 | 0.427173 | 0.7766084 |
| IFN.gamma | 0.32192 | 0.7776 | 0.369948 | 0.417256 | 0.8658591 |
| IgA | 0.282742 | 0.60941 | 0.264177 | 0.415269 | 0.8418357 |
| IgE | 0.170498 | 0.729679 | 0.22617 | 0.335662 | 0.8658591 |
| IGF.BP.2 | 0.773225 | 0.817819 | 0.715304 | 0.601227 | 0.8658591 |
| IGF.1 | 0.6255 | 0.117266 | 0.147098 | 0.54399 | 0.5666864 |
| IgM | 0.242657 | 0.002629 | 0.002949 | 0.39391 | 0.1680878 |
| IL.10 | 0.075118 | 0.97022 | 0.198798 | 0.218273 | 0.8924090 |
| IL.12p40 | 0.624876 | 0.32263 | 0.29792 | 0.54399 | 0.7183870 |
| IL.13 | 0.000158 | 0.23938 | 0.00038 | 0.004868 | 0.7073399 |
| IL.15 | 0.066258 | 0.475608 | 0.071598 | 0.218273 | 0.7897084 |
| IL.16 | 0.495675 | 0.013625 | 0.025532 | 0.48792 | 0.3484039 |
| IL.18 | 0.957756 | 0.361521 | 0.496517 | 0.646866 | 0.7183870 |
| IL.1alpha | 0.264787 | 0.739705 | 0.307833 | 0.39584 | 0.8658591 |
| IL.1beta | 0.436583 | 0.104103 | 0.089848 | 0.459054 | 0.5545758 |
| IL.1ra | 0.923034 | 0.722244 | 0.750466 | 0.645313 | 0.8658591 |
| IL.2 | 0.083942 | 0.1933 | 0.032386 | 0.21842 | 0.6864963 |

| | | | | | |
|---|---|---|---|---|---|
| IL.3 | 0.144316 | 0.750043 | 0.209561 | 0.292364 | 0.8658591 |
| IL.4 | 0.89889 | 0.649876 | 0.682712 | 0.639798 | 0.8565789 |
| IL.5 | 0.925671 | 0.637383 | 0.691159 | 0.645313 | 0.851291 |
| IL.6 | 0.809972 | 0.397116 | 0.443984 | 0.624552 | 0.7183870 |
| IL.7 | 0.130059 | 0.687404 | 0.176328 | 0.273507 | 0.8658591 |
| IL.8 | 0.567911 | 0.717106 | 0.511283 | 0.520282 | 0.8658591 |
| Insulin | 0.028355 | 0.639206 | 0.059454 | 0.126639 | 0.851291 |
| Leptin | 0.993983 | 0.343582 | 0.501263 | 0.661674 | 0.7183870 |
| LH..Luteinizing.Hormone. | 0.843421 | 0.542398 | 0.570245 | 0.628537 | 0.8355049 |
| Lymphotactin | 0.321213 | 0.322325 | 0.162654 | 0.417256 | 0.7183870 |
| MCP.1 | 0.077846 | 0.904011 | 0.182923 | 0.218273 | 0.8868466 |
| MCP.3 | 0.008904 | 0.259545 | 0.007894 | 0.082388 | 0.7073399 |
| M.CSF | 0.198106 | 0.088476 | 0.034682 | 0.352512 | 0.5545758 |

| probeID | cor.TX-vs-allOther.Set_1 | cor.TX-vs-allOther.Set_2 | Z.TX-vs-allOther.Set_1 | Z.TX-vs-allOther.Set_2 | Z.TX-vs-allOther.Meta |
|---|---|---|---|---|---|
| FGF.basic | 0.356752 | 0.316687 | 3.166363 | 2.644104 | 4.1086206 |
| FGF.4 | 0.06623 | 0.008563 | 0.562808 | 0.069042 | 0.4467855 |
| Fibrinogen | 0.132147 | 0.249889 | 1.127901 | 2.058246 | 2.2529458 |
| FSH..Follicle.Stimulating.Hormone. | 0.012906 | 0.061867 | 0.109517 | 0.499429 | 0.4305902 |
| G.CSF | -0.01698 | 0.405008 | 0.144122 | 3.46375 | 2.5511503 |
| GLP.1.Total | 0.241127 | 0.162682 | 2.087127 | 1.323338 | 2.4115632 |
| Glucagon | 0.185841 | 0.131348 | 1.595454 | 1.065118 | 1.8813087 |
| GST | -0.0809 | -0.16161 | 0.687938 | 1.314491 | 1.4159305 |
| GM.CSF | 0.088836 | -0.14659 | 0.755793 | 1.190439 | 1.3761937 |
| GRO.alpha | -0.14033 | 0.107268 | 1.198624 | 0.868159 | 1.4614365 |
| Growth.Hormone | 0.188339 | 0.111215 | 1.617414 | 0.900372 | 1.7803435 |
| Haptoglobin | -0.1792 | 0.264684 | 1.537199 | 2.185987 | 2.6326905 |
| HB.EGF | 0.596784 | 0.123921 | 5.83904 | 1.004246 | 4.8389340 |
| HCC.4 | 0.165811 | 0.293487 | 1.420061 | 2.437845 | 2.7279509 |
| HGF..Hepatocyte.growth.factor. | 0.108858 | 0.405882 | 0.927364 | 3.472177 | 3.1109453 |
| I.309 | 0.457165 | -0.0519 | 4.189365 | 0.418845 | 3.258496 |
| ICAM.1 | -0.03442 | 0.061185 | 0.292143 | 0.493903 | 0.5558190 |
| IFN.gamma | 0.22996 | -0.19664 | 1.986801 | 1.606287 | 2.540696 |
| IgA | 0.001203 | 0.024518 | 0.010208 | 0.197706 | 0.1470177 |
| IgE | 0.204969 | -0.18301 | 1.764207 | 1.492286 | 2.3026883 |
| IGF.BP.2 | 0.408558 | 0.441961 | 3.681589 | 3.826873 | 5.3092843 |
| IGF.1 | 0.064733 | 0.121021 | 0.55005 | 0.980509 | 1.0822685 |
| IgM | 0.256502 | 0.128106 | 2.226198 | 1.03853 | 2.308511 |
| IL.10 | 0.581771 | 0.2282 | 5.643864 | 1.872776 | 5.3150671 |
| IL.12p40 | 0.321709 | 0.064355 | 2.830287 | 0.519561 | 2.3687006 |
| IL.13 | 0.382378 | -0.23417 | 3.418226 | 1.923601 | 3.7772421 |
| IL.15 | 0.247069 | 0.060215 | 2.140746 | 0.486057 | 1.8574300 |
| IL.16 | 0.12106 | 0.08091 | 1.032293 | 0.653747 | 1.1922103 |
| IL.18 | 0.382994 | 0.253633 | 3.424348 | 2.090475 | 3.8995684 |
| IL.1alpha | -0.04039 | -0.22307 | 0.342891 | 1.829202 | 1.5359015 |
| IL.1beta | 0.140032 | -0.19632 | 1.196074 | 1.603589 | 1.9796605 |
| IL.1ra | 0.14626 | 0.419959 | 1.250024 | 3.609005 | 3.4358524 |
| IL.2 | -0.13059 | 0.062868 | 1.114424 | 0.507524 | 1.1468903 |
| IL.3 | 0.07995 | -0.40659 | 0.679847 | 3.47906 | 2.9407910 |
| IL.4 | 0.074635 | 0.14047 | 0.634481 | 1.140043 | 1.254777 |
| IL.5 | 0.147486 | -0.12791 | 1.260655 | 1.036912 | 1.624625 |
| IL.6 | 0.154938 | 0.12264 | 1.32537 | 0.993758 | 1.6398710 |
| IL.7 | 0.252337 | -0.04699 | 2.188413 | 0.379134 | 1.8155305 |
| IL.8 | 0.181431 | 0.400897 | 1.556724 | 3.424176 | 3.5220287 |
| Insulin | -0.04682 | -0.03783 | 0.397611 | 0.305102 | 0.4968928 |
| Leptin | -0.02293 | -0.04998 | 0.194563 | 0.403321 | 0.4227678 |
| LH..Luteinizing.Hormone. | -0.06446 | 0.002961 | 0.547687 | 0.023871 | 0.4041524 |
| Lymphotactin | -0.1349 | -0.09284 | 1.15168 | 0.750673 | 1.3451668 |
| MCP.1 | -0.1098 | 0.114437 | 0.935437 | 0.923398 | 1.3143948 |
| MCP.3 | 0.053832 | 0.022672 | 0.457225 | 0.182822 | 0.4525815 |
| M.CSF | 0.26953 | 0.378849 | 2.344968 | 3.214547 | 3.9311712 |

| probeID | p.TX-vs-allOther.Set_1 | p.TX-vs-allOther.Set_2 | p.TX-vs-allOther.Meta | q.TX-vs-allOther.Set_1 | q.TX-vs-allOther.Set_ |
|---|---|---|---|---|---|
| FGF.basic | 0.001679 | 0.00851 | 3.98E-05 | 0.005437 | 0.016869 |

| | | | | | |
|---|---|---|---|---|---|
| FGF.4 | 0.572373 | 0.944745 | 0.65503 | 0.388933 | 0.4090265 |
| Fibrinogen | 0.258405 | 0.039864 | 0.024263 | 0.223477 | 0.0526827 |
| FSH..Follicle.Stimulating.Hormone. | 0.912491 | 0.616235 | 0.666766 | 0.517096 | 0.3382822 |
| G.CSF | 0.885011 | 0.000613 | 0.010737 | 0.511167 | 0.0026027 |
| GLP.1.Total | 0.03716 | 0.185011 | 0.015884 | 0.057715 | 0.1507222 |
| Glucagon | 0.110413 | 0.285665 | 0.05993 | 0.129277 | 0.2139371 |
| GST | 0.490223 | 0.187952 | 0.156796 | 0.349051 | 0.1510495 |
| GM.CSF | 0.448497 | 0.232919 | 0.168762 | 0.328201 | 0.1846914 |
| GRO.alpha | 0.229825 | 0.383935 | 0.143896 | 0.209121 | 0.2506732 |
| Growth.Hormone | 0.105623 | 0.366574 | 0.07502 | 0.125601 | 0.2422273 |
| Haptoglobin | 0.123958 | 0.029166 | 0.008471 | 0.138732 | 0.0456455 |
| HB.EGF | 1.60E-08 | 0.314021 | 1.31E-06 | 6.04E-07 | 0.2200194 |
| HCC.4 | 0.155112 | 0.015139 | 0.006373 | 0.171084 | 0.0250091 |
| HGF..Hepatocyte.growth.factor. | 0.352543 | 0.000595 | 0.001865 | 0.279482 | 0.0026027 |
| I.309 | 3.74E-05 | 0.674219 | 0.00112 | 0.000219 | 0.3517222 |
| ICAM.1 | 0.769422 | 0.62014 | 0.578335 | 0.472234 | 0.3382822 |
| IFN.gamma | 0.047173 | 0.108008 | 0.011063 | 0.067738 | 0.1058752 |
| IgA | 0.991827 | 0.842686 | 0.883118 | 0.543044 | 0.3820397 |
| IgE | 0.077724 | 0.135226 | 0.021296 | 0.09697 | 0.1182643 |
| IGF.BP.2 | 0.000274 | 0.000161 | 1.10E-07 | 0.001225 | 0.0011981 |
| IGF.1 | 0.581105 | 0.325567 | 0.279133 | 0.389024 | 0.2200194 |
| IgM | 0.026326 | 0.297829 | 0.020971 | 0.046594 | 0.2139371 |
| IL.10 | 4.40E-08 | 0.061252 | 1.07E-07 | 6.70E-07 | 0.0714255 |
| IL.12p40 | 0.004885 | 0.602099 | 0.017851 | 0.01282 | 0.3382822 |
| IL.13 | 0.00071 | 0.054605 | 0.000159 | 0.002704 | 0.0649498 |
| IL.15 | 0.032598 | 0.625704 | 0.06325 | 0.052785 | 0.3382822 |
| IL.16 | 0.300849 | 0.511883 | 0.233179 | 0.248871 | 0.3044420 |
| IL.18 | 0.000695 | 0.036889 | 9.64E-05 | 0.002704 | 0.0525834 |
| IL.1alpha | 0.730818 | 0.067476 | 0.124563 | 0.452187 | 0.0771697 |
| IL.1beta | 0.230815 | 0.108598 | 0.047742 | 0.209121 | 0.1058752 |
| IL.1ra | 0.210525 | 0.000364 | 0.000591 | 0.197803 | 0.0021620 |
| IL.2 | 0.264121 | 0.610534 | 0.251427 | 0.22464 | 0.3382822 |
| IL.3 | 0.495335 | 0.00058 | 0.003274 | 0.349051 | 0.0026027 |
| IL.4 | 0.524521 | 0.253228 | 0.209559 | 0.366227 | 0.1981533 |
| IL.5 | 0.206685 | 0.29858 | 0.104242 | 0.197695 | 0.2139371 |
| IL.6 | 0.184409 | 0.319088 | 0.101032 | 0.194262 | 0.2200194 |
| IL.7 | 0.028957 | 0.703555 | 0.069442 | 0.047908 | 0.3566911 |
| IL.8 | 0.119279 | 0.000704 | 0.000428 | 0.136508 | 0.0027922 |
| Insulin | 0.689952 | 0.759421 | 0.619265 | 0.433957 | 0.3642205 |
| Leptin | 0.845212 | 0.685631 | 0.672465 | 0.502539 | 0.3545655 |
| LH..Luteinizing.Hormone. | 0.58273 | 0.980882 | 0.686101 | 0.389024 | 0.4168077 |
| Lymphotactin | 0.248531 | 0.451435 | 0.178571 | 0.222398 | 0.2809668 |
| MCP.1 | 0.348375 | 0.354472 | 0.188713 | 0.279482 | 0.2368620 |
| MCP.3 | 0.646446 | 0.854391 | 0.65085 | 0.416381 | 0.3820397 |
| M.CSF | 0.019364 | 0.001444 | 8.45E-05 | 0.035944 | 0.0043360 |

| probeID | cor.AR-vs-allOther.Set_1 | cor.AR-vs-allOther.Set_2 | Z.AR-vs-allOther.Set_1 | Z.AR-vs-allOther.Set_2 | Z.AR-vs-allOther.Meta |
|---|---|---|---|---|---|
| FGF.basic | 0.077346 | 0.043972 | 0.657616 | 0.354742 | 0.7158449 |
| FGF.4 | 0.180766 | 0.150036 | 1.550891 | 1.218826 | 1.9584855 |
| Fibrinogen | -0.08417 | -0.2218 | 0.715929 | 1.818409 | 1.7920471 |
| FSH..Follicle.Stimulating.Hormone. | 0.09398 | 0.00072 | 0.799805 | 0.005806 | 0.5696530 |
| G.CSF | 0.078596 | 0.011635 | 0.668291 | 0.093809 | 0.5388863 |
| GLP.1.Total | -0.02292 | -0.14895 | 0.19453 | 1.20989 | 0.9930746 |
| Glucagon | -0.00253 | -0.03584 | 0.021437 | 0.289062 | 0.2195556 |
| GST | -0.19299 | -0.05798 | 1.658401 | 0.467994 | 1.503588 |
| GM.CSF | 0.162092 | 0.030194 | 1.387639 | 0.243502 | 1.1533909 |
| GRO.alpha | -0.0411 | 0.06681 | 0.348935 | 0.53944 | 0.6281754 |
| Growth.Hormone | -0.05927 | -0.12021 | 0.503544 | 0.97387 | 1.044689 |
| Haptoglobin | 0.108033 | -0.32387 | 0.920281 | 2.708659 | 2.5660480 |
| HB.EGF | -0.16269 | 0.03826 | 1.392949 | 0.308631 | 1.2031277 |
| HCC.4 | -0.26526 | -0.24324 | 2.305957 | 2.001176 | 3.0456028 |
| HGF..Hepatocyte.growth.factor. | -0.04898 | -0.24028 | 0.415941 | 1.975811 | 1.6912236 |
| I.309 | -0.07944 | 0.169972 | 0.675508 | 1.383789 | 1.4561426 |
| ICAM.1 | -0.07212 | -0.13676 | 0.613012 | 1.109506 | 1.2180041 |
| IFN.gamma | -0.16881 | 0.086655 | 1.446227 | 0.70039 | 1.5178878 |

EP 2 806 272 A1

| | | | | | |
|---|---|---|---|---|---|
| IgA | 0.109341 | 0.045344 | 0.931511 | 0.365824 | 0.917354 |
| IgE | -0.27505 | 0.116888 | 2.395516 | 0.946706 | 2.363307{ |
| IGF.BP.2 | -0.22848 | -0.19373 | 1.973557 | 1.581898 | 2.514086{ |
| IGF.1 | -0.00761 | 0.107491 | 0.064549 | 0.869985 | 0.660815{ |
| IgM | 0.008175 | 0.282064 | 0.069372 | 2.337435 | 1.701869{ |
| IL.10 | -0.13785 | -0.10496 | 1.177176 | 0.849368 | 1.432982 |
| IL.12p40 | -0.12047 | -0.14403 | 1.027189 | 1.169301 | 1.553152{ |
| IL.13 | 0.194134 | 0.230269 | 1.668458 | 1.89039 | 2.516485 |
| IL.15 | 0.072723 | 0.049583 | 0.618166 | 0.400082 | 0.720010{ |
| IL.16 | 0.036664 | 0.216975 | 0.311242 | 1.777558 | 1.477004{ |
| IL.18 | -0.19097 | -0.02089 | 1.640597 | 0.168406 | 1.279158 |
| IL.1alpha | 0.131015 | 0.136905 | 1.118128 | 1.110737 | 1.576045{ |
| IL.1beta | -0.15057 | 0.136774 | 1.287437 | 1.109661 | 1.69500{ |
| IL.1ra | -0.0782 | -0.1333 | 0.664886 | 1.081138 | 1.234625 |
| IL.2 | 0.238462 | -0.1795 | 2.063134 | 1.463062 | 2.493397 |
| IL.3 | 0.111105 | 0.214391 | 0.946666 | 1.755711 | 1.910869 |
| IL.4 | -0.0262 | -0.01034 | 0.22234 | 0.08333 | 0.216141{ |
| IL.5 | -0.06083 | 0.007354 | 0.516792 | 0.059292 | 0.407353{ |
| IL.6 | -0.09943 | -0.1447 | 0.846526 | 1.174848 | 1.429327{ |
| IL.7 | 0.021694 | -0.02205 | 0.18411 | 0.17778 | 0.255894{ |
| IL.8 | -0.14541 | -0.23241 | 1.242657 | 1.908651 | 2.228310{ |
| Insulin | 0.224752 | -0.03302 | 1.940204 | 0.266341 | 1.560262{ |
| Leptin | 0.031308 | -0.07819 | 0.265743 | 0.631708 | 0.634593 |
| LH..Luteinizing.Hormone. | 0.079306 | 0.051326 | 0.67435 | 0.414169 | 0.769699{ |
| Lymphotactin | -0.01584 | 0.132733 | 0.134411 | 1.076479 | 0.856228{ |
| MCP.1 | 0.234783 | -0.0559 | 2.030067 | 0.451177 | 1.754504{ |
| MCP.3 | 0.219624 | 0.09841 | 1.894437 | 0.795985 | 1.90241{ |
| M.CSF | -0.00806 | 0.006047 | 0.068372 | 0.048756 | 0.08282 |

| probeID | p.AR-vs-allOther.Set_1 | p.AR-vs-allOther.Set_2 | p.AR-vs-allOther.Meta | q.AR-vs-allOther.Set_1 | q.AR-vs-allOther.Set_ |
|---|---|---|---|---|---|
| FGF.basic | 0.509527 | 0.721802 | 0.474087 | 0.553516 | 0.756466{ |
| FGF.4 | 0.120662 | 0.222005 | 0.050173 | 0.38479 | 0.523705{ |
| Fibrinogen | 0.472761 | 0.069096 | 0.073125 | 0.553516 | 0.364263{ |
| FSH..Follicle.Stimulating.Hormone. | 0.422555 | 0.99535 | 0.568913 | 0.54787 | 0.811088{ |
| G.CSF | 0.502685 | 0.924974 | 0.589965 | 0.553516 | 0.797781{ |
| GLP.1.Total | 0.845238 | 0.2254 | 0.320674 | 0.643381 | 0.523705{ |
| Glucagon | 0.982838 | 0.771706 | 0.826217 | 0.671626 | 0.770308{ |
| GST | 0.097129 | 0.638593 | 0.132687 | 0.356984 | 0.754909 |
| GM.CSF | 0.164714 | 0.806904 | 0.24875 | 0.42514 | 0.77994 |
| GRO.alpha | 0.726264 | 0.588289 | 0.529889 | 0.631649 | 0.723964{ |
| Growth.Hormone | 0.613455 | 0.328845 | 0.296167 | 0.575382 | 0.603848{ |
| Haptoglobin | 0.356225 | 0.007055 | 0.010286 | 0.528951 | 0.202616 |
| HB.EGF | 0.163141 | 0.756727 | 0.228927 | 0.42514 | 0.769217{ |
| HCC.4 | 0.021447 | 0.045635 | 0.002322 | 0.256481 | 0.336242{ |
| HGF..Hepatocyte.growth.factor. | 0.676456 | 0.04842 | 0.090794 | 0.604824 | 0.336242{ |
| I.309 | 0.498088 | 0.165824 | 0.145353 | 0.553516 | 0.419530{ |
| ICAM.1 | 0.538635 | 0.266122 | 0.223222 | 0.553516 | 0.528322{ |
| IFN.gamma | 0.147679 | 0.482274 | 0.129043 | 0.42514 | 0.667465{ |
| IgA | 0.350398 | 0.713491 | 0.358957 | 0.528951 | 0.756466{ |
| IgE | 0.01693 | 0.342484 | 0.018113 | 0.256481 | 0.609239{ |
| IGF.BP.2 | 0.048652 | 0.11343 | 0.011934 | 0.298848 | 0.409993{ |
| IGF.1 | 0.948354 | 0.382938 | 0.508731 | 0.652724 | 0.652574{ |
| IgM | 0.944501 | 0.019787 | 0.08878 | 0.652724 | 0.30608{ |
| IL.10 | 0.238243 | 0.394293 | 0.151863 | 0.497507 | 0.652574{ |
| IL.12p40 | 0.30324 | 0.241291 | 0.120387 | 0.528951 | 0.528322{ |
| IL.13 | 0.095131 | 0.058876 | 0.011853 | 0.356984 | 0.336242{ |
| IL.15 | 0.535229 | 0.688021 | 0.471519 | 0.553516 | 0.756466{ |
| IL.16 | 0.754821 | 0.075521 | 0.139674 | 0.63491 | 0.364263{ |
| IL.18 | 0.100749 | 0.865759 | 0.200841 | 0.356984 | 0.77994 |
| IL.1alpha | 0.262541 | 0.265594 | 0.115015 | 0.497507 | 0.528322{ |
| IL.1beta | 0.197239 | 0.266055 | 0.090075 | 0.471744 | 0.528322{ |
| IL.1ra | 0.504862 | 0.278502 | 0.21697 | 0.553516 | 0.528322{ |
| IL.2 | 0.039372 | 0.142999 | 0.012653 | 0.298848 | 0.419530{ |
| IL.3 | 0.342631 | 0.079155 | 0.056021 | 0.528951 | 0.364263{ |
| IL.4 | 0.823457 | 0.933334 | 0.828878 | 0.643381 | 0.798939{ |

74

| | | | | | |
|---|---|---|---|---|---|
| IL.5 | 0.604158 | 0.952538 | 0.683749 | 0.572273 | 0.804033 |
| IL.6 | 0.396009 | 0.239073 | 0.15291 | 0.531527 | 0.5283228 |
| IL.7 | 0.85343 | 0.858364 | 0.798032 | 0.643381 | 0.779941 |
| IL.8 | 0.213217 | 0.056494 | 0.02586 | 0.497507 | 0.3362428 |
| Insulin | 0.05255 | 0.789205 | 0.118698 | 0.298848 | 0.7745690 |
| Leptin | 0.789742 | 0.526199 | 0.525693 | 0.63491 | 0.6846704 |
| LH..Luteinizing.Hormone. | 0.498824 | 0.677648 | 0.441478 | 0.553516 | 0.7564665 |
| Lymphotactin | 0.89271 | 0.280572 | 0.391871 | 0.65242 | 0.5283228 |
| MCP.1 | 0.042604 | 0.650694 | 0.079344 | 0.298848 | 0.755925 |
| MCP.3 | 0.058325 | 0.424634 | 0.057117 | 0.298848 | 0.6525745 |
| M.CSF | 0.945301 | 0.960964 | 0.933993 | 0.652724 | 0.804033 |

| probeID | cor.CAN-vs-allOther.Set_1 | cor.CAN-vs-allOther.Set_2 | Z.CAN-vs-allOther.Set_1 | Z.CAN-vs-allOther.Set_2 | Z.CAN-vs-allOther.M(e |
|---|---|---|---|---|---|
| FGF.basic | -0.4341 | -0.33374 | 3.945108 | 2.797836 | 4.7679818 |
| FGF.4 | -0.247 | -0.15787 | 2.140085 | 1.283531 | 2.4208620 |
| Fibrinogen | -0.04797 | -0.00685 | 0.407382 | 0.055214 | 0.3271054 |
| FSH..Follicle.Stimulating.Hormone. | -0.10689 | -0.05733 | 0.910434 | 0.462703 | 0.9709544 |
| G.CSF | -0.06161 | -0.38221 | 0.523468 | 3.246267 | 2.6656055 |
| GLP.1.Total | -0.21821 | 9.97E-05 | 1.88179 | 0.000804 | 1.3311943 |
| Glucagon | -0.18331 | -0.08434 | 1.573261 | 0.681625 | 1.5944452 |
| GST | 0.27389 | 0.205857 | 2.384914 | 1.683731 | 2.8769664 |
| GM.CSF | -0.25093 | 0.103937 | 2.175656 | 0.841003 | 2.1331001 |
| GRO.alpha | 0.181426 | -0.16496 | 1.556685 | 1.342202 | 2.0498227 |
| Growth.Hormone | -0.12906 | 0.018448 | 1.101294 | 0.148753 | 0.8839165 |
| Haptoglobin | 0.071172 | 0.08169 | 0.604934 | 0.660073 | 0.8944952 |
| HB.EGF | -0.43409 | -0.07512 | 3.945064 | 0.606817 | 3.2186655 |
| HCC.4 | 0.099451 | -0.0253 | 0.846667 | 0.203999 | 0.7429331 |
| HGF..Hepatocyte.growth.factor. | -0.05988 | -0.1311 | 0.50869 | 1.063082 | 1.1114102 |
| I.309 | -0.37772 | -0.12248 | 3.372064 | 0.992446 | 3.0861749 |
| ICAM.1 | 0.106535 | 0.080772 | 0.907419 | 0.652623 | 1.1031165 |
| IFN.gamma | -0.06115 | 0.093269 | 0.519536 | 0.754154 | 0.9006346 |
| IgA | -0.11054 | -0.06778 | 0.941844 | 0.547276 | 1.0529667 |
| IgE | 0.070077 | 0.050565 | 0.595598 | 0.408016 | 0.7096624 |
| IGF.BP.2 | -0.18008 | -0.21066 | 1.544854 | 1.724209 | 2.3115769 |
| IGF.1 | -0.05713 | -0.21822 | 0.485261 | 1.788138 | 1.6075360 |
| IgM | -0.26468 | -0.39928 | 2.300624 | 3.408658 | 4.0370718 |
| IL.10 | -0.44392 | -0.10384 | 4.048377 | 0.840194 | 3.4567416 |
| IL.12p40 | -0.20124 | 0.085142 | 1.731222 | 0.688099 | 1.7107185 |
| IL.13 | -0.57651 | -0.01601 | 5.576717 | 0.129079 | 4.0346071 |
| IL.15 | -0.31979 | -0.10468 | 2.812157 | 0.847057 | 2.5874548 |
| IL.16 | -0.15772 | -0.29101 | 1.349599 | 2.415981 | 2.6626673 |
| IL.18 | -0.19202 | -0.21119 | 1.649835 | 1.728653 | 2.3889522 |
| IL.1alpha | -0.09063 | 0.067202 | 0.771112 | 0.54262 | 0.9289487 |
| IL.1beta | 0.01054 | 0.042856 | 0.089437 | 0.345728 | 0.3077078 |
| IL.1ra | -0.06806 | -0.25096 | 0.578425 | 2.067446 | 1.8709130 |
| IL.2 | -0.10788 | 0.121981 | 0.918932 | 0.988365 | 1.3486623 |
| IL.3 | -0.19105 | 0.15764 | 1.641322 | 1.281618 | 2.0668311 |
| IL.4 | -0.04844 | -0.11819 | 0.411334 | 0.95738 | 0.9678267 |
| IL.5 | -0.08666 | 0.109681 | 0.737155 | 0.887849 | 1.1490518 |
| IL.6 | -0.0555 | 0.032485 | 0.471452 | 0.261992 | 0.5186228 |
| IL.7 | -0.27403 | 0.065044 | 2.386205 | 0.525142 | 2.0586329 |
| IL.8 | -0.03602 | -0.1344 | 0.305775 | 1.090194 | 0.9870989 |
| Insulin | -0.17793 | 0.067633 | 1.526005 | 0.54611 | 1.4652063 |
| Leptin | -0.00838 | 0.123929 | 0.071129 | 1.004309 | 0.7604492 |
| LH..Luteinizing.Hormone. | -0.01485 | -0.05404 | 0.126016 | 0.436072 | 0.3974564 |
| Lymphotactin | 0.150739 | -0.04778 | 1.288881 | 0.385539 | 1.1839940 |
| MCP.1 | -0.12499 | -0.04844 | 1.06611 | 0.390825 | 1.0302088 |
| MCP.3 | -0.27346 | -0.11916 | 2.380938 | 0.965246 | 2.3661096 |
| M.CSF | -0.26147 | -0.35269 | 2.271413 | 2.971055 | 3.7069845 |

| probeID | p.CAN-vs-allOther.Set_1 | p.CAN-vs-allOther.Set_2 | p.CAN-vs-allOther.Meta | q.CAN-vs-allOther.Set_1 | q.CAN-vs-allOther.Se |
|---|---|---|---|---|---|
| FGF.basic | 9.98E-05 | 0.005415 | 1.86E-06 | 0.000689 | 0.0358380 |
| FGF.4 | 0.032651 | 0.198519 | 0.015484 | 0.042171 | 0.2506120 |
| Fibrinogen | 0.682744 | 0.955798 | 0.743588 | 0.344738 | 0.461768 |

| | | | | | |
|---|---|---|---|---|---|
| FSH..Follicle.Stimulating.Hormone. | 0.361385 | 0.64239 | 0.331571 | 0.24473 | 0.372969' |
| G.CSF | 0.599498 | 0.001298 | 0.007685 | 0.333955 | 0.014317: |
| GLP.1.Total | 0.060012 | 0.999356 | 0.183125 | 0.064261 | 0.472466f |
| Glucagon | 0.115427 | 0.494072 | 0.110836 | 0.110289 | 0.338260< |
| GST | 0.017417 | 0.092151 | 0.004015 | 0.027331 | 0.178671; |
| GM.CSF | 0.029895 | 0.398958 | 0.032917 | 0.039504 | 0.314716f |
| GRO.alpha | 0.119289 | 0.178854 | 0.040382 | 0.112251 | 0.249248< |
| Growth.Hormone | 0.269773 | 0.8813 | 0.376741 | 0.206847 | 0.443264f |
| Haptoglobin | 0.543995 | 0.507812 | 0.371057 | 0.32607 | 0.339284f |
| HB.EGF | 9.98E-05 | 0.542611 | 0.001288 | 0.000689 | 0.345330( |
| HCC.4 | 0.395931 | 0.837748 | 0.457522 | 0.261594 | 0.433193f |
| HGF..Hepatocyte.growth.factor. | 0.609837 | 0.286585 | 0.266392 | 0.335174 | 0.296895< |
| I.309 | 0.000835 | 0.319726 | 0.002027 | 0.003051 | 0.296895< |
| ICAM.1 | 0.362974 | 0.512608 | 0.269977 | 0.24473 | 0.339284f |
| IFN.gamma | 0.602241 | 0.449343 | 0.367783 | 0.333955 | 0.336477( |
| IgA | 0.34509 | 0.582885 | 0.292356 | 0.243549 | 0.355892f |
| IgE | 0.550223 | 0.682172 | 0.477913 | 0.32607 | 0.389237: |
| IGF.BP.2 | 0.122107 | 0.084645 | 0.020801 | 0.113188 | 0.169772 |
| IGF.1 | 0.626388 | 0.073812 | 0.107937 | 0.337728 | 0.162848: |
| IgM | 0.021747 | 0.000744 | 5.41E-05 | 0.03141 | 0.011747: |
| IL.10 | 6.63E-05 | 0.399411 | 0.000547 | 0.000588 | 0.314716f |
| IL.12p40 | 0.083399 | 0.489984 | 0.087133 | 0.08779 | 0.338260< |
| IL.13 | 6.20E-08 | 0.896904 | 5.47E-05 | 1.93E-06 | 0.443264f |
| IL.15 | 0.005161 | 0.395579 | 0.009669 | 0.010684 | 4716f |
| IL.16 | 0.176547 | 0.016059 | 0.007752 | 0.152287 | 7675< |
| IL.18 | 0.098857 | 0.083852 | 0.016896 | 0.100407 | 6977: |
| IL.1alpha | 0.439366 | 0.586093 | 0.352916 | 0.284244 | 5892f |
| IL.1beta | 0.928488 | 0.728586 | 0.758305 | 0.426132 | 1863: |
| IL.1ra | 0.561771 | 0.038994 | 0.061357 | 0.32607 | 3238( |
| IL.2 | 0.356929 | 0.321715 | 0.177445 | 0.24473 | 6895< |
| IL.3 | 0.100599 | 0.199186 | 0.03875 | 0.100407 | 0612( |
| IL.4 | 0.679838 | 0.337082 | 0.333131 | 0.344738 | 7476< |
| IL.5 | 0.459751 | 0.373264 | 0.250535 | 0.294366 | 9219f |
| IL.6 | 0.636234 | 0.792557 | 0.604024 | 0.337728 | 0.419667' |
| IL.7 | 0.017357 | 0.598208 | 0.039529 | 0.027331 | 0.35814f |
| IL.8 | 0.758992 | 0.274508 | 0.323594 | 0.371167 | 0.296895< |
| Insulin | 0.126704 | 0.583688 | 0.142865 | 0.115722 | 0.355892f |
| Leptin | 0.943099 | 0.31399 | 0.446986 | 0.427536 | 0.296895< |
| LH..Luteinizing.Hormone. | 0.899374 | 0.661643 | 0.691031 | 0.423158 | 0.380806f |
| Lymphotactin | 0.196739 | 0.698792 | 0.236415 | 0.162916 | 0.391962f |
| MCP.1 | 0.285331 | 0.69487 | 0.302912 | 0.213504 | 0.391962f |
| MCP.3 | 0.017603 | 0.333136 | 0.017976 | 0.027331 | 0.297476< |
| M.CSF | 0.023454 | 0.003179 | 0.00021 | 0.033105 | 0.024607: |

| probeID | cor.TX-vs-AR.Set_1 | cor.TX-vs-AR.Set_2 | Z.TX-vs-AR.Set_1 | Z.TX-vs-AR.Set_2 | Z.TX-vs-AR.Meta |
|---|---|---|---|---|---|
| MDC | 0.020738 | -0.00217 | 0.14219 | 0.01375 | 0.110266 |
| MIF | 0.141713 | 0.364004 | 0.978121 | 2.412778 | 2.397727 |
| MIP.1alpha | 0.271918 | 0.311548 | 1.912272 | 2.038147 | 2.793368 |
| MIP.1beta | 0.022338 | 0.272983 | 0.153165 | 1.771405 | 1.360876 |
| MMP.2 | 0.166311 | 0.184576 | 1.150864 | 1.180899 | 1.648805 |
| MMP.3 | 0.441913 | 0.448621 | 3.253728 | 3.054585 | 4.460651 |
| MMP.9 | 0.02408 | 0.115966 | 0.165113 | 0.73675 | 0.637713 |
| Myeloperoxidase | 0.040536 | 0.113764 | 0.278052 | 0.722634 | 0.707592 |
| Myoglobin | 0.315895 | 0.324539 | 2.242354 | 2.129553 | 3.091405 |
| NGF.beta | -0.14286 | 0.117298 | 0.986124 | 0.745286 | 1.224692 |
| NrCAM | 0.334256 | 0.394634 | 2.383111 | 2.63909 | 3.551232 |
| PAI.1 | -0.07952 | -0.04094 | 0.5463 | 0.259099 | 0.569503 |
| Pancreatic.Polypeptide | 0.110162 | 0.119287 | 0.758313 | 0.758045 | 1.072227 |
| PAPP.A | 0.260508 | 0.148734 | 1.828085 | 0.947706 | 1.96278 |
| PDGF | -0.24143 | -0.10628 | 1.688463 | 0.674692 | 1.671003 |
| Progesterone | 0.162804 | 0.408228 | 1.126152 | 2.741585 | 2.734903 |
| Prolactin | 0.365822 | 0.193777 | 2.629764 | 1.241247 | 2.737218 |
| Prostate.Specific.Antigen..Free | 0.07475 | 0.122761 | 0.513415 | 0.780348 | 0.914828 |

| | | | | | |
|---|---|---|---|---|---|
| Prostatic. Acid.Phos phatase | 0.090012 | -0.21533 | 0.618763 | 1.383534 | 1.415838 |
| PARC | 0.160196 | 0.160555 | 1.107793 | 1.024304 | 1.50762 |
| Peptide.Y Y | 0.454903 | 0.412226 | 3.365202 | 2.771987 | 4.339648 |
| RANTES | -0.074 | -0.08958 | 0.508249 | 0.568059 | 0.761065 |
| Resistin | 0.445155 | 0.265246 | 3.281401 | 1.718656 | 3.535574 |
| Secretin | 0.084473 | 0.219683 | 0.580499 | 1.412419 | 1.409206 |
| Serum.Am yloid.P | 0.055371 | 0.388127 | 0.379994 | 2.59049 | 2.100449 |
| SGOT | 0.138216 | -0.16549 | 0.953667 | 1.056337 | 1.421288 |
| SHBG | -0.16855 | -0.22307 | 1.166651 | 1.434921 | 1.839589 |
| SOD | -0.02863 | 0.04003 | 0.196302 | 0.253307 | 0.317921 |
| Sortilin | 0.105133 | 0.061774 | 0.723429 | 0.391191 | 0.788156 |
| sRAGE | -0.25618 | 0.01949 | 1.796305 | 0.12328 | 1.357352 |
| Stem.Cell. Factor | 0.414095 | 0.511028 | 3.020215 | 3.567812 | 4.658439 |
| Tenascin. C | 0.315288 | 0.380769 | 2.237727 | 2.535887 | 3.375455 |
| Testostero ne | 0.07893 | 0.021649 | 0.542242 | 0.136939 | 0.480253 |
| TGF.alpha | -0.13341 | 0.285182 | 0.920091 | 1.855082 | 1.962343 |
| TGF.b3 | -0.08839 | 0.132805 | 0.607575 | 0.844925 | 1.027072 |
| Thrombop oietin | -0.39584 | -0.2175 | 2.870466 | 1.397888 | 3.018182 |
| TECK | 0.187007 | 0.440059 | 1.297326 | 2.987114 | 3.029556 |
| Thyroid.St imulating. Hormone | -0.04246 | -0.26793 | 0.291238 | 1.73696 | 1.434152 |
| Thyroxine. Binding.Gl obulin | -0.31751 | -0.29424 | 2.254635 | 1.917629 | 2.950236 |
| TIMP.1 | 0.287496 | 0.473723 | 2.02814 | 3.256258 | 3.736634 |
| Tissue.Fa ctor | 0.073635 | -0.05403 | 0.505732 | 0.342037 | 0.599463 |
| TNF.RII | 0.565472 | 0.609502 | 4.393389 | 4.478595 | 6.273439 |
| TNF.alpha | 0.33396 | 0.380832 | 2.380825 | 2.536354 | 3.476971 |
| TNF.beta | 0.055466 | -0.13252 | 0.380644 | 0.843087 | 0.865309 |

| | | | | | |
|---|---|---|---|---|---|
| TRAIL.R3 | 0.437182 | 0.291242 | 3.213527 | 1.89688 | 3.613604 |
| VCAM.1 | 0.444814 | 0.329508 | 3.278484 | 2.164746 | 3.848945 |
| VEGF | 0.515781 | 0.550142 | 3.911663 | 3.912274 | 5.532359 |
| von.Willeb rand.Fact or | 0.122292 | 0.417867 | 0.842611 | 2.815093 | 2.586388 |

| probeID | p.TX-vs- AR.Set_1 | p.TX-vs- AR.Set_2 | p.TX-vs- AR.Meta | q.TX-vs- AR.Set_1 | q.TX-vs- AR.Set_2 | q.TX-vs- AR.Meta |
|---|---|---|---|---|---|---|
| MDC | 0.886335 | 0.988961 | 0.912198 | 0.374357 | 0.556195 | 0.05684 |
| MIF | 0.326255 | 0.016415 | 0.016497 | 0.227793 | 0.040389 | 0.003115 |
| MIP.1alph a | 0.056097 | 0.041983 | 0.005216 | 0.086356 | 0.082641 | 0.00123 |
| MIP.1beta | 0.877629 | 0.076539 | 0.173553 | 0.374357 | 0.128222 | 0.017121 |
| MMP.2 | 0.248371 | 0.236058 | 0.099188 | 0.204286 | 0.26552 | 0.011537 |
| MMP.3 | 0.001314 | 0.002551 | 8.17E-06 | 0.007335 | 0.015448 | 7.92E-06 |
| MMP.9 | 0.868168 | 0.458974 | 0.52366 | 0.374047 | 0.360957 | 0.037754 |
| Myelopero xidase | 0.779864 | 0.467606 | 0.479199 | 0.351953 | 0.360957 | 0.035171 |
| Myoglobin | 0.025431 | 0.03373 | 0.001992 | 0.052262 | 0.06989 | 0.000617 |
| NGF.beta | 0.322325 | 0.453798 | 0.220842 | 0.227793 | 0.360957 | 0.021171 |
| NrCAM | 0.01767 | 0.008825 | 0.000383 | 0.043958 | 0.025734 | 0.000152 |
| PAI.1 | 0.583057 | 0.794334 | 0.569015 | 0.312583 | 0.500046 | 0.039324 |
| Pancreatic .Polypepti de | 0.446307 | 0.446124 | 0.283618 | 0.272928 | 0.360957 | 0.024991 |
| PAPP.A | 0.067674 | 0.341157 | 0.049672 | 0.098686 | 0.316638 | 0.007111 |
| PDGF | 0.091211 | 0.497592 | 0.094721 | 0.115273 | 0.36961 | 0.011319 |
| Progester one | 0.258628 | 0.006576 | 0.00624 | 0.204286 | 0.020566 | 0.001396 |
| Prolactin | 0.008987 | 0.213103 | 0.006196 | 0.025554 | 0.250433 | 0.001396 |
| Prostate.S pecific.Ant igen..Free | 0.605915 | 0.432892 | 0.360282 | 0.312583 | 0.358783 | 0.029933 |
| Prostatic. Acid.Phos phatase | 0.534176 | 0.165519 | 0.156823 | 0.306864 | 0.213645 | 0.015908 |
| PARC | 0.266437 | 0.303715 | 0.131652 | 0.207572 | 0.297054 | 0.014356 |

| | | | | | | |
|---|---|---|---|---|---|---|
| Peptide.YY | 0.000903 | 0.006018 | 1.43E-05 | 0.007335 | 0.020566 | 1.24E-05 |
| RANTES | 0.609542 | 0.567838 | 0.446618 | 0.312583 | 0.40645 | 0.0333 |
| Resistin | 0.001198 | 0.08561 | 0.000407 | 0.007335 | 0.132169 | 0.000154 |
| Secretin | 0.559726 | 0.156931 | 0.158774 | 0.309054 | 0.209428 | 0.01592 |
| Serum.Amyloid.P | 0.702518 | 0.010117 | 0.035689 | 0.332945 | 0.027849 | 0.005535 |
| SGOT | 0.338455 | 0.288909 | 0.155233 | 0.227793 | 0.297054 | 0.015908 |
| SHBG | 0.241971 | 0.150482 | 0.065829 | 0.20397 | 0.204283 | 0.008701 |
| SOD | 0.843565 | 0.798831 | 0.750545 | 0.366221 | 0.500046 | 0.047791 |
| Sortilin | 0.46746 | 0.693936 | 0.430606 | 0.279845 | 0.46574 | 0.032664 |
| sRAGE | 0.072532 | 0.901276 | 0.17467 | 0.103126 | 0.533559 | 0.017121 |
| Stem.Cell.Factor | 0.002794 | 0.000462 | 3.19E-06 | 0.011351 | 0.003639 | 3.97E-06 |
| Tenascin.C | 0.025731 | 0.011769 | 0.000737 | 0.052262 | 0.029893 | 0.000257 |
| Testosterone | 0.585856 | 0.890405 | 0.631047 | 0.312583 | 0.533559 | 0.042674 |
| TGF.alpha | 0.355683 | 0.063788 | 0.049723 | 0.235213 | 0.111609 | 0.007111 |
| TGF.b3 | 0.541585 | 0.395898 | 0.304386 | 0.308009 | 0.347248 | 0.026291 |
| Thrombopoietin | 0.004435 | 0.161208 | 0.002543 | 0.015765 | 0.211549 | 0.000693 |
| TECK | 0.193463 | 0.003148 | 0.002449 | 0.183376 | 0.015517 | 0.000689 |
| Thyroid.Stimulating.Hormone | 0.769716 | 0.082367 | 0.151529 | 0.3502 | 0.129706 | 0.015737 |
| Thyroxine.Binding.Globulin | 0.024652 | 0.05546 | 0.003175 | 0.052262 | 0.101552 | 0.000815 |
| TIMP.1 | 0.042924 | 0.001333 | 0.000186 | 0.075 | 0.008748 | 0.000102 |
| Tissue.Factor | 0.611313 | 0.730772 | 0.548864 | 0.312583 | 0.483516 | 0.038927 |
| TNF.RII | 1.88E-05 | 1.44E-05 | 3.53E-10 | 0.000356 | 0.000566 | 1.54E-09 |
| TNF.alpha | 0.017777 | 0.011754 | 0.000507 | 0.043958 | 0.029893 | 0.000184 |
| TNF.beta | 0.702034 | 0.396923 | 0.386869 | 0.332945 | 0.347248 | 0.030154 |
| TRAIL.R3 | 0.001501 | 0.058115 | 0.000302 | 0.007335 | 0.103994 | 0.000138 |
| VCAM.1 | 0.00121 | 0.03095 | 0.000119 | 0.007335 | 0.067691 | 6.90E-05 |
| VEGF | 0.000126 | 0.000133 | 3.16E-08 | 0.001197 | 0.001491 | 6.89E-08 |
| von.Willeb | 0.397526 | 0.0053 | 0.009699 | 0.254022 | 0.019871 | 0.001968 |

rand.Fact
or

| probeID | cor.TX-vs-CAN.Set_1 | cor.TX-vs-CAN.Set_2 | Z.TX-vs-CAN.Set_1 | Z.TX-vs-CAN.Set_2 | Z.TX-vs-CAN.Meta |
|---|---|---|---|---|---|
| MDC | 0.184673 | -0.20418 | 1.280746 | 1.30977 | 1.831771 |
| MIF | -0.03028 | 0.173263 | 0.207656 | 1.106978 | 0.929587 |
| MIP.1alpha | 0.313204 | 0.271348 | 2.221875 | 1.760243 | 2.815783 |
| MIP.1beta | -0.04866 | 0.217295 | 0.333889 | 1.396561 | 1.223613 |
| MMP.2 | 0.289563 | 0.218129 | 2.043599 | 1.402093 | 2.436472 |
| MMP.3 | 0.590033 | 0.498382 | 4.646195 | 3.460484 | 5.732288 |
| MMP.9 | 0.147526 | -0.0469 | 1.018823 | 0.29681 | 0.930293 |
| Myeloperoxidase | -0.32289 | 0.164726 | 2.295728 | 1.051399 | 2.366777 |
| Myoglobin | 0.485366 | 0.439982 | 3.633357 | 2.986508 | 4.680952 |
| NGF.beta | -0.14286 | 0.289265 | 0.986124 | 1.883221 | 2.028934 |
| NrCAM | 0.4261 | 0.387812 | 3.120157 | 2.588144 | 4.036379 |
| PAI.1 | -0.24008 | 0.078037 | 1.678639 | 0.494557 | 1.536681 |
| Pancreatic.Polypeptide | 0.334016 | 0.318936 | 2.381255 | 2.090024 | 3.161671 |
| PAPP.A | 0.371781 | 0.036952 | 2.677054 | 0.233814 | 2.058295 |
| PDGF | -0.35852 | 0.1521 | 2.572147 | 0.969488 | 2.504314 |
| Progesterone | 0.112085 | 0.140025 | 0.771657 | 0.891451 | 1.175995 |
| Prolactin | 0.606472 | 0.30348 | 4.821735 | 1.981791 | 4.810819 |
| Prostate.Specific.Antigen..Free | 0.171232 | 0.083955 | 1.185586 | 0.532233 | 1.214682 |
| Prostatic.Acid.Phosphatase | 0.125276 | -0.34453 | 0.863383 | 2.271932 | 2.217002 |
| PARC | 0.155144 | -0.10163 | 1.072272 | 0.645016 | 1.214306 |
| Peptide.YY | 0.305702 | 0.359425 | 2.165 | 2.379455 | 3.213415 |
| RANTES | -0.37216 | 0.036513 | 2.680046 | 0.231032 | 2.058443 |

| | | | | | |
|---|---|---|---|---|---|
| Resistin | 0.741489 | 0.465578 | 6.538783 | 3.190168 | 6.879407 |
| Secretin | 0.223501 | 0.236074 | 1.558552 | 1.521769 | 2.178115 |
| Serum.Amyloid.P | -0.10503 | 0.184815 | 0.722717 | 1.182458 | 1.347162 |
| SGOT | 0.478992 | -0.11072 | 3.576428 | 0.703143 | 3.026114 |
| SHBG | 0.361378 | 0.086673 | 2.594657 | 0.549548 | 2.223289 |
| SOD | -0.64202 | 0.10839 | 5.221275 | 0.688222 | 4.178645 |
| Sortilin | -0.20894 | 0.031076 | 1.453793 | 0.196605 | 1.167008 |
| sRAGE | 0.194518 | 0.018859 | 1.350757 | 0.119287 | 1.039478 |
| Stem.Cell.Factor | 0.621128 | 0.653251 | 4.982956 | 4.939153 | 7.01599 |
| Tenascin.C | 0.315348 | 0.448986 | 2.238184 | 3.057479 | 3.744599 |
| Testosterone | -0.06065 | -0.233 | 0.41628 | 1.50117 | 1.355842 |
| TGF.alpha | -0.06146 | 0.318665 | 0.421897 | 2.08812 | 1.774849 |
| TGF.b3 | 0.026359 | 0.185642 | 0.180747 | 1.187878 | 0.967764 |
| Thrombopoietin | -0.31356 | -0.38236 | 2.224602 | 2.547646 | 3.374489 |
| TECK | 0.32947 | 0.334517 | 2.346236 | 2.200346 | 3.214919 |
| Thyroid.Stimulating.Hormone | 0.050745 | -0.31956 | 0.348192 | 2.094415 | 1.727184 |
| Thyroxine.Binding.Globulin | 0.043194 | -0.07695 | 0.29631 | 0.487635 | 0.554333 |
| TIMP.1 | 0.169449 | 0.399849 | 1.172995 | 2.678252 | 2.723243 |
| Tissue.Factor | 0.297355 | 0.047158 | 2.10205 | 0.298473 | 1.697426 |
| TNF.RII | 0.68866 | 0.680426 | 5.795793 | 5.248792 | 7.8097 |
| TNF.alpha | 0.386616 | 0.31424 | 2.795844 | 2.057015 | 3.431489 |
| TNF.beta | 2.08E-17 | -0.02166 | 0 | 0.136981 | 0.09686 |
| TRAIL.R3 | 0.607785 | 0.409098 | 4.835988 | 2.748192 | 5.362825 |
| VCAM.1 | 0.510293 | 0.382044 | 3.860593 | 2.545326 | 4.529669 |
| VEGF | 0.691197 | 0.727241 | 5.828977 | 5.83659 | 8.248802 |
| von.Willebrand.Factor | 0.009654 | 0.412935 | 0.066189 | 2.777391 | 2.010714 |

81

| probeID | p.TX-vs-CAN.Set_1 | p.TX-vs-CAN.Set_2 | p.TX-vs-CAN.Meta | q.TX-vs-CAN.Set_1 | q.TX-vs-CAN.Set_2 | q.TX-vs-CAN.Meta |
|---|---|---|---|---|---|---|
| MDC | 0.199184 | 0.189067 | 0.066986 | 0.171894 | 0.179082 | 0.007235 |
| MIF | 0.834645 | 0.266521 | 0.352585 | 0.487639 | 0.206125 | 0.024971 |
| MIP.1alpha | 0.026779 | 0.078389 | 0.004866 | 0.038441 | 0.096979 | 0.000914 |
| MIP.1beta | 0.73717 | 0.161603 | 0.221098 | 0.448937 | 0.160596 | 0.017644 |
| MMP.2 | 0.041384 | 0.159961 | 0.014831 | 0.055357 | 0.160596 | 0.002288 |
| MMP.3 | 6.50E-06 | 0.000671 | 9.91E-09 | 3.25E-05 | 0.003697 | 1.22E-08 |
| MMP.9 | 0.306596 | 0.765233 | 0.352219 | 0.234349 | 0.372511 | 0.024971 |
| Myeloperoxidase | 0.022193 | 0.291158 | 0.017944 | 0.034634 | 0.210119 | 0.002628 |
| Myoglobin | 0.000353 | 0.003154 | 2.86E-06 | 0.001244 | 0.010621 | 1.45E-06 |
| NGF.beta | 0.322325 | 0.05992 | 0.042465 | 0.243883 | 0.080719 | 0.004962 |
| NrCAM | 0.002033 | 0.010183 | 5.43E-05 | 0.005077 | 0.025721 | 1.95E-05 |
| PAI.1 | 0.093091 | 0.618903 | 0.124371 | 0.104077 | 0.323211 | 0.011306 |
| Pancreatic.Polypeptide | 0.017757 | 0.037109 | 0.001569 | 0.028916 | 0.057945 | 0.000339 |
| PAPP.A | 0.007851 | 0.814012 | 0.039562 | 0.015078 | 0.377854 | 0.004748 |
| PDGF | 0.01057 | 0.330217 | 0.012269 | 0.017994 | 0.224918 | 0.001927 |
| Progesterone | 0.438363 | 0.370482 | 0.239597 | 0.315736 | 0.246337 | 0.01865 |
| Prolactin | 3.04E-06 | 0.04789 | 1.50E-06 | 1.75E-05 | 0.067513 | 8.66E-07 |
| Prostate.Specific.Antigen..Free | 0.23445 | 0.592473 | 0.224487 | 0.1908 | 0.321288 | 0.017644 |
| Prostatic.Acid.Phosphatase | 0.386024 | 0.023671 | 0.026623 | 0.28349 | 0.043483 | 0.003594 |
| PARC | 0.282009 | 0.516661 | 0.224631 | 0.217778 | 0.295471 | 0.017644 |
| Peptide.YY | 0.030854 | 0.017925 | 0.001312 | 0.0428 | 0.03622 | 0.000291 |
| RANTES | 0.007783 | 0.816185 | 0.039548 | 0.015078 | 0.377854 | 0.004748 |
| Resistin | 7.31E-10 | 0.001654 | 6.01E-12 | 5.47E-08 | 0.007164 | 8.65E-12 |
| Secretin | 0.118706 | 0.127494 | 0.029397 | 0.120161 | 0.145824 | 0.003908 |
| Serum.Am | 0.467898 | 0.235443 | 0.177928 | 0.33065 | 0.193128 | 0.015072 |

| yloid.P | | | | | | |
|---|---|---|---|---|---|
| SGOT | 0.000433 | 0.479673 | 0.002477 | 0.001381 | 0.291815 | 0.000522 |
| SHBG | 0.009924 | 0.580505 | 0.026196 | 0.017287 | 0.321288 | 0.003593 |
| SOD | 5.03E-07 | 0.489026 | 2.93E-05 | 4.38E-06 | 0.291815 | 1.13E-05 |
| Sortilin | 0.145351 | 0.843184 | 0.243207 | 0.140169 | 0.387226 | 0.018762 |
| sRAGE | 0.175855 | 0.904459 | 0.298582 | 0.163295 | 0.404243 | 0.021679 |
| Stem.Cell. Factor | 1.49E-06 | 2.05E-06 | 2.28E-12 | 1.01E-05 | 2.48E-05 | 3.95E-12 |
| Tenascin. C | 0.025701 | 0.002527 | 0.000181 | 0.038441 | 0.009013 | 5.20E-05 |
| Testostero ne | 0.675675 | 0.132679 | 0.17515 | 0.428923 | 0.146794 | 0.014983 |
| TGF.alpha | 0.671556 | 0.037279 | 0.075923 | 0.428923 | 0.057945 | 0.007903 |
| TGF.b3 | 0.855818 | 0.233316 | 0.333162 | 0.492675 | 0.193128 | 0.023988 |
| Thrombop oietin | 0.026596 | 0.011394 | 0.00074 | 0.038441 | 0.026737 | 0.000173 |
| TECK | 0.019469 | 0.028341 | 0.001305 | 0.031029 | 0.049956 | 0.000291 |
| Thyroid.St imulating. Hormone | 0.726358 | 0.03672 | 0.084135 | 0.445978 | 0.057945 | 0.008453 |
| Thyroxine. Binding.Gl obulin | 0.765822 | 0.623815 | 0.579351 | 0.458924 | 0.323211 | 0.038212 |
| TIMP.1 | 0.239432 | 0.007894 | 0.006464 | 0.1908 | 0.020807 | 0.001188 |
| Tissue.Fa ctor | 0.035982 | 0.763957 | 0.089616 | 0.049006 | 0.372511 | 0.0089 |
| TNF.RII | 3.24E-08 | 5.17E-07 | 5.73E-15 | 4.04E-07 | 7.84E-06 | 1.34E-14 |
| TNF.alpha | 0.005548 | 0.040151 | 0.0006 | 0.011231 | 0.059364 | 0.000148 |
| TNF.beta | 1 | 0.890371 | 0.922837 | 0.535051 | 0.402793 | 0.056954 |
| TRAIL.R3 | 2.85E-06 | 0.006451 | 8.19E-08 | 1.75E-05 | 0.017776 | 7.87E-08 |
| VCAM.1 | 0.000153 | 0.011467 | 5.91E-06 | 0.000574 | 0.026737 | 2.69E-06 |
| VEGF | 2.75E-08 | 3.33E-08 | 1.60E-16 | 4.04E-07 | 1.58E-06 | 9.46E-16 |
| von.Willeb rand.Fact or | 0.946947 | 0.005923 | 0.044356 | 0.521567 | 0.017098 | 0.00511 |

| probeID | cor.AR-vs-CAN.Set_ | cor.AR-vs-CAN.Set_ | Z.AR-vs-CAN.Set_ | Z.AR-vs-CAN.Set_ | Z.AR-vs-CAN.Meta |
|---|---|---|---|---|---|

| | 1 | 2 | 1 | 2 | |
|---|---|---|---|---|---|
| MDC | 0.103413 | -0.167 | 0.711505 | 1.155692 | 1.320308 |
| MIF | -0.10158 | -0.1924 | 0.698807 | 1.335643 | 1.438573 |
| MIP.1alpha | 0.045648 | -0.02223 | 0.313167 | 0.152442 | 0.329236 |
| MIP.1beta | -0.04728 | -0.04417 | 0.324356 | 0.303 | 0.443608 |
| MMP.2 | 0.161158 | 0.121929 | 1.114561 | 0.840083 | 1.382142 |
| MMP.3 | 0.125146 | 0.076195 | 0.862482 | 0.523379 | 0.979952 |
| MMP.9 | 0.110438 | -0.14408 | 0.760225 | 0.994719 | 1.240933 |
| Myeloperoxidase | -0.4953 | 0.040019 | 3.723039 | 0.274501 | 2.826688 |
| Myoglobin | 0.280657 | 0.197606 | 1.977136 | 1.37278 | 2.368748 |
| NGF.beta | NA | 0.017836 | NA | 0.12229 | 0.12229 |
| NrCAM | 0.087839 | 0.028086 | 0.603747 | 0.192599 | 0.563102 |
| PAI.1 | -0.16141 | 0.128355 | 1.116356 | 0.88484 | 1.415059 |
| Pancreatic.Polypeptide | 0.187375 | 0.17151 | 1.299934 | 1.187552 | 1.758919 |
| PAPP.A | 0.092539 | -0.14486 | 0.636233 | 1.000163 | 1.157106 |
| PDGF | -0.02785 | 0.241131 | 0.190953 | 1.686315 | 1.327429 |
| Progesterone | -0.07019 | -0.25156 | 0.481967 | 1.76246 | 1.58705 |
| Prolactin | 0.442612 | 0.162334 | 3.259687 | 1.122836 | 3.098911 |
| Prostate.Specific.Antigen..Free | 0.145264 | -0.35282 | 1.002975 | 2.527376 | 2.496336 |
| Prostatic.Acid.Phosphatase | -0.02486 | -0.13245 | 0.170494 | 0.913422 | 0.766444 |
| PARC | -0.0276 | -0.16478 | 0.189263 | 1.140064 | 0.939976 |
| Peptide.YY | 0.011431 | -0.05506 | 0.078371 | 0.377834 | 0.322586 |
| RANTES | -0.22576 | 0.137612 | 1.574834 | 0.949447 | 1.784936 |
| Resistin | 0.300631 | 0.209598 | 2.126714 | 1.458547 | 2.535163 |
| Secretin | 0.086681 | -0.01147 | 0.595753 | 0.078617 | 0.476852 |
| Serum.Amyloid.P | -0.15056 | -0.27262 | 1.040095 | 1.917472 | 2.091316 |
| SGOT | 0.258178 | 0.048635 | 1.810955 | 0.333687 | 1.516491 |

| | | | | | |
|---|---|---|---|---|---|
| SHBG | 0.424867 | 0.224435 | 3.109835 | 1.565293 | 3.305814 |
| SOD | -0.58662 | 0.125594 | 4.610396 | 0.865603 | 3.872116 |
| Sortilin | -0.31869 | -0.03931 | 2.263645 | 0.269604 | 1.791278 |
| sRAGE | 0.316927 | 0.001461 | 2.25021 | 0.010013 | 1.598219 |
| Stem.Cell.Factor | 0.236723 | 0.24356 | 1.654269 | 1.704009 | 2.374661 |
| Tenascin.C | 0.058677 | 0.182305 | 0.402735 | 1.263948 | 1.178523 |
| Testosterone | -0.16059 | -0.29424 | 1.110594 | 2.078635 | 2.255126 |
| TGF.alpha | 0.18877 | 0.032865 | 1.309849 | 0.22539 | 1.085578 |
| TGF.b3 | 0.115033 | 0.085377 | 0.792132 | 0.586742 | 0.975012 |
| Thrombopoietin | 0.138222 | -0.17447 | 0.953708 | 1.208463 | 1.528886 |
| TECK | 0.117975 | -0.01031 | 0.812578 | 0.070668 | 0.624549 |
| Thyroid.Stimulating.Hormone | 0.105729 | -0.10608 | 0.72756 | 0.730024 | 1.030667 |
| Thyroxine.Binding.Globulin | 0.399715 | 0.22207 | 2.902068 | 1.548231 | 3.146837 |
| TIMP.1 | -0.14848 | -0.03518 | 1.025495 | 0.241276 | 0.895742 |
| Tissue.Factor | 0.309481 | 0.100877 | 2.193614 | 0.69394 | 2.041809 |
| TNF.RII | 0.150577 | 0.272693 | 1.040216 | 1.918015 | 2.091785 |
| TNF.alpha | 0.095617 | -0.07475 | 0.657525 | 0.513393 | 0.827964 |
| TNF.beta | -0.05864 | 0.077571 | 0.402446 | 0.532868 | 0.661367 |
| TRAIL.R3 | 0.228393 | 0.175987 | 1.593894 | 1.219199 | 1.989157 |
| VCAM.1 | -0.01033 | 0.122415 | 0.070845 | 0.843465 | 0.646515 |
| VEGF | 0.344022 | 0.269567 | 2.458765 | 1.894885 | 3.078495 |
| von.Willebrand.Factor | -0.13041 | -0.01308 | 0.899169 | 0.089651 | 0.699201 |

| probeID | p.AR-vs-CAN.Set_1 | p.AR-vs-CAN.Set_2 | p.AR-vs-CAN.Meta | q.AR-vs-CAN.Set_1 | q.AR-vs-CAN.Set_2 | q.AR-vs-CAN.Meta |
|---|---|---|---|---|---|---|
| MDC | 0.474818 | 0.246401 | 0.186732 | 0.482798 | 0.70734 | 0.012673 |

| | | | | | | |
|---|---|---|---|---|---|---|
| MIF | 0.482723 | 0.180709 | 0.150272 | 0.483563 | 0.67953 | 0.012314 |
| MIP.1alpha | 0.752927 | 0.878202 | 0.741978 | 0.596784 | 0.886847 | 0.027883 |
| MIP.1beta | 0.744405 | 0.760696 | 0.657326 | 0.596784 | 0.865859 | 0.026983 |
| MMP.2 | 0.26354 | 0.398941 | 0.166928 | 0.39584 | 0.718387 | 0.012314 |
| MMP.3 | 0.386519 | 0.598946 | 0.32711 | 0.43615 | 0.841836 | 0.017117 |
| MMP.9 | 0.445164 | 0.31814 | 0.214631 | 0.462817 | 0.718387 | 0.013656 |
| Myeloperoxidase | 0.000255 | 0.782603 | 0.004703 | 0.005903 | 0.865859 | 0.001911 |
| Myoglobin | 0.048356 | 0.16896 | 0.017848 | 0.186431 | 0.654602 | 0.003955 |
| NGF.beta | NA | 0.902155 | 0.902669 | NA | 0.886847 | 0.032819 |
| NrCAM | 0.544132 | 0.846478 | 0.573366 | 0.513556 | 0.866332 | 0.02412 |
| PAI.1 | 0.262774 | 0.374361 | 0.157051 | 0.39584 | 0.718387 | 0.012314 |
| Pancreatic.Polypeptide | 0.192574 | 0.233679 | 0.078591 | 0.349387 | 0.70734 | 0.008511 |
| PAPP.A | 0.522712 | 0.315508 | 0.247229 | 0.498621 | 0.718387 | 0.014633 |
| PDGF | 0.847774 | 0.091619 | 0.184367 | 0.628537 | 0.554576 | 0.012673 |
| Progesterone | 0.628145 | 0.07802 | 0.112501 | 0.54399 | 0.554576 | 0.010805 |
| Prolactin | 0.001288 | 0.260026 | 0.001942 | 0.023839 | 0.70734 | 0.001324 |
| Prostate.Specific.Antigen..Free | 0.314154 | 0.011968 | 0.012548 | 0.417256 | 0.348404 | 0.003757 |
| Prostatic.Acid.Phosphatase | 0.863914 | 0.35917 | 0.443412 | 0.634424 | 0.718387 | 0.020733 |
| PARC | 0.849105 | 0.252817 | 0.34723 | 0.628537 | 0.70734 | 0.017853 |
| Peptide.YY | 0.937201 | 0.704128 | 0.747009 | 0.645313 | 0.865859 | 0.027883 |
| RANTES | 0.114936 | 0.340591 | 0.074272 | 0.253214 | 0.718387 | 0.008329 |
| Resistin | 0.033892 | 0.144049 | 0.01124 | 0.142544 | 0.613899 | 0.003576 |
| Secretin | 0.549469 | 0.937005 | 0.633468 | 0.513556 | 0.886847 | 0.026215 |
| Serum.Amyloid.P | 0.296644 | 0.05544 | 0.0365 | 0.417256 | 0.554576 | 0.005993 |
| SGOT | 0.070258 | 0.737323 | 0.129395 | 0.218273 | 0.865859 | 0.011975 |
| SHBG | 0.002102 | 0.117134 | 0.000947 | 0.027785 | 0.566686 | 0.000964 |
| SOD | 7.57E-06 | 0.384807 | 0.000108 | 0.000701 | 0.718387 | 0.000275 |

| | | | | | | |
|---|---|---|---|---|---|---|
| Sortilin | 0.024093 | 0.786385 | 0.073249 | 0.123421 | 0.865859 | 0.008329 |
| sRAGE | 0.02493 | 0.991968 | 0.109994 | 0.123421 | 0.905896 | 0.010767 |
| Stem.Cell.Factor | 0.097891 | 0.088298 | 0.017565 | 0.244804 | 0.554576 | 0.003955 |
| Tenascin.C | 0.685649 | 0.205107 | 0.238588 | 0.576931 | 0.70734 | 0.014618 |
| Testosterone | 0.265235 | 0.038068 | 0.024125 | 0.39584 | 0.554576 | 0.004912 |
| TGF.alpha | 0.189223 | 0.820757 | 0.277666 | 0.349387 | 0.865859 | 0.015704 |
| TGF.b3 | 0.426336 | 0.555517 | 0.329555 | 0.458704 | 0.841836 | 0.017117 |
| Thrombopoietin | 0.338435 | 0.225589 | 0.126293 | 0.417256 | 0.70734 | 0.011904 |
| TECK | 0.414521 | 0.943363 | 0.532267 | 0.45124 | 0.886847 | 0.022767 |
| Thyroid.Stimulating.Hormone | 0.464926 | 0.463419 | 0.302697 | 0.477993 | 0.789428 | 0.016218 |
| Thyroxine.Binding.Globulin | 0.004029 | 0.121146 | 0.00165 | 0.046602 | 0.566686 | 0.001324 |
| TIMP.1 | 0.303451 | 0.808363 | 0.37039 | 0.417256 | 0.865859 | 0.018666 |
| Tissue.Factor | 0.028741 | 0.485772 | 0.04117 | 0.126639 | 0.796072 | 0.00635 |
| TNF.RII | 0.296588 | 0.055372 | 0.036458 | 0.417256 | 0.554576 | 0.005993 |
| TNF.alpha | 0.508915 | 0.605931 | 0.407691 | 0.493124 | 0.841836 | 0.019764 |
| TNF.beta | 0.685862 | 0.592344 | 0.508377 | 0.576931 | 0.841836 | 0.022243 |
| TRAIL.R3 | 0.110645 | 0.221515 | 0.046684 | 0.253214 | 0.70734 | 0.006601 |
| VCAM.1 | 0.943221 | 0.397049 | 0.517946 | 0.645313 | 0.718387 | 0.022342 |
| VEGF | 0.014435 | 0.058341 | 0.00208 | 0.102742 | 0.554576 | 0.001324 |
| von.Willebrand.Factor | 0.366696 | 0.928185 | 0.484426 | 0.427173 | 0.886847 | 0.021821 |

| probeID | cor.TX-vs-allOther.Set_1 | cor.TX-vs-allOther.Set_2 | Z.TX-vs-allOther.Set_1 | Z.TX-vs-allOther.Set_2 | Z.TX-vs-allOther.Meta |
|---|---|---|---|---|---|
| MDC | 0.085351 | -0.08747 | 0.725997 | 0.706989 | 1.013274 |
| MIF | 0.016748 | 0.262886 | 0.142128 | 2.170413 | 1.635214 |
| MIP.1alph | 0.28169 | 0.253312 | 2.456629 | 2.087715 | 3.213336 |

a

| | | | | | |
|---|---|---|---|---|---|
| MIP.1beta | -0.0122 | 0.213583 | 0.103531 | 1.748883 | 1.309854 |
| MMP.2 | 0.216655 | 0.190024 | 1.867983 | 1.550873 | 2.417496 |
| MMP.3 | 0.477651 | 0.433179 | 4.411809 | 3.7393 | 5.763705 |
| MMP.9 | 0.080224 | 0.024681 | 0.682187 | 0.199023 | 0.62311 |
| Myeloperoxidase | -0.14194 | 0.121186 | 1.212547 | 0.981862 | 1.551681 |
| Myoglobin | 0.370785 | 0.34722 | 3.303594 | 2.920786 | 4.401301 |
| NGF.beta | -0.1644 | 0.130386 | 1.407747 | 1.057225 | 1.742999 |
| NrCAM | 0.356109 | 0.343946 | 3.160111 | 2.890808 | 4.278646 |
| PAI.1 | -0.15094 | 0.024729 | 1.290667 | 0.199409 | 1.053643 |
| Pancreatic.Polypeptide | 0.23187 | 0.210082 | 2.003922 | 1.719335 | 2.63274 |
| PAPP.A | 0.306516 | 0.191152 | 2.687255 | 1.560309 | 3.003482 |
| PDGF | -0.28215 | 0.025756 | 2.460882 | 0.207698 | 1.886971 |
| Progesterone | 0.150933 | 0.242195 | 1.290568 | 1.99222 | 2.321282 |
| Prolactin | 0.454698 | 0.199473 | 4.162936 | 1.630054 | 4.096262 |
| Prostate.Specific.Antigen..Free | 0.109009 | -0.04202 | 0.928658 | 0.338956 | 0.896338 |
| Prostatic.Acid.Phosphatase | 0.072363 | -0.25033 | 0.615094 | 2.062057 | 1.893032 |
| PARC | 0.148516 | 0.043472 | 1.269588 | 0.350704 | 1.14572 |
| Peptide.YY | 0.363236 | 0.34968 | 3.229568 | 2.943362 | 4.364921 |
| RANTES | -0.20822 | -0.02349 | 1.793052 | 0.189391 | 1.401799 |
| Resistin | 0.518695 | 0.323071 | 4.875244 | 2.701438 | 5.357523 |
| Secretin | 0.125258 | 0.189078 | 1.068462 | 1.542963 | 1.846556 |
| Serum.Amyloid.P | -0.01967 | 0.242773 | 0.166901 | 1.997174 | 1.530232 |
| SGOT | 0.264746 | -0.12352 | 2.301251 | 1.000984 | 2.335033 |
| SHBG | 0.112484 | -0.06857 | 0.958512 | 0.553691 | 1.069289 |
| SOD | -0.30918 | 0.08366 | 2.712198 | 0.676069 | 2.395867 |
| Sortilin | -0.04429 | 0.034962 | 0.376087 | 0.28199 | 0.465331 |
| sRAGE | -0.05465 | 0.019511 | 0.464221 | 0.15732 | 0.439496 |

| Stem.Cell. Factor | 0.485245 | 0.5154 | 4.49569 | 4.595932 | 6.428748 |
|---|---|---|---|---|---|
| Tenascin. C | 0.304279 | 0.39645 | 2.66632 | 3.381555 | 4.276493 |
| Testosterone | 0.007738 | -0.09854 | 0.065663 | 0.797077 | 0.61005 |
| TGF.alpha | -0.10708 | 0.274568 | 0.912099 | 2.271924 | 2.251444 |
| TGF.b3 | -0.02423 | 0.129616 | 0.205642 | 1.050909 | 0.888516 |
| Thrombopoietin | -0.33237 | -0.23915 | 2.931586 | 1.966158 | 3.463228 |
| TECK | 0.214433 | 0.339491 | 1.848212 | 2.850142 | 3.322237 |
| Thyroid.Stimulating. Hormone | 1.53E-05 | -0.25996 | 0.000129 | 2.145112 | 1.516914 |
| Thyroxine. Binding.Globulin | -0.13412 | -0.17346 | 1.144912 | 1.412786 | 1.808566 |
| TIMP.1 | 0.21861 | 0.391884 | 1.88539 | 3.33797 | 3.693474 |
| Tissue.Factor | 0.149151 | 0.002911 | 1.275101 | 0.023467 | 0.918226 |
| TNF.RII | 0.58186 | 0.578802 | 5.645007 | 5.326405 | 7.757959 |
| TNF.alpha | 0.342067 | 0.309458 | 3.024426 | 2.579487 | 3.962565 |
| TNF.beta | 0.027471 | -0.05735 | 0.233157 | 0.462882 | 0.492174 |
| TRAIL.R3 | 0.480544 | 0.32049 | 4.443666 | 2.67823 | 5.035941 |
| VCAM.1 | 0.441897 | 0.315102 | 4.026989 | 2.62991 | 4.707138 |
| VEGF | 0.590959 | 0.581946 | 5.76268 | 5.364633 | 7.868198 |
| von.Willebrand.Factor | 0.060914 | 0.38273 | 0.51751 | 3.251136 | 2.664835 |

| probeID | p.TX-vs-allOther.Set_1 | p.TX-vs-allOther.Set_2 | p.TX-vs-allOther.Meta | q.TX-vs-allOther.Set_1 | q.TX-vs-allOther.Set_2 | q.TX-vs-allOther.Meta |
|---|---|---|---|---|---|---|
| MDC | 0.466565 | 0.478162 | 0.310929 | 0.338171 | 0.293162 | 0.001164 |
| MIF | 0.886591 | 0.030321 | 0.102004 | 0.511167 | 0.046236 | 0.000517 |
| MIP.1alpha | 0.014352 | 0.037136 | 0.001312 | 0.028744 | 0.052583 | 1.53E-05 |
| MIP.1beta | 0.917257 | 0.080319 | 0.190245 | 0.517096 | 0.086848 | 0.000811 |

| | | | | | | |
|---|---|---|---|---|---|---|
| MMP.2 | 0.061899 | 0.120637 | 0.015628 | 0.082646 | 0.110375 | 0.000118 |
| MMP.3 | 1.47E-05 | 0.000224 | 8.23E-09 | 0.000124 | 0.001483 | 5.91E-10 |
| MMP.9 | 0.493853 | 0.841653 | 0.533212 | 0.349051 | 0.38204 | 0.001852 |
| Myeloperoxidase | 0.224477 | 0.324901 | 0.120739 | 0.208339 | 0.220019 | 0.000598 |
| Myoglobin | 0.001058 | 0.00372 | 1.08E-05 | 0.003833 | 0.009217 | 2.73E-07 |
| NGF.beta | 0.158707 | 0.28924 | 0.081334 | 0.172549 | 0.213937 | 0.000433 |
| NrCAM | 0.001715 | 0.004081 | 1.88E-05 | 0.005437 | 0.009708 | 3.89E-07 |
| PAI.1 | 0.196122 | 0.841349 | 0.292046 | 0.196946 | 0.38204 | 0.001111 |
| Pancreatic.Polypeptide | 0.045318 | 0.085522 | 0.00847 | 0.066325 | 0.090822 | 6.89E-05 |
| PAPP.A | 0.007481 | 0.118408 | 0.002669 | 0.017792 | 0.110028 | 2.87E-05 |
| PDGF | 0.014186 | 0.834849 | 0.059164 | 0.028744 | 0.38204 | 0.000354 |
| Progesterone | 0.196156 | 0.046602 | 0.020272 | 0.196946 | 0.057739 | 0.000141 |
| Prolactin | 4.16E-05 | 0.102926 | 4.20E-05 | 0.000226 | 0.105536 | 7.86E-07 |
| Prostate.Specific.Antigen..Free | 0.351872 | 0.733697 | 0.370072 | 0.279482 | 0.357652 | 0.001351 |
| Prostatic.Acid.Phosphatase | 0.537258 | 0.039502 | 0.058354 | 0.37171 | 0.052683 | 0.000354 |
| PARC | 0.203498 | 0.724838 | 0.251911 | 0.197695 | 0.357652 | 0.000987 |
| Peptide.YY | 0.00136 | 0.003467 | 1.27E-05 | 0.004704 | 0.008965 | 3.04E-07 |
| RANTES | 0.073024 | 0.849221 | 0.160975 | 0.092798 | 0.38204 | 0.000722 |
| Resistin | 1.87E-06 | 0.007206 | 8.44E-08 | 2.37E-05 | 0.015304 | 4.31E-09 |
| Secretin | 0.284272 | 0.122531 | 0.064811 | 0.237743 | 0.110409 | 0.000372 |
| Serum.Amyloid.P | 0.866995 | 0.046065 | 0.125959 | 0.50756 | 0.057739 | 0.000603 |
| SGOT | 0.021711 | 0.315591 | 0.019542 | 0.039342 | 0.220019 | 0.000138 |
| SHBG | 0.336638 | 0.57848 | 0.284939 | 0.275482 | 0.334006 | 0.001096 |
| SOD | 0.006954 | 0.497595 | 0.016581 | 0.01764 | 0.298913 | 0.000121 |
| Sortilin | 0.705926 | 0.77714 | 0.641695 | 0.440365 | 0.369737 | 0.002115 |
| sRAGE | 0.641416 | 0.874519 | 0.660302 | 0.416381 | 0.385247 | 0.002123 |
| Stem.Cell.Factor | 1.02E-05 | 6.87E-06 | 1.29E-10 | 0.000111 | 8.18E-05 | 1.11E-11 |

| | | | | | | |
|---|---|---|---|---|---|---|
| Tenascin.C | 0.007951 | 0.000817 | 1.90E-05 | 0.018338 | 0.003038 | 3.89E-07 |
| Testosterone | 0.947465 | 0.424 | 0.541829 | 0.526328 | 0.271136 | 0.001867 |
| TGF.alpha | 0.360509 | 0.023457 | 0.024357 | 0.282852 | 0.037703 | 0.000159 |
| TGF.b3 | 0.83652 | 0.292123 | 0.374263 | 0.501287 | 0.213937 | 0.001355 |
| Thrombopoietin | 0.003575 | 0.049516 | 0.000534 | 0.009718 | 0.060098 | 6.97E-06 |
| TECK | 0.064688 | 0.004622 | 0.000893 | 0.084881 | 0.010573 | 1.10E-05 |
| Thyroid.Stimulating.Hormone | 0.999896 | 0.03228 | 0.129288 | 0.543552 | 0.047994 | 0.000605 |
| Thyroxine.Binding.Globulin | 0.251314 | 0.157174 | 0.070519 | 0.222398 | 0.133532 | 0.000393 |
| TIMP.1 | 0.059528 | 0.00095 | 0.000221 | 0.080899 | 0.003324 | 3.07E-06 |
| Tissue.Factor | 0.20155 | 0.981206 | 0.3585 | 0.197695 | 0.416808 | 0.00132 |
| TNF.RII | 4.38E-08 | 2.34E-07 | 8.63E-15 | 6.70E-07 | 3.49E-06 | 1.24E-15 |
| TNF.alpha | 0.002666 | 0.010231 | 7.41E-05 | 0.007515 | 0.017896 | 1.28E-06 |
| TNF.beta | 0.815021 | 0.642262 | 0.622596 | 0.49228 | 0.341034 | 0.002095 |
| TRAIL.R3 | 1.28E-05 | 0.00771 | 4.76E-07 | 0.000122 | 0.015811 | 1.58E-08 |
| VCAM.1 | 7.22E-05 | 0.008864 | 2.51E-06 | 0.000366 | 0.017004 | 6.76E-08 |
| VEGF | 2.38E-08 | 1.94E-07 | 3.60E-15 | 6.04E-07 | 3.49E-06 | 7.75E-16 |
| von.Willebrand.Factor | 0.603656 | 0.001277 | 0.007703 | 0.39949 | 0.004218 | 6.64E-05 |

| probeID | cor.AR-vs-allOther.Set_1 | cor.AR-vs-allOther.Set_2 | Z.AR-vs-allOther.Set_1 | Z.AR-vs-allOther.Set_2 | Z.AR-vs-allOther.Meta |
|---|---|---|---|---|---|
| MDC | 0.044152 | -0.10158 | 0.374886 | 0.821797 | 0.846182 |
| MIF | -0.07082 | -0.26011 | 0.601937 | 2.146353 | 1.943335 |
| MIP.1alpha | -0.11093 | -0.13617 | 0.945173 | 1.104693 | 1.449474 |
| MIP.1beta | -0.03377 | -0.13205 | 0.286655 | 1.07088 | 0.959922 |
| MMP.2 | 0.01271 | -0.00345 | 0.107854 | 0.027818 | 0.095934 |
| MMP.3 | -0.14378 | -0.14949 | 1.228546 | 1.214319 | 1.727366 |

| | | | | | |
|---|---|---|---|---|---|
| MMP.9 | 0.035644 | -0.09215 | 0.302578 | 0.745027 | 0.740768 |
| Myelopero xidase | -0.25356 | -0.02434 | 2.199488 | 0.196244 | 1.694038 |
| Myoglobin | -0.01328 | -0.01769 | 0.112693 | 0.142663 | 0.180564 |
| NGF.beta | 0.082199 | -0.04409 | 0.699063 | 0.355716 | 0.745841 |
| NrCAM | -0.09995 | -0.1394 | 0.85092 | 1.131277 | 1.401625 |
| PAI.1 | -0.03942 | 0.09006 | 0.334695 | 0.72806 | 0.751481 |
| Pancreatic .Polypepti de | 0.024146 | 0.071138 | 0.204922 | 0.574506 | 0.551139 |
| PAPP.A | -0.0615 | -0.19526 | 0.522541 | 1.594702 | 1.497117 |
| PDGF | 0.11224 | 0.175323 | 0.956416 | 1.428259 | 1.68622 |
| Progester one | -0.10165 | -0.31134 | 0.865538 | 2.596236 | 2.447844 |
| Prolactin | 0.052892 | 0.016995 | 0.449223 | 0.137034 | 0.414547 |
| Prostate.S pecific.Ant igen..Free | 0.047086 | -0.2301 | 0.399836 | 1.888941 | 1.61841 |
| Prostatic. Acid.Phos phatase | -0.01399 | 0.000403 | 0.118733 | 0.003249 | 0.086255 |
| PARC | -0.09259 | -0.17231 | 0.787868 | 1.403169 | 1.549298 |
| Peptide.Y Y | -0.21813 | -0.21381 | 1.881145 | 1.750815 | 2.568184 |
| RANTES | -0.07018 | 0.105098 | 0.596467 | 0.850469 | 1.023138 |
| Resistin | -0.07343 | -0.00506 | 0.624197 | 0.0408 | 0.470224 |
| Secretin | 0.00886 | -0.0961 | 0.075181 | 0.777203 | 0.602727 |
| Serum.Am yloid.P | -0.10016 | -0.32046 | 0.852751 | 2.677962 | 2.496591 |
| SGOT | 0.060979 | 0.097092 | 0.518064 | 0.785253 | 0.921585 |
| SHBG | 0.287531 | 0.214779 | 2.510558 | 1.758993 | 3.019029 |
| SOD | -0.26796 | 0.04512 | 2.330599 | 0.364018 | 1.905381 |
| Sortilin | -0.19383 | -0.04644 | 1.665734 | 0.37466 | 1.442776 |
| sRAGE | 0.2925 | -0.0175 | 2.556594 | 0.141099 | 1.907557 |
| Stem.Cell. Factor | -0.08241 | -0.07763 | 0.700877 | 0.627097 | 0.939019 |
| Tenascin. C | -0.13667 | -0.08143 | 1.166982 | 0.657936 | 1.290412 |
| Testostero | -0.1135 | -0.17285 | 0.967285 | 1.407653 | 1.679335 |

ne

| | | | | | |
|---|---|---|---|---|---|
| TGF.alpha | 0.108733 | -0.09836 | 0.926292 | 0.795538 | 1.217517 |
| TGF.b3 | 0.089999 | 0.015428 | 0.765735 | 0.124393 | 0.629415 |
| Thrombopoietin | 0.240406 | -0.03481 | 2.080632 | 0.280745 | 1.669746 |
| TECK | -0.02747 | -0.18083 | 0.233126 | 1.474139 | 1.207218 |
| Thyroid.Stimulating.Hormone | 0.072018 | 0.043569 | 0.612154 | 0.351487 | 0.681397 |
| Thyroxine.Binding.Globulin | 0.334454 | 0.248786 | 2.951476 | 2.048769 | 3.535707 |
| TIMP.1 | -0.20876 | -0.2303 | 1.797789 | 1.890625 | 2.608103 |
| Tissue.Factor | 0.131736 | 0.075439 | 1.12435 | 0.609366 | 1.225922 |
| TNF.RII | -0.16053 | -0.08516 | 1.374063 | 0.688222 | 1.458256 |
| TNF.alpha | -0.10733 | -0.19093 | 0.914248 | 1.558468 | 1.748474 |
| TNF.beta | -0.05494 | 0.086686 | 0.466667 | 0.700646 | 0.825415 |
| TRAIL.R3 | -0.09299 | -0.02429 | 0.791365 | 0.195899 | 0.698101 |
| VCAM.1 | -0.17067 | -0.06317 | 1.462463 | 0.510008 | 1.394748 |
| VEGF | -0.06845 | -0.06687 | 0.581686 | 0.53991 | 0.793088 |
| von.Willebrand.Factor | -0.11453 | -0.17101 | 0.976073 | 1.392382 | 1.674751 |

| probeID | p.AR-vs-allOther.Set_1 | p.AR-vs-allOther.Set_2 | p.AR-vs-allOther.Meta | q.AR-vs-allOther.Set_1 | q.AR-vs-allOther.Set_2 | q.AR-vs-allOther.Meta |
|---|---|---|---|---|---|---|
| MDC | 0.706821 | 0.409795 | 0.397451 | 0.620378 | 0.652575 | 0.091697 |
| MIF | 0.545991 | 0.032182 | 0.051976 | 0.553516 | 0.336243 | 0.041592 |
| MIP.1alpha | 0.343391 | 0.268195 | 0.147205 | 0.528951 | 0.528323 | 0.052399 |
| MIP.1beta | 0.773634 | 0.283075 | 0.337095 | 0.63491 | 0.528323 | 0.082684 |
| MMP.2 | 0.913815 | 0.977722 | 0.923573 | 0.65242 | 0.806612 | 0.155457 |
| MMP.3 | 0.218442 | 0.223713 | 0.084102 | 0.497507 | 0.523705 | 0.044623 |
| MMP.9 | 0.761434 | 0.454841 | 0.458834 | 0.63491 | 0.663886 | 0.09809 |
| Myeloperoxidase | 0.028163 | 0.843835 | 0.090258 | 0.298848 | 0.779942 | 0.044623 |

| | | | | | | |
|---|---|---|---|---|---|---|
| Myoglobin | 0.909965 | 0.886125 | 0.85671 | 0.65242 | 0.779942 | 0.145716 |
| NGF.beta | 0.483241 | 0.72107 | 0.455763 | 0.553516 | 0.756467 | 0.09809 |
| NrCAM | 0.393566 | 0.256883 | 0.161027 | 0.531527 | 0.528323 | 0.053526 |
| PAI.1 | 0.73701 | 0.465163 | 0.452363 | 0.63491 | 0.667466 | 0.09809 |
| Pancreatic .Polypepti de | 0.837084 | 0.564293 | 0.581538 | 0.643381 | 0.713822 | 0.108408 |
| PAPP.A | 0.600144 | 0.110557 | 0.134363 | 0.572273 | 0.409994 | 0.051327 |
| PDGF | 0.337693 | 0.152702 | 0.091753 | 0.528951 | 0.419531 | 0.044623 |
| Progester one | 0.385504 | 0.009757 | 0.014371 | 0.531527 | 0.202616 | 0.022325 |
| Prolactin | 0.65222 | 0.890589 | 0.678474 | 0.594262 | 0.779942 | 0.122557 |
| Prostate.S pecific.Ant igen..Free | 0.688308 | 0.059068 | 0.105574 | 0.609723 | 0.336243 | 0.04731 |
| Prostatic. Acid.Phos phatase | 0.905161 | 0.997397 | 0.931264 | 0.65242 | 0.811089 | 0.155457 |
| PARC | 0.429502 | 0.160004 | 0.12131 | 0.54787 | 0.419531 | 0.049593 |
| Peptide.Y Y | 0.060099 | 0.079989 | 0.010223 | 0.298848 | 0.364264 | 0.02106 |
| RANTES | 0.549642 | 0.393682 | 0.306242 | 0.553516 | 0.652575 | 0.077566 |
| Resistin | 0.531258 | 0.96733 | 0.638195 | 0.553516 | 0.804034 | 0.116182 |
| Secretin | 0.939863 | 0.435628 | 0.54669 | 0.652724 | 0.652575 | 0.103569 |
| Serum.Am yloid.P | 0.392551 | 0.007716 | 0.012539 | 0.531527 | 0.202616 | 0.02106 |
| SGOT | 0.603269 | 0.430896 | 0.356745 | 0.572273 | 0.652575 | 0.085351 |
| SHBG | 0.012373 | 0.0786 | 0.002536 | 0.256481 | 0.364264 | 0.014773 |
| SOD | 0.02011 | 0.714844 | 0.056731 | 0.256481 | 0.756467 | 0.041592 |
| Sortilin | 0.095669 | 0.70689 | 0.149084 | 0.356984 | 0.756467 | 0.052399 |
| sRAGE | 0.01088 | 0.887365 | 0.056449 | 0.256481 | 0.779942 | 0.041592 |
| Stem.Cell. Factor | 0.482107 | 0.52922 | 0.347721 | 0.553516 | 0.68467 | 0.084402 |
| Tenascin. C | 0.242319 | 0.509185 | 0.196908 | 0.497507 | 0.682001 | 0.061579 |
| Testostero ne | 0.332243 | 0.15868 | 0.093087 | 0.528951 | 0.419531 | 0.044623 |
| TGF.alpha | 0.353098 | 0.424894 | 0.223407 | 0.528951 | 0.652575 | 0.063162 |
| TGF.b3 | 0.442559 | 0.900627 | 0.529077 | 0.553516 | 0.782712 | 0.102045 |

| | | | | | | |
|---|---|---|---|---|---|---|
| Thrombopoietin | 0.037748 | 0.778098 | 0.09497 | 0.298848 | 0.770309 | 0.044623 |
| TECK | 0.815046 | 0.140013 | 0.227348 | 0.643381 | 0.419531 | 0.063162 |
| Thyroid.Stimulating.Hormone | 0.539204 | 0.724249 | 0.49562 | 0.553516 | 0.756467 | 0.09956 |
| Thyroxine.Binding.Globulin | 0.00336 | 0.040776 | 0.000407 | 0.236366 | 0.336243 | 0.009477 |
| TIMP.1 | 0.072275 | 0.058845 | 0.009105 | 0.314295 | 0.336243 | 0.02106 |
| Tissue.Factor | 0.259902 | 0.540919 | 0.220228 | 0.497507 | 0.691943 | 0.063162 |
| TNF.RII | 0.168866 | 0.489907 | 0.14477 | 0.42514 | 0.667466 | 0.052399 |
| TNF.alpha | 0.359381 | 0.11884 | 0.080382 | 0.528951 | 0.409994 | 0.044623 |
| TNF.beta | 0.639661 | 0.482114 | 0.409136 | 0.594137 | 0.667466 | 0.09256 |
| TRAIL.R3 | 0.42746 | 0.844106 | 0.485114 | 0.54787 | 0.779942 | 0.099106 |
| VCAM.1 | 0.143207 | 0.608788 | 0.163092 | 0.42514 | 0.737338 | 0.053526 |
| VEGF | 0.559569 | 0.587964 | 0.427726 | 0.557643 | 0.723964 | 0.095835 |
| von.Willebrand.Factor | 0.327879 | 0.163224 | 0.093983 | 0.528951 | 0.419531 | 0.044623 |

| probeID | cor.CAN-vs-allOther.Set_1 | cor.CAN-vs-allOther.Set_2 | Z.CAN-vs-allOther.Set_1 | Z.CAN-vs-allOther.Set_2 | Z.CAN-vs-allOther.Meta |
|---|---|---|---|---|---|
| MDC | -0.1295 | 0.181611 | 1.105078 | 1.480622 | 1.828367 |
| MIF | 0.054072 | 0.019567 | 0.459262 | 0.157778 | 0.436313 |
| MIP.1alpha | -0.17076 | -0.09561 | 1.463268 | 0.77319 | 1.581414 |
| MIP.1beta | 0.04597 | -0.06338 | 0.390347 | 0.511637 | 0.637798 |
| MMP.2 | -0.22937 | -0.17042 | 1.981477 | 1.387508 | 2.382232 |
| MMP.3 | -0.33387 | -0.24687 | 2.945889 | 2.032273 | 3.520092 |
| MMP.9 | -0.11587 | 0.069564 | 0.987606 | 0.561753 | 1.095563 |
| Myeloperoxidase | 0.395494 | -0.08655 | 3.549356 | 0.699525 | 3.004413 |
| Myoglobin | -0.3575 | -0.30001 | 3.173678 | 2.495514 | 4.008724 |
| NGF.beta | 0.082199 | -0.07521 | 0.699063 | 0.607507 | 0.923885 |

| | | | | | |
|---|---|---|---|---|---|
| NrCAM | -0.25616 | -0.1753 | 2.223113 | 1.428091 | 2.581792 |
| PAI.1 | 0.190368 | -0.11269 | 1.635274 | 0.912383 | 1.801466 |
| Pancreatic.Polypeptide | -0.25602 | -0.26336 | 2.221782 | 2.17453 | 3.108662 |
| PAPP.A | -0.24501 | 0.020356 | 2.122164 | 0.16414 | 1.616661 |
| PDGF | 0.169911 | -0.19889 | 1.455865 | 1.625162 | 2.178615 |
| Progesterone | -0.04928 | 0.089729 | 0.418499 | 0.725371 | 0.808838 |
| Prolactin | -0.50759 | -0.19951 | 4.747326 | 1.630377 | 4.509717 |
| Prostate.Specific.Antigen..Free | -0.15609 | 0.268545 | 1.335426 | 2.2195 | 2.513712 |
| Prostatic.Acid.Phosphatase | -0.05837 | 0.228648 | 0.495857 | 1.876594 | 1.677576 |
| PARC | -0.05593 | 0.132529 | 0.475082 | 1.074804 | 1.095935 |
| Peptide.YY | -0.1451 | -0.10614 | 1.239988 | 0.858987 | 1.484199 |
| RANTES | 0.278402 | -0.08361 | 2.426357 | 0.675646 | 2.193447 |
| Resistin | -0.44527 | -0.29055 | 4.062575 | 2.41193 | 4.578167 |
| Secretin | -0.13412 | -0.0769 | 1.144928 | 0.621232 | 1.248864 |
| Serum.Amyloid.P | 0.119828 | 0.098325 | 1.02168 | 0.795292 | 1.284793 |
| SGOT | -0.32572 | 0.01593 | 2.868346 | 0.128445 | 2.119051 |
| SHBG | -0.40001 | -0.15204 | 3.594924 | 1.235355 | 3.415524 |
| SOD | 0.577136 | -0.12167 | 5.584658 | 0.985806 | 4.646019 |
| Sortilin | 0.238118 | 0.014447 | 2.060044 | 0.116485 | 1.539038 |
| sRAGE | -0.23785 | -0.00035 | 2.057585 | 0.002844 | 1.456944 |
| Stem.Cell.Factor | -0.40283 | -0.39396 | 3.62344 | 3.357771 | 4.936462 |
| Tenascin.C | -0.16761 | -0.28132 | 1.435763 | 2.330941 | 2.663461 |
| Testosterone | 0.105766 | 0.263012 | 0.900824 | 2.171504 | 2.172464 |
| TGF.alpha | -0.00165 | -0.15287 | 0.014028 | 1.242231 | 0.888309 |
| TGF.b3 | -0.06577 | -0.13403 | 0.558868 | 1.087087 | 1.163866 |
| Thrombopoietin | 0.091965 | 0.253628 | 0.782556 | 2.090432 | 2.031509 |

| | | | | | |
|---|---|---|---|---|---|
| TECK | -0.18697 | -0.1298 | 1.605344 | 1.052396 | 1.879306 |
| Thyroid.Stimulating.Hormone | -0.07203 | 0.194295 | 0.612284 | 1.586624 | 1.554863 |
| Thyroxine.Binding.Globulin | -0.20034 | -0.09007 | 1.723227 | 0.728136 | 1.733376 |
| TIMP.1 | -0.00985 | -0.12827 | 0.083603 | 1.039904 | 0.794439 |
| Tissue.Factor | -0.28089 | -0.0781 | 2.449232 | 0.630965 | 2.178028 |
| TNF.RII | -0.42133 | -0.44444 | 3.812464 | 3.851766 | 5.419429 |
| TNF.alpha | -0.23474 | -0.09222 | 2.029656 | 0.745616 | 1.962414 |
| TNF.beta | 0.027471 | -0.03421 | 0.233157 | 0.275927 | 0.359977 |
| TRAIL.R3 | -0.38755 | -0.26895 | 3.469748 | 2.223041 | 4.02541 |
| VCAM.1 | -0.27123 | -0.22514 | 2.360531 | 1.846785 | 2.975022 |
| VEGF | -0.52251 | -0.46561 | 4.919693 | 4.066991 | 6.354545 |
| von.Willebrand.Factor | 0.053613 | -0.17919 | 0.455358 | 1.460421 | 1.35466 |

| probeID | p.CAN-vs-allOther.Set_1 | p.CAN-vs-allOther.Set_2 | p.CAN-vs-allOther.Meta | q.CAN-vs-allOther.Set_1 | q.CAN-vs-allOther.Set_2 | q.CAN-vs-allOther.Meta |
|---|---|---|---|---|---|---|
| MDC | 0.268136 | 0.138288 | 0.067495 | 0.206847 | 0.223243 | 0.000181 |
| MIF | 0.64498 | 0.874157 | 0.662609 | 0.338084 | 0.443265 | 0.000898 |
| MIP.1alpha | 0.142988 | 0.437998 | 0.113783 | 0.128659 | 0.336477 | 0.000255 |
| MIP.1beta | 0.695328 | 0.607646 | 0.523605 | 0.345474 | 0.358149 | 0.000749 |
| MMP.2 | 0.047763 | 0.164695 | 0.017208 | 0.052971 | 0.242241 | 6.80E-05 |
| MMP.3 | 0.003419 | 0.042408 | 0.000431 | 0.008494 | 0.107958 | 3.92E-06 |
| MMP.9 | 0.322208 | 0.572964 | 0.27327 | 0.235426 | 0.355893 | 0.000482 |
| Myeloperoxidase | 0.000445 | 0.482815 | 0.002661 | 0.001974 | 0.33826 | 1.61E-05 |
| Myoglobin | 0.001639 | 0.012932 | 6.10E-05 | 0.004848 | 0.071331 | 7.39E-07 |
| NGF.beta | 0.483241 | 0.542153 | 0.355546 | 0.306248 | 0.34533 | 0.000567 |
| NrCAM | 0.026533 | 0.15275 | 0.009829 | 0.035944 | 0.23512 | 4.25E-05 |
| PAI.1 | 0.101852 | 0.360229 | 0.07163 | 0.100407 | 0.308986 | 0.000186 |

| | | | | | | |
|---|---|---|---|---|---|---|
| Pancreatic .Polypepti de | 0.026623 | 0.030012 | 0.001879 | 0.035944 | 0.094657 | 1.31E-05 |
| PAPP.A | 0.034121 | 0.869127 | 0.105951 | 0.042383 | 0.443265 | 0.000247 |
| PDGF | 0.145011 | 0.103956 | 0.02936 | 0.128659 | 0.181069 | 9.85E-05 |
| Progester one | 0.674581 | 0.466811 | 0.418609 | 0.344738 | 0.336477 | 0.000629 |
| Prolactin | 3.35E-06 | 0.102858 | 6.49E-06 | 4.16E-05 | 0.181069 | 1.47E-07 |
| Prostate.S pecific.Ant igen..Free | 0.181115 | 0.02681 | 0.011947 | 0.154088 | 0.094657 | 5.05E-05 |
| Prostatic. Acid.Phos phatase | 0.618878 | 0.06073 | 0.09343 | 0.33716 | 0.143558 | 0.000223 |
| PARC | 0.63364 | 0.281319 | 0.273107 | 0.337728 | 0.296895 | 0.000482 |
| Peptide.Y Y | 0.214198 | 0.38897 | 0.137756 | 0.172767 | 0.314717 | 0.000294 |
| RANTES | 0.015582 | 0.497864 | 0.028275 | 0.027024 | 0.33826 | 9.85E-05 |
| Resistin | 6.26E-05 | 0.016235 | 4.69E-06 | 0.000588 | 0.076754 | 1.22E-07 |
| Secretin | 0.251307 | 0.533075 | 0.211715 | 0.197567 | 0.34533 | 0.000409 |
| Serum.Am yloid.P | 0.305834 | 0.425037 | 0.198865 | 0.226122 | 0.330969 | 0.000389 |
| SGOT | 0.004349 | 0.897407 | 0.034086 | 0.009646 | 0.443265 | 0.000109 |
| SHBG | 0.000377 | 0.215823 | 0.000637 | 0.001801 | 0.250612 | 5.26E-06 |
| SOD | 5.96E-08 | 0.322966 | 3.38E-06 | 1.93E-06 | 0.296895 | 1.02E-07 |
| Sortilin | 0.039665 | 0.906914 | 0.123795 | 0.047654 | 0.444643 | 0.000268 |
| sRAGE | 0.039899 | 0.997722 | 0.145132 | 0.047654 | 0.472467 | 0.000303 |
| Stem.Cell. Factor | 0.00034 | 0.000887 | 7.96E-07 | 0.001757 | 0.011747 | 3.61E-08 |
| Tenascin. C | 0.150618 | 0.020126 | 0.007734 | 0.131751 | 0.088808 | 3.69E-05 |
| Testostero ne | 0.366466 | 0.030238 | 0.029821 | 0.24473 | 0.094657 | 9.85E-05 |
| TGF.alpha | 0.988769 | 0.213289 | 0.374374 | 0.438634 | 0.250612 | 0.00057 |
| TGF.b3 | 0.575063 | 0.275874 | 0.244478 | 0.326829 | 0.296895 | 0.000458 |
| Thrombop oietin | 0.432615 | 0.036893 | 0.042203 | 0.282823 | 0.101744 | 0.000126 |
| TECK | 0.108235 | 0.291443 | 0.060203 | 0.105032 | 0.296895 | 0.000171 |
| Thyroid.St | 0.539117 | 0.112363 | 0.119979 | 0.32607 | 0.190694 | 0.000263 |

| | | | | | | |
|---|---|---|---|---|---|---|
| imulating. Hormone Thyroxine. Binding.Gl obulin | 0.084825 | 0.465116 | 0.083029 | 0.087803 | 0.336477 | 0.00021 |
| TIMP.1 | 0.933141 | 0.297192 | 0.42694 | 0.426132 | 0.296895 | 0.000636 |
| Tissue.Fa ctor | 0.014644 | 0.526685 | 0.029404 | 0.02675 | 0.34515 | 9.85E-05 |
| TNF.RII | 0.000167 | 0.000147 | 5.98E-08 | 0.001037 | 0.003301 | 5.43E-09 |
| TNF.alpha | 0.042646 | 0.454485 | 0.049714 | 0.049973 | 0.336477 | 0.000143 |
| TNF.beta | 0.815021 | 0.781808 | 0.718864 | 0.392387 | 0.417308 | 0.00096 |
| TRAIL.R3 | 0.000592 | 0.026571 | 5.69E-05 | 0.002298 | 0.094657 | 7.38E-07 |
| VCAM.1 | 0.018584 | 0.064904 | 0.00293 | 0.02815 | 0.148133 | 1.72E-05 |
| VEGF | 1.52E-06 | 6.31E-05 | 2.09E-10 | 2.36E-05 | 0.003301 | 3.80E-11 |
| von.Willeb rand.Fact or | 0.647791 | 0.143718 | 0.175526 | 0.338084 | 0.226486 | 0.000354 |

It should be appreciated by those of skill in the art that the techniques disclosed in the examples above represent techniques discovered by the inventors to function well in the practice of the invention. Those of skill in the art should, however, in light of the present disclosure, appreciate that many changes can be made in the specific embodiments that are disclosed and still obtain a like or similar result without departing from the spirit and scope of the invention, therefore all matter set forth or shown in the accompanying drawings is to be interpreted as illustrative and not in a limiting sense.

Furthermore, the present invention relates to the following items:

1. A method for diagnosing, monitoring, or determining kidney transplant rejection or an associated disorder in a mammal, the method comprising:

a. providing a test sample comprising a sample of bodily fluid taken from the mammal;
b. determining sample concentrations for sample analytes in the test sample, wherein the sample analytes are microalbumin, VEGF, BLC, CD40, IGF BP2, MMP3, peptide YY, stem cell factor, TNF RII, AXL, Eotaxin 3, FABP, FGF basic, myoglobin, resistin, TRAIL R3, endothelin 1, NrCAM, Tenascin C, VCAM1, and cortisol;
c. comparing the combination of sample concentrations to a data set comprising at least one entry, wherein each entry of the data set comprises a list comprising three or more minimum diagnostic concentrations indicative of kidney transplant rejection or an associated disorder, wherein each minimum diagnostic concentration comprises a maximum of a range of analyte concentrations for a healthy mammal;
d. determining a matching entry of the dataset in which all minimum diagnostic concentrations are less than the corresponding sample concentrations; and,
e. identifying an indicated disorder comprising the particular disorder of the matching entry.

2. A method for diagnosing, monitoring, or determining kidney transplant rejection or an associated disorder in a mammal, the method comprising:

a. providing a test sample comprising a sample of bodily fluid taken from the mammal;
b. determining a combination of sample concentrations for three or more sample analytes in the test sample, wherein the sample analytes are selected from the group consisting of alpha-1 microglobulin, beta-2 microglobulin, calbindin, clusterin, CTGF, creatinine, cystatin C, GST-alpha, KIM-1, microalbumin, NGAL, osteopontin, THP, TIMP-1, TFF-3, VEGF, BLC, CD40, IGF BP2, MMP3, peptide YY, stem cell factor, TNF RII, AXL, Eotaxin 3, FABP, FGF basic, myoglobin, resistin, TRAIL R3, endothelin 1, NrCAM, Tenascin C, VCAM1, and cortisol;
c. comparing the combination of sample concentrations to a data set comprising at least one entry, wherein each entry of the data set comprises a list comprising three or more minimum diagnostic concentrations indicative of kidney transplant rejection or an associated disorder, wherein each minimum diagnostic concentration comprises a maximum of a range of analyte concentrations for a healthy mammal;
d. determining a matching entry of the dataset in which all minimum diagnostic concentrations are less than the corresponding sample concentrations; and,

e. identifying an indicated disorder comprising the particular disorder of the matching entry.

3. The method of item 2, wherein the mammal is selected from the group consisting of humans, apes, monkeys, rats, mice, dogs, cats, pigs, and livestock including cattle and oxen.

4. The method of item 2, wherein the bodily fluid is selected from the group consisting of urine, blood, plasma, serum, saliva, semen, and tissue lysates.

5. The method of item 2, wherein the minimum diagnostic concentration in human plasma of alpha-1 microglobulin is about 16 $\mu$g/ml, beta-2 microglobulin is about 2.2 $\mu$g/ml, calbindin is greater than about 5 ng/ml, clusterin is about 134 $\mu$g/ml, CTGF is about 16 ng/ml, cystatin C is about 1170 ng/ml, GST-alpha is about 62 ng/ml, KIM-1 is about 0.57 ng/ml, NGAL is about 375 ng/ml, osteopontin is about 25 ng/ml, THP is about 0.052 $\mu$g/ml, TIMP-1 is about 131 ng/ml, TFF-3 is about 0.49 $\mu$g/ml, and VEGF is about 855 $\mu$g/ml.

6. The method of item 2, wherein the minimum diagnostic concentration in human sera of alpha-1 microglobulin is about 17 $\mu$g/ml, beta-2 microglobulin is about 2.6$\mu$ g/ml, calbindin is greater than about 2.6 ng/ml, clusterin is about 152 $\mu$g/ml, CTGF is greater than about 8.2 ng/ml, cystatin C is about 1250 ng/ml, GST-alpha is about 52 ng/ml, KIM-1 is greater than about 0.35 ng/ml, NGAL is about 822 ng/ml, osteopontin is about 12 ng/ml, THP is about 0.053 $\mu$g/ml, TIMP-1 is about 246 ng/ml, TFF-3 is about 0.17 $\mu$g/ml, and VEGF is about 1630 $\mu$g/ml.

7. The method of item 2, wherein the minimum diagnostic concentration in human urine of alpha-1 microglobulin is about 233 $\mu$g/ml, beta-2 microglobulin is greater than about 0.17 $\mu$g/ml, calbindin is about 233 ng/ml, clusterin is greater than about 0.089 $\mu$g/ml, CTGF is greater than about 0.90 ng/ml, cystatin C is about 1170 ng/ml, GST-alpha is greater than about 26 ng/ml, KIM-1 is about 0.67 ng/ml, NGAL is about 81 ng/ml, osteopontin is about 6130 ng/ml, THP is about 2.6 $\mu$g/ml, TIMP-1 is greater than about 3.9 ng/ml, TFF-3 is greater than about 21 $\mu$g/ml, and VEGF is about 517 $\mu$g/ml.

8. The method of item 2, wherein a combination of sample concentrations for six or more sample analytes in the test sample are determined.

9. The method of item 8, wherein sample concentrations are determined for the analytes selected from the group consisting of BLC, CD40, IGF BP2, MMP3, peptide YY, stem cell factor, TNF RII, AXL, Eotaxin 3, FABP, FGF basic, myoglobin, resistin, TRAIL R3, endothelin 1, NrCAM, Tenascin C, VCAM1, and cortisol.

10. The method of item 2, wherein the kidney transplant rejection is acute rejection.

11. The method of item 2, wherein the kidney transplant rejection is chronic allograft nephropathy.

12. A method for diagnosing, monitoring, or determining kidney transplant rejection or an associated disorder in a mammal, the method comprising:

a. providing a test sample comprising a sample of bodily fluid taken from the mammal;

b. determining the concentrations of three or more sample analytes in a panel of biomarkers in the test sample, wherein the sample analytes are selected from the group consisting of alpha-1 microglobulin, beta-2 microglobulin, calbindin, clusterin, CTGF, creatinine, cystatin C, GST-alpha, KIM-1, microalbumin, NGAL, osteopontin, THP, TIMP-1, TFF-3, VEGF, BLC, CD40, IGF BP2, MMP3, peptide YY, stem cell factor, TNF RII, AXL, Eotaxin 3, FABP, FGF basic, myoglobin, resistin, TRAIL R3, endothelin 1, NrCAM, Tenascin C, VCAM1, and cortisol;

c. identifying diagnostic analytes in the test sample, wherein the diagnostic analytes are the sample analytes whose concentrations are statistically different from concentrations found in a control group of humans who do not suffer from kidney transplant rejection or an associated disorder;

d. comparing the combination of diagnostic analytes to a dataset comprising at least one entry, wherein each entry of the dataset comprises a combination of three or more diagnostic analytes reflective of kidney transplant rejection or an associated disorder; and,

e. identifying the particular disorder having the combination of diagnostic analytes that essentially match the combination of sample analytes.

13. The method of item 12, wherein the mammal is selected from the group consisting of humans, apes, monkeys, rats, mice, dogs, cats, pigs, and livestock including cattle and oxen.

14. The method of item 12, wherein the bodily fluid is selected from the group consisting of urine, blood, plasma, serum, saliva, semen, and tissue lysates.

15. The method of item 12, wherein the kidney transplant rejection is acute rejection.

16. The method of item 12, wherein the kidney transplant rejection is chronic allograft nephropathy.

17. A method for diagnosing, monitoring, or determining kidney transplant rejection or an associated disorder in a mammal, the method comprising:

a. providing an analyte concentration measurement device comprising three or more detection antibodies ,

wherein each detection antibody comprises an antibody coupled to an indicator, wherein the antigenic determinants of the antibodies are sample analytes associated with kidney transplant rejection or an associated disorder, and wherein the sample analytes are selected from the group consisting of alpha-1 microglobulin, beta-2 microglobulin, calbindin, clusterin, CTGF, creatinine, cystatin C, GST-alpha, KIM-1, microalbumin, NGAL, osteopontin, THP, TIMP-1, TFF-3, VEGF, BLC, CD40, IGF BP2, MMP3, peptide YY, stem cell factor, TNF RII, AXL, Eotaxin 3, FABP, FGF basic, myoglobin, resistin, TRAIL R3, endothelin 1, NrCAM, Tenascin C, VCAM1, and cortisol;

b. providing a test sample comprising three or more sample analytes and a bodily fluid taken from the mammal;

c. contacting the test sample with the detection antibodies and allowing the detection antibodies to bind to the sample analytes;

d. determining the concentrations of the sample analytes by detecting the indicators of the detection antibodies bound to the sample analytes in the test sample; and,

e. comparing the concentrations of each sample analyte to a corresponding minimum diagnostic concentration reflective of kidney transplant rejection or an associated disorder.

18. The method of item 17, wherein the bodily fluid is selected from the group consisting of urine, blood, plasma, serum, saliva, semen, and tissue lysates.

19. The method of item 17, wherein the analyte concentration measurement device comprises six or more detection antibodies.

20. The method of item 17, wherein the analyte concentration measurement device comprises sixteen detection antibodies.

21. The method of item 17, wherein the sample analytes are selected from the group consisting of alpha-1 microglobulin, beta-2 microglobulin, cystatin C, KIM-1, THP, and TIMP-1.

22. The method of item 17, wherein the kidney transplant rejection is acute rejection.

23. The method of item 17, wherein the kidney transplant rejection is chronic allograft nephropathy.

24. A method for diagnosing, monitoring, or determining kidney transplant rejection or an associated disorder in a mammal, the method comprising:

a. providing a test sample comprising a sample of bodily fluid taken from the mammal;

b. determining sample concentrations for sample analytes in the test sample, wherein the sample analytes are microalbumin, VEGF, BLC, CD40, IGF BP2, MMP3, peptide YY, stem cell factor, TNF RII, AXL, Eotaxin 3, FABP, FGF basic, myoglobin, resistin, TRAIL R3, endothelin 1, NrCAM, Tenascin C, VCAM1, and cortisol;

c. comparing the combination of sample concentrations to a data set comprising at least one entry, wherein each entry of the data set comprises a list comprising three or more minimum diagnostic concentrations indicative of kidney transplant rejection or an associated disorder, wherein each minimum diagnostic concentration comprises a maximum of a range of analyte concentrations for a healthy mammal;

d. determining a matching entry of the dataset in which all minimum diagnostic concentrations are less than the corresponding sample concentrations; and,

e. identifying an indicated disorder comprising the particular disorder of the matching entry.

25. A method for diagnosing, monitoring, or determining kidney transplant rejection or an associated disorder in a mammal, the method comprising:

a. providing a test sample comprising a sample of bodily fluid taken from the mammal;

b. determining sample concentrations for sample analytes in the test sample, wherein the sample analytes are alpha-1 microglobulin, beta-2 microglobulin, calbindin, clusterin, CTGF, creatinine, cystatin C, GST-alpha, KIM-1, microalbumin, NGAL, osteopontin, THP, TIMP-1, TFF-3, VEGF, BLC, CD40, IGF BP2, MMP3, peptide YY, stem cell factor, TNF RII, AXL, Eotaxin 3, FABP, FGF basic, myoglobin, resistin, TRAIL R3, endothelin 1, NrCAM, Tenascin C, VCAM1, and cortisol;

c. comparing the combination of sample concentrations to a data set comprising at least one entry, wherein each entry of the data set comprises a list comprising three or more minimum diagnostic concentrations indicative of kidney transplant rejection or an associated disorder, wherein each minimum diagnostic concentration comprises a maximum of a range of analyte concentrations for a healthy mammal;

d. determining a matching entry of the dataset in which all minimum diagnostic concentrations are less than the corresponding sample concentrations; and,

e. identifying an indicated disorder comprising the particular disorder of the matching entry.

**Claims**

1. A method for diagnosing, monitoring, or determining kidney transplant rejection or an associated disorder in a mammal, the method comprising:

    a. providing an analyte concentration measurement device comprising three or more detection antibodies, wherein each detection antibody comprises an antibody coupled to an indicator, wherein the antigenic determinants of the antibodies are sample analytes associated with kidney transplant rejection or an associated disorder, and wherein the sample analytes are selected from the group consisting of TFF-3, beta-2 microglobulin, alpha-1 microglobulin, calbindin, clusterin, CTGF, creatinine, cystatin C, GST-alpha, KIM-1, microalbumin, NGAL, osteopontin, THP, TIMP-1, VEGF, BLC, CD40, IGF BP2, MMP3, peptide YY, stem cell factor, TNF RII, AXL, Eotaxin 3, FABP, FGF basic, myoglobin, resistin, TRAIL R3, endothelin 1, NrCAM, Tenascin C, VCAM1, and cortisol;
    b. contacting a test sample comprising three or more sample analytes and a bodily fluid taken from the mammal with the detection antibodies and allowing the detection antibodies to bind to the sample analytes;
    c. determining the concentrations of the sample analytes by detecting the indicators of the detection antibodies bound to the sample analytes in the test sample; and,
    d. comparing the concentrations of each sample analyte to a corresponding minimum diagnostic concentration reflective of kidney transplant rejection or an associated disorder.

2. The method of claim 1, wherein the analyte concentration measurement device comprises six or more detection antibodies.

3. The method of claim 1, wherein the analyte concentration measurement device comprises sixteen detection antibodies.

4. The method of claim 1, wherein the sample analytes are selected from the group consisting of alpha-1 microglobulin, beta-2 microglobulin, cystatin C, KIM-1, THP, and TIMP-1.

5. The method of any one of claims 1 to 4, wherein the mammal is selected from the group consisting of humans, apes, monkeys, rats, mice, dogs, cats, pigs, and livestock including cattle and oxen.

6. The method of any one of claims 1 to 5, wherein the bodily fluid is selected from the group consisting of urine, blood, plasma, serum, saliva, semen, and tissue lysates.

7. The method of any one of claims 1 to 6, wherein the kidney transplant rejection is acute rejection or chronic allograft nephropathy.

FIG. 1

## FIG. 2A

Protein significance for status
TX vs. AR on all samples
cor=0.57, p=3.5e-14

Protein significance for status
TX vs. CAN on all samples
cor=0.45, p=1.4e-08

EP 2 806 272 A1

FIG. 2B

Protein significance for status
AR vs. CAN on all samples
cor=0.34, p=3.6e-05

Protein significance for status
TX vs. allOther on all samples
cor=0.52, p=1.5e-11

EP 2 806 272 A1

# FIG. 2C

Protein significance for status
AR vs. allOther on all samples
cor=0.44, p=3.3e-08

Protein significance for status
CAN vs. allOther on all samples
cor=0.42, p=1.6e-07

EP 2 806 272 A1

FIG. 3

FIG. 4

FIG. 5

FIG. 6

FIG. 7

EP 2 806 272 A1

FIG. 8

FIG. 9

EP 2 806 272 A1

Europäisches
Patentamt

European
Patent Office

Office européen
des brevets

# EUROPEAN SEARCH REPORT

Application Number

EP 14 18 0229

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2004/042346 A2 (EXPRESSION DIAGNOSTICS INC [US]; WOHLGEMUTH JAY [US]; FRY KIRK [US]; W) 21 May 2004 (2004-05-21) * the whole document * * abstract * * table 8 * * claims 17, 21, 42 * | 1-7 | INV. G01N33/48 G01N33/53 G01N33/68 |
| A | GILLESPIE A ET AL: "Biomarkers in renal transplantation", BIOMARKERS IN MEDICINE, FUTURE MEDICINE, LONDON, vol. 2, no. 6, 1 December 2008 (2008-12-01), pages 603-612, XP008158885, ISSN: 1752-0363, DOI: 10.2217/17520363.2.6.603 * the whole document * * abstract * * page 604, right-hand column, last paragraph - page 606, left-hand column, paragraph 1 * * page 605; table 1 * * page 606, left-hand column, last paragraph - right-hand column, paragraph 3 * * page 607, left-hand column, paragraph 2 - paragraph 3 * * Executive summary; page 610 * | 1-7 | TECHNICAL FIELDS SEARCHED (IPC) G01N |

-/--

The present search report has been drawn up for all claims

| Place of search | Date of completion of the search | Examiner |
|---|---|---|
| The Hague | 8 October 2014 | Gall-Truchot, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EUROPEAN SEARCH REPORT**

Europäisches Patentamt
European Patent Office
Office européen des brevets

Application Number

EP 14 18 0229

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | UWE POGE ET AL: "Time course of low molecular weight proteins in the early kidney transplantation period--influence of corticosteroids", NEPHROLOGY DIALYSIS TRANSPLANTATION, vol. 19, no. 11, 22 September 2004 (2004-09-22), pages 2858-2863, XP55048192, DOI: 10.1093/ndt/gfh341 * the whole document * * abstract * * page 2858, right-hand column, paragraph 1 - paragraph 3 * * page 2863, left-hand column, last paragraph * | 1-7 | |
| A,P | NICKERSON ET AL: "Post-transplant monitoring of renal allografts: are we there yet?", CURRENT OPINION IN IMMUNOLOGY, ELSEVIER, OXFORD, GB, vol. 21, no. 5, 26 August 2009 (2009-08-26), - 1 October 2009 (2009-10-01), pages 563-568, XP026682570, ISSN: 0952-7915, DOI: 10.1016/J.COI.2009.07.014 [retrieved on 2009-08-26] * the whole document * * abstract * * page 563, right-hand column, paragraph 2 * | 1-7 | TECHNICAL FIELDS SEARCHED (IPC) |

-/--

The present search report has been drawn up for all claims

| Place of search | Date of completion of the search | Examiner |
|---|---|---|
| The Hague | 8 October 2014 | Gall-Truchot, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**EUROPEAN SEARCH REPORT**

Application Number

EP 14 18 0229

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,P | TRUONG D Q ET AL: "The immunological monitoring of kidney and liver transplants in adult and pediatric recipients", TRANSPLANT IMMUNOLOGY, ELSEVIER, NL, vol. 22, no. 1-2, 1 December 2009 (2009-12-01), pages 18-27, XP026755846, ISSN: 0966-3274, DOI: 10.1016/J.TRIM.2009.09.008 [retrieved on 2009-09-30] * the whole document * * abstract * * page 19, left-hand column, paragraph 1 * ----- | 1-7 | |

TECHNICAL FIELDS
SEARCHED      (IPC)

The present search report has been drawn up for all claims

| Place of search | Date of completion of the search | Examiner |
|---|---|---|
| The Hague | 8 October 2014 | Gall-Truchot, A |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons
...............................................................................
& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 14 18 0229

This annex lists the patent family members relating to the patent documents cited in the above-mentioned European search report.
The members are as contained in the European Patent Office EDP file on
The European Patent Office is in no way liable for these particulars which are merely given for the purpose of information.

08-10-2014

| Patent document cited in search report | Publication date | Patent family member(s) | | Publication date |
|---|---|---|---|---|
| WO 2004042346 A2 | 21-05-2004 | AU | 2003299465 A1 | 07-06-2004 |
| | | CA | 2483481 A1 | 21-05-2004 |
| | | EP | 1585972 A2 | 19-10-2005 |
| | | EP | 2194145 A1 | 09-06-2010 |
| | | EP | 2253719 A2 | 24-11-2010 |
| | | EP | 2292786 A2 | 09-03-2011 |
| | | JP | 2005536230 A | 02-12-2005 |
| | | JP | 2010022375 A | 04-02-2010 |
| | | US | 2006088836 A1 | 27-04-2006 |
| | | US | 2007031890 A1 | 08-02-2007 |
| | | US | 2010099098 A1 | 22-04-2010 |
| | | WO | 2004042346 A2 | 21-05-2004 |

EPO FORM P0459

For more details about this annex : see Official Journal of the European Patent Office, No. 12/82

**EP 2 806 272 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 61327389 A

- US 61232091 A